# EUROPEAN PATENT SPECIFICATION

(11) **EP 3 361 857 B1**
(45) Date of publication and mention of the grant of the patent: **25.11.2026**
(21) Application number: 16790795.5
(22) Date of filing: 14.10.2016
(51) Int. Cl.: A01H 5/06

(54) **GUAYULE MOLECULAR MARKERS AND PRIMERS AND USE OF MOLECULAR MARKERS, PRIMERS AND RATE OF APOMIXIS FOR GUAYULE IDENTIFICATION, CHARACTERIZATION AND BREEDING**
MOLEKULARE MARKER FÜR GUAYUL UND PRIMER SOWIE VERWENDUNG VON MOLEKULAREN MARKERN, PRIMERN UND APOMIXISGESCHWINDIGKEIT ZUR IDENTIFIZIERUNG VON GUAYUL, CHARAKTERISIERUNG UND ZÜCHTUNG
MARQUEURS MOLÉCULAIRES ET AMORCES DE GAYULE ET UTILISATION DE MARQUEURS MOLÉCULAIRES, D'AMORCES ET VITESSE DE L'APOMIXIE POUR L'IDENTIFICATION, LA CARACTÉRISATION ET LA CULTURE DE GUAYULE

(30) Priority: 16.10.2015 US 201562242960 P; 16.10.2015 US 201562242951 P; 26.09.2016 US 201662399993 P
(43) Date of publication of application: 22.08.2018
(73) Proprietor: Bridgestone Corporation, Tokyo 104-8340 (JP)
(72) Inventor: CRUZ, Vonmark, V., Eloy, AZ 85131 (US); DIERIG, David, A., Eloy, AZ 85131 (US)
(74) Representative: Marks & Clerk LLP
(86) International application number: PCT/US2016/057227
(87) International publication number: WO 2017/066710

(56) References cited:
- US-A1- 2002 170 086
- US-A1- 2002 170 086
- US-A1- 2006 217 512
- US-A1- 2014 325 699
- US-A1- 2014 325 699
- ESTILAI ET AL: "Isozyme markers in breeding guayule (Parthenium argentatum) for increased rubber production", ENZYME AND MICROBIAL TECHNOLOGY, STONEHAM, MA, US, vol. 13, no. 6, 1 June 1991 (1991-06-01), pages 523, XP023791103, ISSN: 0141-0229, [retrieved on 19910601], DOI: 10.1016/0141-0229(91)90033-7
- ERIC J MATHUR: "Molecular Breeding & Crop Improvement of Jatropha and Guayule Hybrid Cultivars", INTERNET CITATION, 8 December 2014 (2014-12-08), pages 1 - 20, XP002766526, Retrieved from the Internet <URL:https://www.bio.org/sites/default/files/Eric%20Mathur.pdf> [retrieved on 1077]
- KUMAR SHASHI ET AL: "Comparative analysis of the complete sequence of the plastid genome of Parthenium argentatum and identification of DNA barcodes to differentiate Parthenium species and lines. Article 131", BMC PLANT BIOLOGY, BIOMED CENTRAL, LONDON, GB, vol. 9, no. 1, 17 November 2009 (2009-11-17), pages 1 - 12, XP021062479, ISSN: 1471-2229, DOI: 10.1186/1471-2229-9-131
- RAY D T ET AL: "Breeding guayule for commercial production", INDUSTRIAL CROPS AND PRODUCTS, ELSEVIER, NL, vol. 22, no. 1, 1 July 2005 (2005-07-01), pages 15 - 25, XP027814348, ISSN: 0926-6690, [retrieved on 20050701], DOI: 10.1016/J.INDCROP.2004.06.005
- TANG S ET AL: "Simple sequence repeat map of the sunflower genome", THEORETICAL AND APPLIED GENETICS ; INTERNATIONAL JOURNAL OF PLANT BREEDING RESEARCH, SPRINGER, BERLIN, DE, vol. 105, no. 8, 1 December 2002 (2002-12-01), pages 1124 - 1136, XP019776194, ISSN: 1432-2242, DOI: 10.1007/S00122-002-0989-Y
- KURAVADI NAGESH ASWATHNARAYANA ET AL: "Identification and Characterization of EST-SSR Markers in Cluster Bean ( Cyamopsis spp.)", CROP SCIENCE, vol. 54, no. 3, 1 May 2014 (2014-05-01), US, pages 1097 - 1102, XP093206396, ISSN: 0011-183X, Retrieved from the Internet <URL:https://onlinelibrary.wiley.com/doi/full-xml/10.2135/cropsci2013.08.0522> DOI: 10.2135/cropsci2013.08.0522
- ESTILAI A ET AL: "Isozyme markers in breeding guayule (Parthenium argentatum) for increased rubber production", ENZYME AND MICROBIAL TECHNOLOGY, vol. 13, no. 6, 1 June 1991 (1991-06-01), STONEHAM, MA, US, pages 523, XP023791103, ISSN: 0141-0229, [retrieved on 19910601], DOI: 10.1016/0141-0229(91)90033-7
- ERIC J MATHUR: "Molecular Breeding & Crop Improvement of Jatropha and Guayule Hybrid Cultivars", 8 December 2014 (2014-12-08), pages 20PP, XP002766526, Retrieved from the Internet <URL:https://www.bio.org/sites/default/files/Eric%20Mathur.pdf> [retrieved on 20170130]
- KUMAR SHASHI ET AL: "Comparative analysis of the complete sequence of the plastid genome of Parthenium argentatum and identification of DNA barcodes to differentiate Parthenium species and lines", BMC PLANT BIOLOGY, vol. 9, no. 1, 131, 17 November 2009 (2009-11-17), BIOMED CENTRAL, LONDON, GB, pages 12PP, XP021062479, ISSN: 1471-2229, DOI: 10.1186/1471-2229-9-131
- RAY D T ET AL: "Breeding guayule for commercial production", INDUSTRIAL CROPS AND PRODUCTS, vol. 22, no. 1, 1 July 2005 (2005-07-01), ELSEVIER, NL, pages 15 - 25, XP027814348, ISSN: 0926-6690, [retrieved on 20050701]
- PERUMAL VENKATACHALAM ET AL: "Natural rubber producing plants: An overview", AFRICAN JOURNAL OF BIOTECHNOLOGY, vol. 12, no. 12, 4 December 2014 (2014-12-04), Victoria Island, pages 1297 - 1310, XP055340127, Retrieved from the Internet <URL:http://www.academicjournals.org/article/article1380725201_Venkatachalam%20et%20al.pdf> [retrieved on 20170130], DOI: 10.5897/AJBX12.016

## Description

### TECHNICAL FIELD OF THE INVENTION

The present invention relates in general to methods of selecting guayule plants suitable for breeding based on the rate of apomixis and molecular markers.

### BACKGROUND ART

Natural rubber is a valuable and strategically important biomaterial. The predominant source of natural rubber cultivated for commercial production is *Hevea brasiliensis,* which is grown mostly in South America, Southeast Asia and areas of Africa and India. To meet the demands for natural rubber and provide a sustainable source of biorubber in alternative geographic locations, other species of rubber producing plants have been studied.

Guayule (*Parthenium argentatum*) is a perennial desert shrub species native to northcentral Mexico and southwestern Texas. Guayule is being studied as an alternative source of natural rubber for potential large scale commercial production. Guayule produces rubber within its cells that generally must be destructively harvested after the plant reaches maturity. The guayule produced rubber also provides a benefit of being free from the proteins responsible for latex allergies that can be caused by *Hevea* sourced natural rubber.

Mathur, "Molecular Breeding & Crop Improvement of Jatropha and Guayule Hybrid Cultivars", Pacific Rim Bio Summit, 8 December 2014, discloses that apomixis restricts genetic gain when breeding guayule, and that ploidy and aneuploidy complicate breeding.

Kumar et al., "Comparative analysis of the complete sequence of the plastid genome of Parthenium argentatum and identification of DNA barcodes to differentiate Parthenium species and lines", BMC Plant Biology, 2009, 9:131, discloses the identification of chloroplast DNA barcodes useful for discriminating between species of *Parthenium.*

Ray et al., "Breeding guayule for commercial production", Industrial Crops and Products, 22 (2005), 15-25, discloses the history of guayule commercialisation and domestication, the reproductive biology of guayule, and current approaches to breeding guayule.

Kuravadi et al., "Identification and characterization of EST-SSR markers in cluster bean (Cyamopsis spp.)", Crop Science, May-June 2014, vol. 54, p.1097-1102, discloses the identification of simple sequence repeats (SSRs) useful for marked-based selection of cluster bean (aka "guar").

There is a need for diversifying the production of natural rubber and improvements in the breeding and characteristics of guayule plants that produce natural rubber.

### SUMMARY OF THE INVENTION

The present invention is directed to a method of selecting two guayule plants for breeding comprising:
(a) determining the rate of apomixis of one or more guayule plants;
(b) determining the genetic pedigree of the guayule plants using DNA analysis; and
(c) using the information regarding the rate of apomixis and genetic pedigrees to identify two plants that are genetically dissimilar suitable for use in guayule plant breeding to produce an advanced guayule plant;
wherein the DNA analysis is performed using DNA of mono-, di-, tri-, tetra-, penta- or hexanucleotide simple sequence repeats (SSRs), characterized in that the SSRs comprise the SSR markers corresponding to 4, 5, 10, 12, 18, 19, 68, 80, 93, 134, 139, 158, 185, 186 and 188 in Table 11.

Preferably, the rate of apomixis is determined by flow cytometry.

Advantageously, the rate of apomixis is determined from leaf samples, seed samples, or both.

Also disclosed herein, but not part of the present invention, are molecular markers for guayule and methods of using those molecular markers to identify, characterize and breed guayule. The molecular markers can also be used in the methods of the present invention. The molecular markers may be single nucleotide polymorphisms (SNPs) or short tandem repeating mono-, di-, tri-, tetra- penta, and hexanucleotide microsatellites (simple sequence repeats or SSRs). The molecular markers may be polymorphic. The molecular markers may be associated with expressed traits of the guayule plant, particularly those associated with improved plant yield, size, uniformity and rubber production. Also disclosed herein but not part of the claimed invention are methods for determining the rate of apomixis in guayule plants and using information regarding the rate of apomixis in a method for breeding a guayule plant, for example, in the identification of guayule plants for breeding, either alone or in combination with a step of determining the genetic pedigree of a plant, e.g., by DNA analysis using SNPs and/or SSRs. In particular, methods which include a step of breeding guayule are not part of the claimed invention.

Also disclosed herein but not part of the claimed invention are primers associated with SNPs or SSRs according to the invention. The primers may be used in a method to amplify a molecular marker such as an SNP or SSR, and that molecular marker may be polymorphic. Also disclosed herein but not part of the claimed invention is a primer kit containing a set of primers to be used in the methods according to the invention and as otherwise disclosed herein.

The molecular markers and primers may be used in a method to evaluate the genetic diversity of guayule germplasm from one or more collections of guayule germplasm.

Alternatively, the molecular markers and primers may be used in a method to quantify genetic similarity or distance among accessions within one or more collections of guayule germplasm.

The molecular markers and primers may be used in a method of parenteral germplasm screening and/or selection.

The molecular markers and primers may be used in a method for the genetic advancement of guayule germplasm.

The molecular markers and primers may be used in a method to screen and select one or more guayule cells, tissues, seedlings, plants, plant parts, hybrids or progeny thereof.

The molecular markers and primers may be used in a method to develop new varieties of guayule.

The molecular markers and primers may be used in a method of selection for one or more traits of guayule cells, tissues, seedlings, plants, plant parts, hybrids or progeny thereof.

The molecular markers and primers may be used in a method of identification, validation and/or quality assessment, including germplasm identity, parental background recovery, and seed purity testing.

In some instances, the molecular markers, primers and methods may be used to identify or improve an expressed trait of a guayule plant including rubber content, rubber quality, rubber yield, average molecular weight of rubber, plant size, plant yield, plant vigor, plant uniformity, salinity tolerance, temperature tolerance, disease resistance, or drought tolerance.

Also disclosed herein but not part of the claimed invention are guayule plant cells, plant tissue, seedlings, plant parts, plants, and progeny thereof produced by any of the methods of the present invention or otherwise disclosed herein. Also disclosed herein but not part of the claimed invention is the rubber composition produced by or extracted from such guayule plants produced by any of the methods of the present invention or otherwise disclosed herein. Those guayule plant cells, plant tissue, seedlings, plant parts, plants, and progeny thereof may have an improved expressed trait, phenotype and/or genotype over naturally occurring guayule or publicly available guayule germplasm.

Also disclosed herein but not part of the claimed invention are products comprising the rubber produced by or extracted from guayule plants disclosed herein or in plants produced by any of the methods of the present invention or otherwise disclosed herein. Those products may include tires and latex products for the medical profession.

### DESCRIPTION OF THE DRAWINGS

For a more complete understanding of the features and advantages of the present invention, reference is now made to the detailed description of the invention along with the accompanying figures.
FIG. 1 is a principal coordinate analysis of guayule accessions showing the 2D relationships among the represented guayule germplasm per ploidy level.
FIG. 2 is a principal coordinate analysis using pairwise genetic distances among individual plant samples from 54 SSR markers.
FIG. 3 is a diagram of Bayesian cluster assignments of guayule germplasm from controlled crosses.
FIG. 4 is a tree representing the genetic relationships among a subset of private and public guayule germplasm (red = private; black = public germplasm) using SSR markers.
FIG. 5 is a general outline of NGS assisted plant breeding from Ray, S. and Sataya, P., 2014 "Next Generation Sequencing Technologies for Next Generation Plant Breeding," Front. Plant Sci.

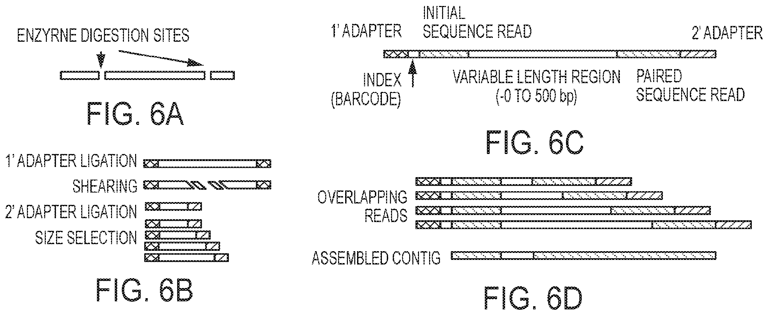

FIG. 6 is a diagram providing an overview of paired-end RAD sequencing. In section A, genomic DNA is digested with a restriction endonuclease. In section B, after ligation with a primary adapter, the fragments are sheared, then ligated with a secondary adapter. In section C, a composite mixture of variable length fragments is recovered from each restriction enzyme digestion site. These fragments are size selected, amplified and sequenced on a next-generation DNA sequencing platform using paired end chemistry. In section D, development of the genomic assembly around each digestion site is then completed bioinformatically.
FIG. 7 is a DNA quality check of four guayule DNA samples.
FIG. 8 is a diagram of a summary neighbor joining tree from Nei's distances showing the relationships among the different diploid HS and USDA germplasm, BART historical germplasm, and other representative polyploids such as G7-11 (Rec ID 88716 and 88717).
FIG. 9 is a diagram of a tree showing genetic similarity among individual plants of each germplasm type (HS or accession). Colors denote original group assignment. Some clusters contain plants from one or more HS (e.g. HS 5 and HS 14) indicating genetic admixture. Polyploid germplasm are shown to separately group from diploids.
FIG. 10 is a diagram of expected heterozygosity of SNP loci within each diploid germplasm.
FIG. 11 is a matrix of average number of pairwise differences among the diploid germplasm. The above diagonal indicates differences between germplasm, the diagonal elements show differences within germplasm, and the below diagonal the Nei pairwise genetic distances.
FIG. 12 is a diagram of the possible reproductive classes from a tetraploid X tetraploid.
FIG. 13 is a graph of seed maturity vs. nuclei staining (red = seed; black = 2x reference).
FIG. 14 is a graph of embryo ploidy detectable at all development stages (red = early; blue = mid; late = black).

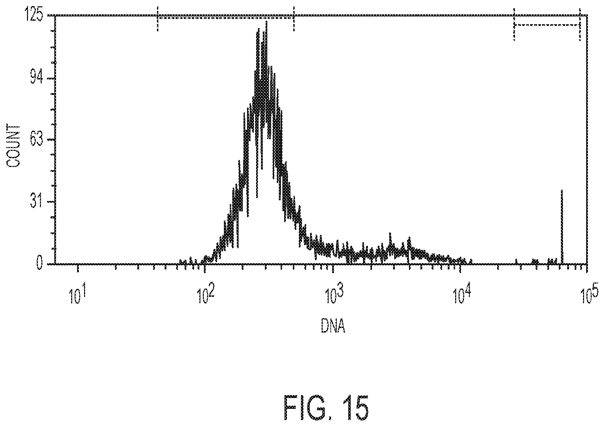

FIG. 15 is a graph of DNA content - seed + internal control.

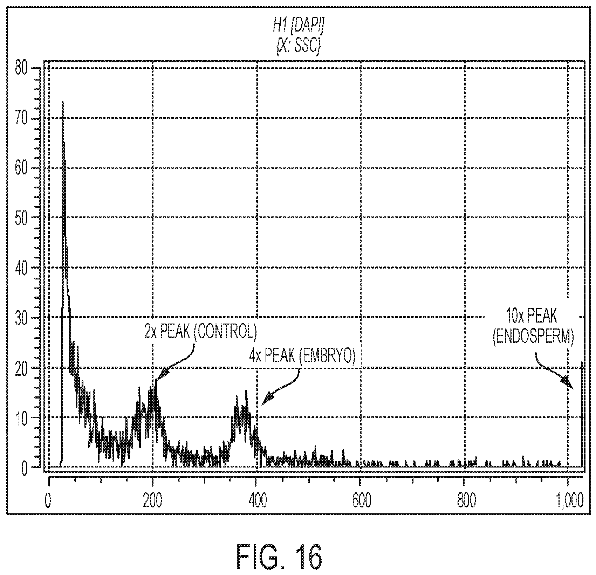

FIG. 16 is a graph of apomictic seed from a 4x plant.

### DETAILED DESCRIPTION OF THE INVENTION

The present invention is directed to a method of selecting two guayule plants suitable for breeding. The method of the invention, as well as other methods and compositions disclosed herein, can be used for the aim of the genetic improvement of guayule, or to improve a guayule crop for higher plant yield, size, uniformity and rubber production, and other traits that will make it suitable for large scale agriculture.

Disclosed herein are molecular markers for guayule and methods of using those molecular markers to identify, characterize and breed guayule. Methods which include a step of breeding guayule are not part of the claimed invention. The molecular markers may be single nucleotide polymorphisms (SNPs) or short tandem repeating mono-, di-, tri-, tetra- penta, and hexanucleotide microsatellites (simple sequence repeats or SSRs). In addition, the molecular markers may be polymorphic. The SNPs may be selected from the SNPs listed in Table 9. The SSRs may be selected from the SSRs listed in Tables 11 and 12.

Also disclosed herein are primers associated with SNPs or SSRs disclosed herein. The primers may be used in a method to amplify a molecular marker such as an SNP or SSR, and that molecular marker may be polymorphic. Also disclosed herein is an isolated nucleic acid molecule, or primer, for detecting a molecular marker representing a polymorphism in guayule plant DNA, wherein said nucleic acid molecule comprises at least 15 nucleotides that include or are immediately adjacent to said polymorphism, wherein said nucleic acid molecule is at least 90 percent identical to a sequence of the same number of consecutive nucleotides in either strand of DNA that include or are immediately adjacent to said polymorphism. The polymorphism may be a polymorphic SNP or SSR, for example, a SNP or SSR identified in Tables 9, 11 and 12. Also disclosed herein is a primer kit containing a set of primers to be used in the methods according to the invention and as otherwise disclosed herein. The set of primers may comprise one or more primers listed in Tables 11 and 12.

The molecular markers and primers disclosed herein may used in a method to evaluate the genetic diversity of guayule germplasm from one or more collections of guayule germplasm. For example, the method may be for determining the contributions of guayule populations to a guayule genome, comprising performing a genotyping assay on a sample obtained from a test guayule plant to determine the identity of one or both alleles present in the test guayule plant genome for each set of markers, wherein the set of markers is indicative of the contribution of guayule populations of the genome of the test guayule plant. The set of markers used in that method may comprise at least five markers or, more preferably, at least ten markers. The set of markers may be selected from single nucleotide polymorphisms (SNPs) or single nucleotide repeat microsatellites, di-, tri-, tetra-, penta- and hexanucleotide simple sequence repeats (SSRs). For example, the set of molecular markers may comprise markers listed in Tables 9, 11 and 12.

The molecular markers and primers disclosed herein may used in a method to quantify genetic similarity or distance among accessions within one or more collections of guayule germplasm.

The molecular markers and primers disclosed herein may be used in a method of parenteral germplasm screening and/or selection. For example, the method may be for determining the genotype of guayule germplasm, comprising the steps of (a) amplifying the guayule germplasm using a set of primers comprising primers identified in Tables 11 and 12; and (b) determining the genotype using a combination of molecular markers comprising molecular markers identified in Tables 11 and 12. Also disclosed herein is a primer kit comprising primers identified in Tables 11 and 12.

The molecular markers and primers disclosed herein may be used in a method for the genetic advancement of guayule germplasm.

The molecular markers and primers disclosed herein may be used in a method to screen and select one or more guayule cells, tissues, seedlings, plants, plant parts, hybrids or progeny thereof.

The molecular markers and primers disclosed herein may be used in a method to develop a new variety of guayule. For example, the method may be directed to a guayule plant breeding method comprising (a) evaluating guayule plants using objective criteria to obtain phenotype scores; (b) determining the genetic pedigree of the guayule plants using DNA analysis; and (c) using the phenotype scores and genetic pedigrees to identify one or more select guayule plants for use in a next generation of guayule plant breeding to produce an advanced generation of guayule plants. The DNA analysis may be performed using DNA of single nucleotide polymorphism (SNP) or single nucleotide repeat microsatellites, di-, tri-, tetra-, penta-, or hexanucleotide simple sequence repeats (SSRs). Two select guayule plants are identified that are genetically dissimilar or highly dissimilar and the two select plants may be crossed to produce an advanced generation of guayule plants.

The molecular markers and primers disclosed herein may be used in a method of selection for one or more traits of guayule cells, tissues, seedlings, plants, plant parts, hybrids or progeny thereof. For example, such methods may be for identifying a genetic marker linked to a trait locus of a guayule plant, said method comprising the steps of: (a) extracting nucleic acid from a guayule plant; (b) amplifying the nucleic acid using a set of primers comprising primers identified in Tables 11 and 12; (c) determining the genotype using a set of molecular markers comprising molecular markers identified in Tables 11 and 12; and (d) associating a genetic marker with an expressed trait of the guayule plant. The genetic marker may be linked to a trait such as rubber content, rubber quality, rubber yield, average molecular weight of rubber, plant size, plant vigor, plant uniformity, salinity tolerance, temperature tolerance, disease resistance, or drought tolerance. The temperature tolerance may be cold tolerance.

The molecular markers and primers disclosed herein may be used in a method of identification, validation and/or quality assessment, including germplasm identity, parental background recovery, and seed purity testing.

The molecular markers and primers disclosed herein, and methods according to the invention and as otherwise disclosed herein, may be used to improve the characteristics of a guayule plant including rubber content, rubber quality, rubber yield, average molecular weight of rubber, plant size, plant vigor, plant uniformity, salinity tolerance, temperature tolerance, disease resistance, or drought tolerance.

Also disclosed herein are guayule plant cells, plant tissue, seedlings, plant parts, plants, and progeny thereof produced by any of the methods according to the invention or as otherwise disclosed herein. Also disclosed herein is the rubber composition produced by or extracted from such guayule plants produced by any of the methods herein. Those guayule plant cells, plant tissue, seedlings, plant parts, plants, and progeny thereof may have an improved expressed trait, phenotype and/or genotype over naturally occurring guayule or publicly available guayule germplasm.

Also disclosed herein are products comprising the rubber produced by or extracted from plants disclosed herein, including tires and latex products for the medical profession.

### EXPERIMENTAL PROCEDURES

A study was conducted to develop microsatellite (SSR) and single nucleotide repeat (SNP) markers that can be used in methods according to the invention and as otherwise disclosed herein, including methods in which molecular markers are used to screen for genetic diversity, perform marker assisted selection, and aid trait introgression for guayule breeding.

Publicly available guayule expressed sequenced tags were used to develop 713 SSR primer pairs and testing was completed on 193 SSRs. A set of SSRs was used to screen the genetic diversity of USDA guayule collection and a private germplasm collection to assess the genetic variation within accessions and among various guayule genetic groups.

In addition to SSRs, SNP markers were developed for guayule breeding. A total of 1,065 SNPs were identified from four guayule sequence libraries and the level of polymorphism was studied. SNPs are identified in Table 9. SSRs and their corresponding primers are identified in Tables 11 and 12. The two marker systems provide unique opportunities for use in guayule molecular breeding for plant improvement.

Molecular markers for guayule were developed to increase the available information for decision making during guayule improvement activities. Research to find markers that are useful for estimating genetic diversity also aimed to identify molecular markers that are tightly linked to traits. Having these useful markers will enable rapid screening of segregating phenotypes at an early stage of development, with potential time and cost savings.

### Example 1 - Development and testing of microsatellite markers (SSR)

Initial work to develop primers for microsatellite (simple sequence repeat or SSR) markers was completed. Expressed sequenced tags (EST) made publicly available by the USDA-ARS Elastomics Project through the NCBI EST online database were downloaded. A total of 11,739 EST sequences were obtained and contigs assembled by using an overlap identity cutoff of 80% and using masking processes to identify repeats, vector, and organelle sequences. From the contigs, 337 microsatellite units were identified while 515 microsatellite units on the singletons (sequences did not contain enough overlap in length to be combined with others). The microsatellites from the contigs have the following proportion of repeat units: 73 (21.7%) di-, 242 (71.8%) tri-, 12 (3.5%) tetra-, and 10 (3.0%) hexa-nucleotides. Primers for microsatellite units in contigs and singletons were designed based on the following parameters: 100-500 amplicon size, 18-24 bp primer size, 40-60% G-C content, 55-60°C with 58°C optimal Tₘ. A total of 198 SSR primers were designed from the contigs and 515 from the singletons (see Tables 11 and 12 for primer sequences and SSRs).

Preliminary primer testing was conducted using seven guayule germplasm (11591, 11604, 141660, 141678, AZ-2, S1, S3) representing various ploidy levels (2X to 6X). Leaf tissue was obtained on 10 to 12 individual plants per accession and the DNA was processed for extraction and genotyping. Two bulks of DNA samples per accession were made by pooling DNA from six different plants (five for the triploid accessions).

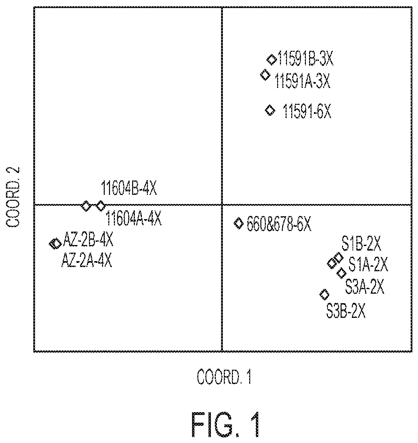

An 18 bps M13 fragment was added to forward primer of all 198 markers and FAM fluorescent dye was used to label the primers. Polymerase chain reaction (PCR) was performed using Applied Biosystems^{®} GeneAmp^{®} PCR System 9700 following 50 cycles with 95°C denaturation, 55°C annealing and 72°C extension. The amplified products were run on ABI 3730xl sequencer in order to separate DNA fragments based on their molecular weights. Out of 198 tested markers 20 (10%) failed to amplify any part of the genome, while 178 (90%) successfully amplified. A total of 93 markers (47%) were polymorphic while 85 primers (43%) were monomorphic. The genetic distances among accessions were computed following the method of Huff et al. (1993) treating the polymorphic marker scores as dominant since the test set included polyploid guayule germplasm. The principal coordinates plot, showing the 2D relationships among the represented guayule germplasm per ploidy level, was computed and are shown in FIG. 1.

The 93 polymorphic markers were further assessed to determine marker polymorphism using individual plant samples and determine their robustness. Two to five individual plants representing each accession were used. Out of the 93 polymorphic markers, 54 markers were found to be most promising and easy to score and can be readily utilized for analyzing the entire guayule germplasm collection. An additional 18 markers were also determined as useful, though these set will need further performance validation using fluorescent labels. The other 21 markers were found to be difficult to score but may have utility in the long term.

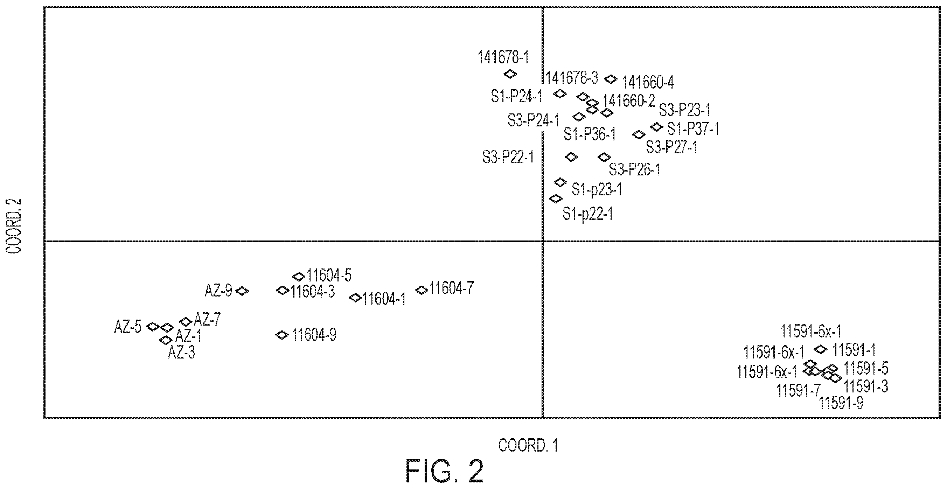

The genetic distances among individual plant samples were determined from results of using the 54 markers. The principal coordinates plot is shown in FIG. 2. The plot shows the genetic similarity among samples of each ploidy level. The tight clustering of samples of 11591 and the low mean pairwise genetic distance (Table 1) suggest that this accession has more narrow genetic diversity than AZ-2. The diploid populations (S1 and S3) have higher mean pairwise distance values than the tetraploid accessions. These preliminary observations remain to be confirmed using larger sets of samples.

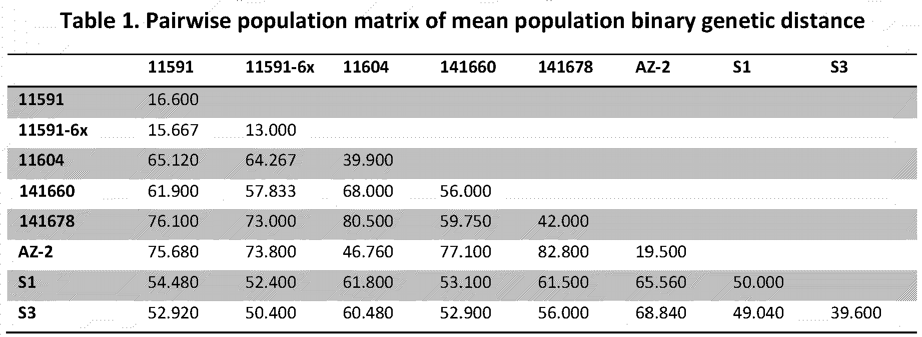

Microsatellite markers have many applications in biological studies of plants, molecular genomics, and in a breeding program. They can be used to determine taxonomic classification, population structure, genetic diversity, and marker-trait associations.

First generation (F1) progenies from polyhaploid (n = 36) x tetraploid (n = 72) guayule germplasm along with parental materials were genotyped using microsatellite markers (SSRs) to test if possible to validate the parentage of the progenies and confirm that they arose from sexual recombination. The SSR markers used were those determined as most robust in the set (54 of 93 SSRs) during preliminary testing. Those 54 SSR markers correspond to 4, 5, 6, 8, 10, 12, 16, 18, 19, 24, 26, 37, 43, 51, 52, 58, 61, 68, 70, 71, 80, 93, 107, 108, 112, 114, 115, 118, 119, 126, 128, 132, 134, 139, 140, 143, 144, 156, 157, 158, 162, 164, 165, 170, 171, 180, 181, 183, 185, 186, 188, 195, 197, and 198 in Table 11.

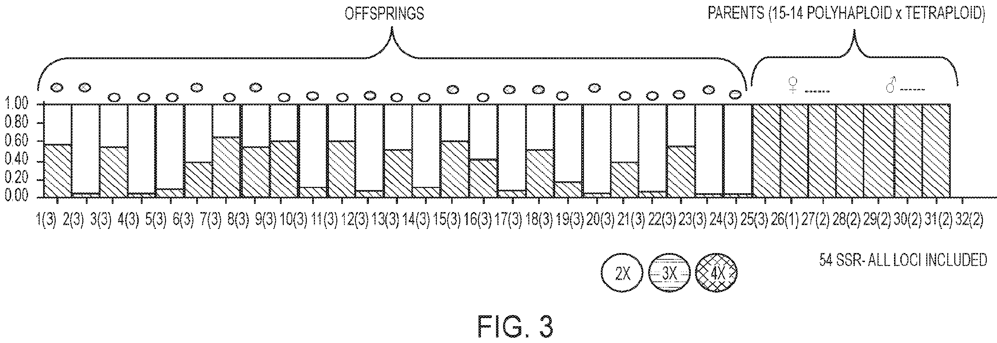

The Bayesian clustering results using the marker data provided additional information about possible paternity and parental mode of reproduction. The cluster assignments of the genotyped germplasm are shown in FIG 3. This method is originally intended for determining population structure but also adapted for this purpose.

Leaf tissues from 140 guayule germplasm were collected for SSR genotyping (Table 2). A subset of 15 guayule SSRs was used to determine the genetic similarity among breeding materials and with the USDA germplasm collection to assist the breeding program. Those 15 SSR markers correspond to 4, 5, 10, 12, 18, 19, 68, 80, 93, 134, 139, 158, 185, 186 and 188 in Table 11.

For genotyping, the M13-tailed SSR primers used during the primer testing phase were replaced by fluorescent-labeled primers which are more stable, reliable than M13-labelled primers, and gave reproducible results needed for routine genotyping.

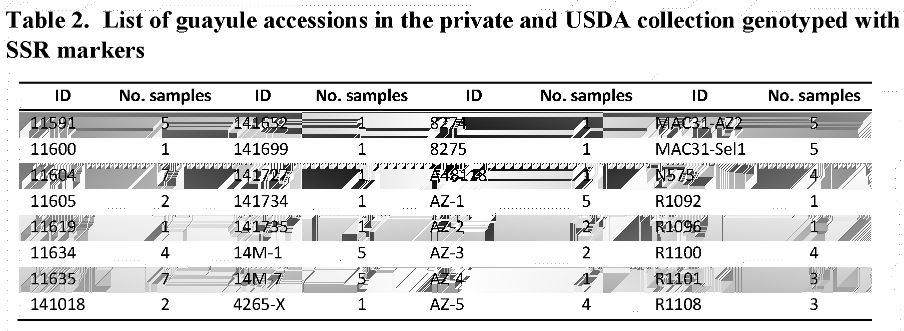

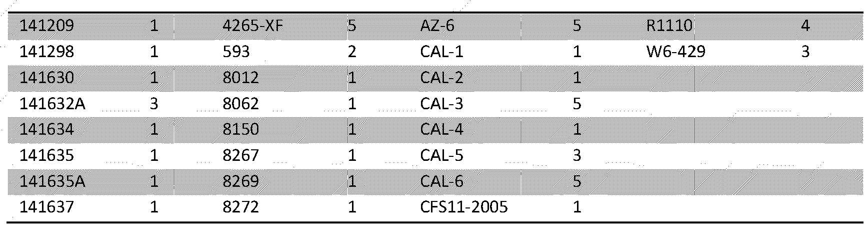

Analysis of SSR data showed that the mean major allele frequency for the marker set was 0.3752 and the polymorphism information content (PIC) was 0.7309 indicating that the marker set is highly informative. Pairwise comparison of all the germplasm using Dice coefficient (Dice, 1945) has a mean of 0.3938, with values ranging from 0.0000 to 0.7857 (9,730 total pairwise values). The highly similar germplasm, as well as extremely dissimilar germplasm pairs are listed in Table 3.

The mean pairwise genetic distance among selected private germplasm and USDA germplasm was computed following the method of Huff et al. (1993) on dominant markers and the results shown in Table 4.

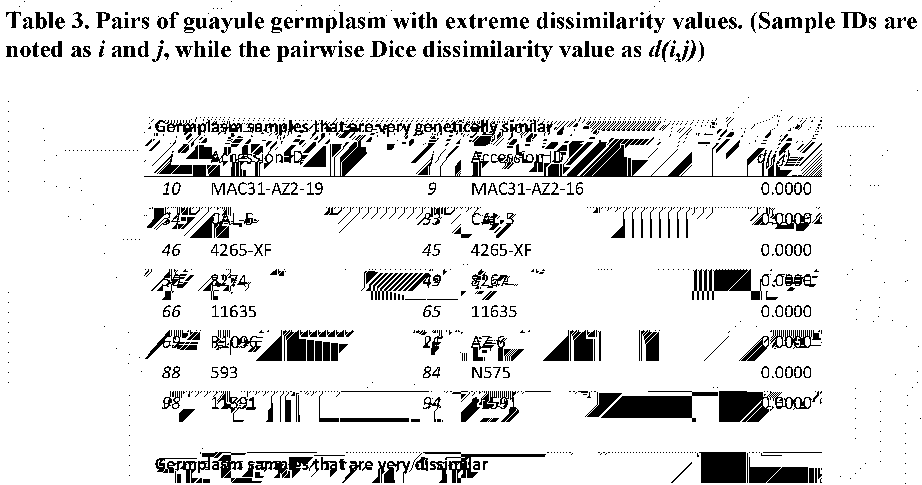

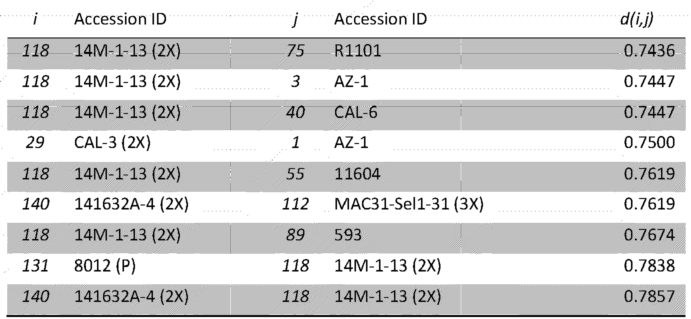

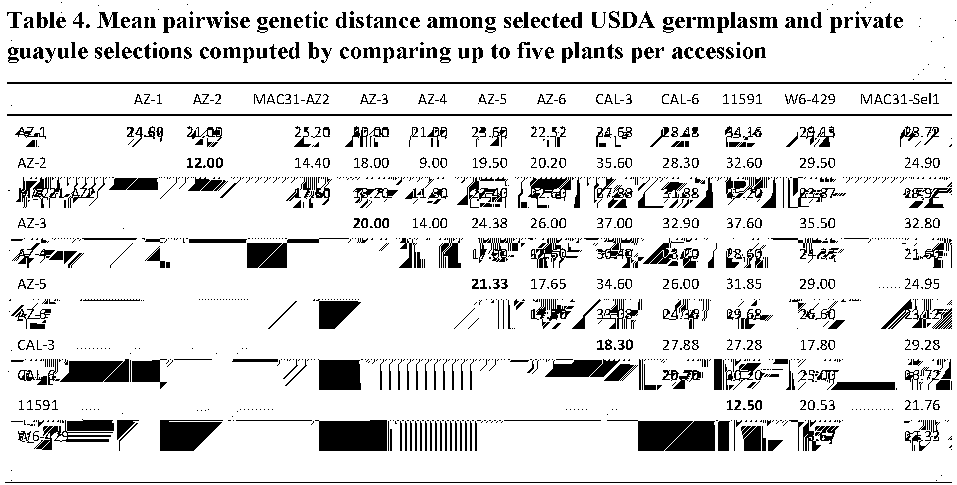

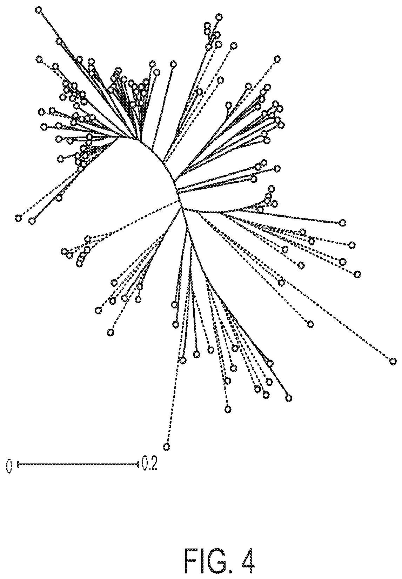

A tree representing the genetic similarity among the different accessions in the private and public collections using 15 SSR markers is shown in FIG. 4.

The SSRs will be useful in establishing a genetic linkage map of guayule and identification of quantitative trait loci (QTL). This can be the basis to develop mapping populations to determine the distribution of the SSR markers in various linkage groups the guayule. Both SSRs and SNPs will be used for estimating recombination events, establishing marker-trait association in quantitative and qualitative traits of guayule.

SSR markers may be used to anchor next-generation genome sequencing data into chromosomes. Contigs where markers were detected may be used to physically map genetic marker sequences and assess their location on the genetic map.

Information from SSR and SNPs can be used to establish genotype information for future proprietary guayule varieties. The information from both molecular marker systems will allow identity and purity testing of seed supply as well as gene flow or potential contamination.

### Reference Example 1 - Development and testing of SNPs (RAD-seq) markers

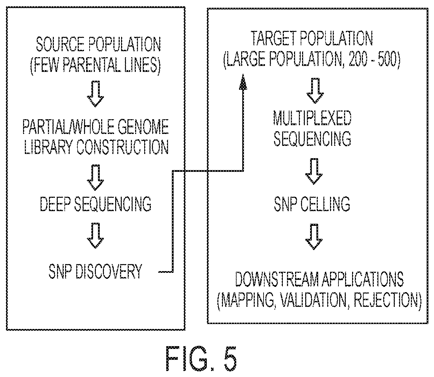

Restriction-site associated DNA sequencing (RAD-seq) as a next generation sequencing (NGS) technology platform was employed to obtain large numbers of SNP markers. The potential of NGS technologies for marker assisted breeding have been reviewed in the literature and FIG. 5 is an example from Ray, S., and Sataya, P., 2014 "Next Generation Sequencing Technologies for Next Generation Plant Breeding," Front. Plant Sci.

Four individual leaf samples of 4 accessions were received for SNP discovery project. Discovery of SNPs can be effectively performed by using next-generation sequencing (NGS) technologies. NGS technologies and the use of a complexity reduction strategy can successfully be applied for SNP discovery in large and complex genomes with no reference genome. Restriction site-associated DNA sequencing (RAD-Seq) is commonly used for SNP detection in genomes and is based on identifying polymorphism next to restriction enzyme digestion sites. Paired-end RAD sequencing was applied for detection of sequence variation and design of SNP markers. An overview of pair-end RAD sequencing is shown in FIG. 6. In section A, genomic DNA is digested with a restriction endonuclease. In section B, after ligation with a primary adapter, the fragments are sheared, then ligated with a secondary adapter. In section C, a composite mixture of variable length fragments is recovered from each restriction enzyme digestion site. These fragments are size selected, amplified and sequenced on a next-generation DNA sequencing platform using paired end chemistry. In section D, development of the genomic assembly around each digestion site is then completed bioinformatically.

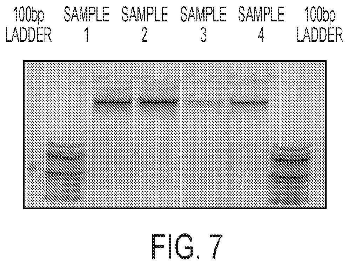

To develop the RAD genotyping platform for guayule, leaf tissue samples were collected from 11591, 8150 (Cal 3), 141632A (W6-429), and 14M-1-12 (S1) germplasm to obtain 3 µg of high molecular weight DNA required for DNA library development. The DNA quality and quantity was confirmed by agarose gel as shown in FIG. 7. DNA sequencing was performed on each sample and development of RAD libraries followed. The associated statistics with the four RAD libraries are shown below. The number of sequencing reads indicates the number of short DNA fragments that have been sequenced.
Total Reads: 30,211,365
Mean Reads per Sample: 6,042,273
Median Reads per Sample: 6,488,899
Read Goal per Sample: 4,000,000
Std. Dev of Reads per Sample: 1,899,084
Number of Samples Meeting Read Goals: 4
Sequencing Coefficient of Variation: 0.31

**Table 5**

| **Sample ID** | **NGS Reads** | **Read Goal** | **RAD Clusters** |
|---|---|---|---|
| **11591 (4X)** | 8,656,563 | 4,000,000 | 29,973 |
| **14M-1-12 (2X)** | 6,705,768 | 4,000,000 | 24,366 |
| **141632A-3 (2X)** | 6,488,899 | 4,000,000 | 20,754 |
| **8150 (2X)** | 4,270,193 | 4,000,000 | 19,587 |

The sequence data for guayule was analyzed to find markers with good characteristics for downstream genotyping. There were 1,065 markers that met the required characteristics for genotyping (80 bp flanking sequence, no other markers within 50 bp). Those SNP molecular markers are shown in Table 9. A total of 970 primers were designed and tested for marker polymorphism and informativeness.

For testing, leaf tissues were harvested from five plants of 12 diploid accessions (14M-7, CAL-3, HS10, HS12, HS14, HS15, HS17, HS18, HS2, HS5, HS6, and W6-429) and genotyped. Two sets of primers were used for the initial screening of the diploid accessions. The first set of 384 primers indicate that 379 SNPs successfully amplified, 5 failed, and 3.6% has missing data. Of the 384 markers, 319 are polymorphic while 60 were monomorphic. The mean allele frequency was 0.65, the PIC was 0.28 and mean heterozygosity was 0.68. The genetic distances among the 12 accessions were estimated using the method of Nei (1972) (Table 6).

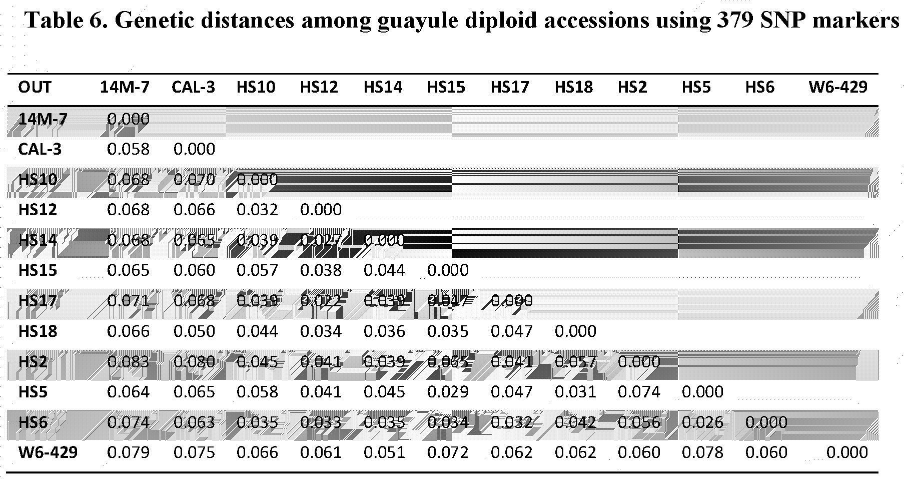

**Table 7. Examples of SNP sequences discovered by RAD-Seq method**

| **#CHROM** | **POS** | **SEQ** | **Sample_1** | **Sample_2** | **Sample_3** | **Sample_4** | **ESTA Name** |
|---|---|---|---|---|---|---|---|
| RAQ_kmer_0000025_1_TGCA | 107 | CGCCACACATAAATAATCAAACCTTATCCGTCACCACTTGAGAAAAACCCTATACA GGGCGGCATGCCGCCACTGTCGCT[A/G]CTGCCCTGTCCGGCCGCCATTTTGTTTTTG TTTTCTGTTTCGTCTTTCTTGTTTCCTGTTCTAAAAAGACTAGGTCTAGGT | G | A | A | A | GYSNP0001 |
| RAD_kmer_0000028_1_TGCA | 262 | AAATCGCAATATACGAATACCACATAAACGGAAAGAATTCGTTTATTAGTTCCTTT ATTTATTTACTATTACGAAAGTAA[G/C]CCATTTATGGTGTTATAATGACTTAATTA TCTAAGATACTAATACATGACTATAAATTTAAAATCTGGCTACGGTTTAAT | C | G | G | - | GYSNP0002 |
| RAD_kmer_0000037_1_TGCA | 446 | TGCAGTAATTTTAGGTAGGAAGTGCAAAATATGGGTCGGTAAAAAATCAGCCGGG TCAAATGGGTCATCAACAAAACCAA[C/T]AAATAACGATAAAATGCCAATATTCA TTACATATTAATTAATAGCATTGACATGTTCATTAAAAAATTAACCAATAACCTT | C | C | T | T | GYSNP0003 |
| RAD_kmer_0000038_1_TGCA | 417 | AGAATAAGATAATTGGTTCTTAGAGTTTTTTTTAGGATGCAACCAATCTGAACTGTT GGATGGCGCAACGAATCATAATC[A/T]TTGTTTGGAACCAAAATTTGAAGACAAG TTAAAGGGCTAGATTTTTTTTAAACATCACTAATTTTAATGATAAATATACA | W | A | T | - | GYSNP0004 |
| RAD_kmer_0000047_1_TGCA | 106 | TACGCATGTACCAGGTAAAGGGTTATGAGAGTTGGGCCAAGGCACCACTCCATGT GGGCCTAAGGTACTCCAAAAATGAT[G/A]GGCTTCACTTTAGCGTAACAGGACAT GGGTATCAAGCATCCGTGATGAAACGCAAGGTGGGCACAAGCCCACCTATTGCA | G | G | A | G | GYSNP0005 |
| RAD_kmer_0000049_1_TGCA | 210 | TCTTCTCTTCTATTCAATATATGTTCTCCGTCACTAAAATTTTTATACTGTTAAAAAT GATTTTTATTGCCCGGGATGGC[G/A]GGATCTCACTGGAAGCAACCTCTCTATCTTC AAAGATGGATGTAAGGCTTGCCTCCATCCCACCCTCCCAGACCCTTACT | G | G | A | A | GYSNP0006 |
| RAD_kmer_0000049_1_TGCA | 339 | TTGCCTCCATCCCACCCTCCCAGACCCTTACTTTCTGTGTGGGATATTACTGGGTAC GCTTGTTTGTTTGTTTGTTTACT[T/C]CTATAATGTAGGTATGTTGGCAATAATATAG AACCAACCCAATTATAAAAAGAAAAAAAAAAGATCATTCGTAGTGCTAG | C | T | T | T | GYSNP0007 |
| RAD_kmer_0000052_1_TGCA | 352 | CTATGGTTATCCGTGAAAGGTATAATTCTGGAACACCCTATGGTTCGGGTTATAATT TTTACTCGGTTTGTAATCGGATC[T/A]CTGACGGGAATTTAAATCAGATTTAAATTA TGTACACAACGGTTCCGCTACTCGATTAATATGTAATATTAATAAACGTA | w | T | A | A | GYSNP0008 |

The second set consisted of 192 SNP assays screened against the same set of 12 diploid accessions. Another 145 polymorphic SNP markers in this set were identified. A total 464 SNPs were found to be informative from both tests which were all used to screen single sample DNA (in contrast to bulked DNA in previous testing). A subsequent stepwise testing of these markers on single plant DNA sample was conducted to evaluate the effectiveness of each marker on individual plants. Four plants per accession were analyzed (total of 48 samples). Among the 464 SNPs, only 348 were found to be polymorphic when single sample DNA was used and these was the preferred set for routine analysis.

### Diversity analysis and parental selection for crossing

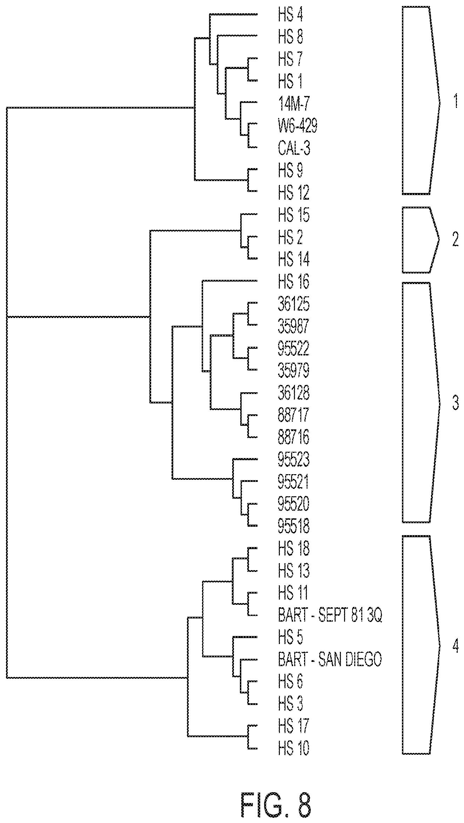

Leaf tissues from 1,144 diploid guayule germplasm were collected for SNP genotyping. The 348 SNPs set was used to determine the genetic similarity among these breeding materials and with the USDA diploid germplasm collection and representative polyploid germplasm. A summary neighbor joining tree from Nei's distances is shown in FIG. 8. There are four major clusters denoting germplasm groups. The USDA diploid germplasm W6-429 and Cal-3 grouped with 14M-7 and six HS. There is a distinct group containing G7-11 (8817, 8816) and other polyploids along with HS 16.

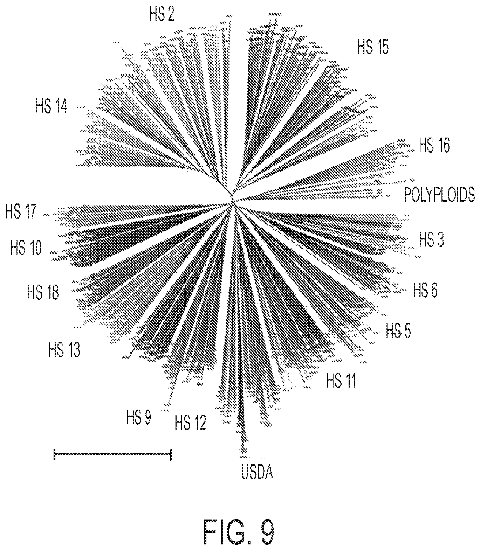

FIG. 9 shows a detailed tree with all individual plants color coded by HS and accession grouping. Some HS are genetically uniform with the individual plants having very similar genotypes (e.g. HS 10, 12, 13, 16, 17, and 18), while others show genetic admixture (e.g. HS 2, 5 and 6).

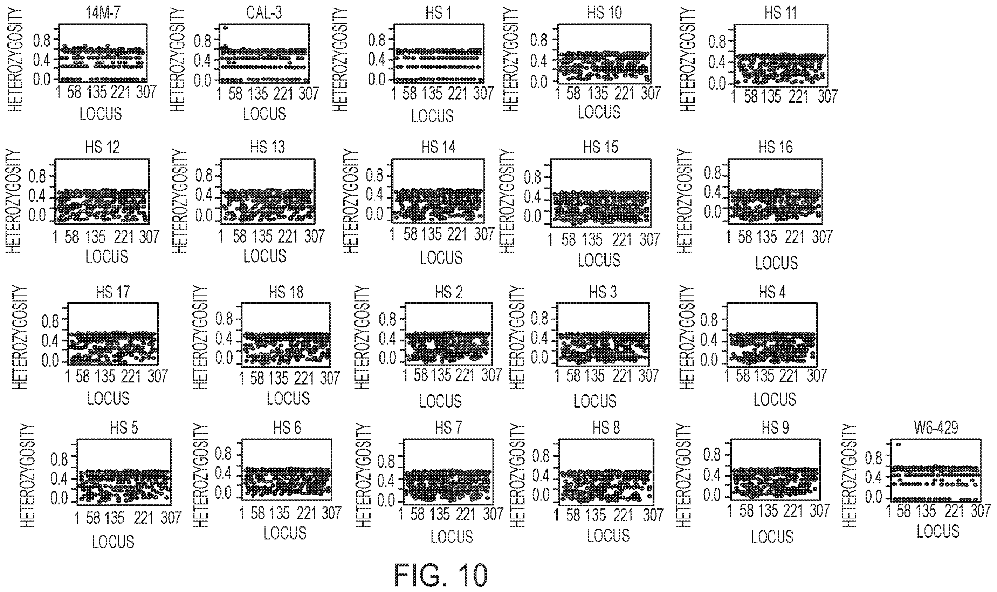

The mean total expected heterozygosity (gene diversity) of the diploid HS from the 348 SNP markers was estimated at 0.37. The highest heterozygosity was observed on 14M-7 (0.43) while the lowest on HS 14 (0.32). FIG. 10 shows the expected heterozygosity on all SNP loci of the diploids.

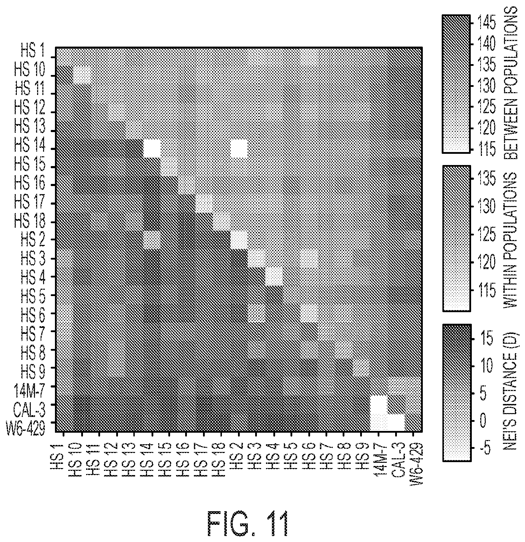

The similarity between and within the diploid accessions is shown in FIG. 11. Information from these genetic relationships are useful in selecting parents for the breeding program that are genetically distant (less similar).

### Example 2 - Analysis of Rate of Apomixis in Guayule Germplasm

As disclosed herein, the rate of apomixis of guayule germplasm may be determined and used in breeding guayule, including the selection of guayule germplasm to produce an advanced guayule plant, seed or plant tissue. There is disclosed herein, but not part of the claimed invention, a method for breeding a guayule plant which comprises determining the rate of apomixis of one or more guayule plants, using the resulting information regarding the rate of apomixis of the plant(s) to identify one more select guayule plant(s) for use in guayule plant breeding to produce an advanced guayule plant, seed or plant tissue. The rate of apomixis may be determined by flow cytometry. The rate of apomixis may be determined from leaf samples, seed samples, or both. Also disclosed herein are guayule plants, seeds, and plant tissue generated from such methods of breeding. The plant tissue referenced in these methods includes the natural rubber produced by the guayule plant.

The present invention relates to a method for selecting two guayule pants for breeding, comprising (a) determining the rate of apomixis of one or more guayule plant(s), (b) determining the genetic pedigree of the guayule plant(s); and (c) using the information regarding rate of apomixis and genetic pedigrees to identify two guayule plants that are genetically dissimilar suitable for use in guayule plant breeding to produce an advanced guayule plant The DNA analysis is performed using DNA of mono-, di-, tri-, tetra-, penta- or hexanucleotide simple sequence repeats (SSRs). In analogous methods which are not part of the claimed invention, the DNA analysis can be performed using DNA of single nucleotide polymorphisms (SNPs). In some embodiments, the rate of apomixes is determined by flow cytometry. In some embodiment, the rate of apomixes is determined from leaf samples, seed samples, or both. Disclosed herein are guayule plants, seeds, and plant tissue generated from such methods. The plant tissue referenced in these methods includes the natural rubber produced by the guayule plant.

The rate of apomixis in guayule germplasm was studied for enhancing guayule breeding. Guayule has a range of reproductive types of populations ranging from sexual diploid plants to facultative apomictic polyploid lines. Plant improvement has been a challenge in guayule through traditional breeding due to complications from facultative apomixis in polyploid collections.

As shown in Table 8, pollen viability also can relate to whether the plant is a polyhaploid, tetraploid or a diploid.

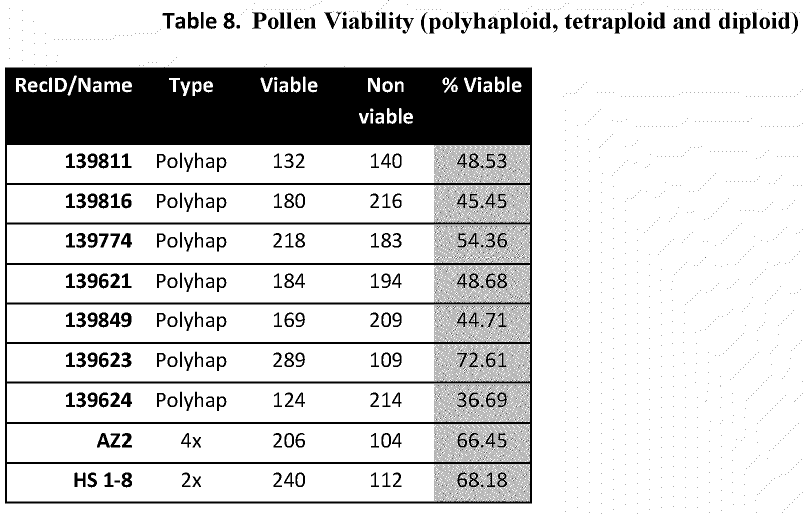

Guayule polyploids range from triploids *(2n = 3x =* 54) to octaploids *(2n = 8x =* 144). Polyhaploids result from reduction of a 4x (4x = 72) maternal plant and no fertilization. On a ploidy analyzer, when sampling leaf tissue, they can appear the same as diploids but differ in reproduction.

Polyploidy was studied during the Emergency Rubber Project in the 1940's by counting chromosomes in root-tips or pollen mother cells, with appropriate stains. Use of new research technologies and equipment provide for advancement of the understanding of polyploidy in a way that was previously too labor-intensive to be practical. An example of this is the use of a desk-top ploidy analyzer (flow cytometry) that is a fast and easy technique used to measure embryo and endosperm ploidy. Flow cytometry (or ploidy analysis) allows hundreds of plants in a population to be analyzed and the ploidy of each to be identified. Since most lines or populations are facultative apomictic, this is very valuable information.

This technique was also expanded to not only analyzing the ploidy of a plant but to also determine the rate of occurrence of sexual and non-sexual types of reproduction. This method combined with molecular markers can be a nondestructive method for identifying new genetic diversity in a guayule breeding program.

In this experiment, a novel method has been employed using seeds produced by a single plant that utilizes the combination of embryo and endosperm ploidy number to detect the type of reproduction that occurs from this single plant, referred to as the rate of apomixis. Although this method is technically destructive, the knowledge of the rate of apomixis is valuable and once it is known, molecular markers can be identified in leaf tissue to distinguish between sexual and non-sexual (apomictic) types and allow selection of plants resulting from sexual reproduction to obtain greater genetic diversity for rubber improvement.

The USDA National Plant Germplasm System (NPGS) contains 51 accessions of guayule *(P. argentatum)* plus accessions that are interspecific hybrids. All except two diploid guayule collections reproduce by apomixis; the two diploid accessions reproduce sexually. The objective of this study was to estimate the rate of apomixis, as well as whether or not there was meiotic reduction in the megaspore mother cell, and whether or not there was fertilization to form an embryo. The germplasm that was the subject of this study included two basic types - 2 accessions of diploids (sexual reproduction) and 48 accessions of polyploids (facultative apomictic). The polypolids range from triploids (3x = 54) to octaploids (8x = 144). All germplasm was publically available in the Germplasm Resources Information Network (GRIN) USDA collections.

Young leaf tissue of maternal plants and 100 progeny seeds from these plants were analyzed to estimate their rate of apomixis. The seed provided additional insight due to the ploidy levels in two different seed structures (embryo and endosperm). Diploid leaf samples were prepared in UV dye as a control to calibrate the ploidy analyzer. The seed samples were chopped in UV dye to prepare the sample with a 2x leaf sample and analyzed in the ploidy analyzer.

The study sought to identify which of the four potential reproductive classes the seed resulted from: (1) meiotic reduction of egg + fertilization; (2) meiotic reduction of egg + no fertilization; (3) no meiotic reduction of egg + fertilization; and (4) no meiotic reduction of egg + no fertilization. The results potentially indicate that the earlier assumptions of: (1) embryo sac development and (2) normal meiosis in the male (pollen) are not always the case. Results from this study indicated that off-type progeny (seed) would not have been identified if only leaf samples were used, resulting in an over-estimate of the rate of apomixis for the accession. Segregation in triploids is an example of this.

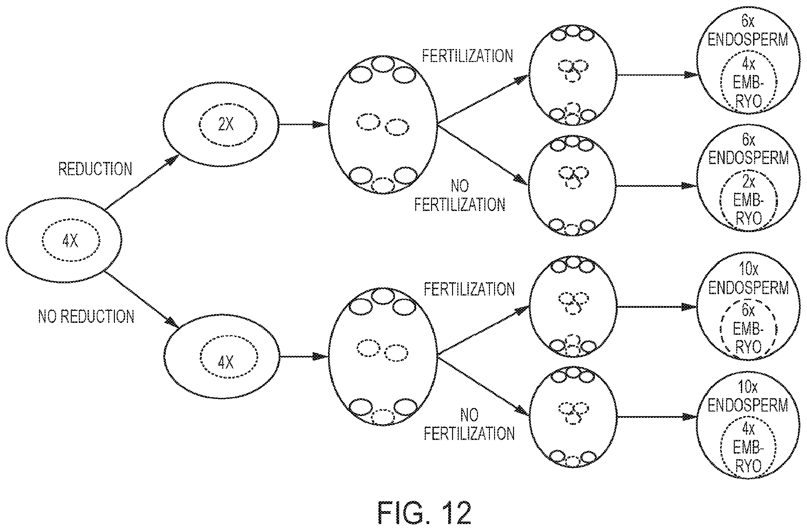

For example, a tetraploid x tetraploid could involve the reproductive classes as depicted in FIG. 12.

These results can help guide a guayule breeding program and better utilize plants resulting from reduction and/or fertilization causing new recombinations. The technique will also help to better characterize new breeding lines and the expected amount of variation from generation to generation.

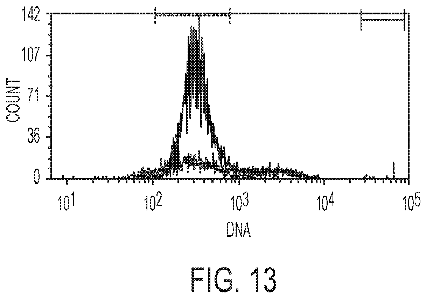

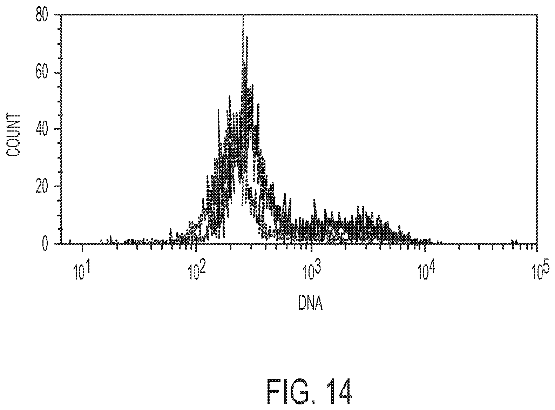

Seed maturity and nuclei staining was also studied. Seeds from plant heads at different stages of development (early, mid and late stage) were collected. As shown in FIGS. 13-14, results indicated that embryo ploidy was detectable at all development stages.

The USDA collection R1100-4x resulted in a 93% rate of apomixis. The USDA collection 11604-2x polyhaploid resulted in a 0% rate of apomixis. Further studies and analysis will further expand the usefulness of this technology in developing improved guayule plants and the rubber produced by such plants.

All of the compositions and/or methods disclosed and claimed herein can be made and executed without undue experimentation in light of the present disclosure.

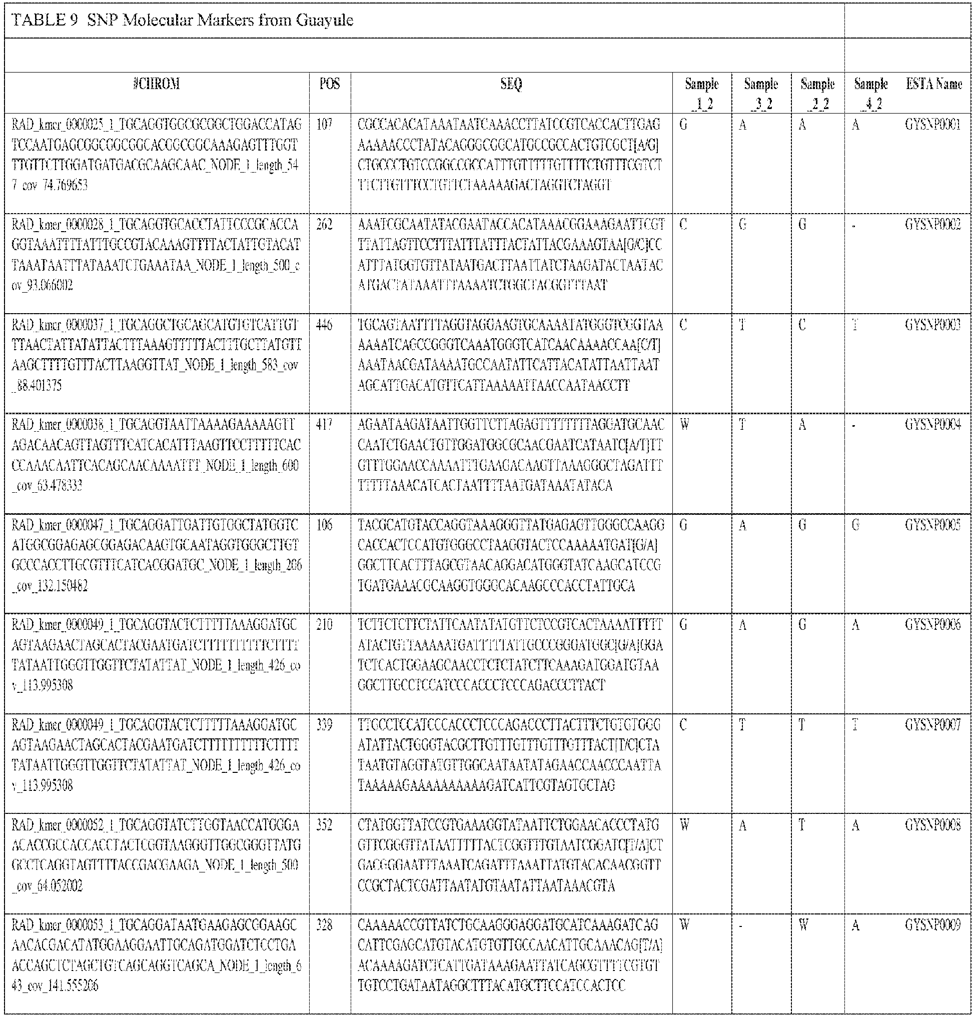

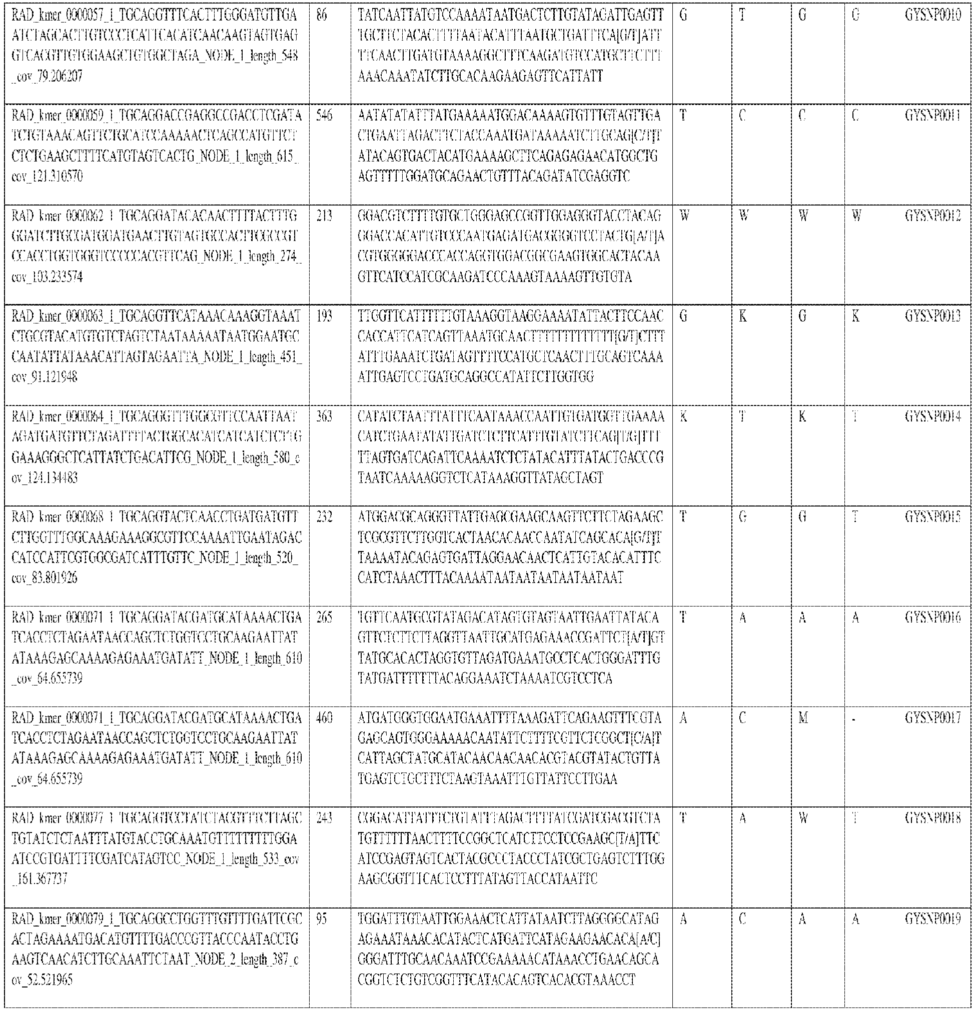

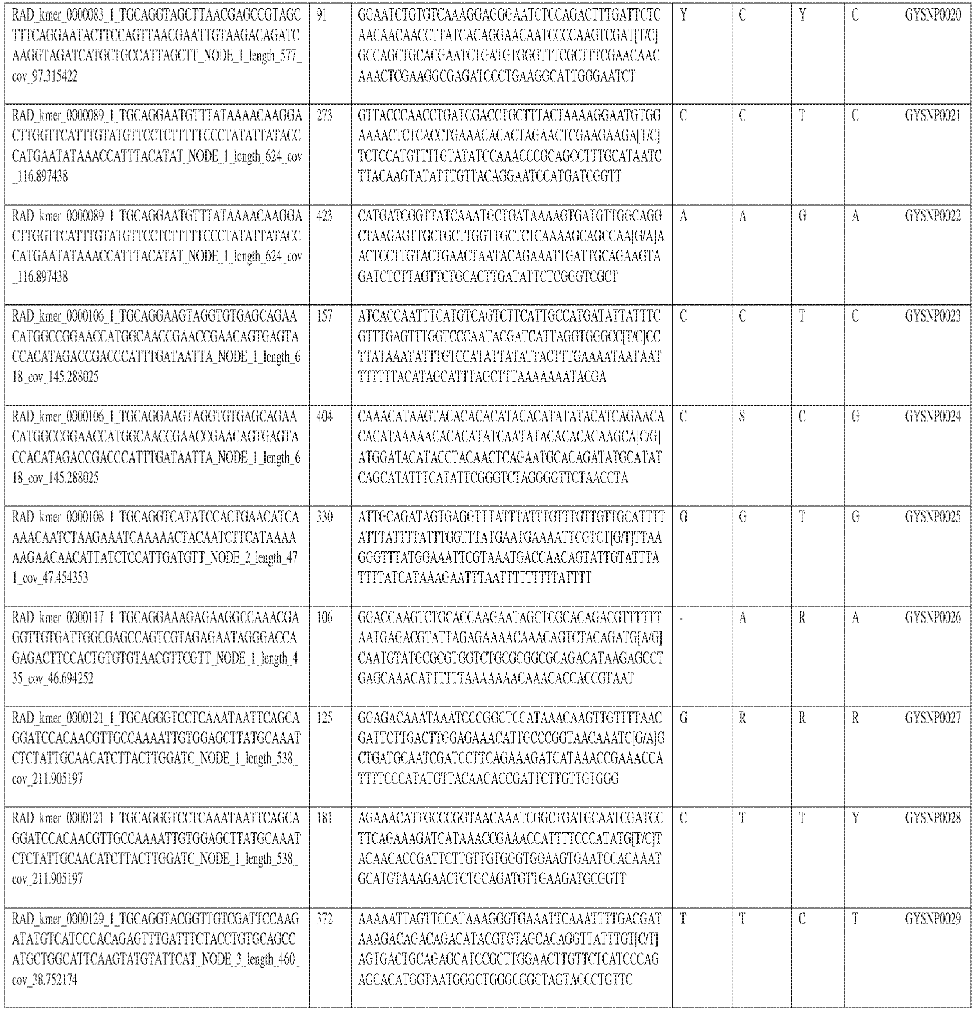

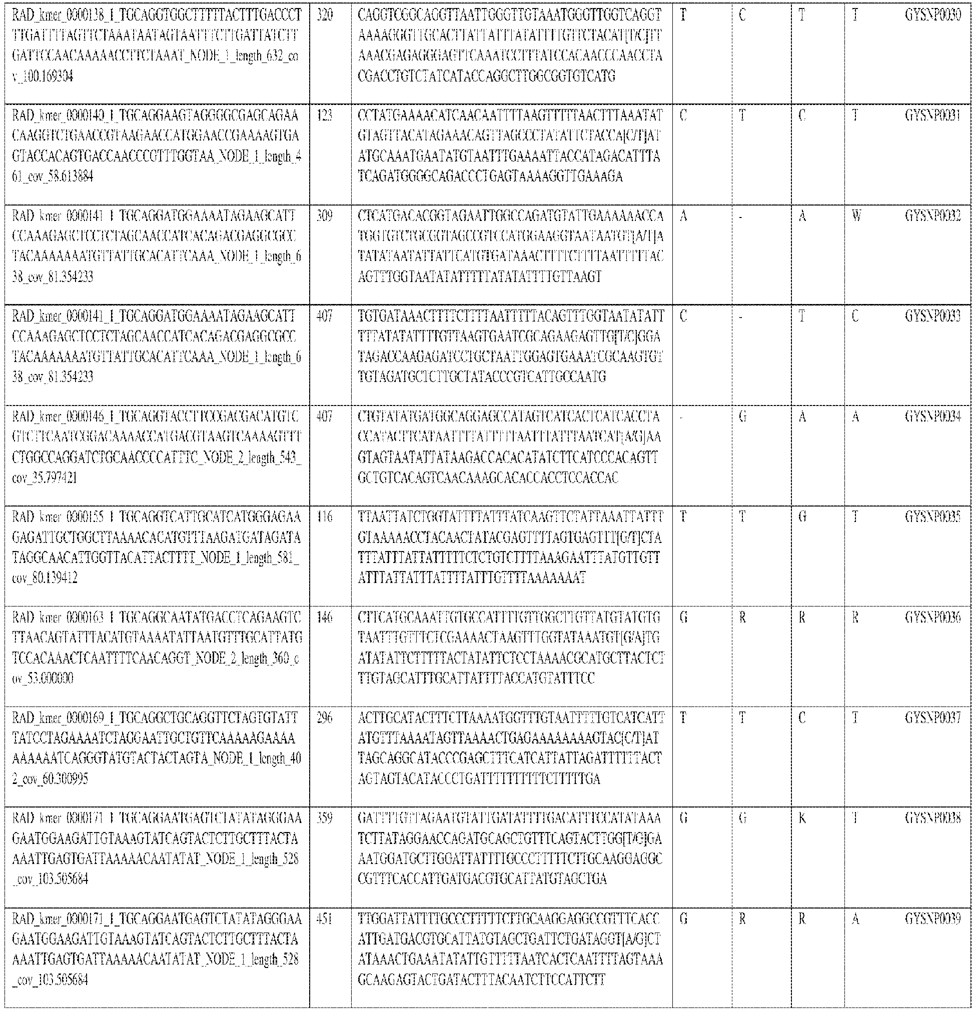

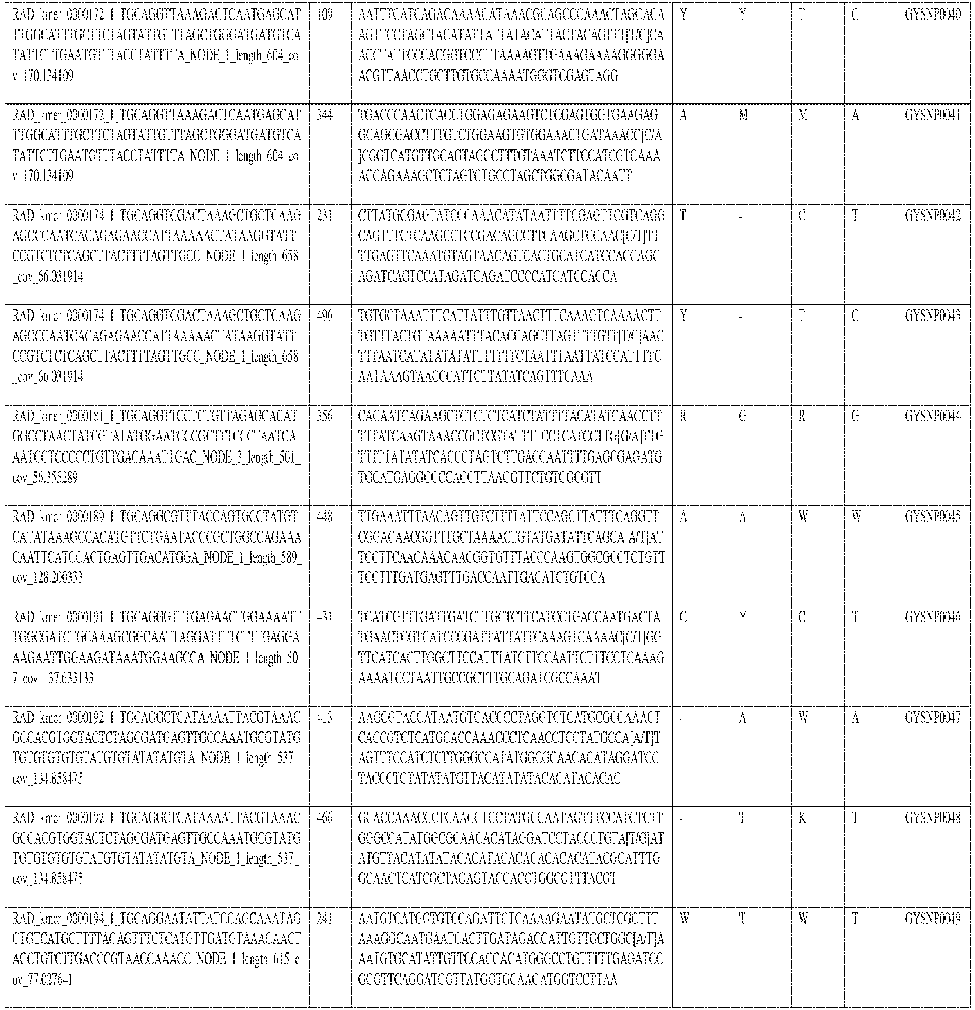

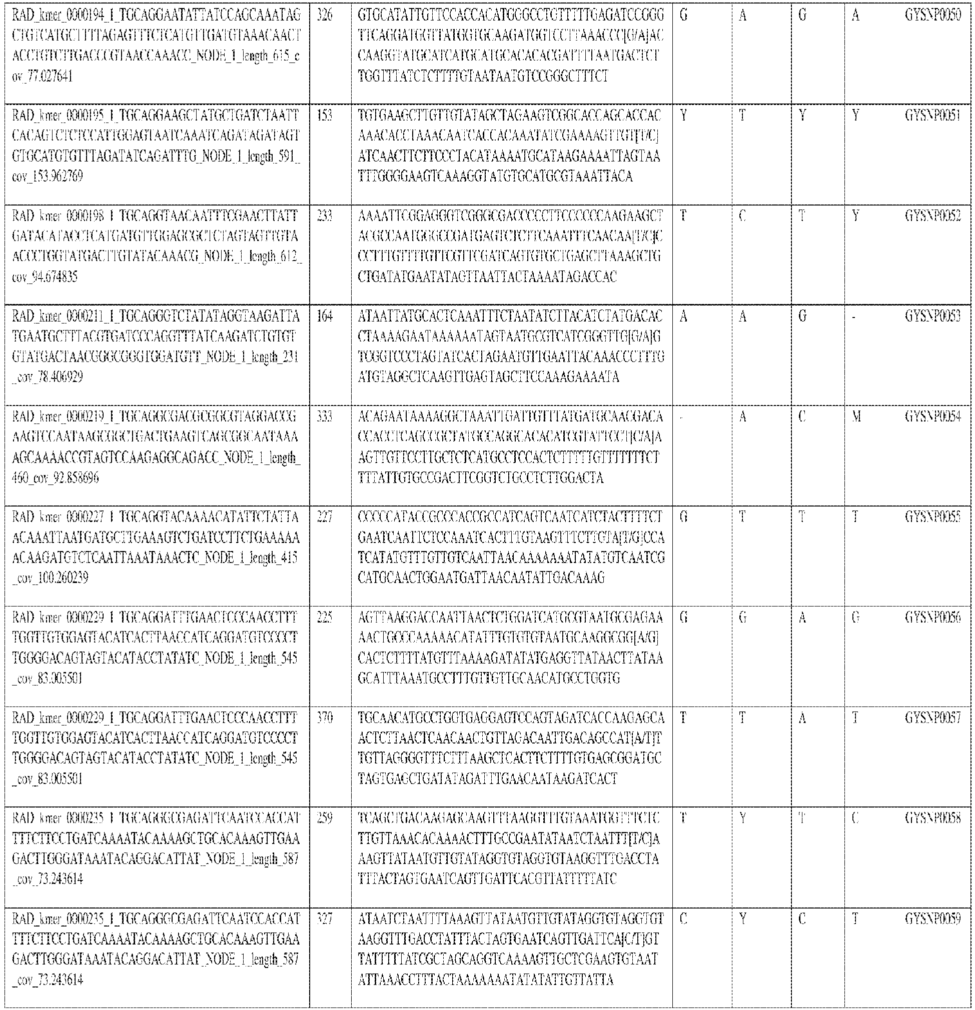

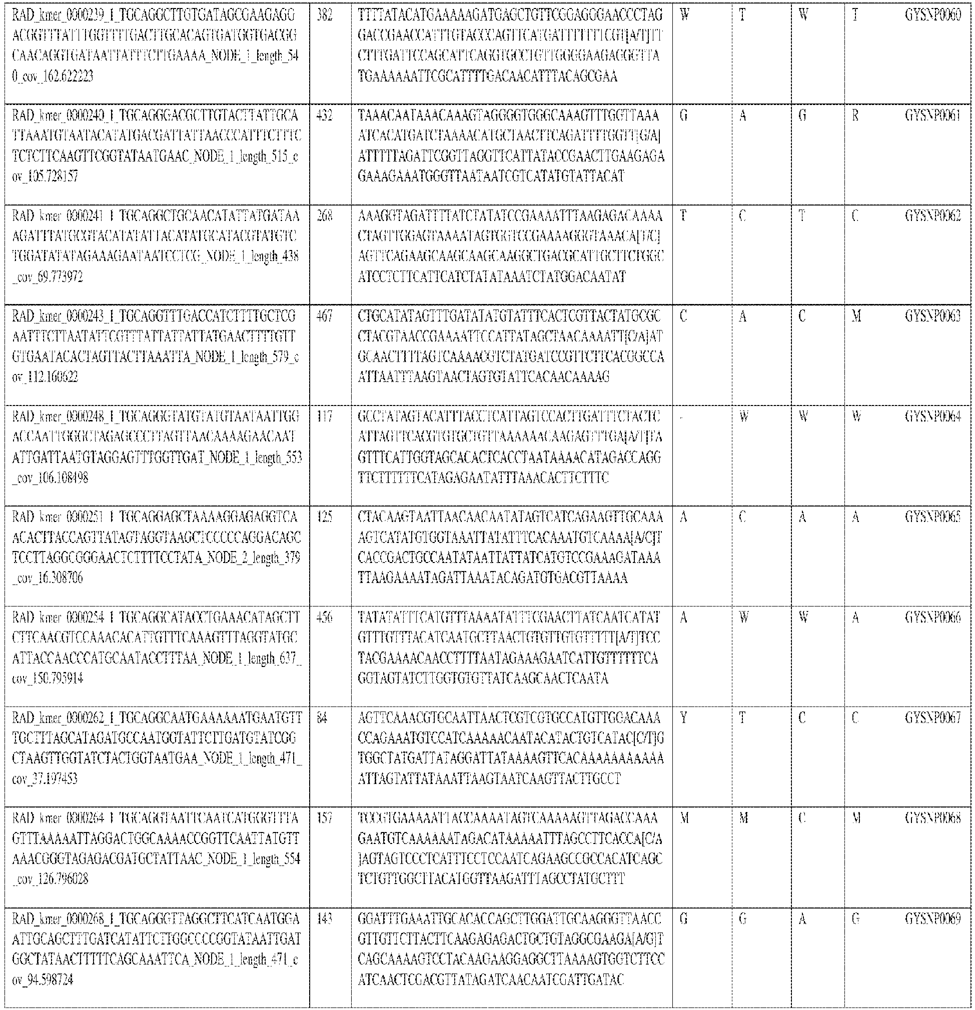

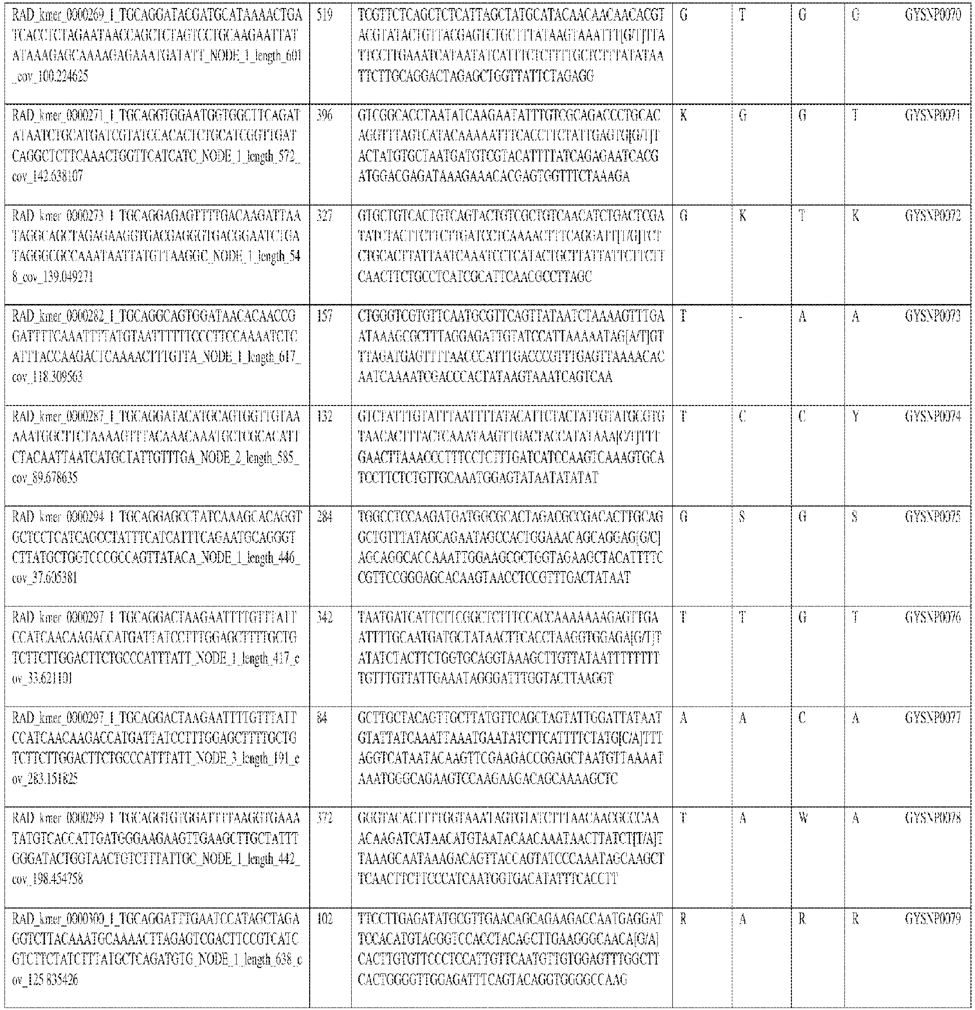

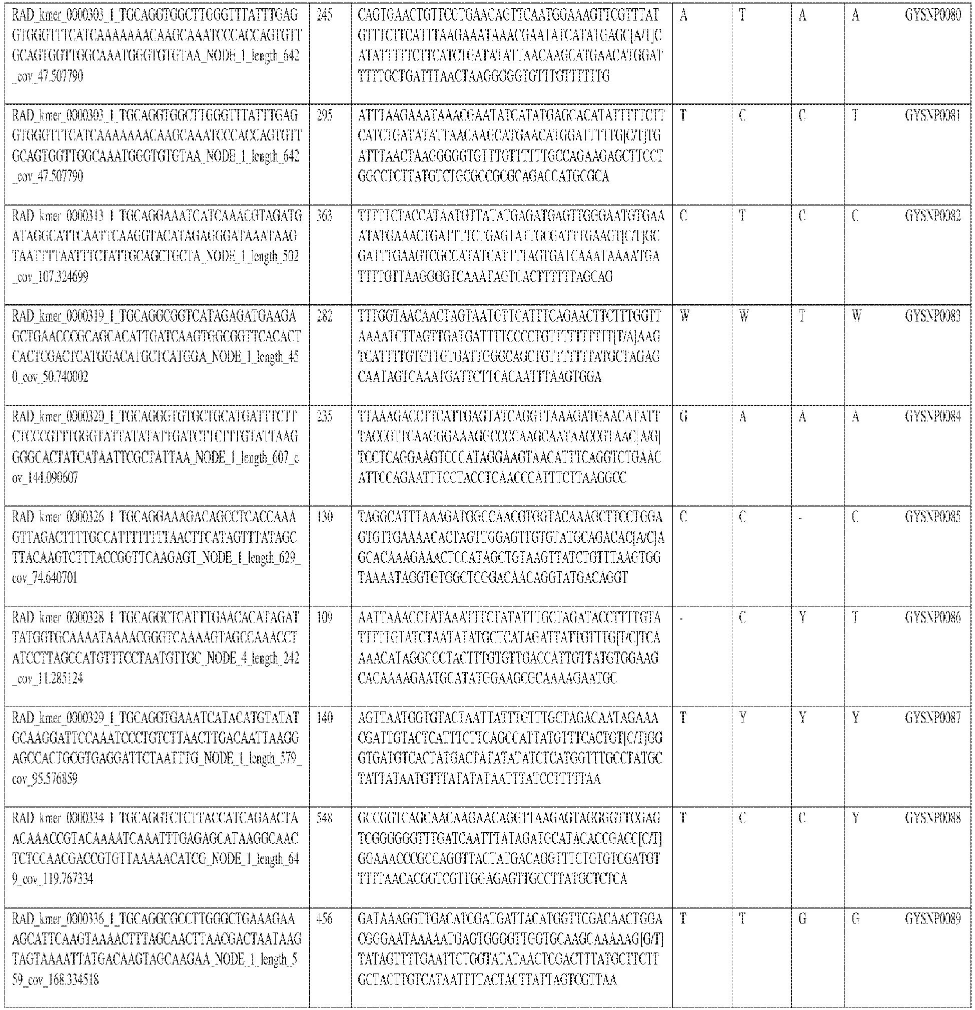

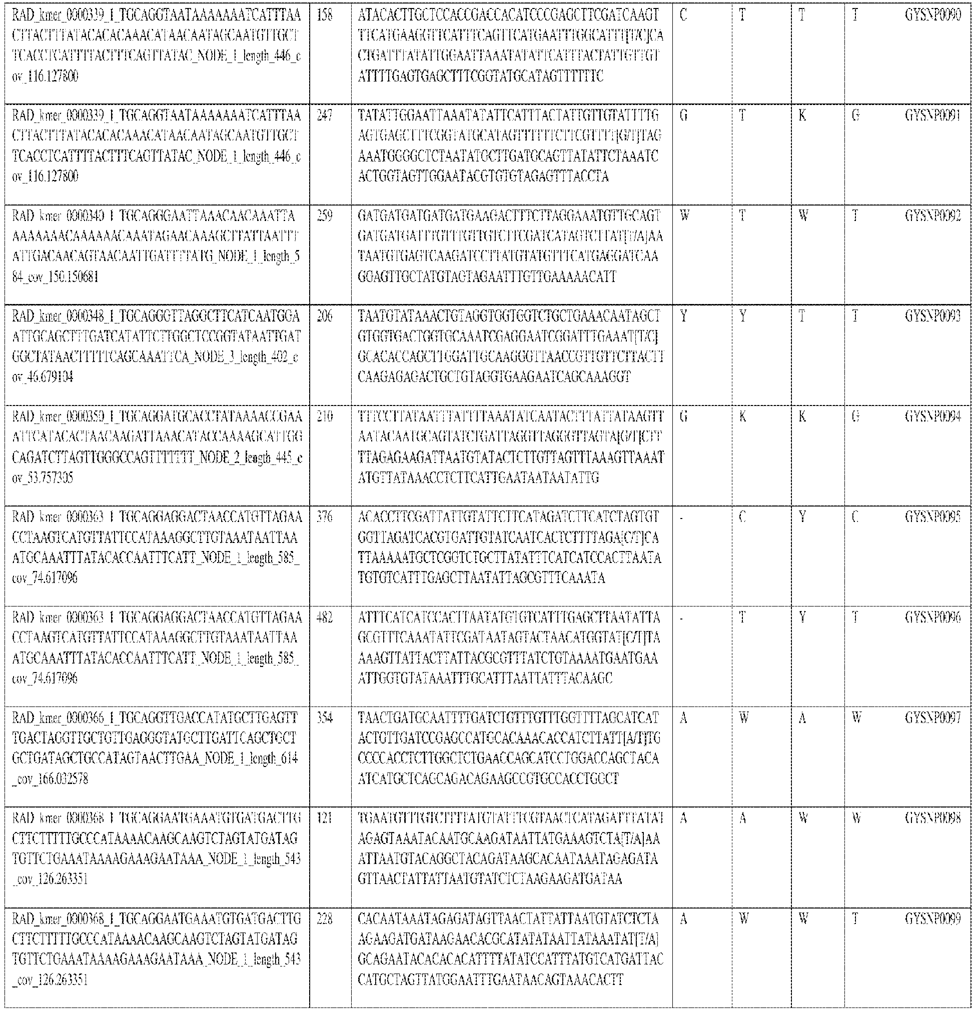

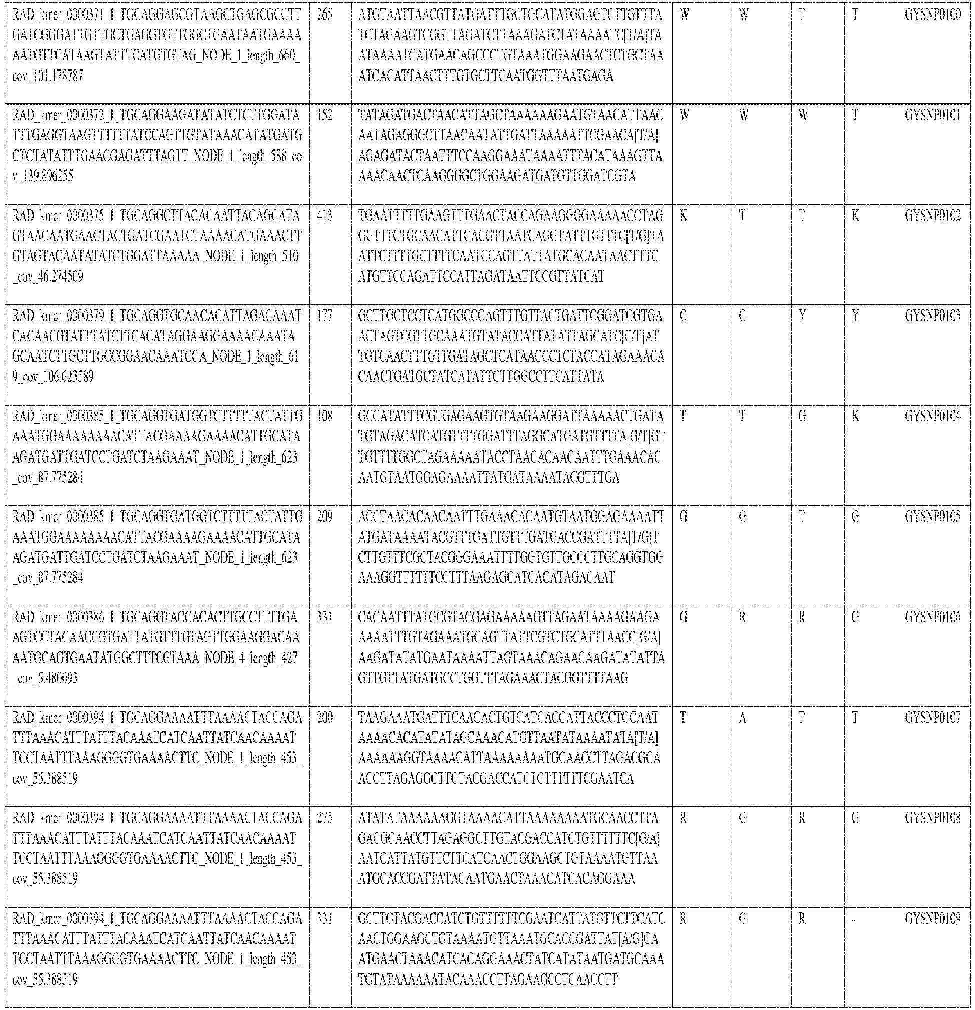

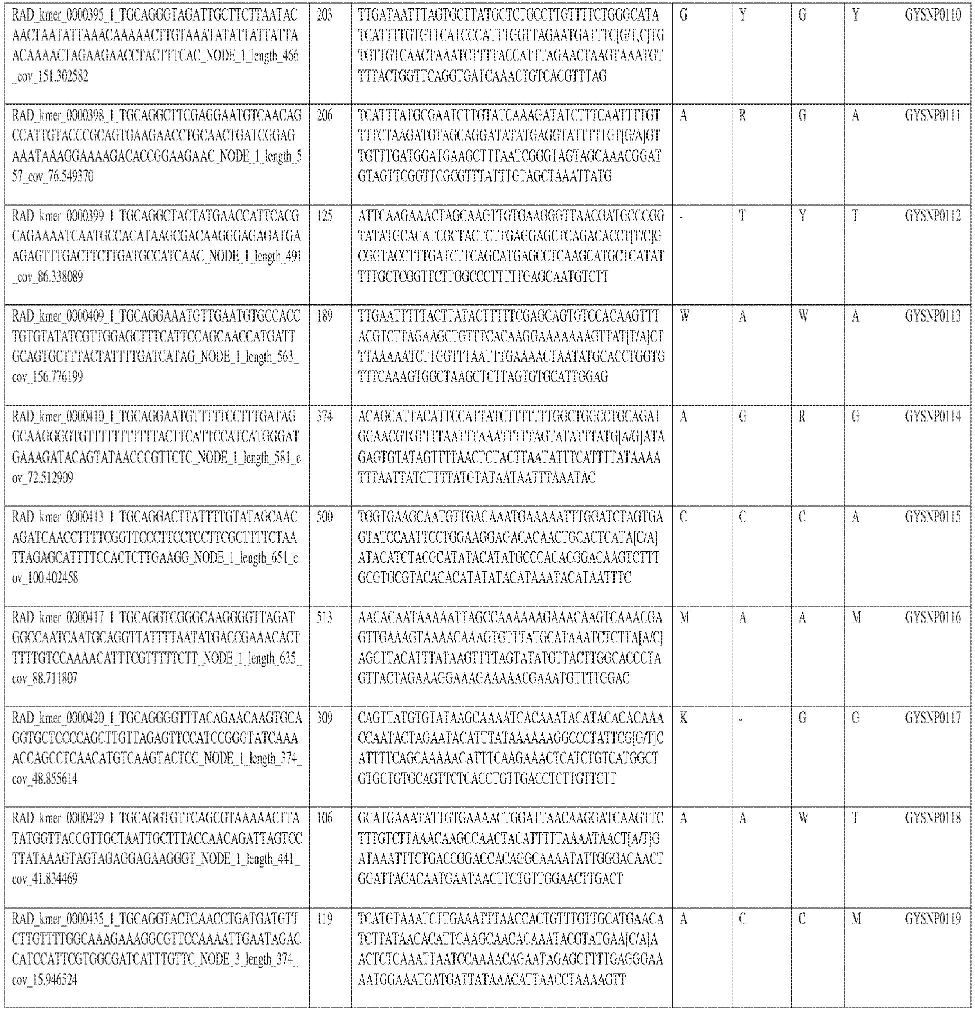

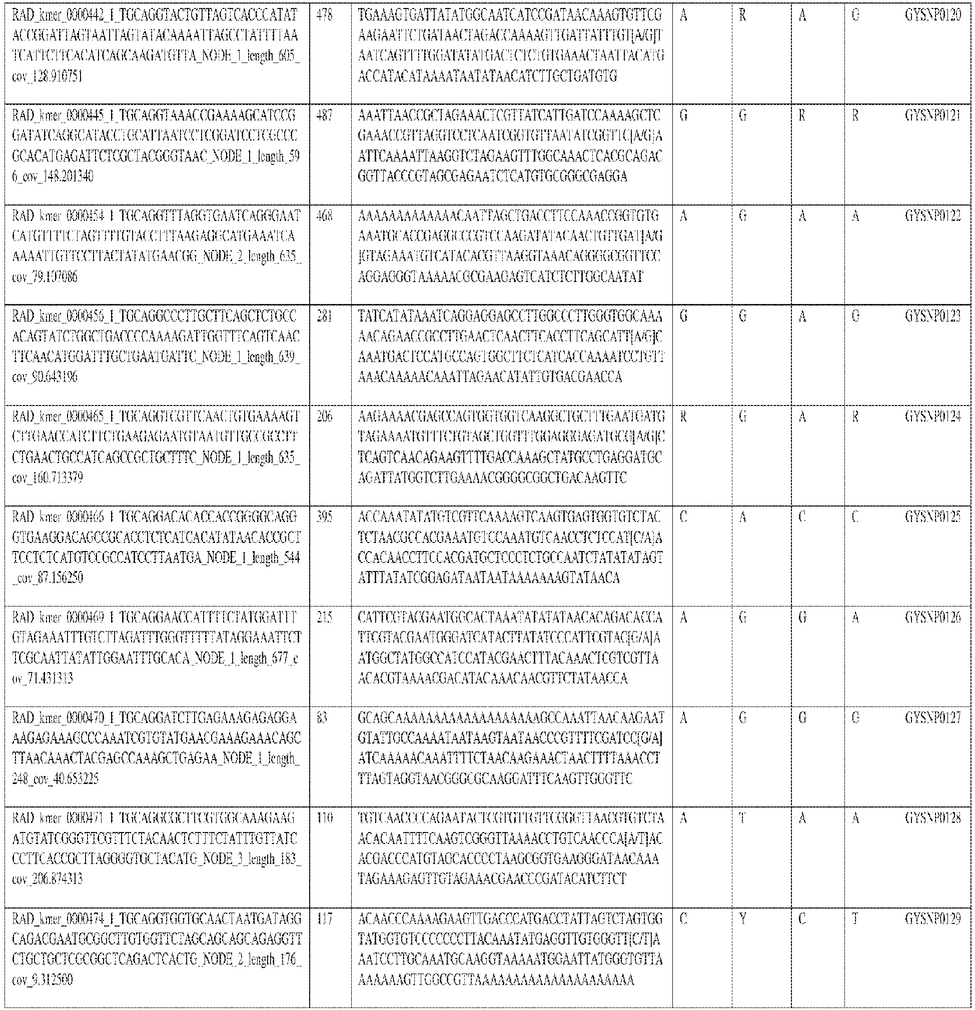

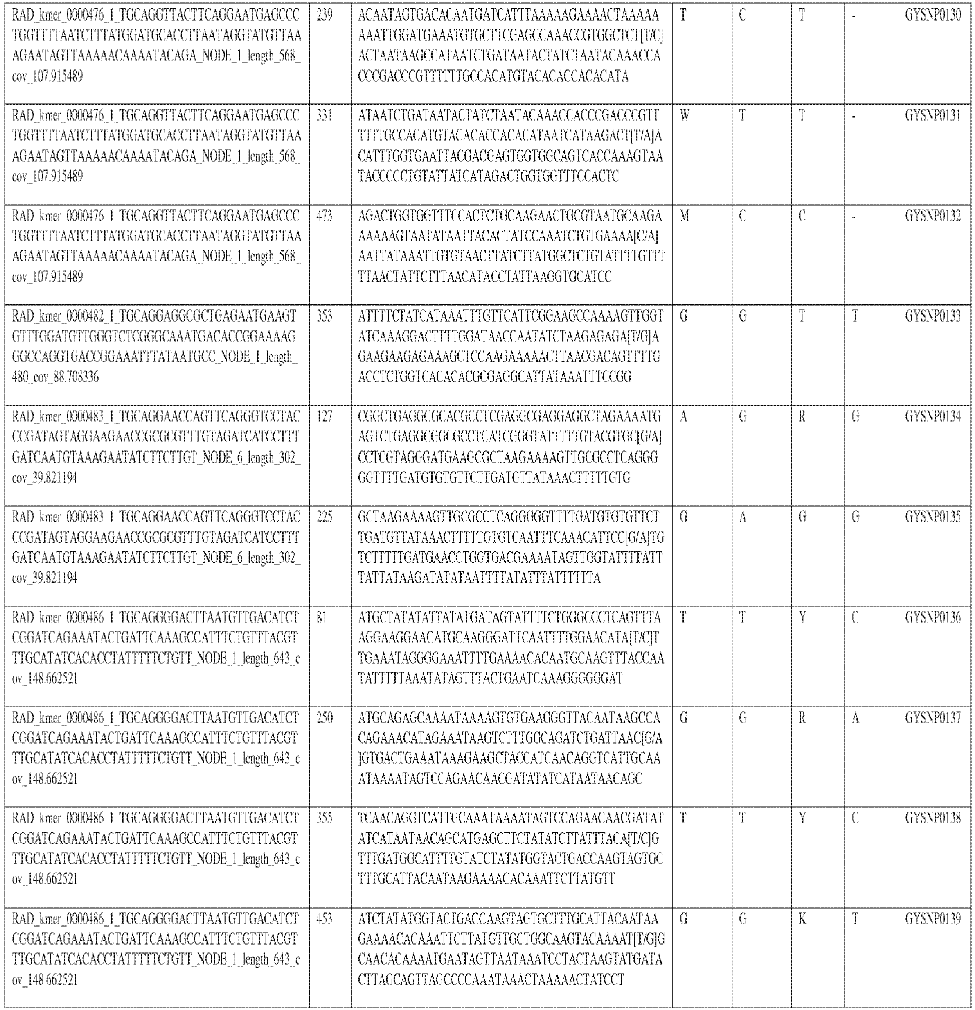

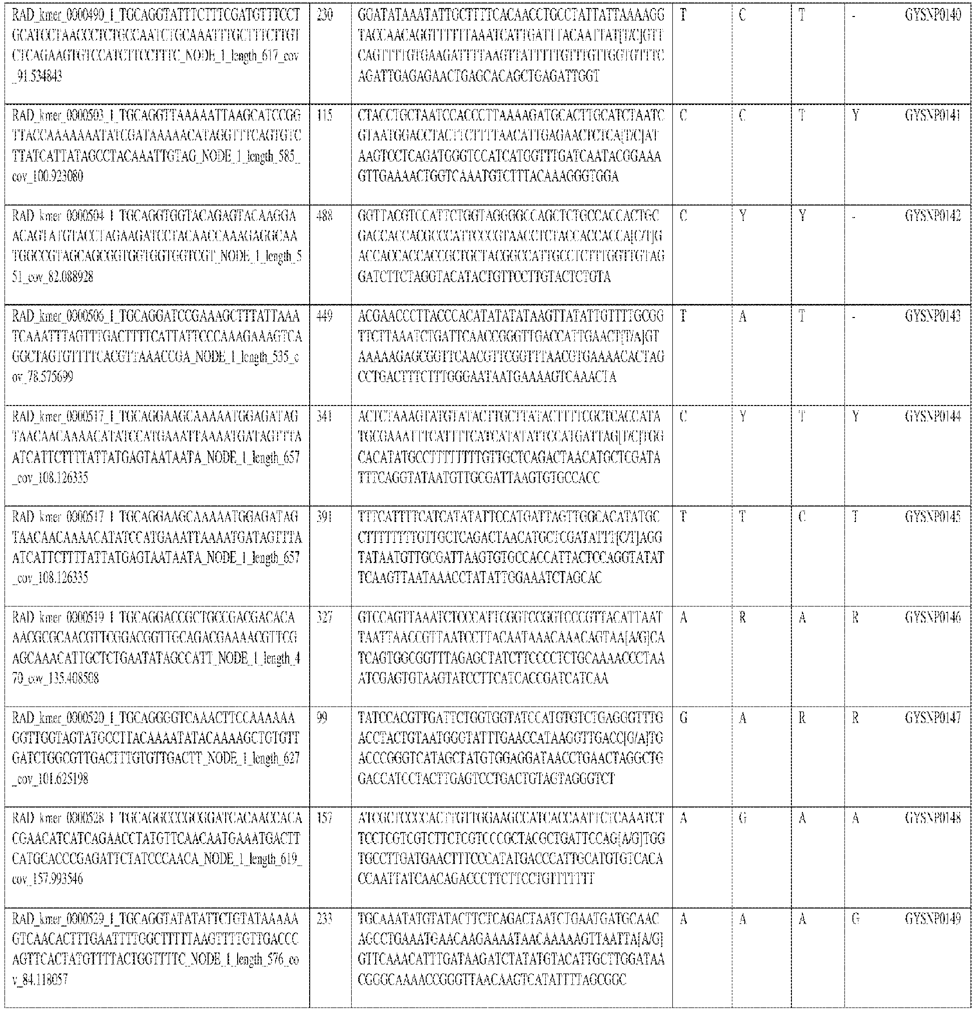

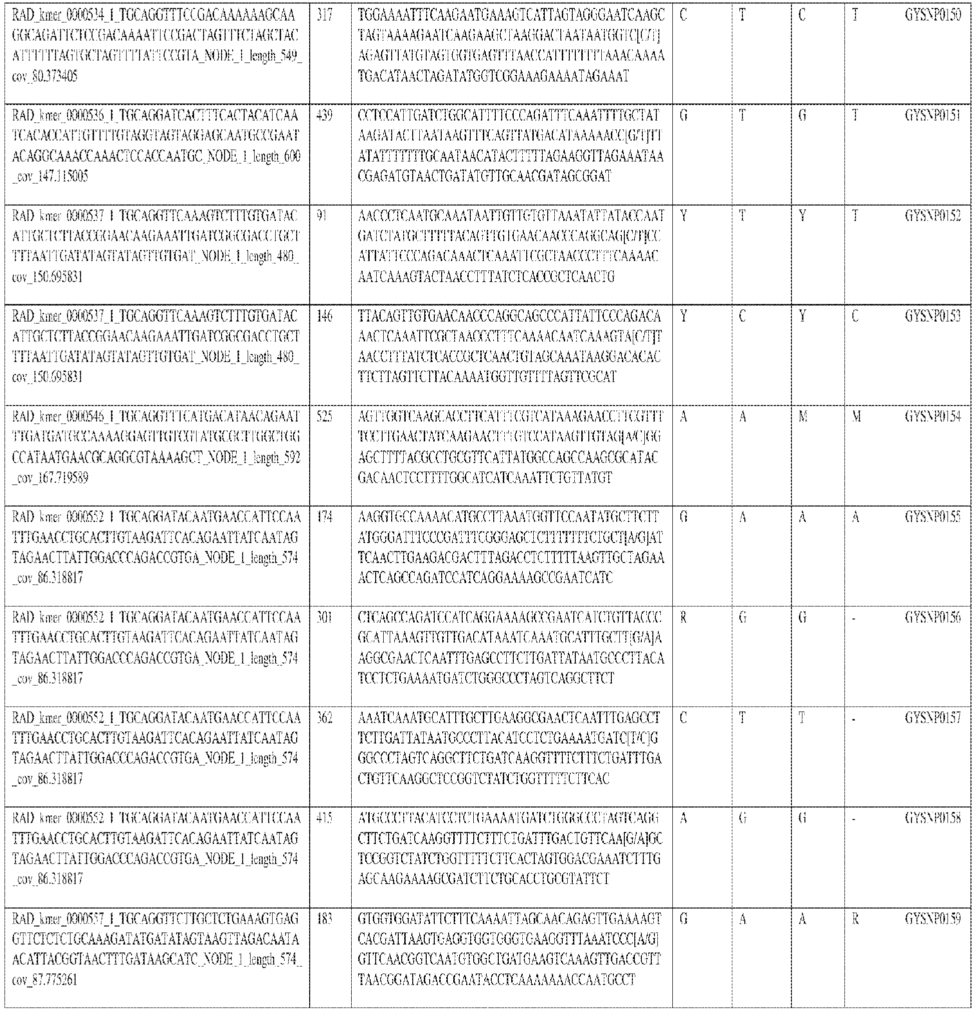

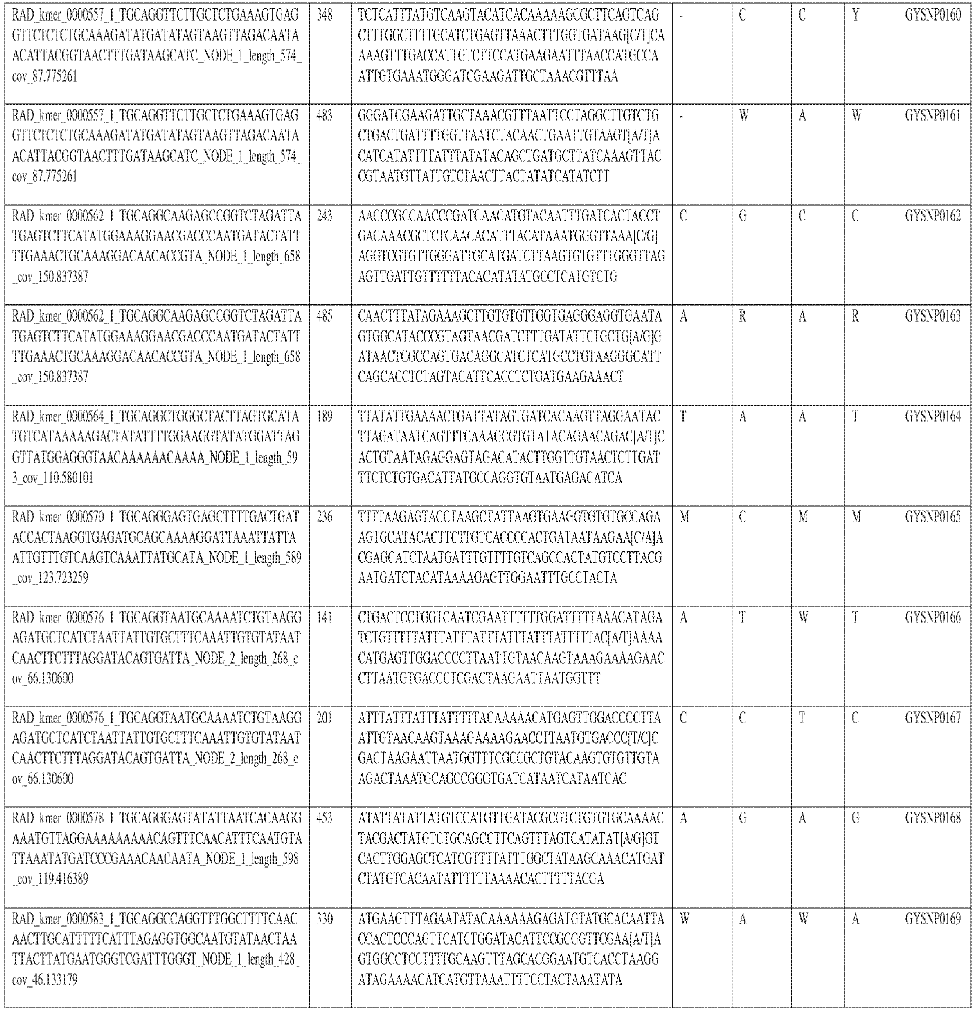

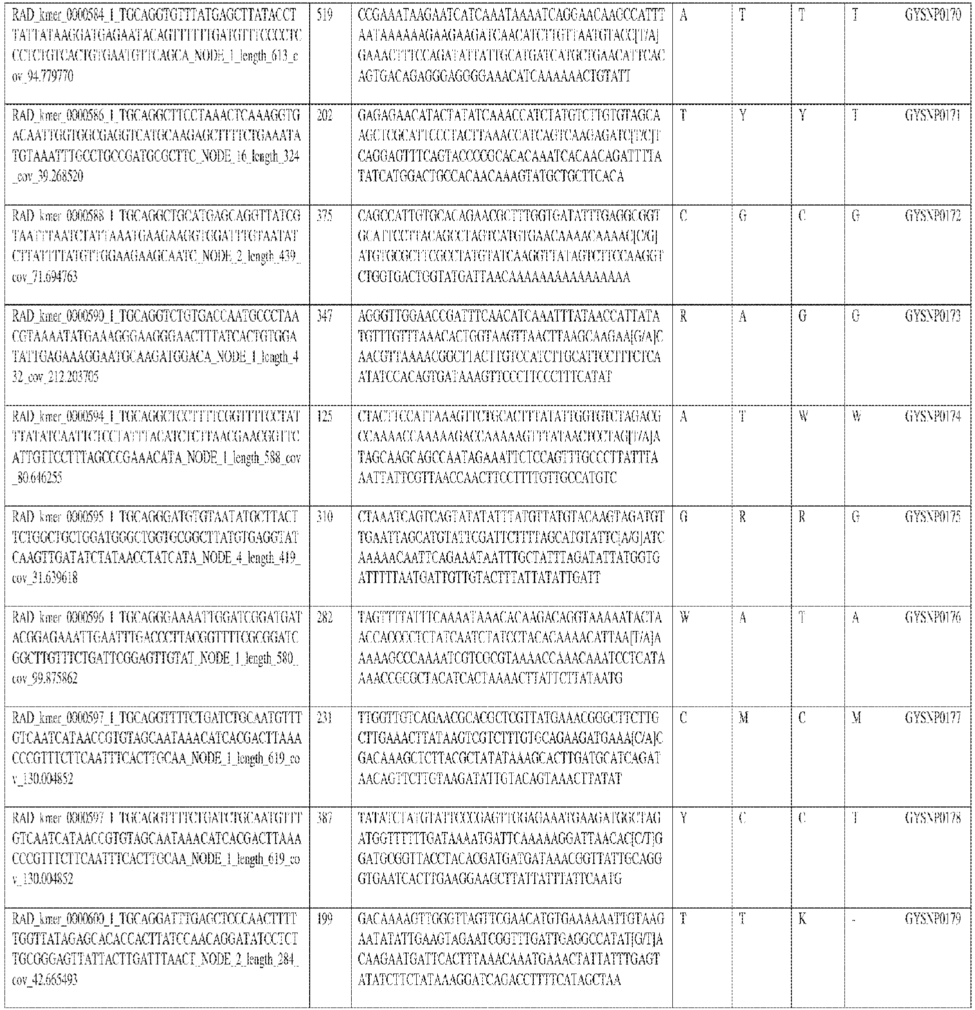

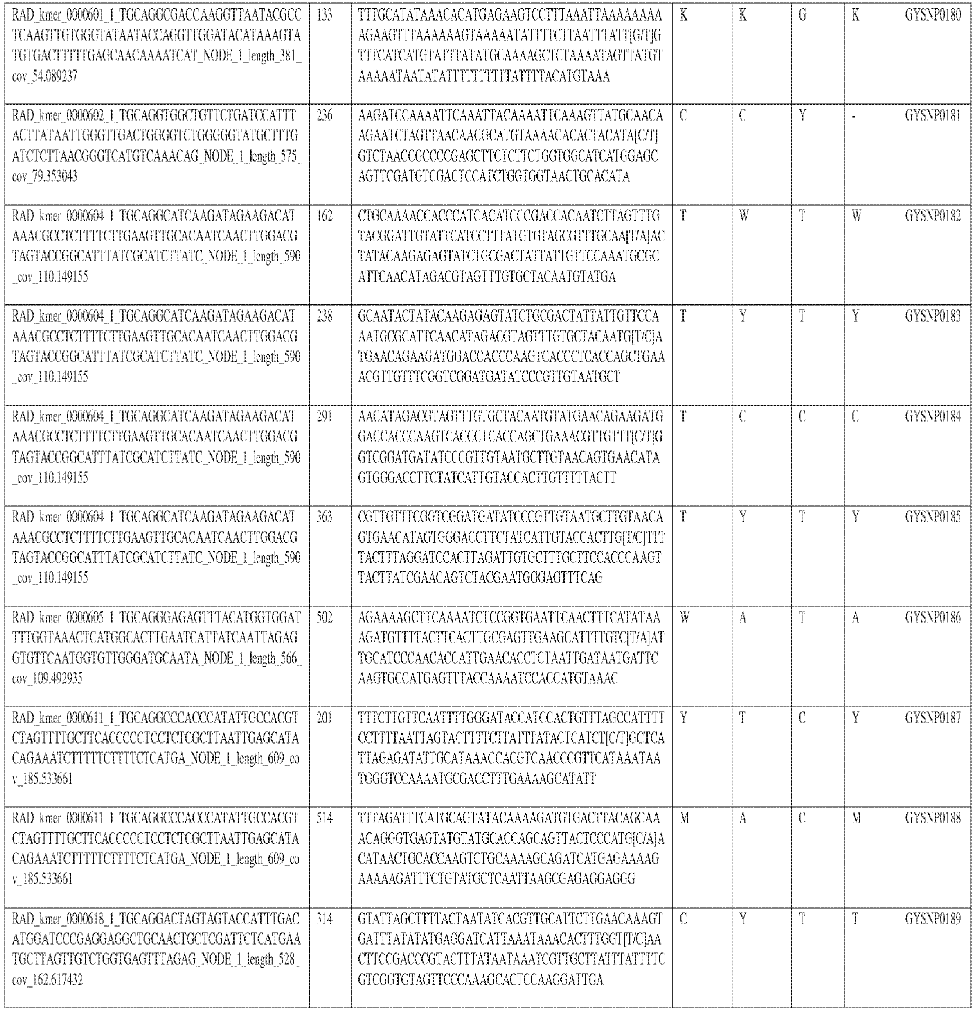

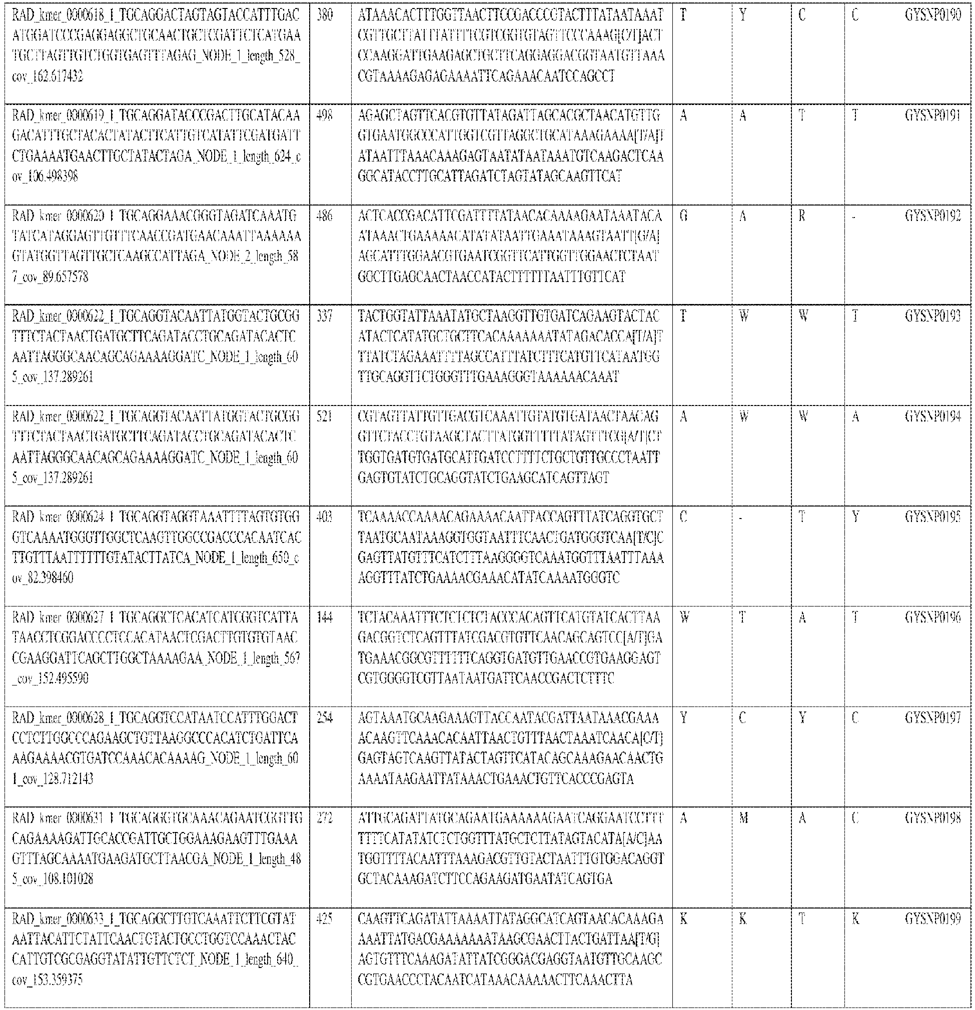

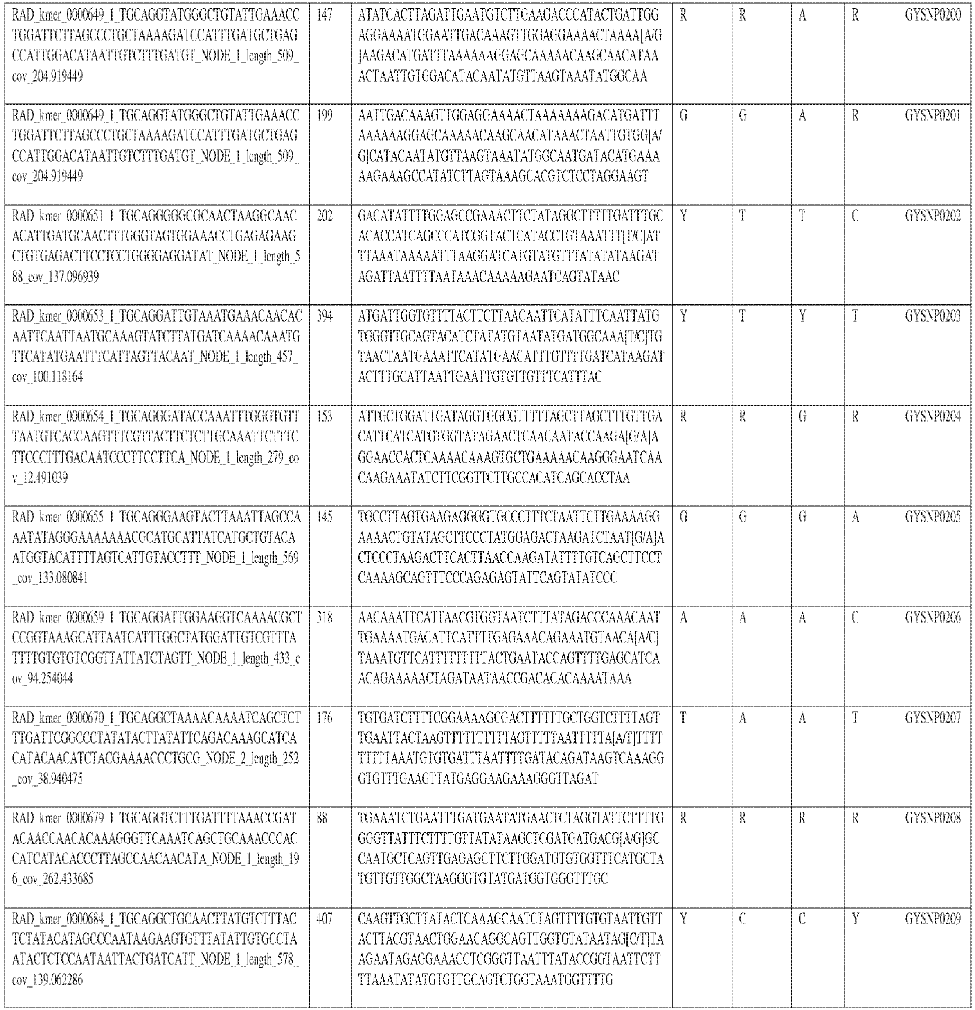

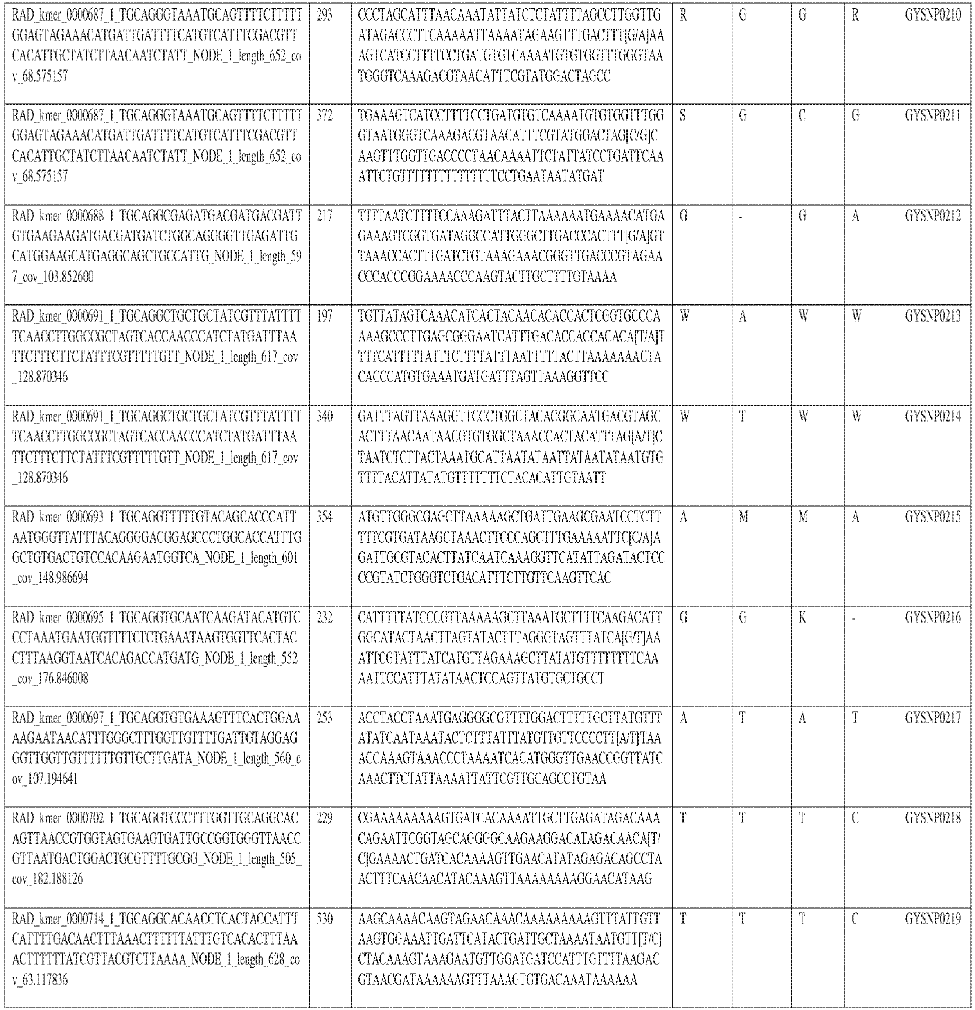

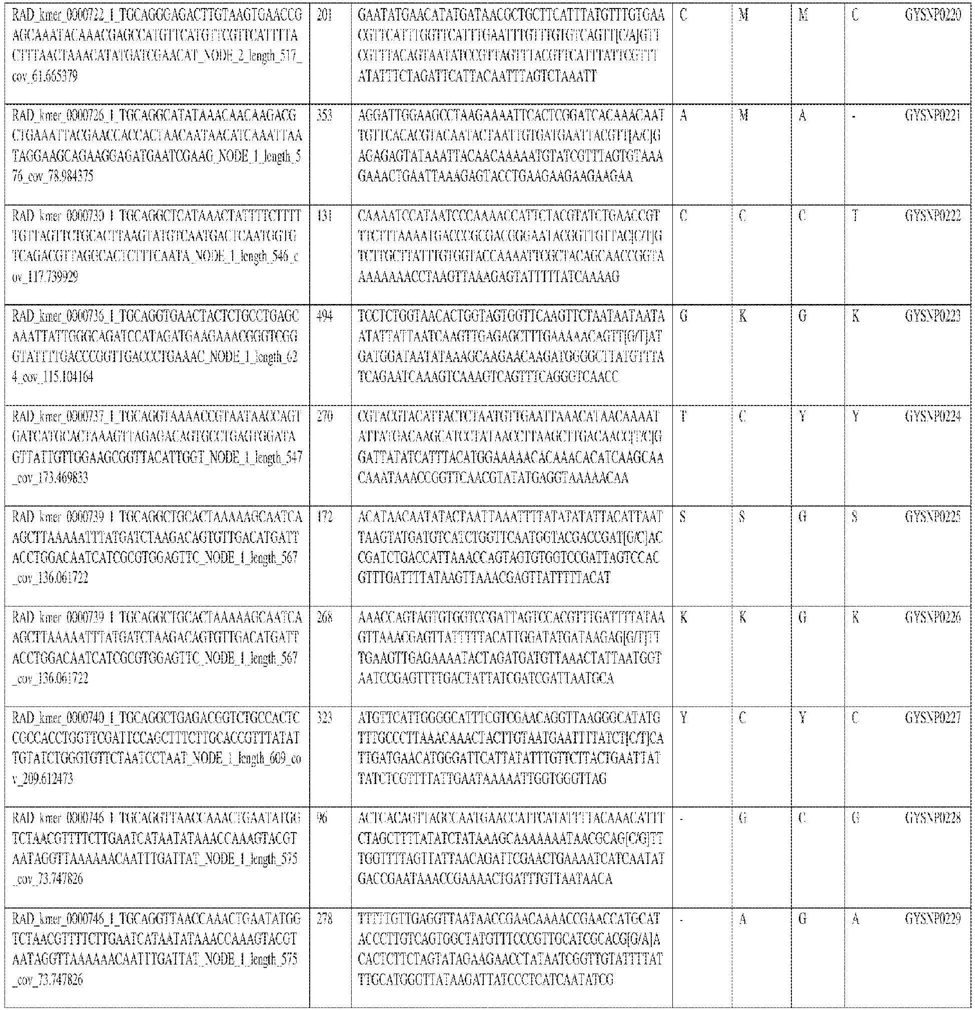

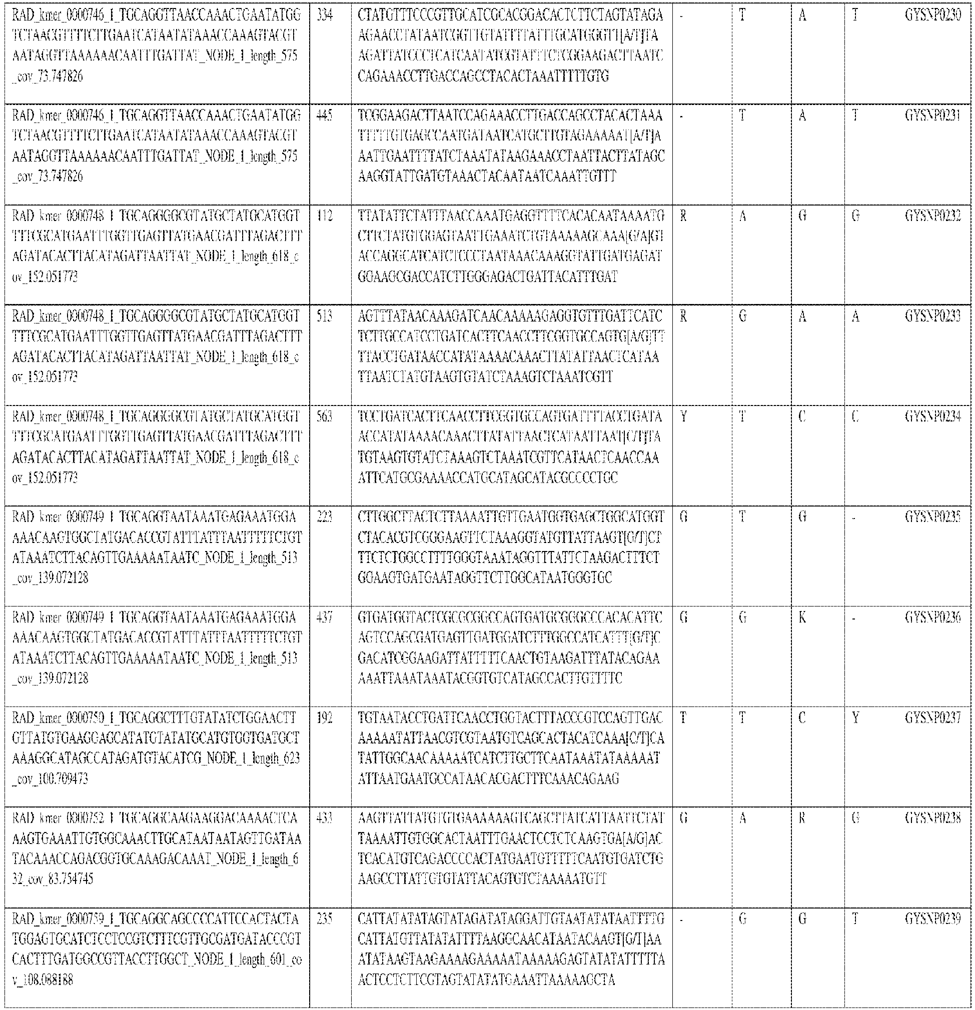

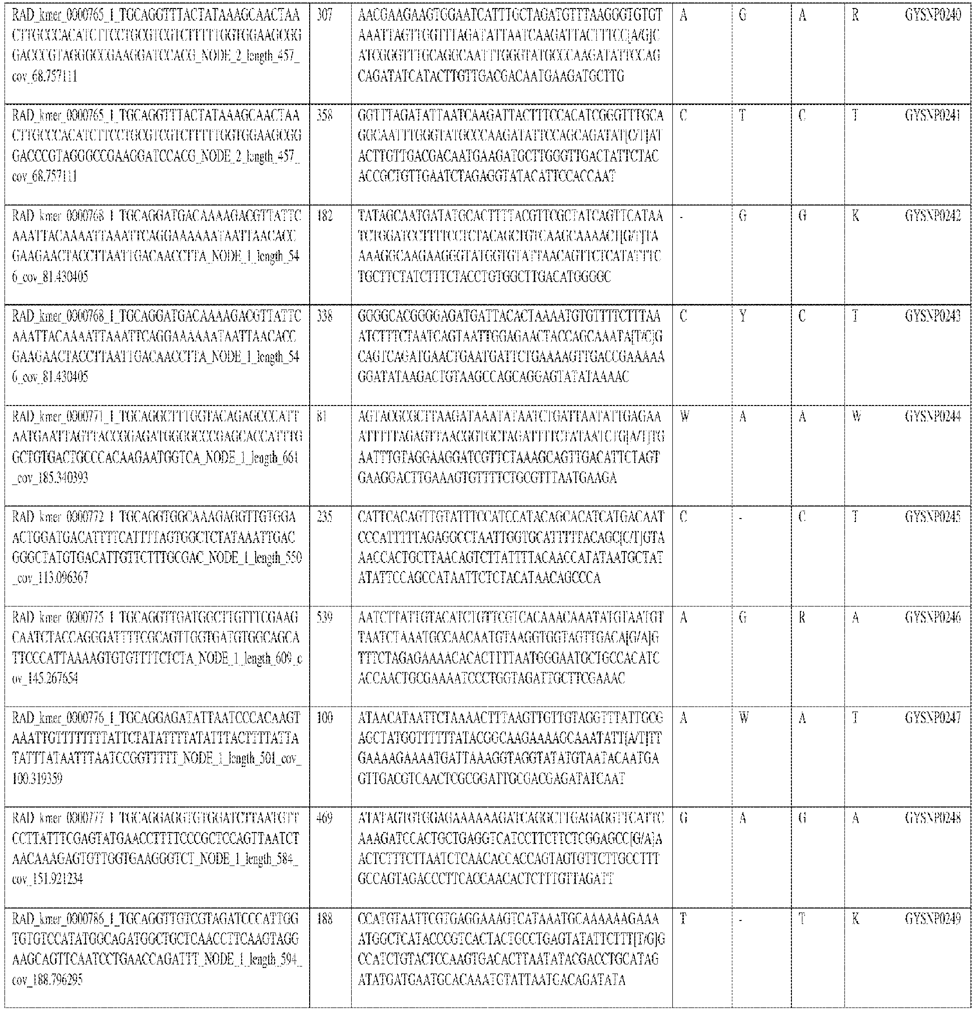

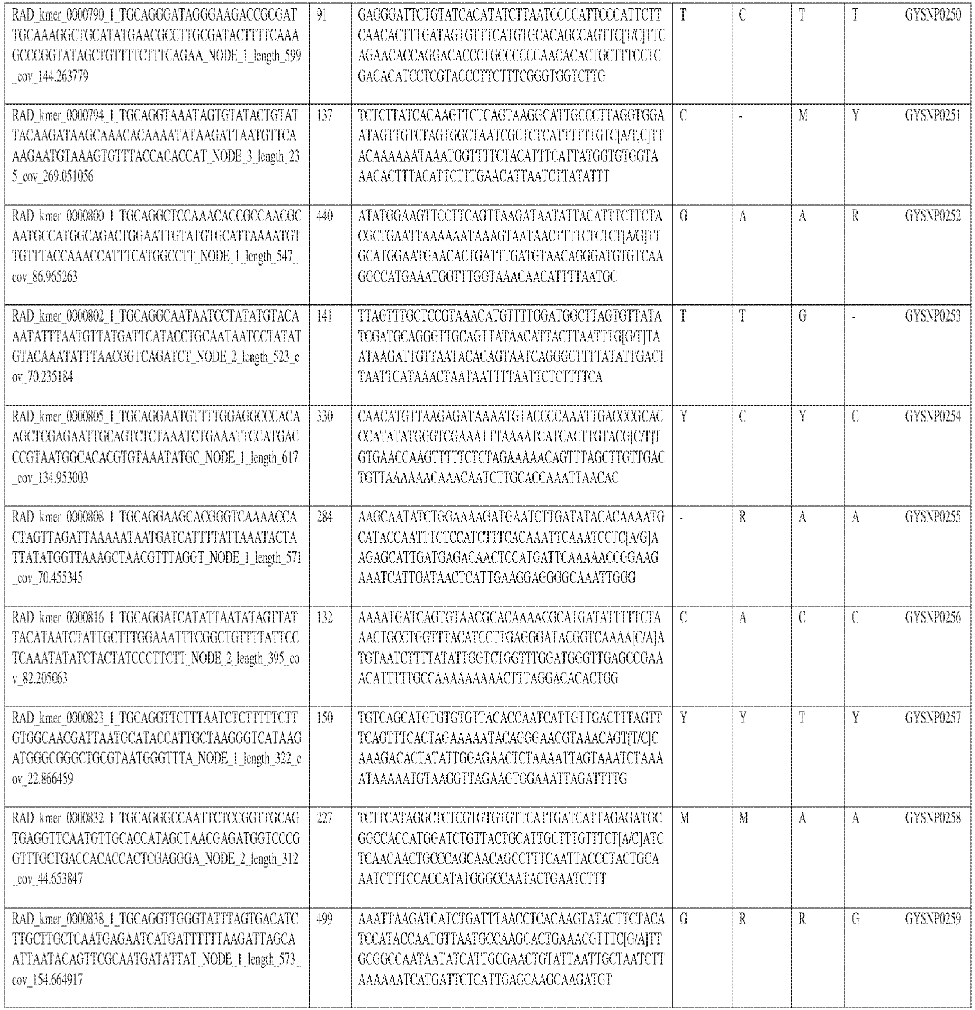

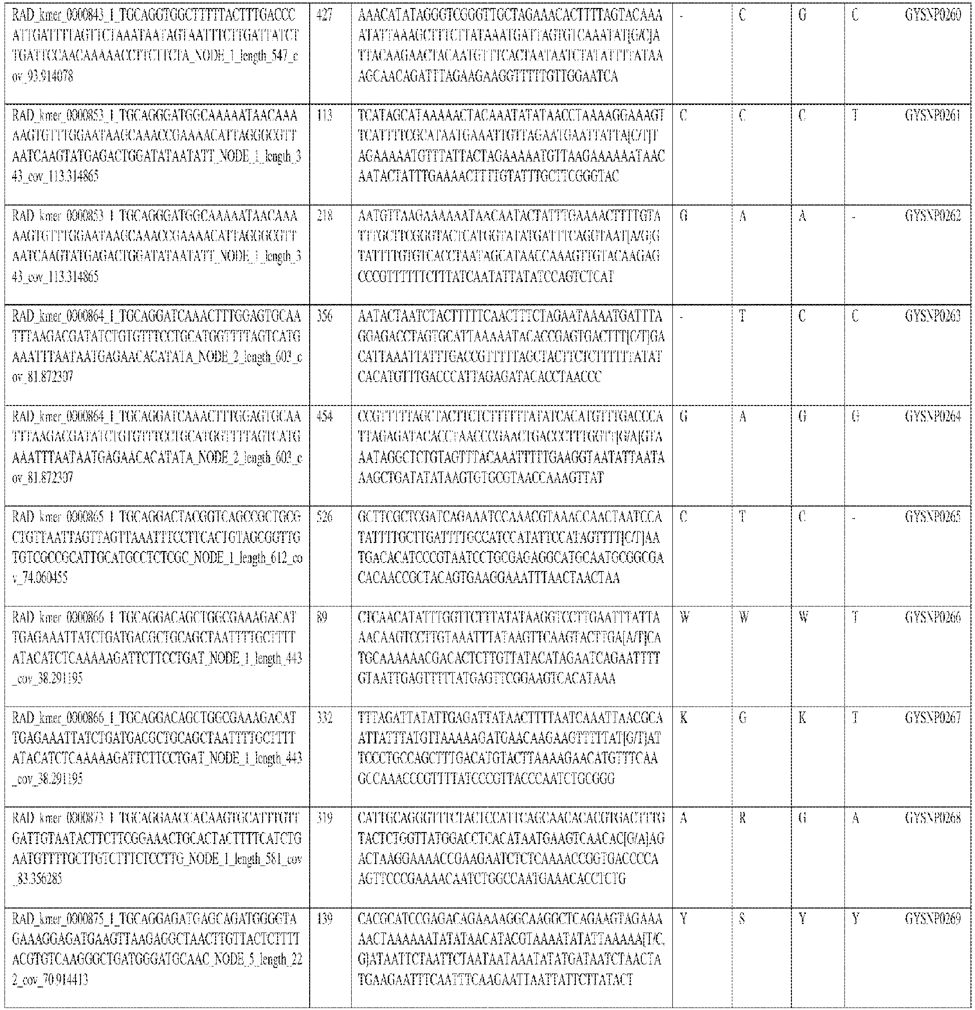

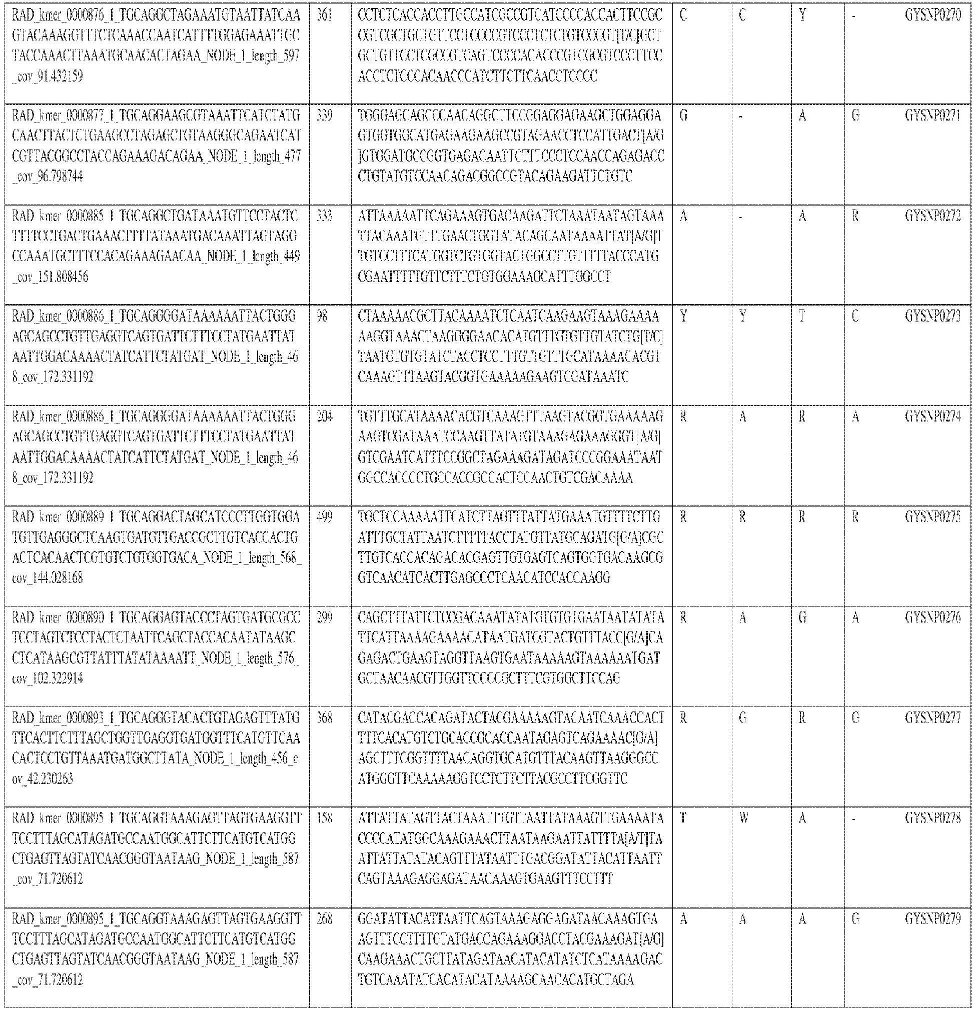

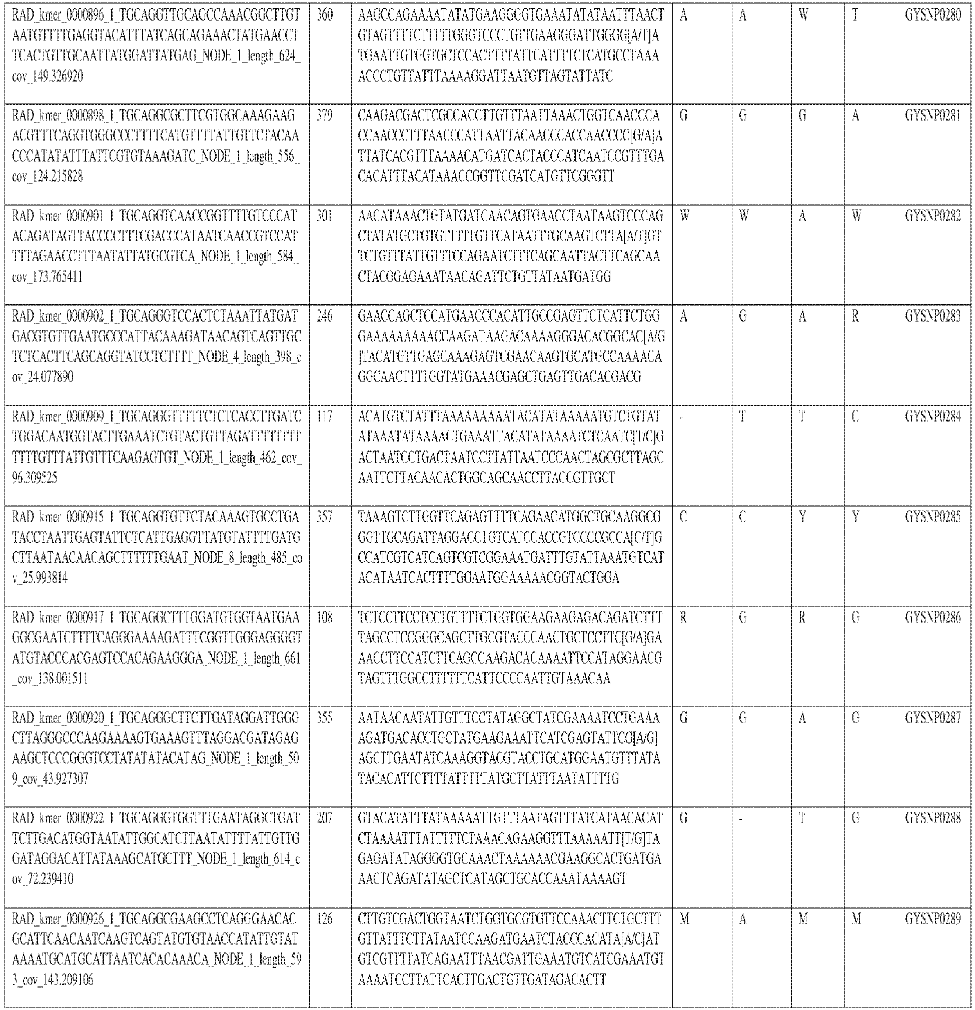

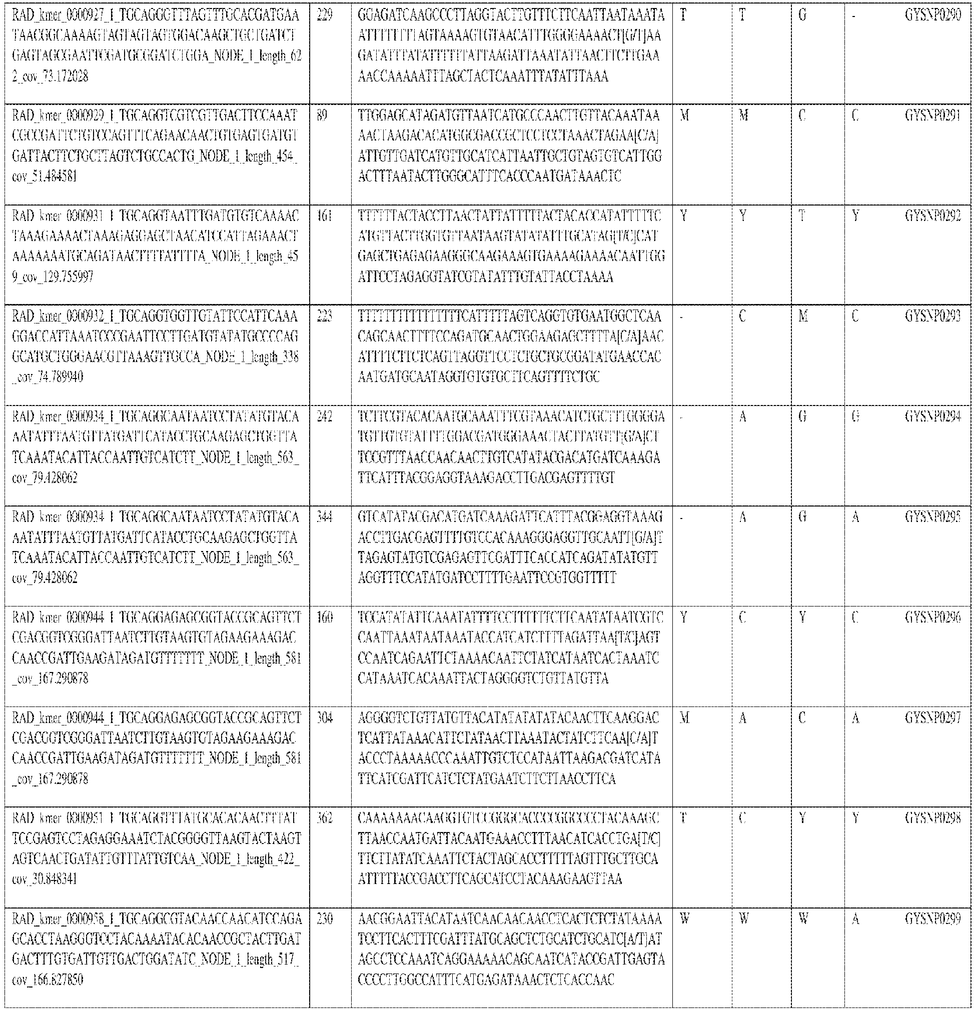

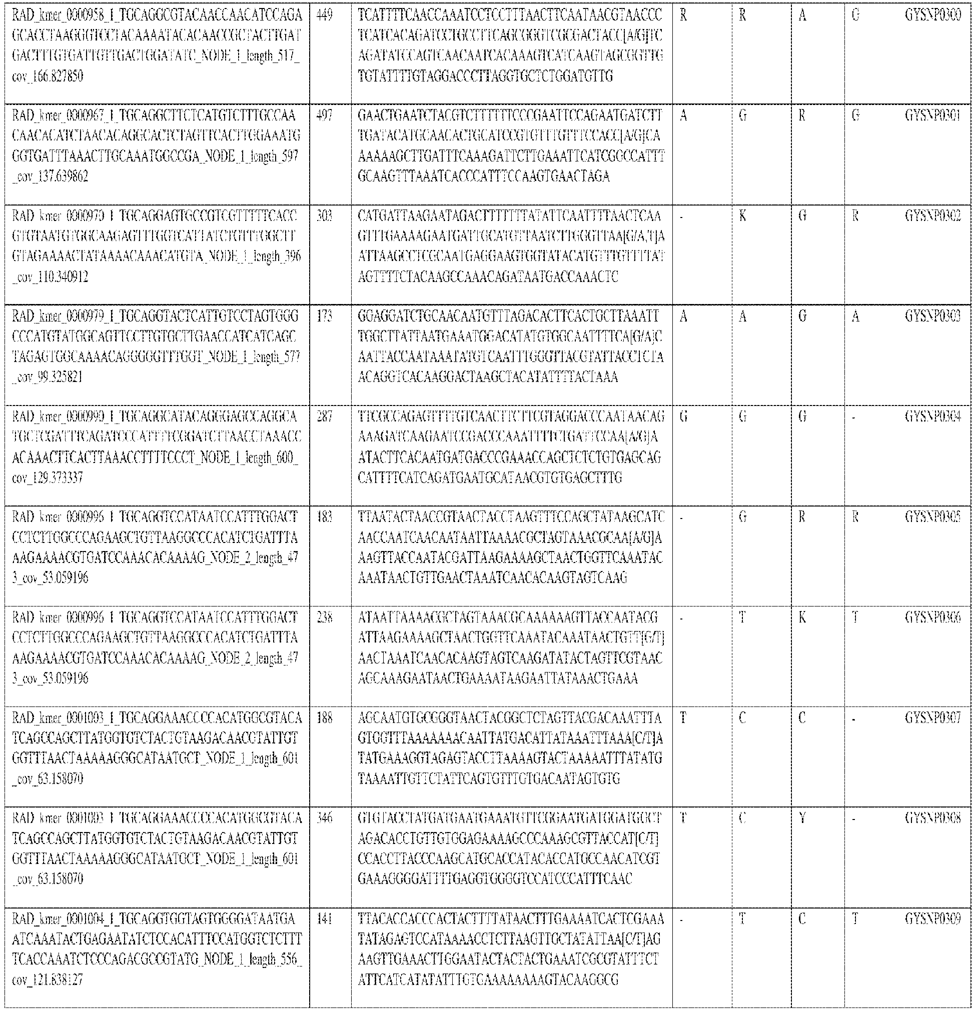

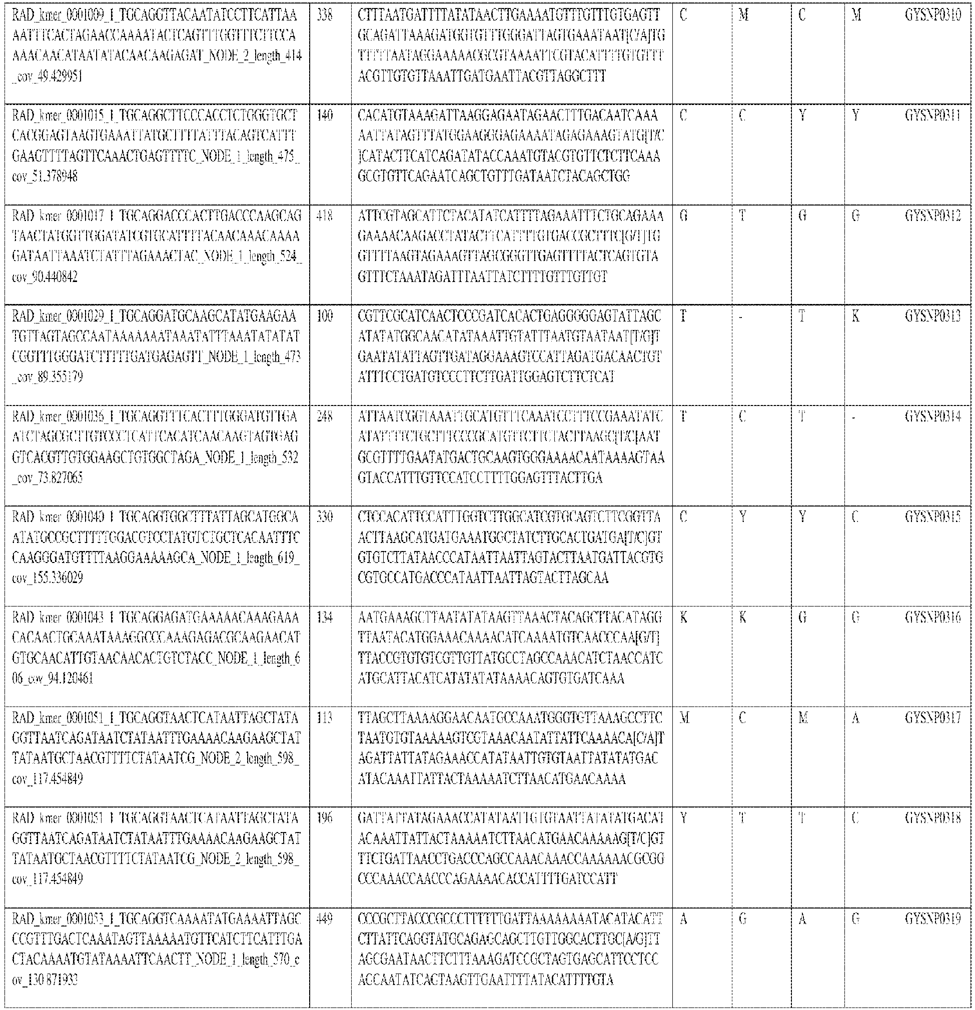

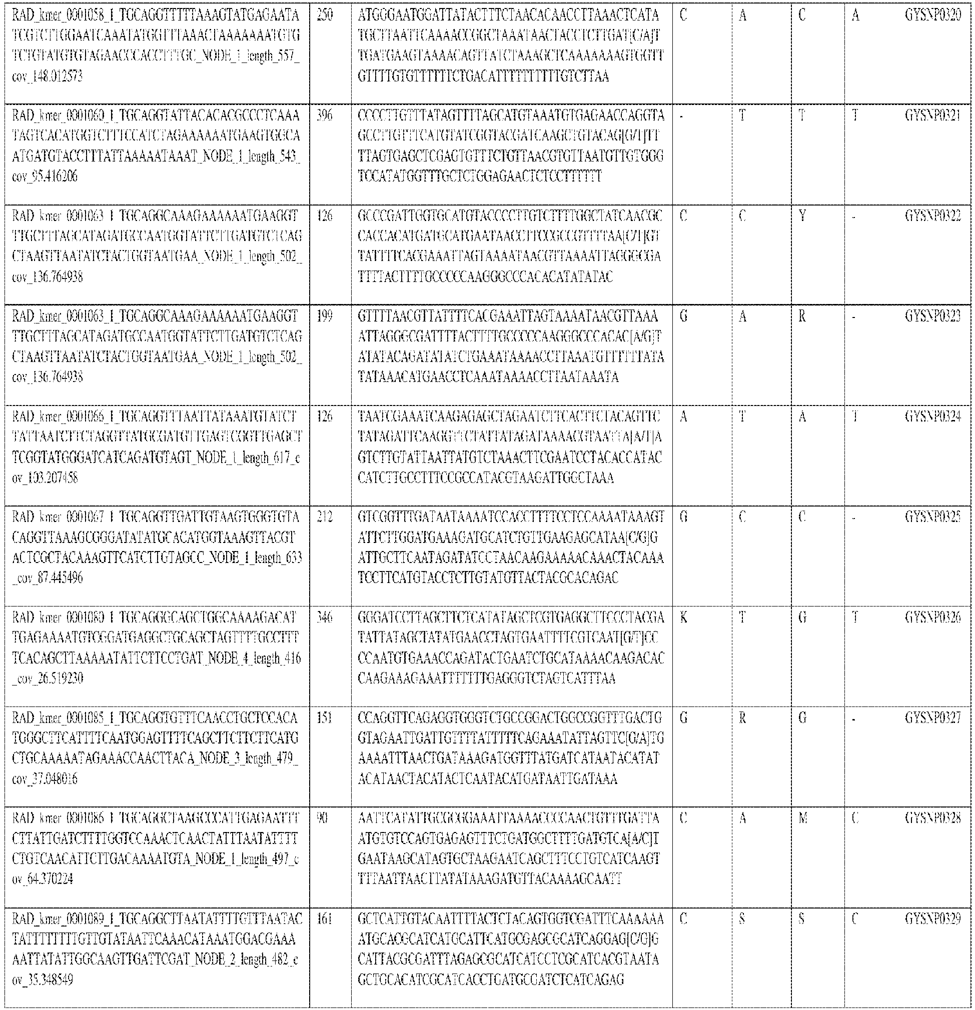

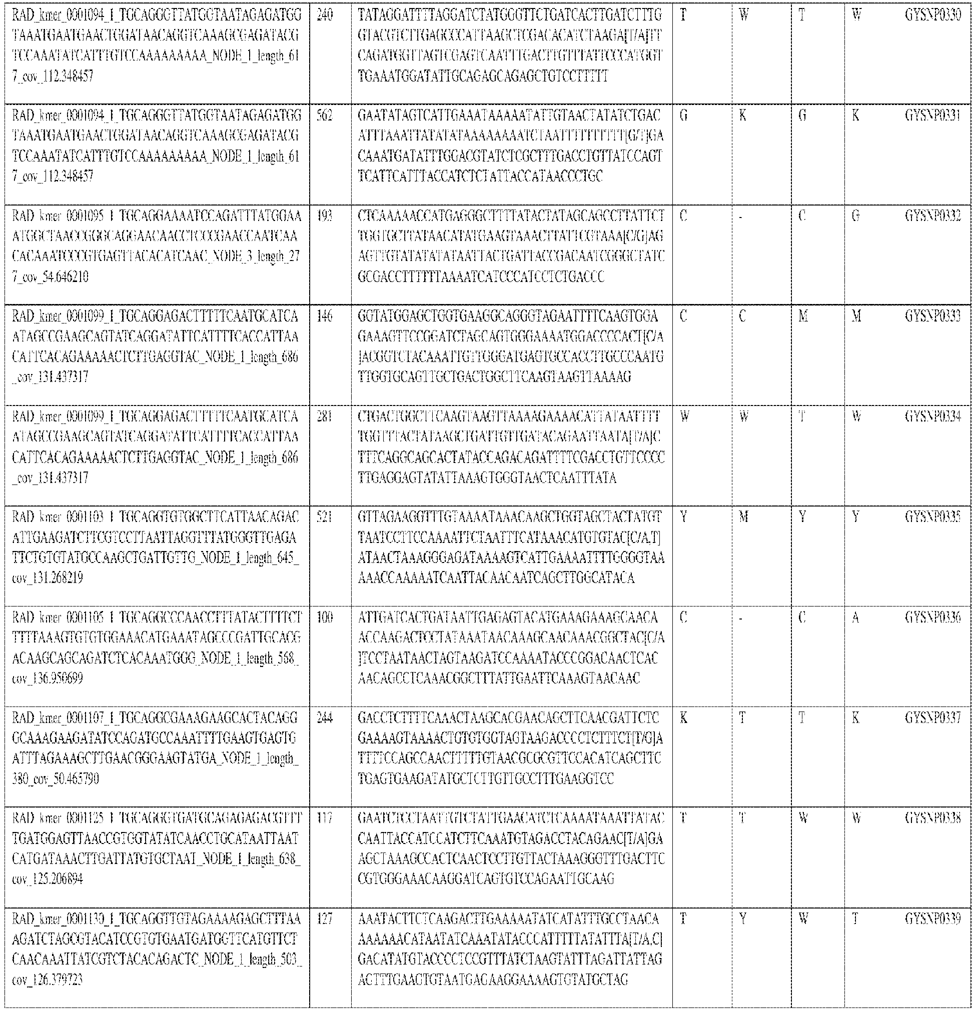

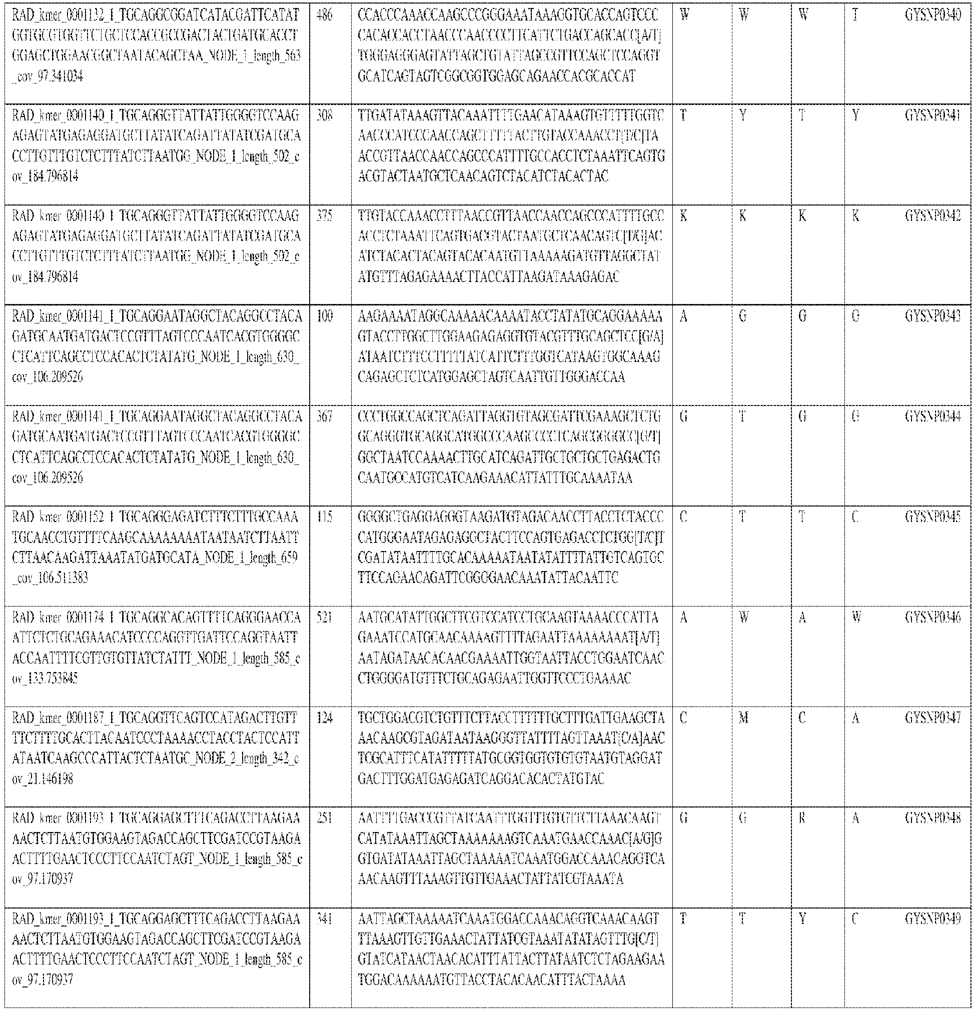

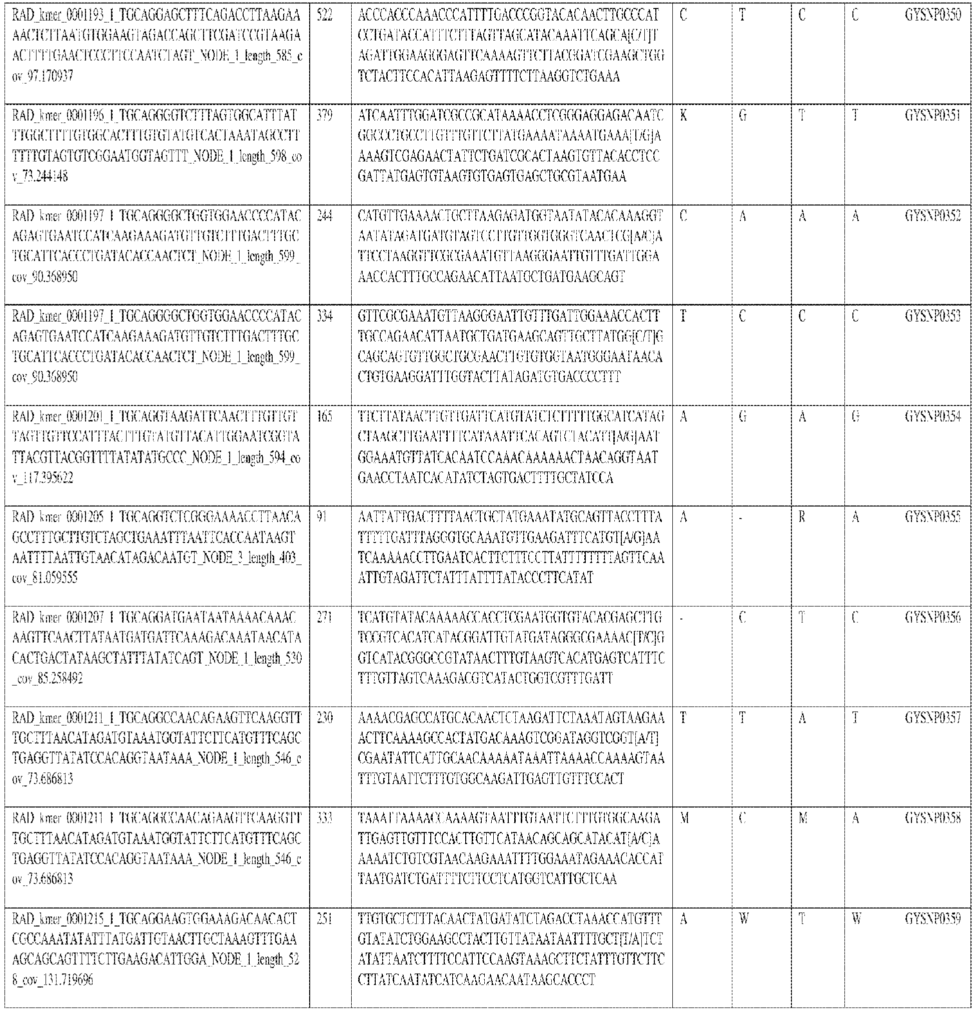

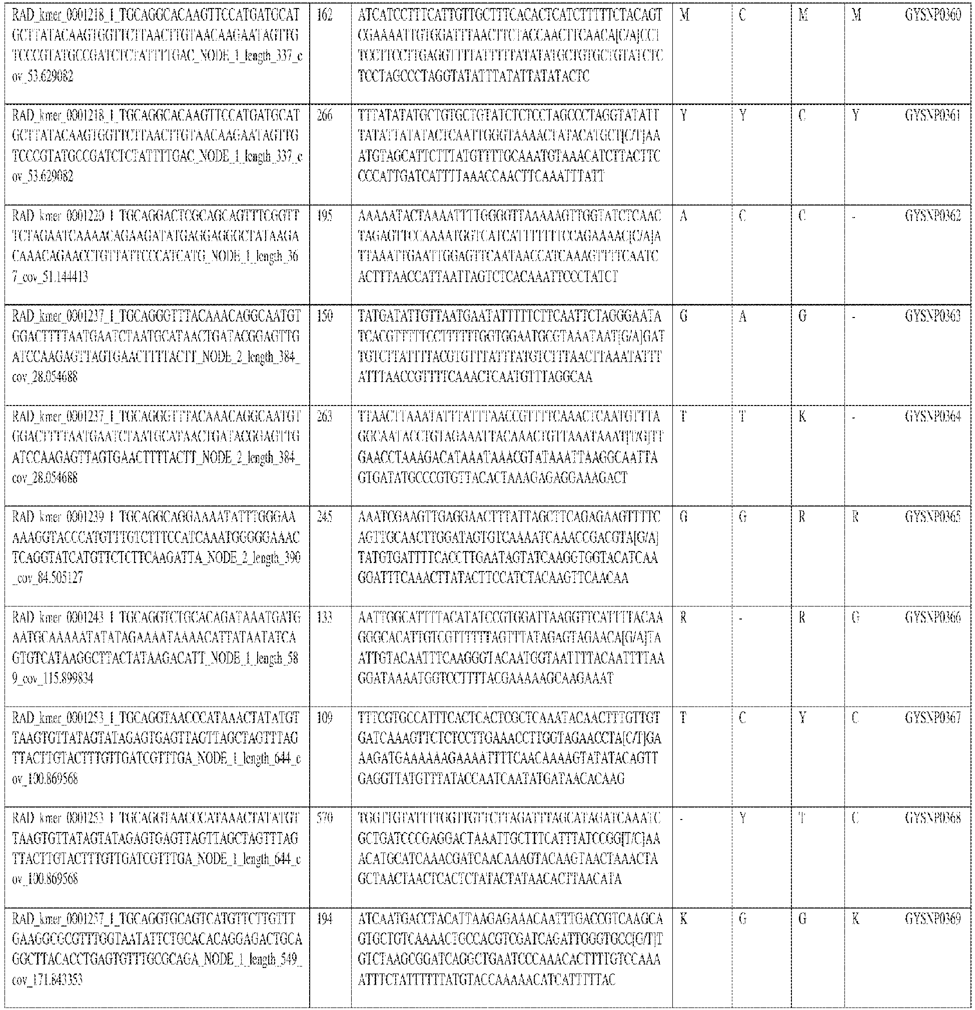

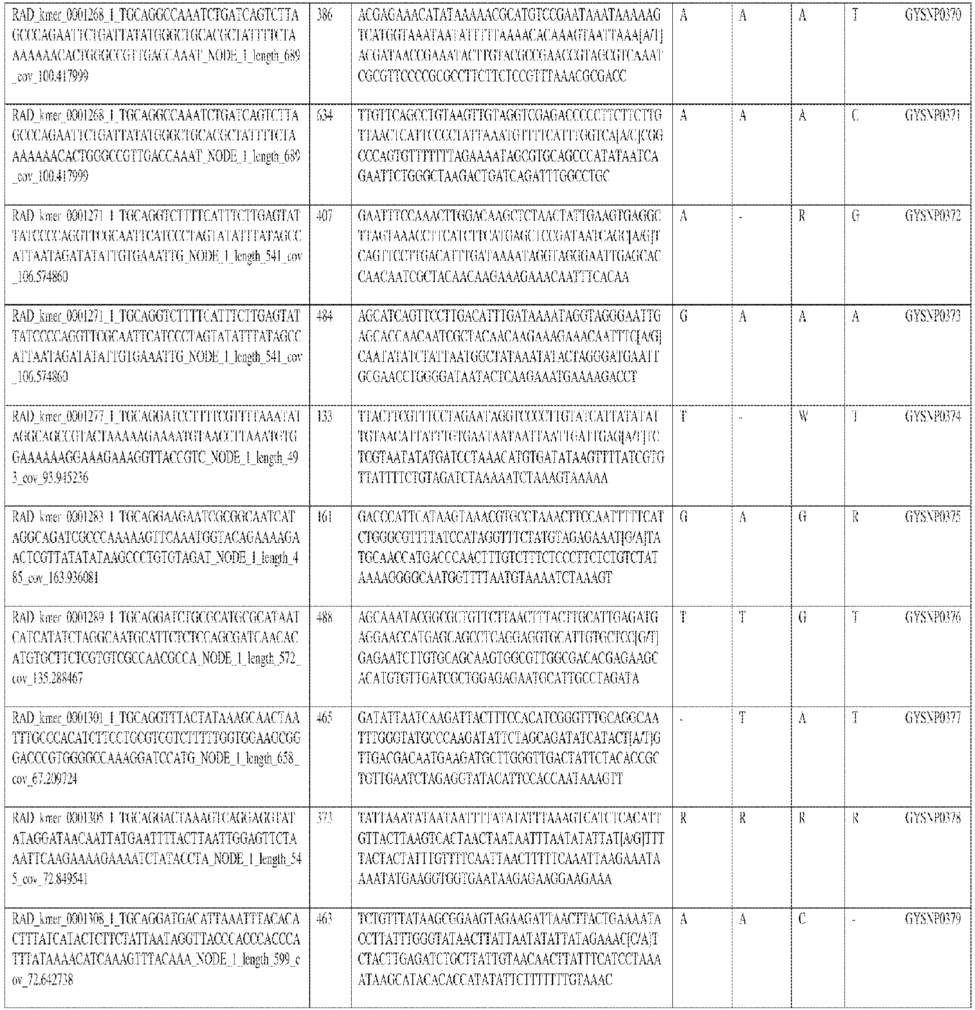

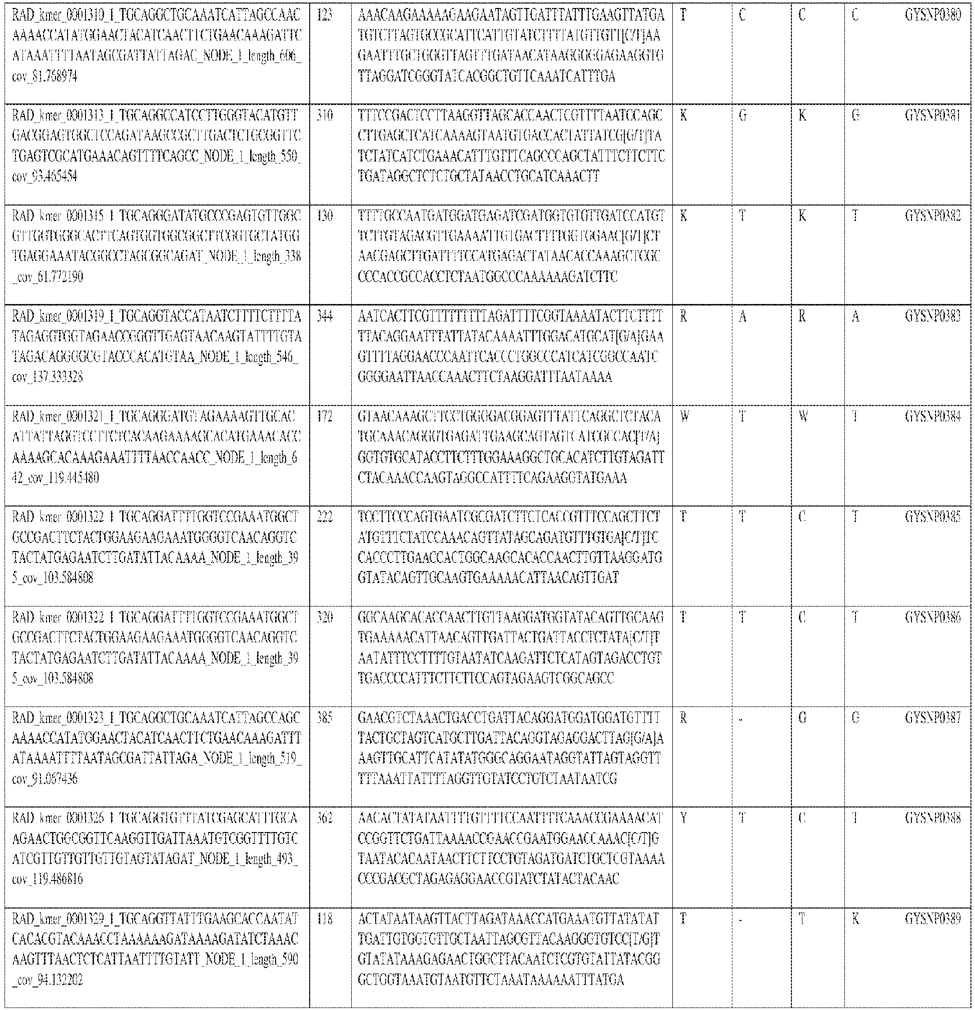

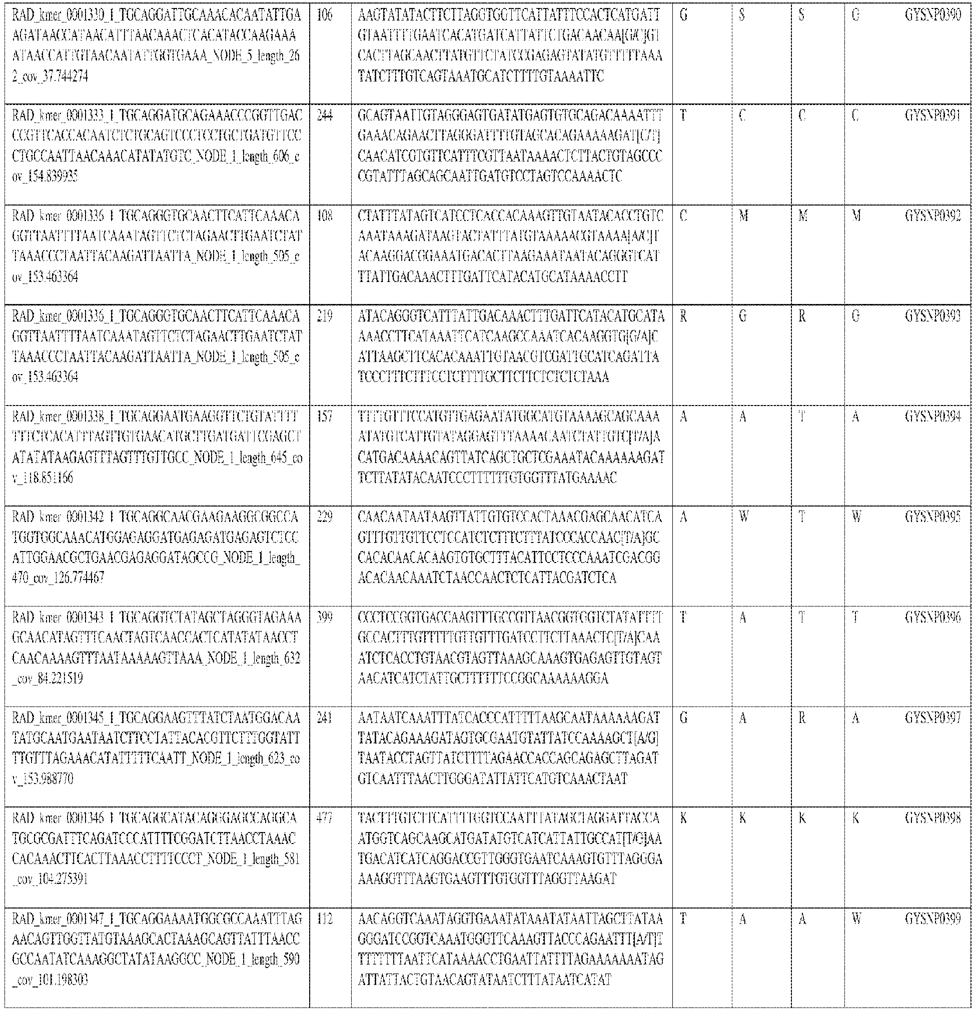

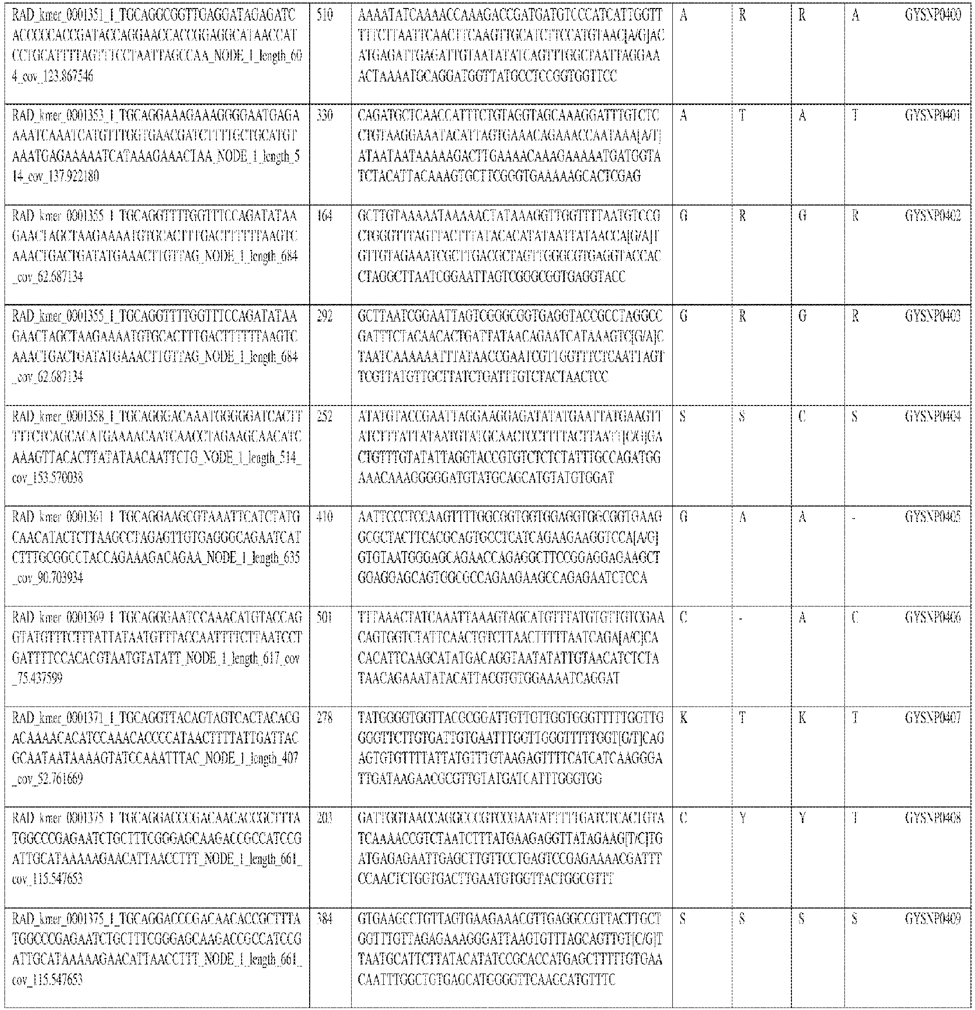

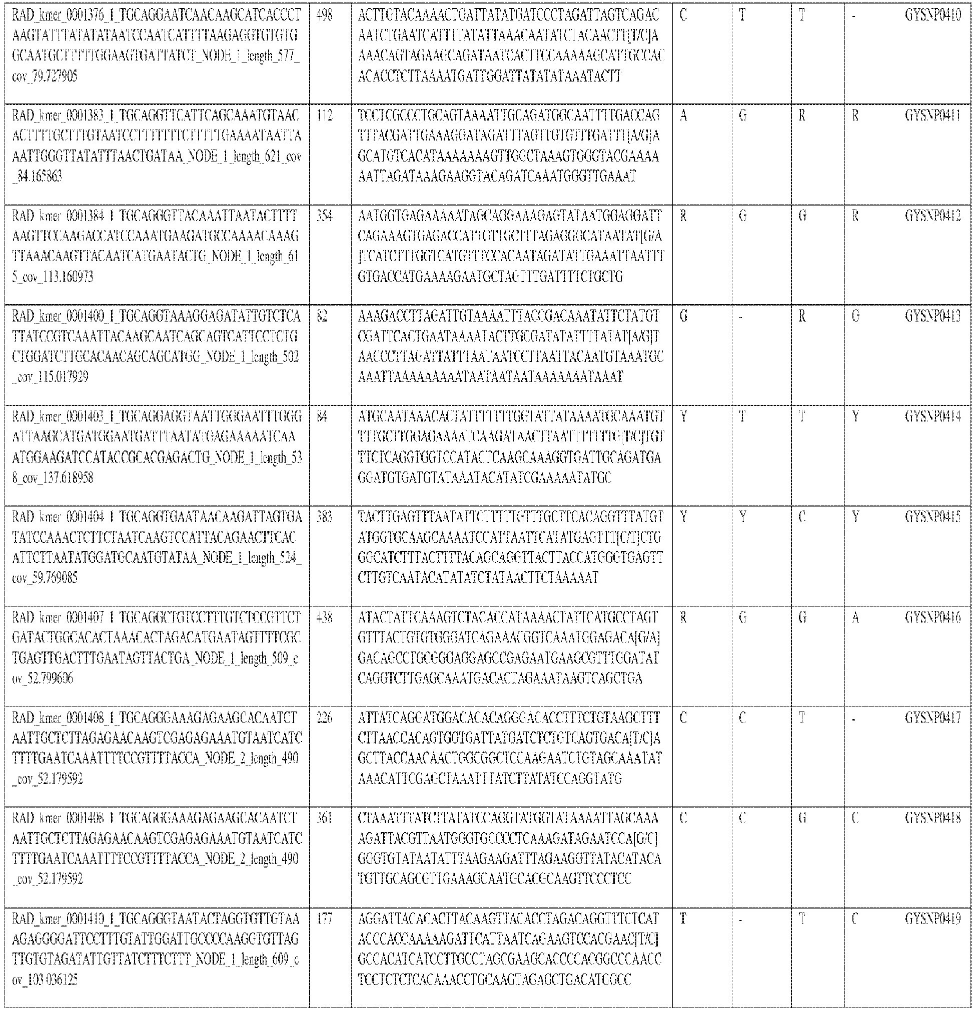

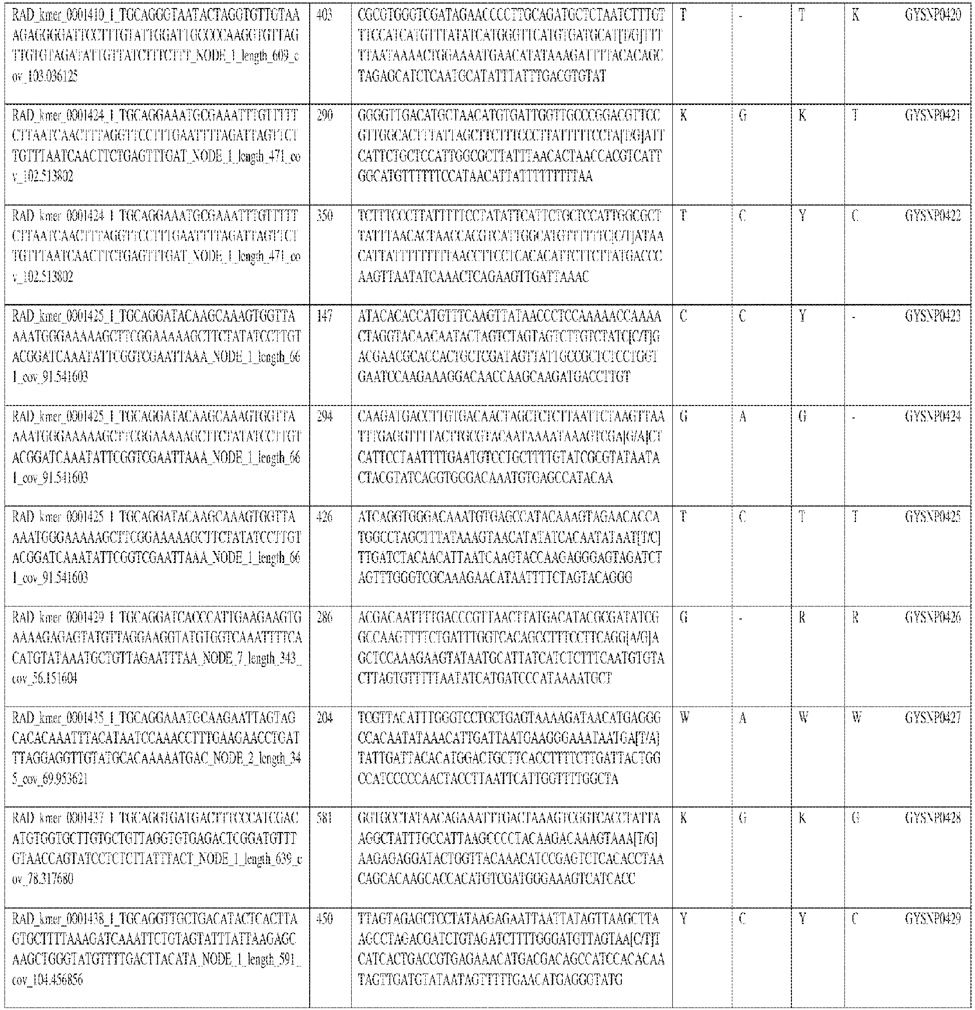

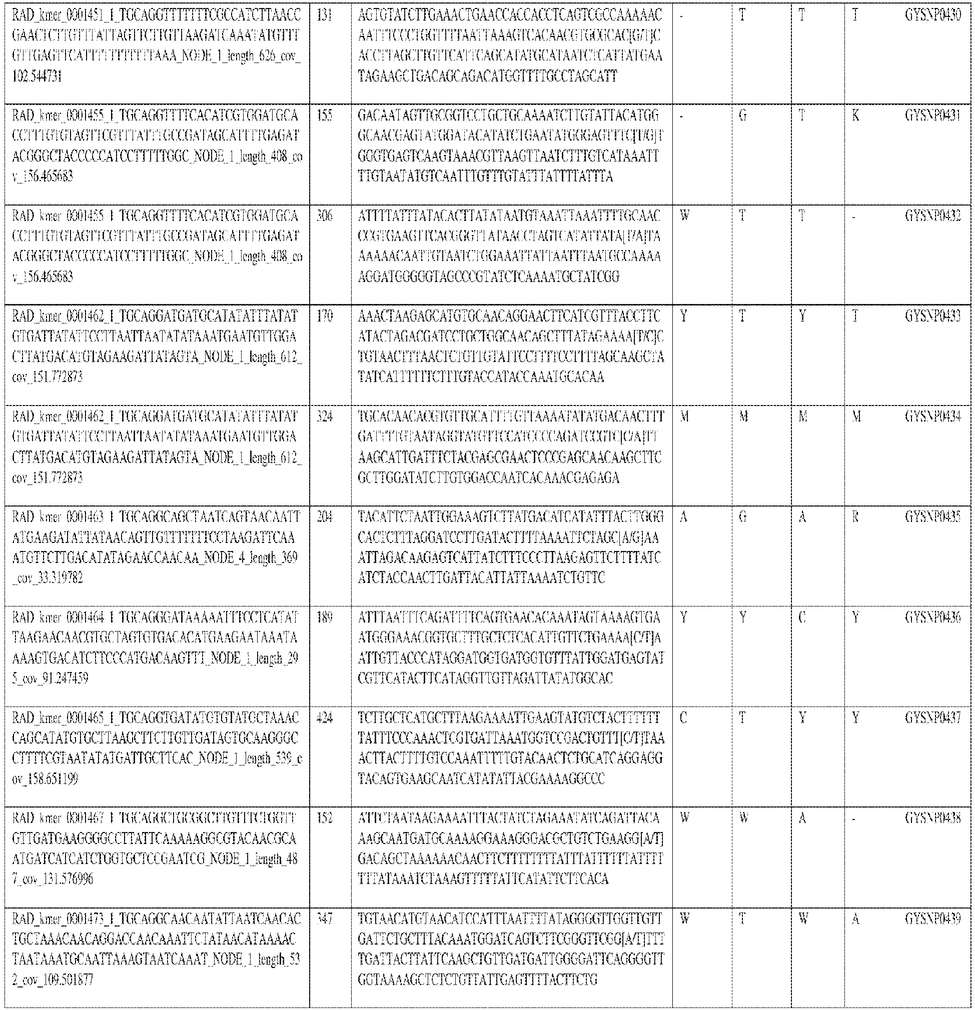

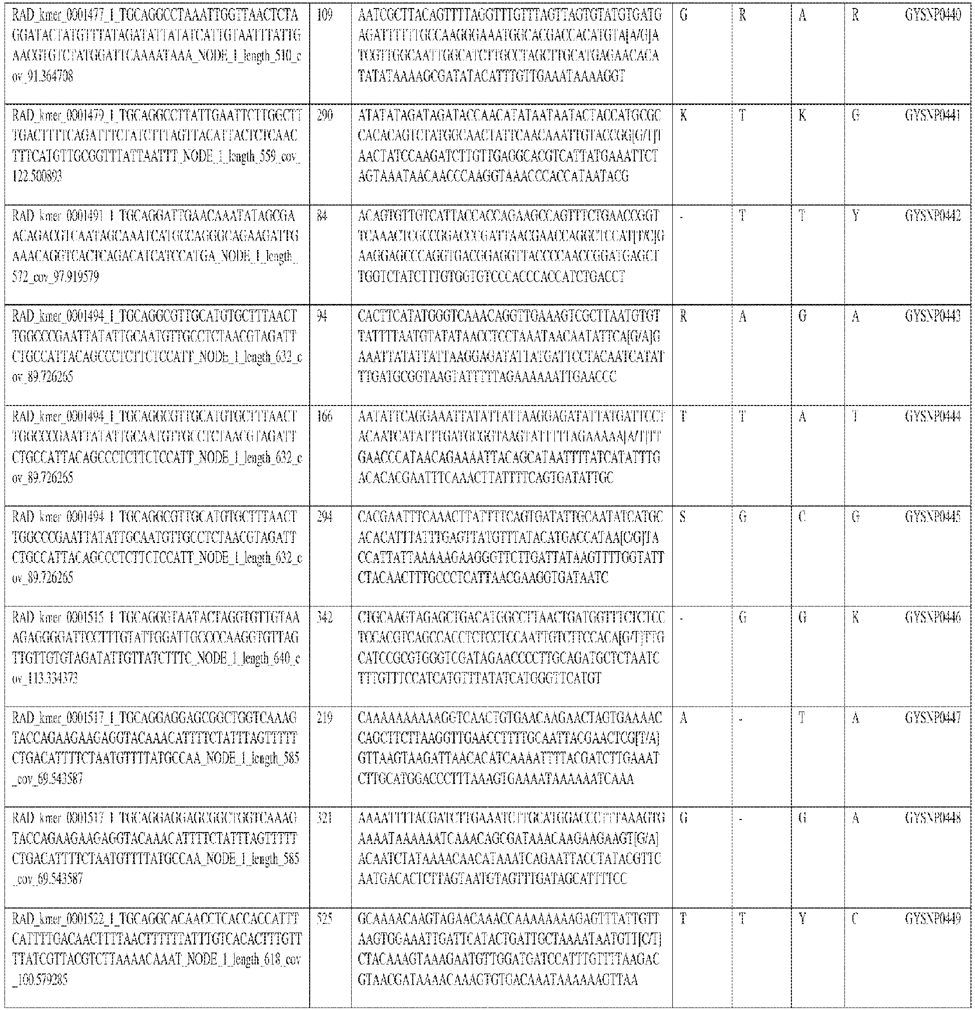

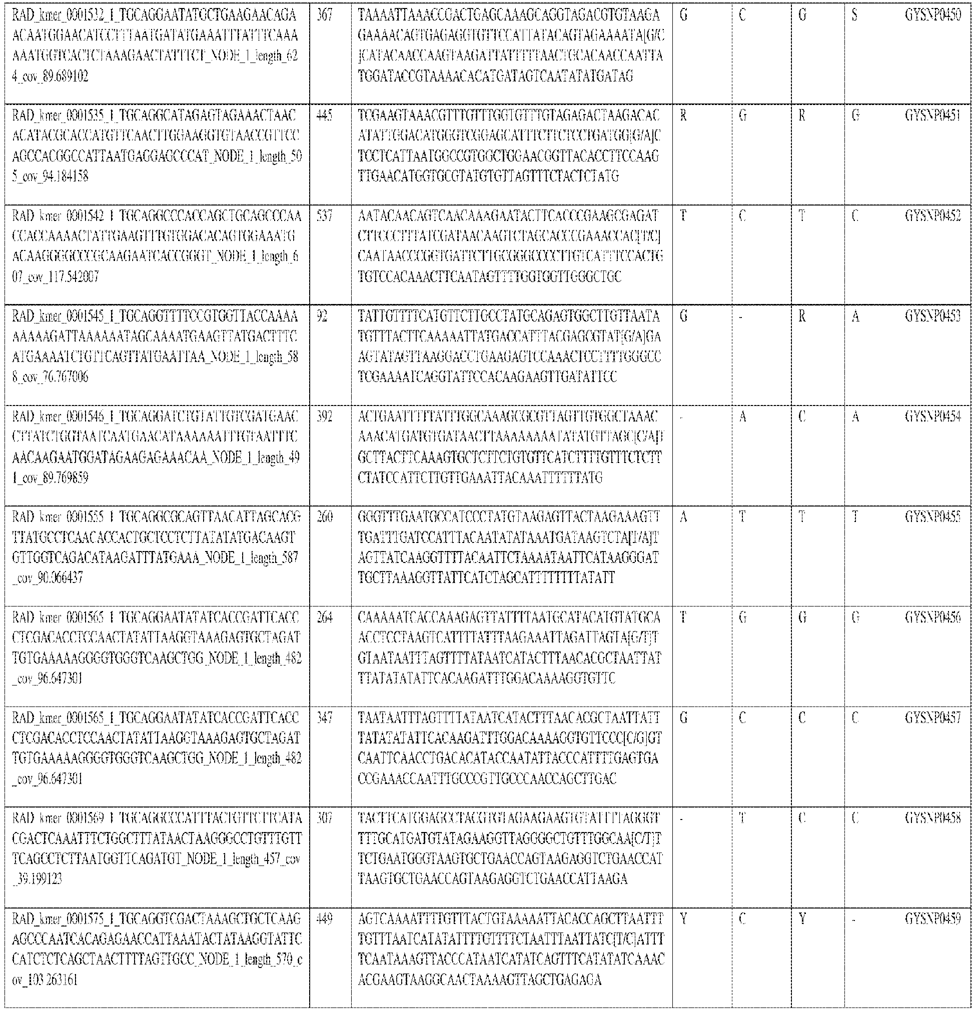

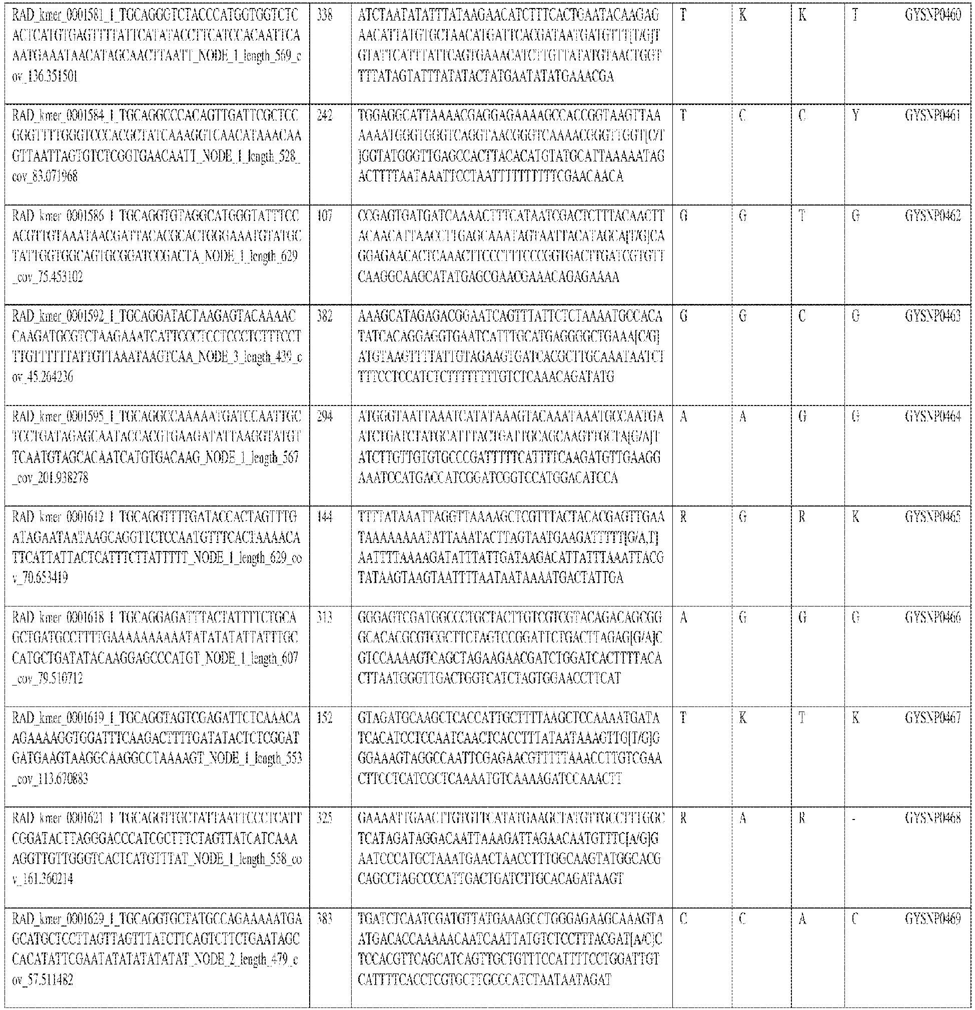

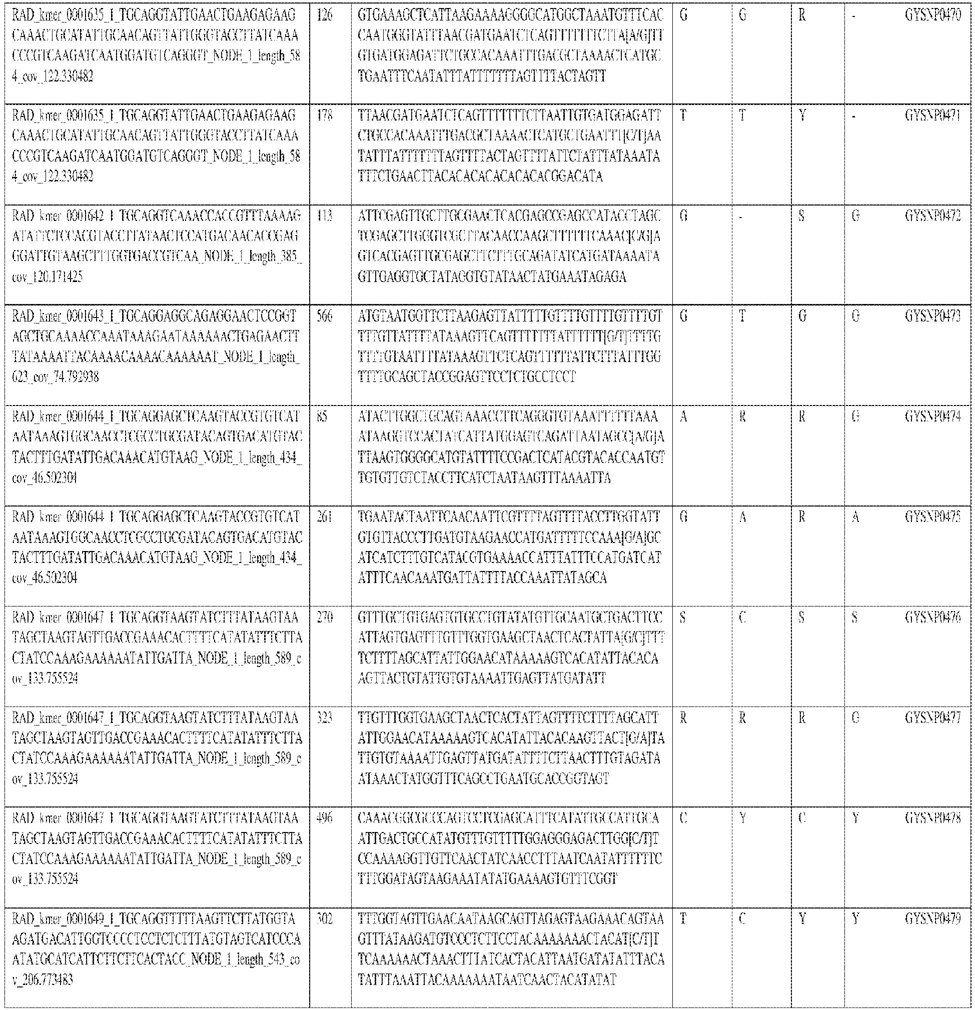

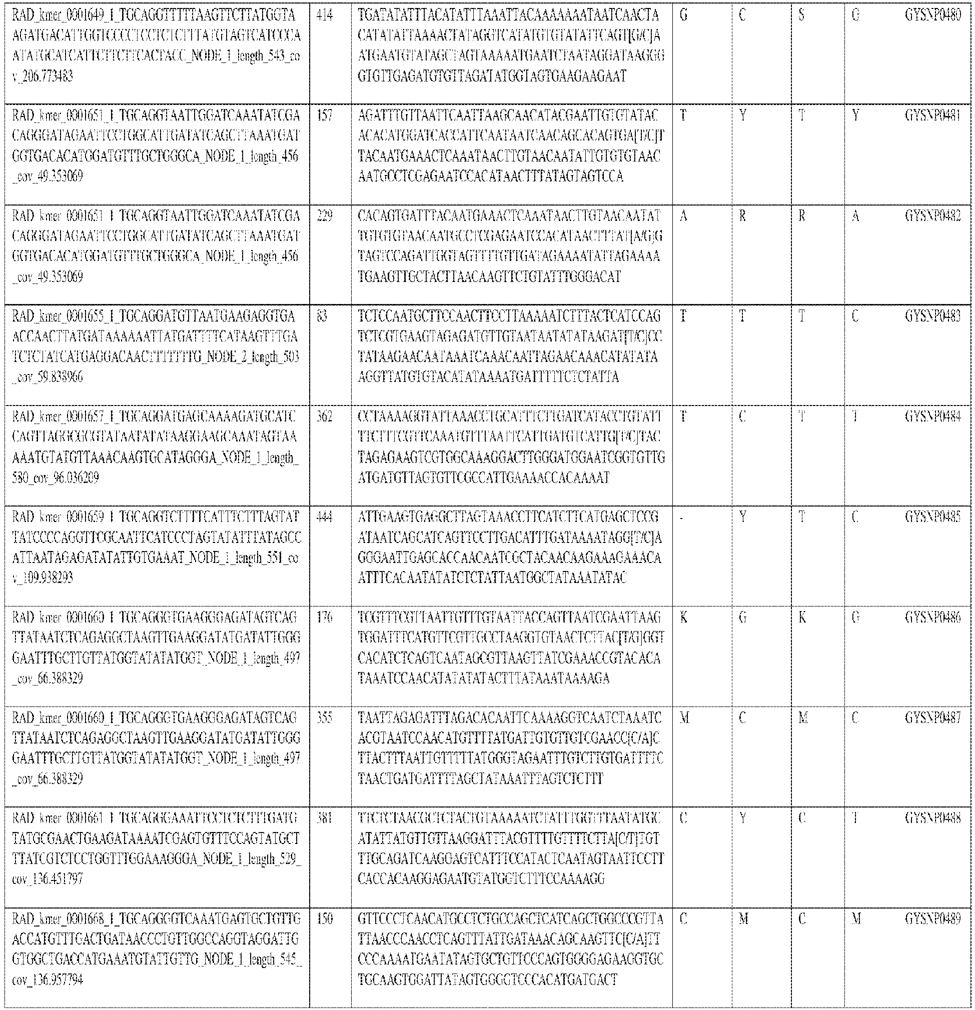

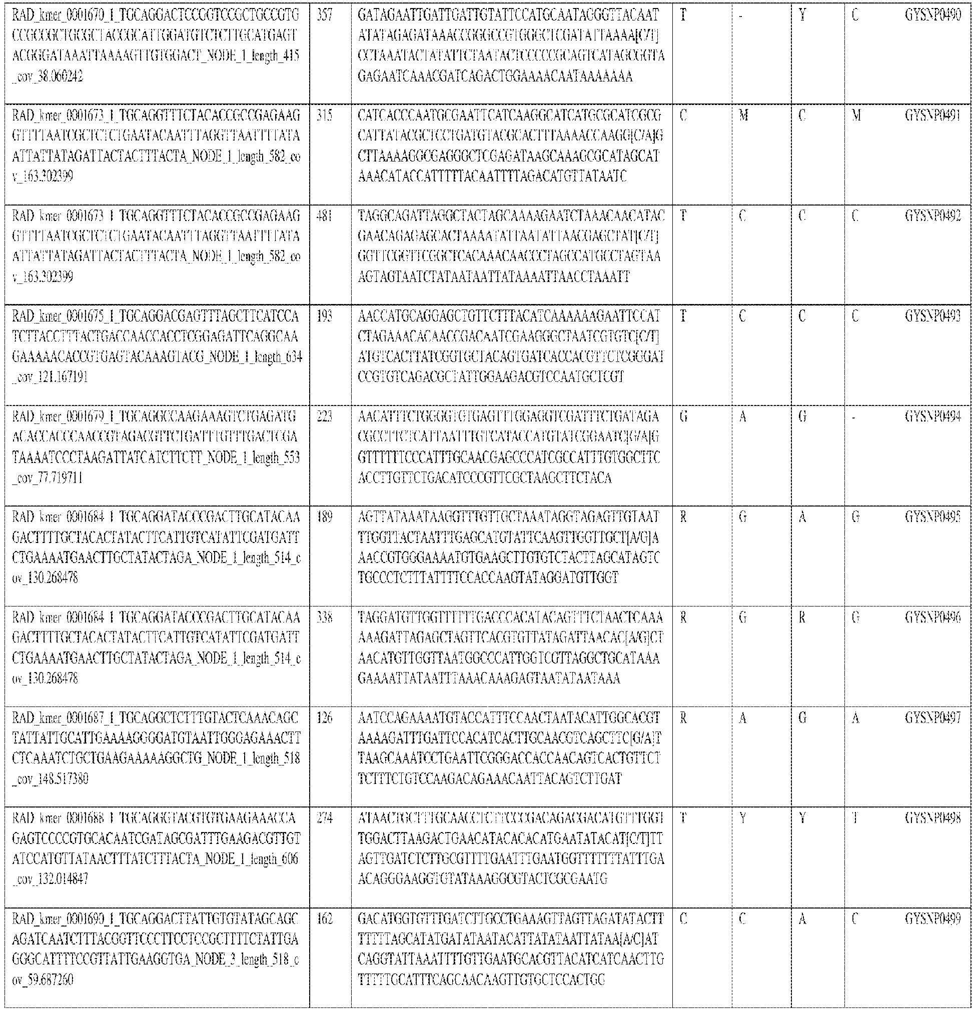

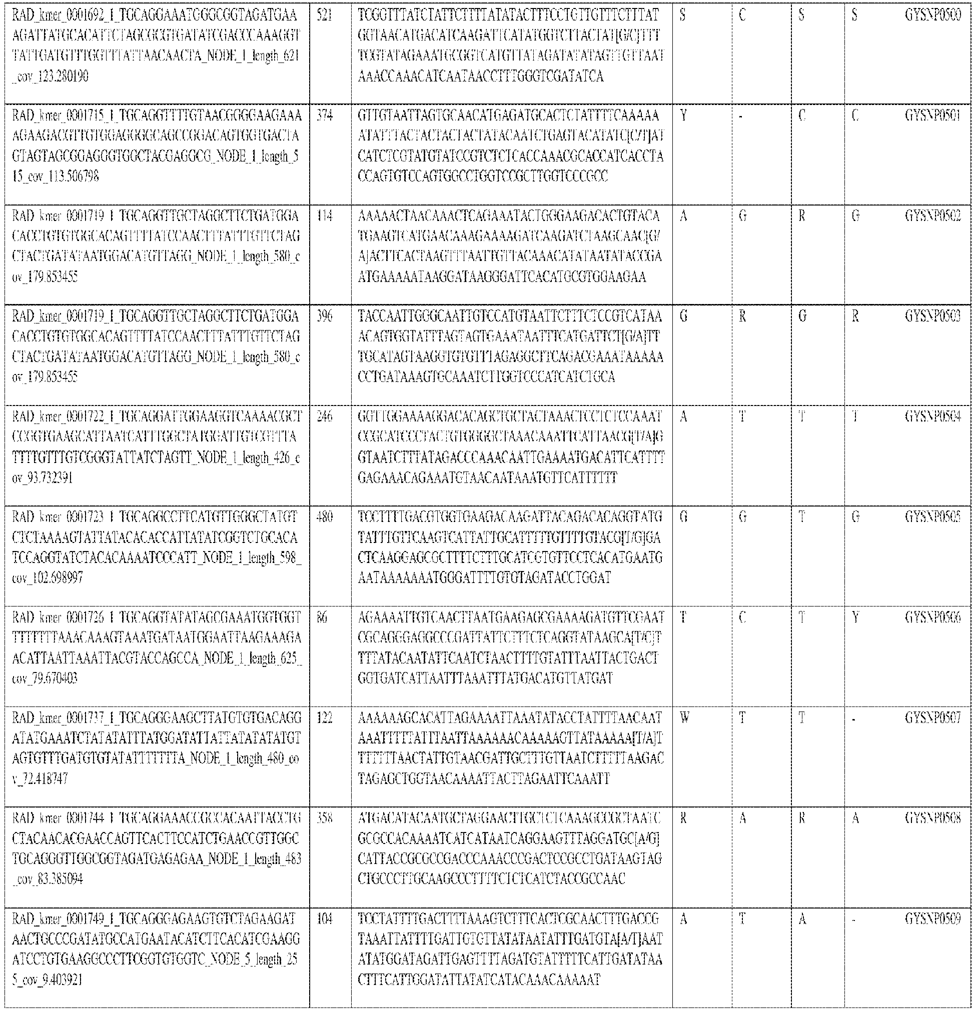

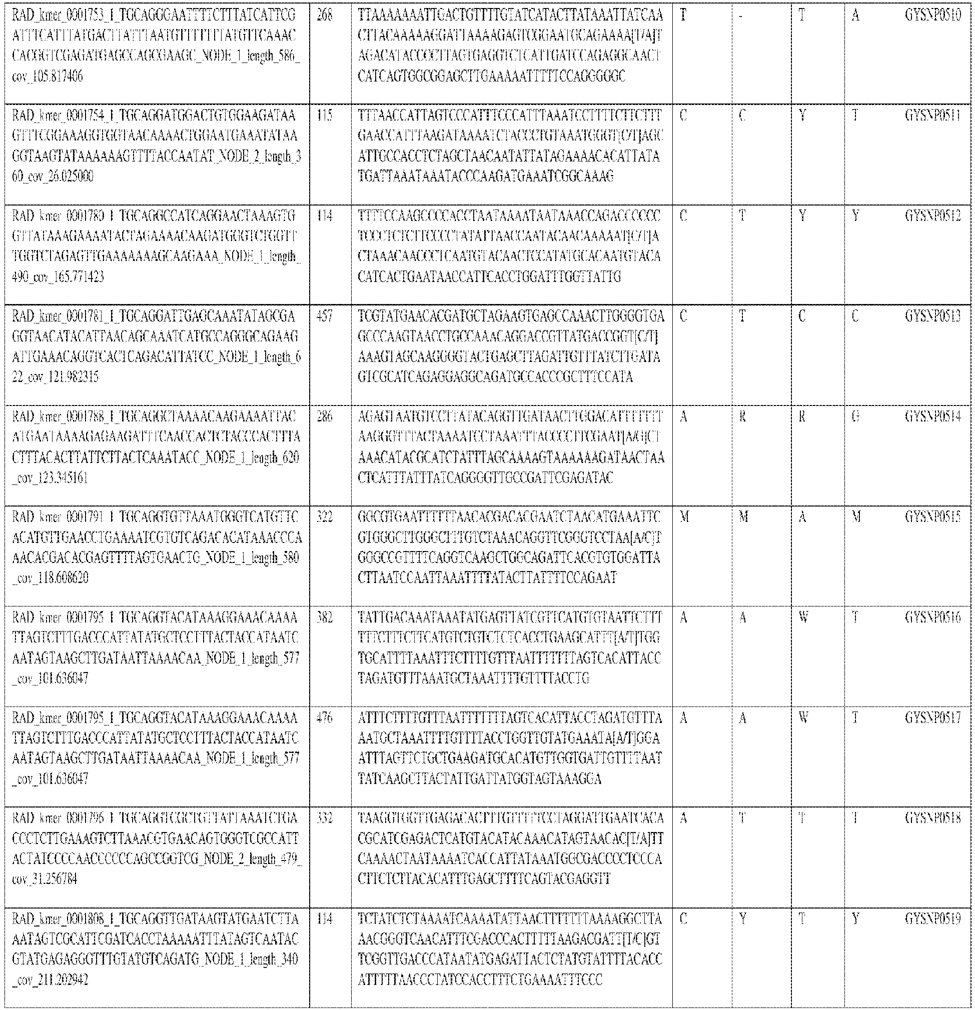

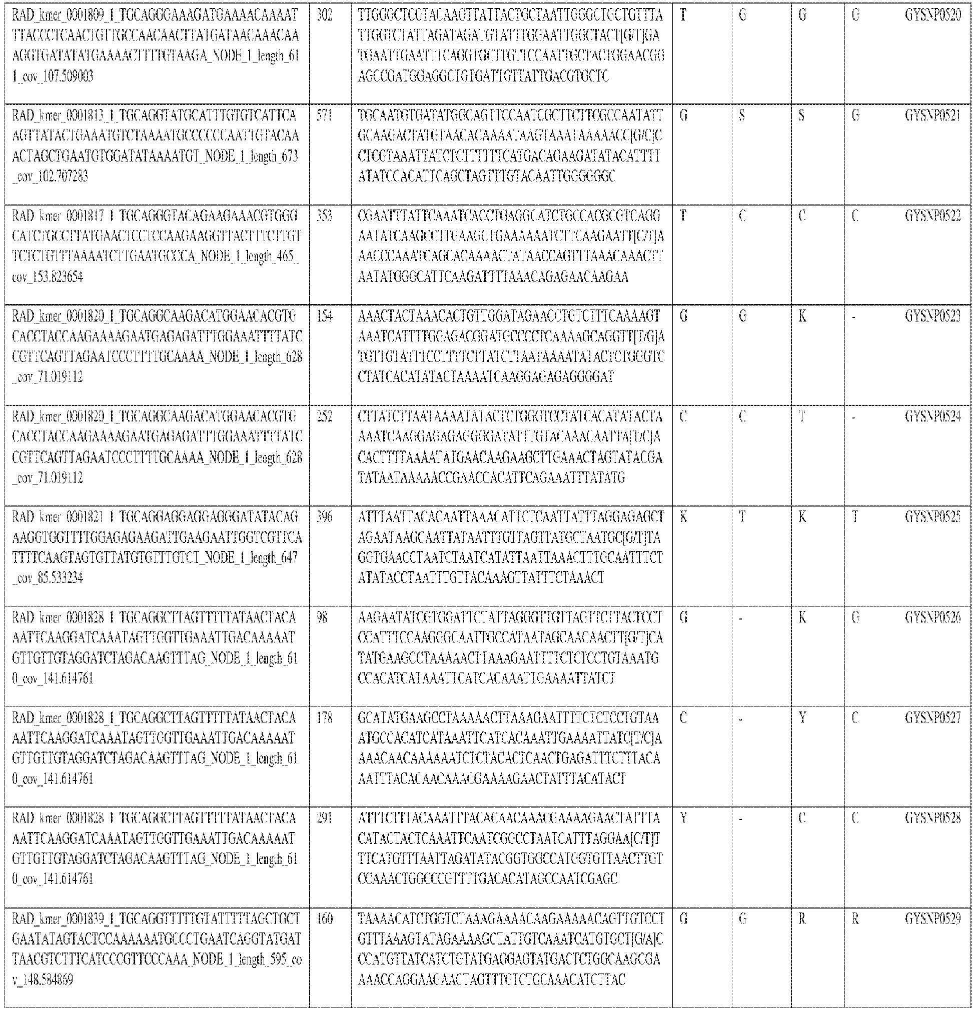

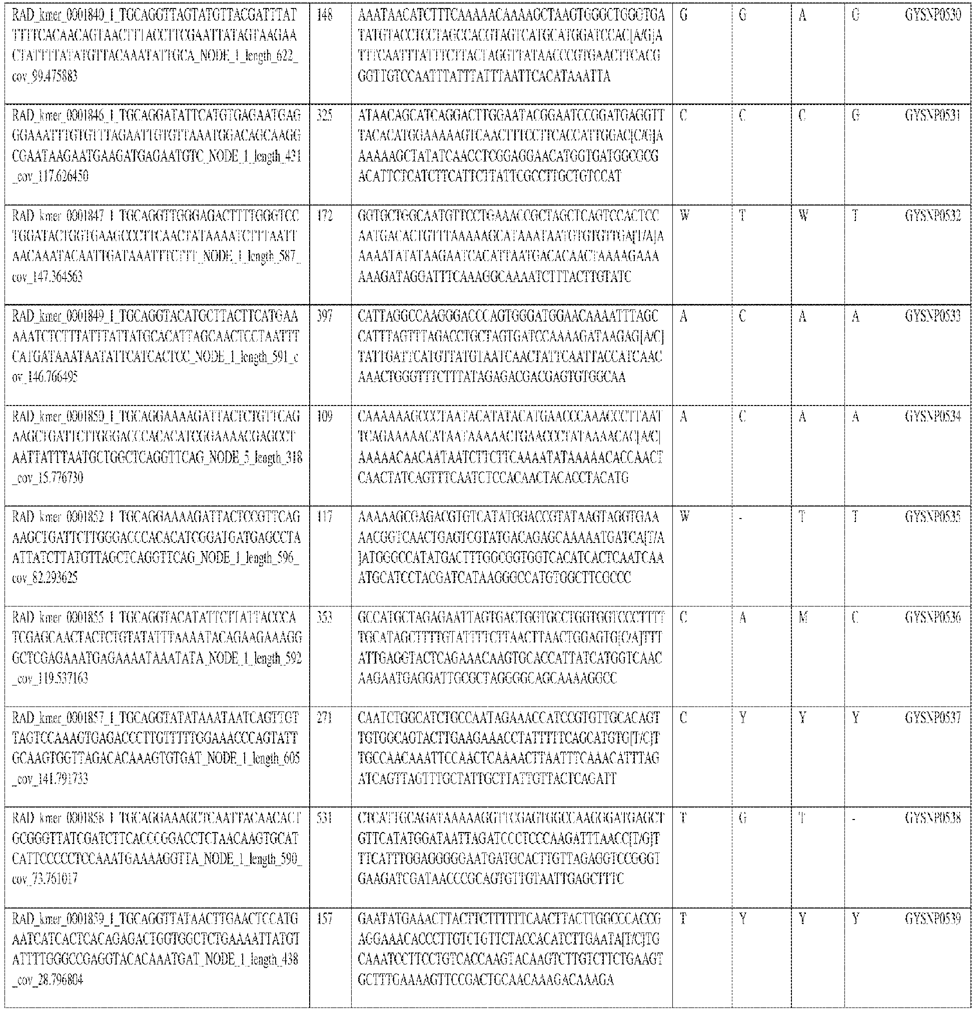

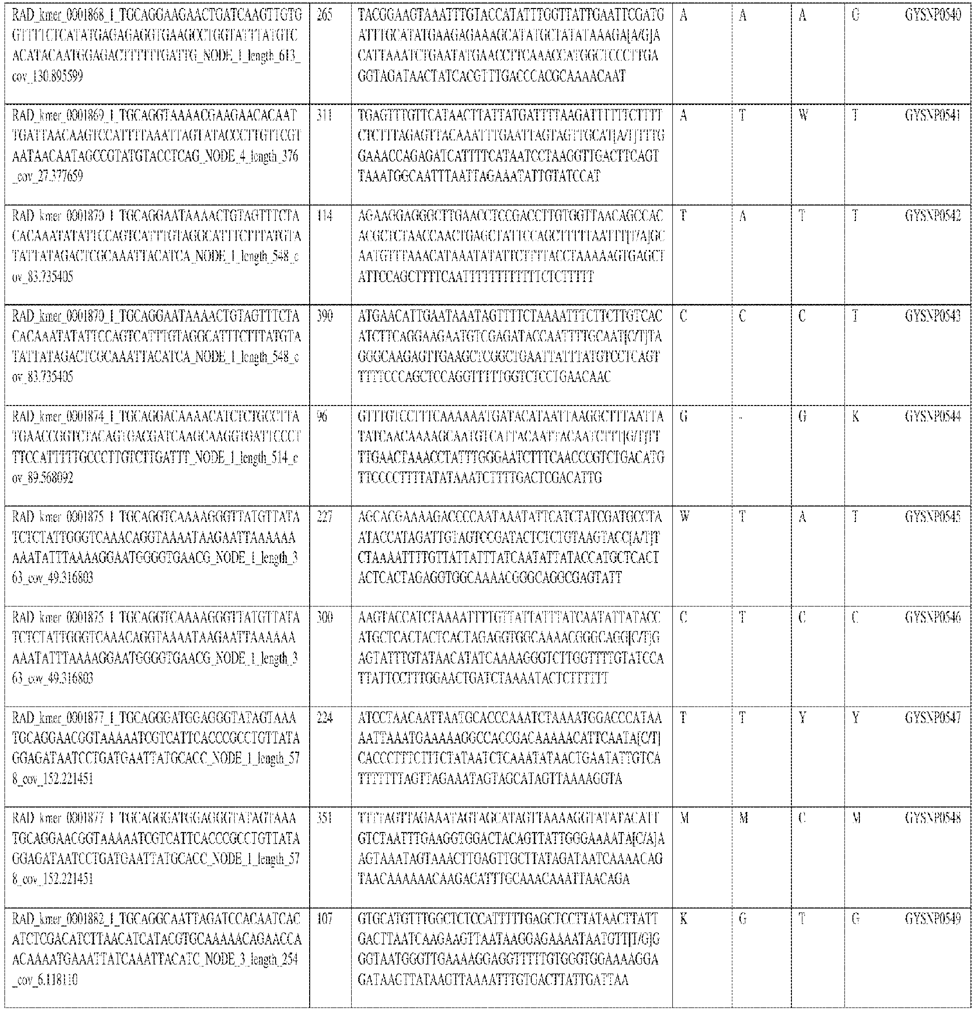

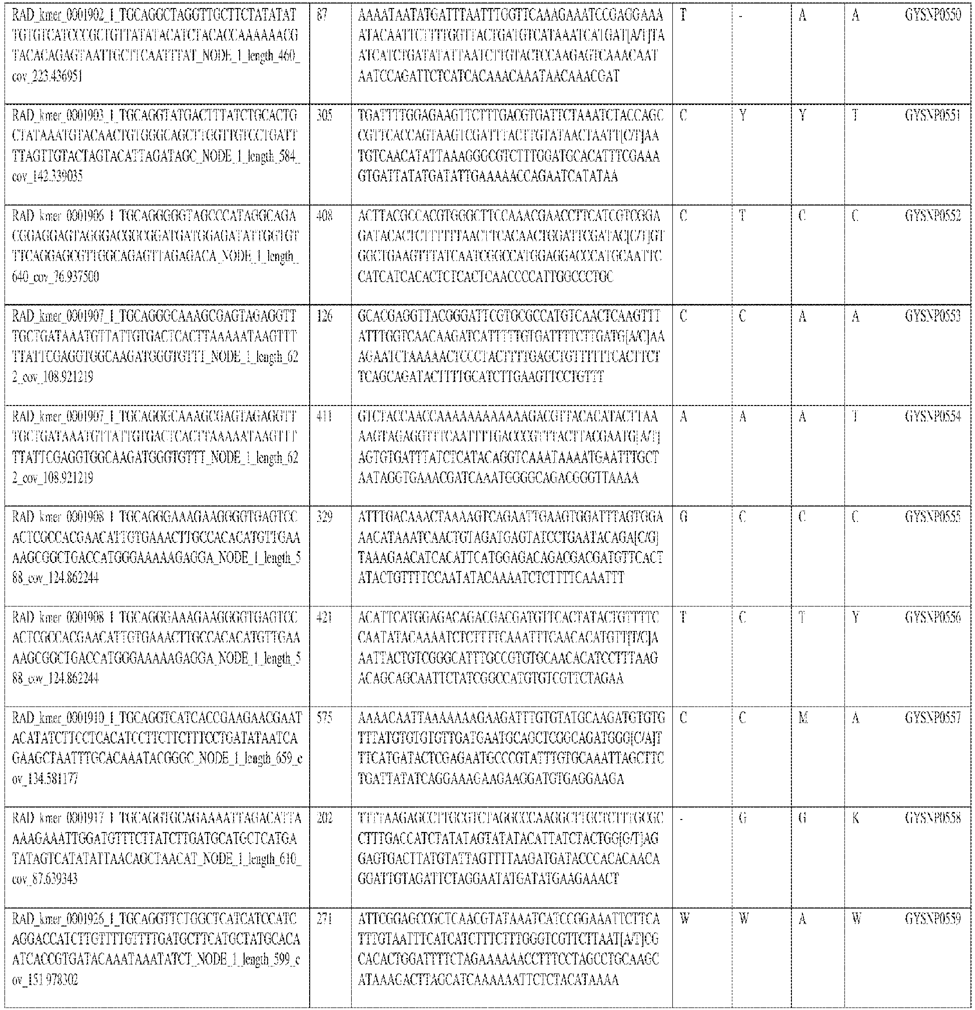

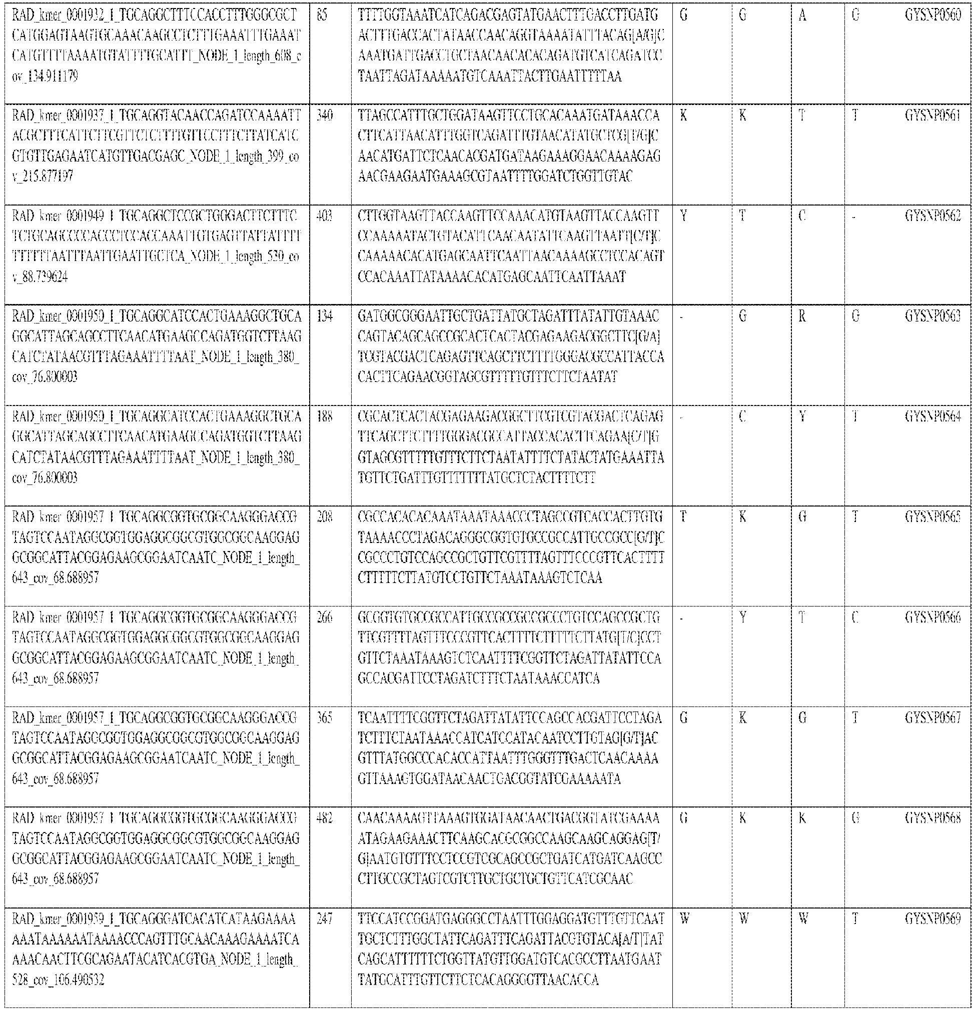

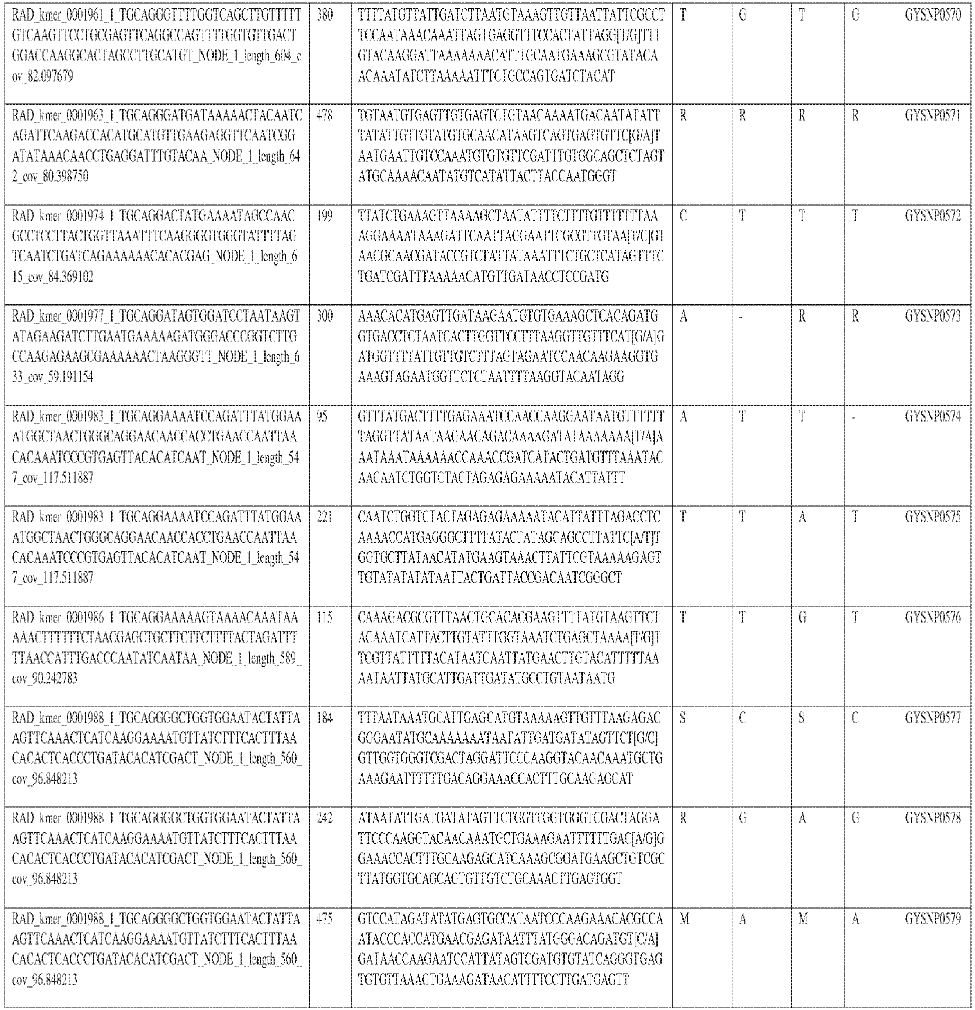

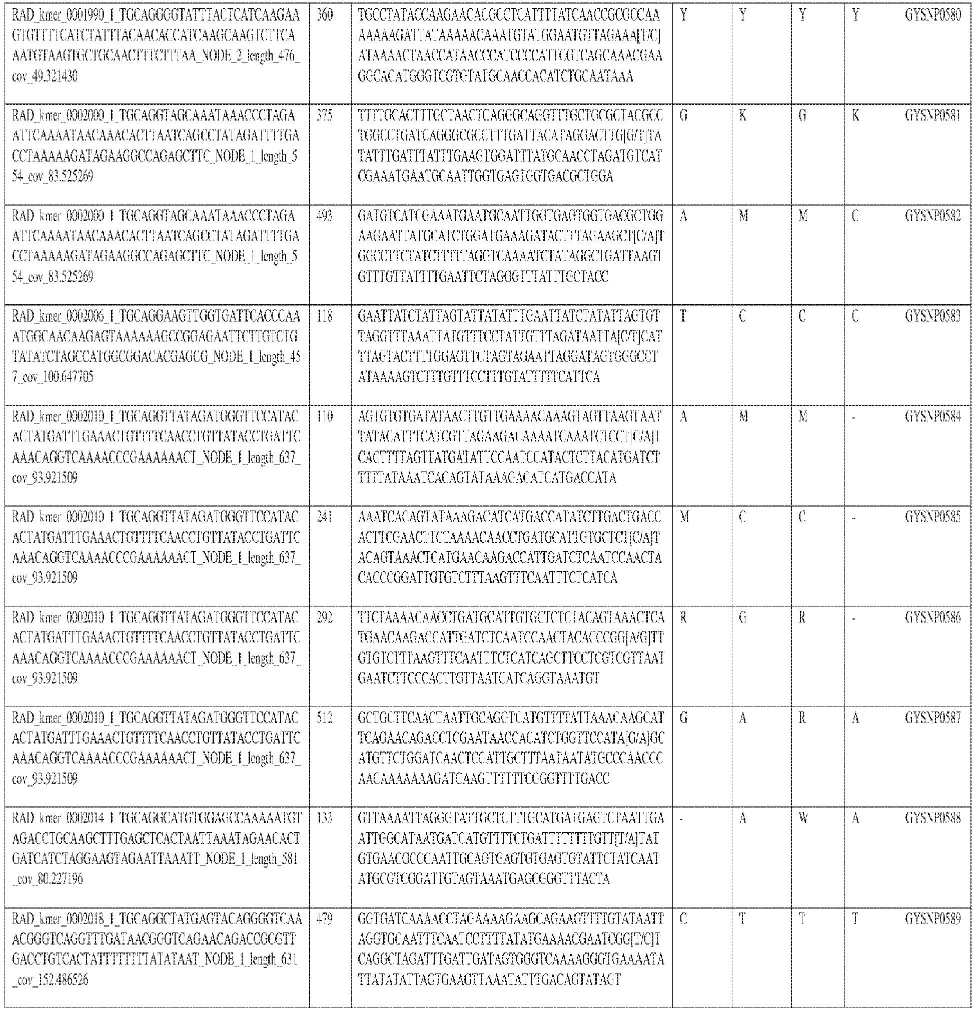

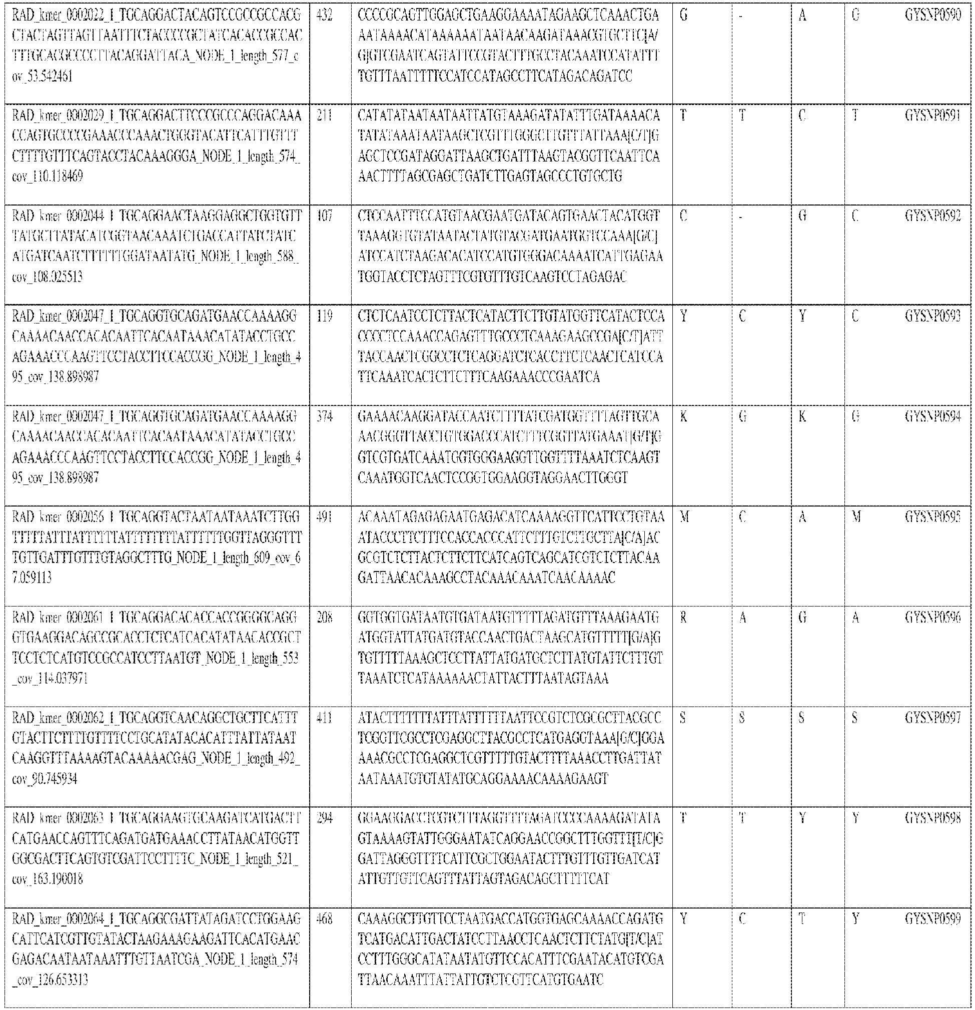

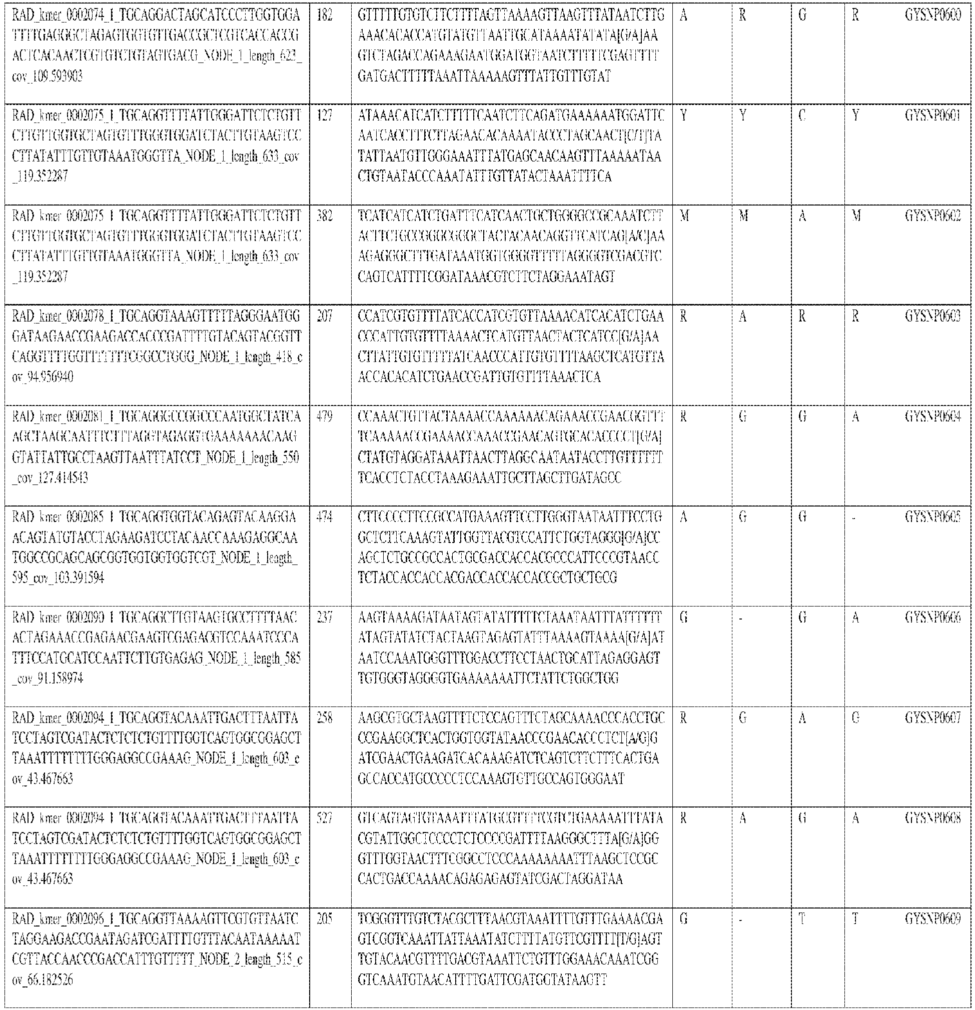

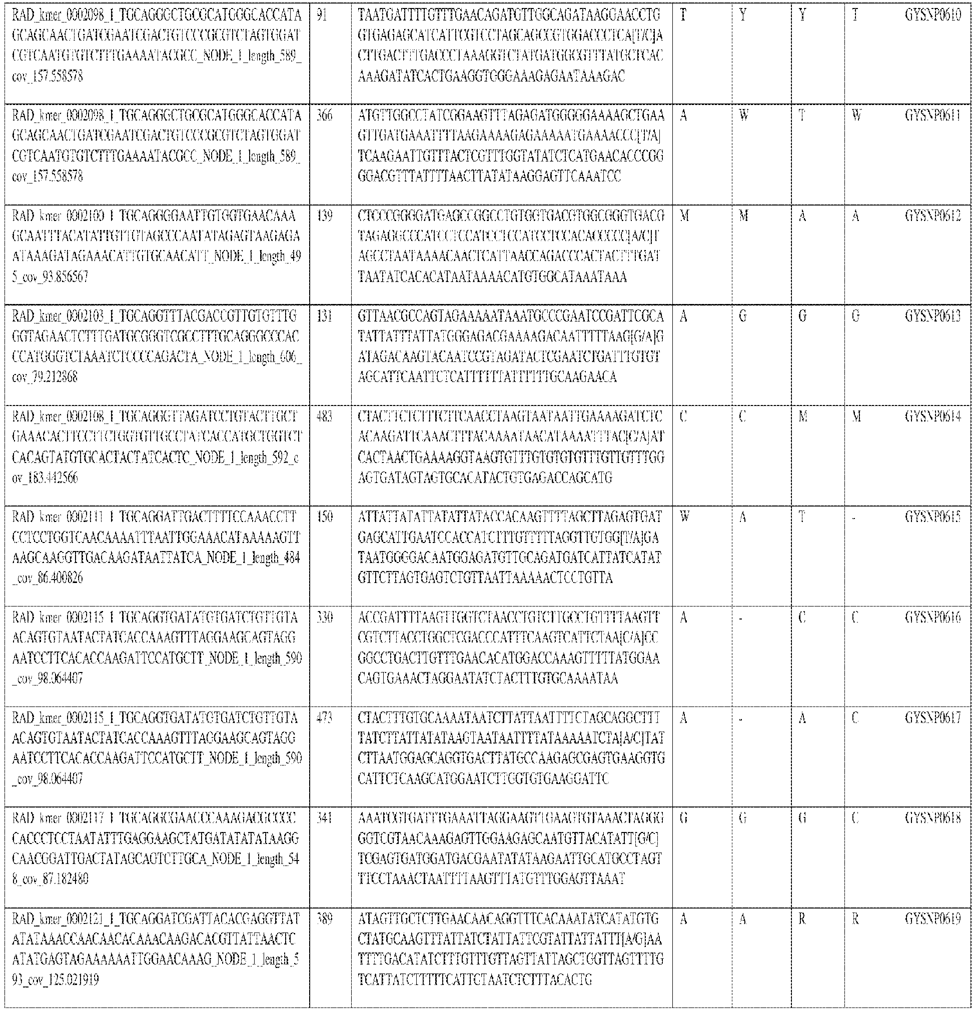

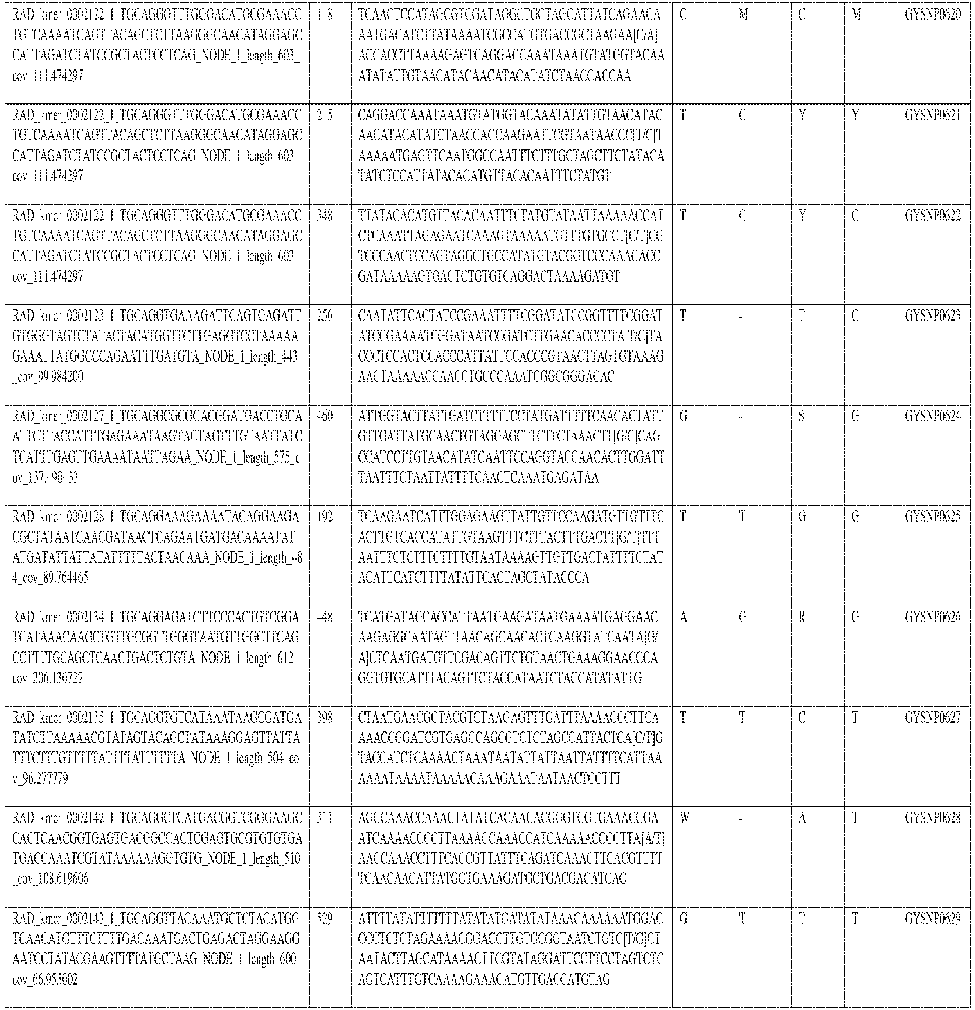

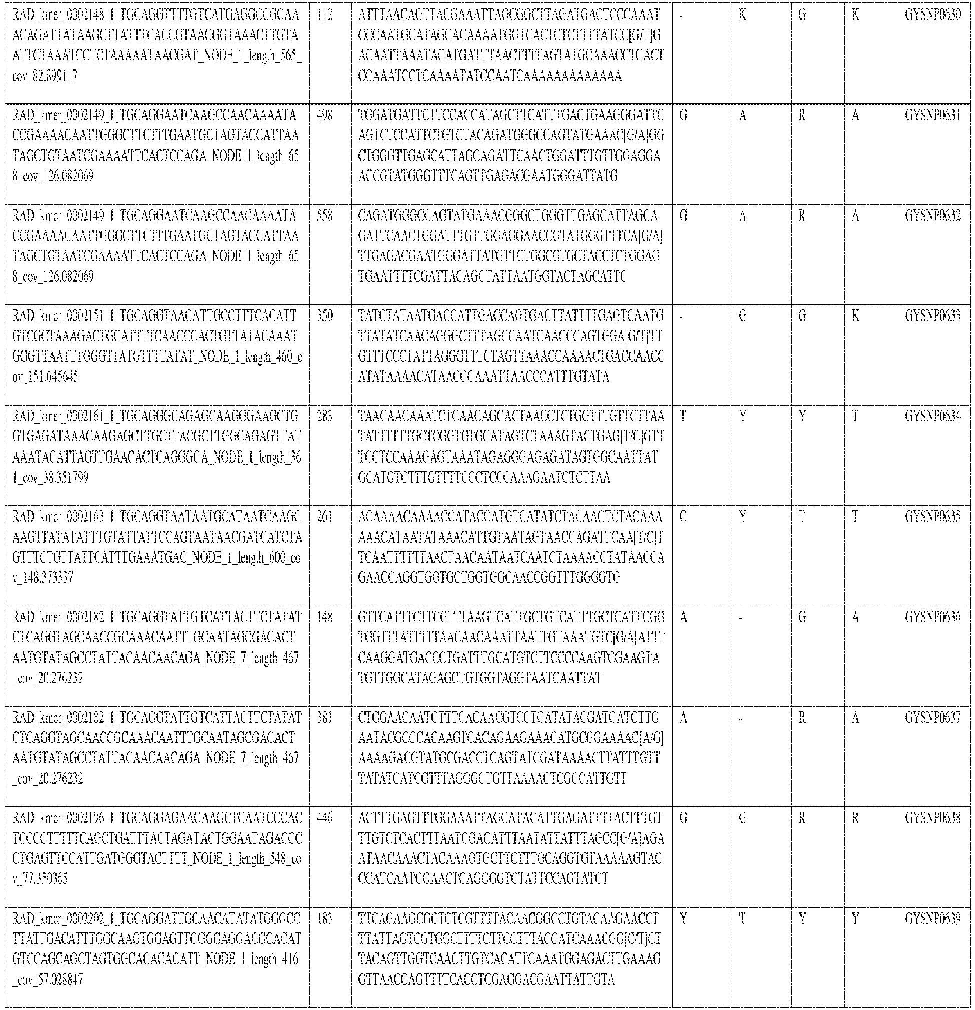

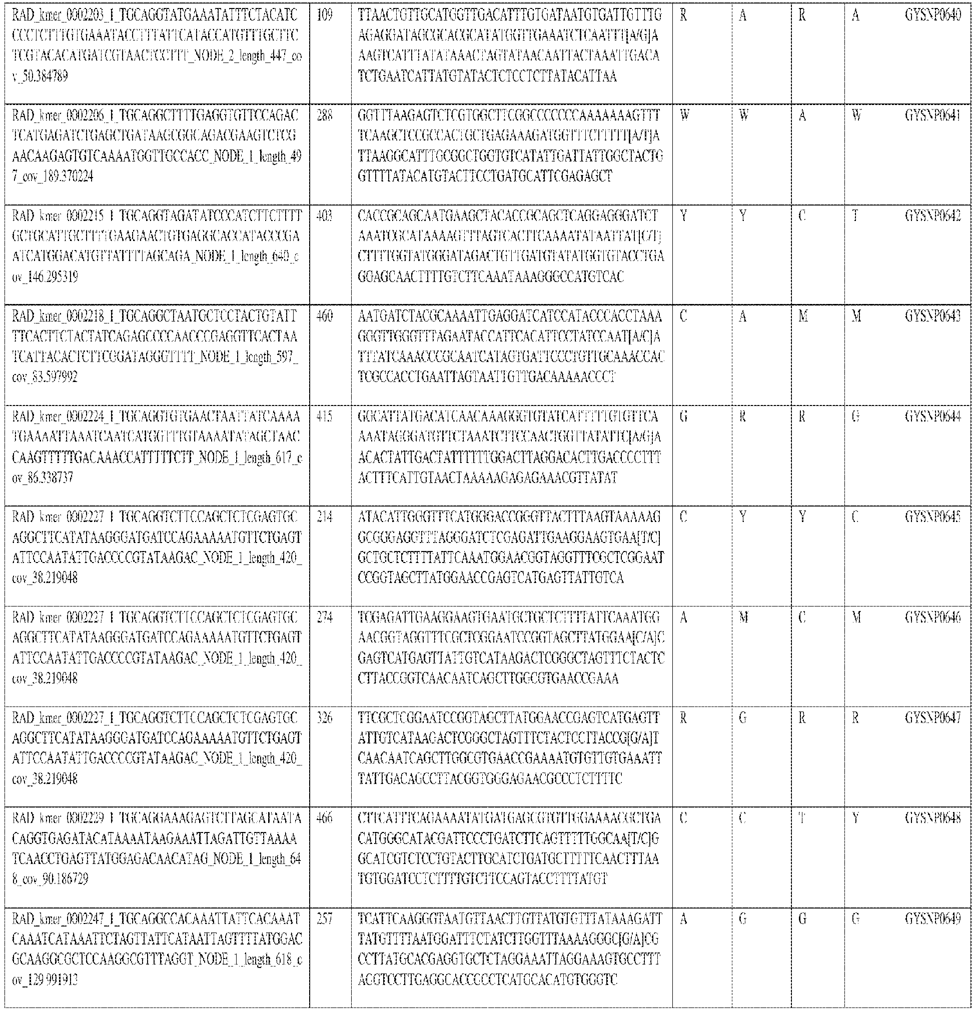

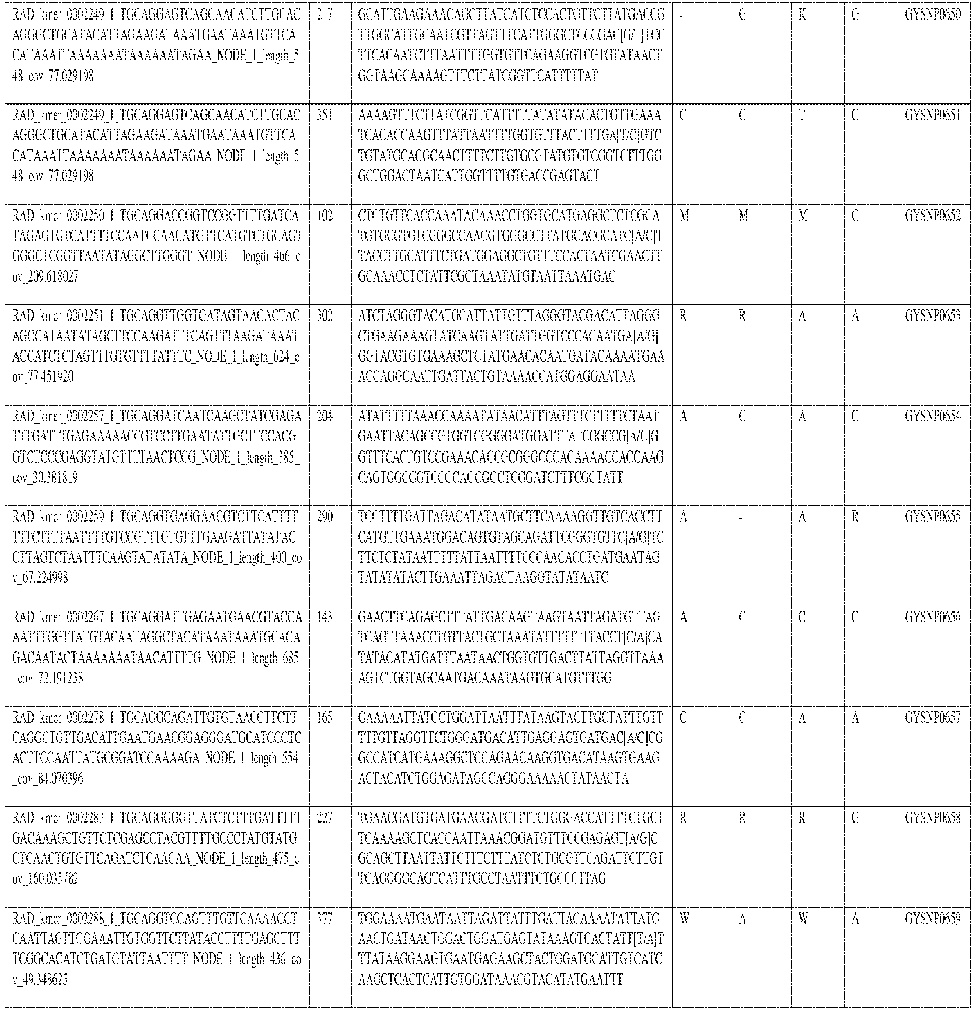

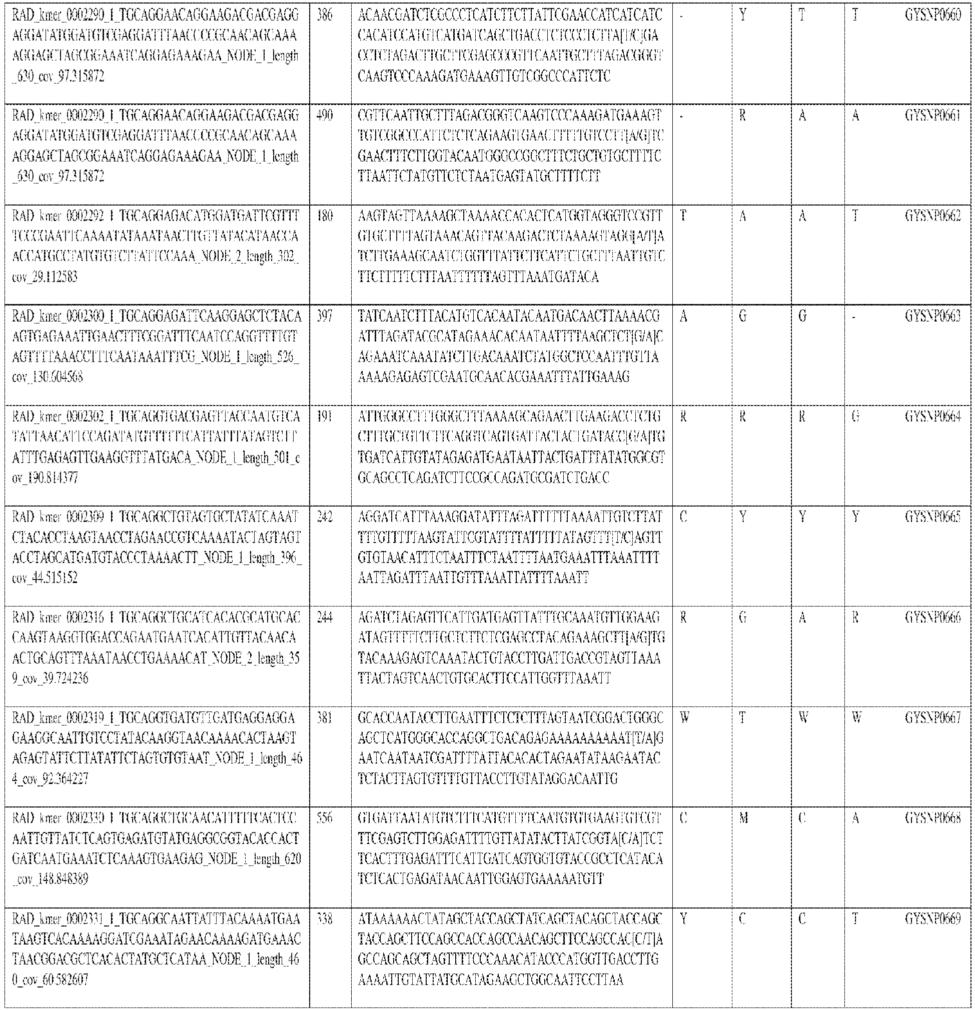

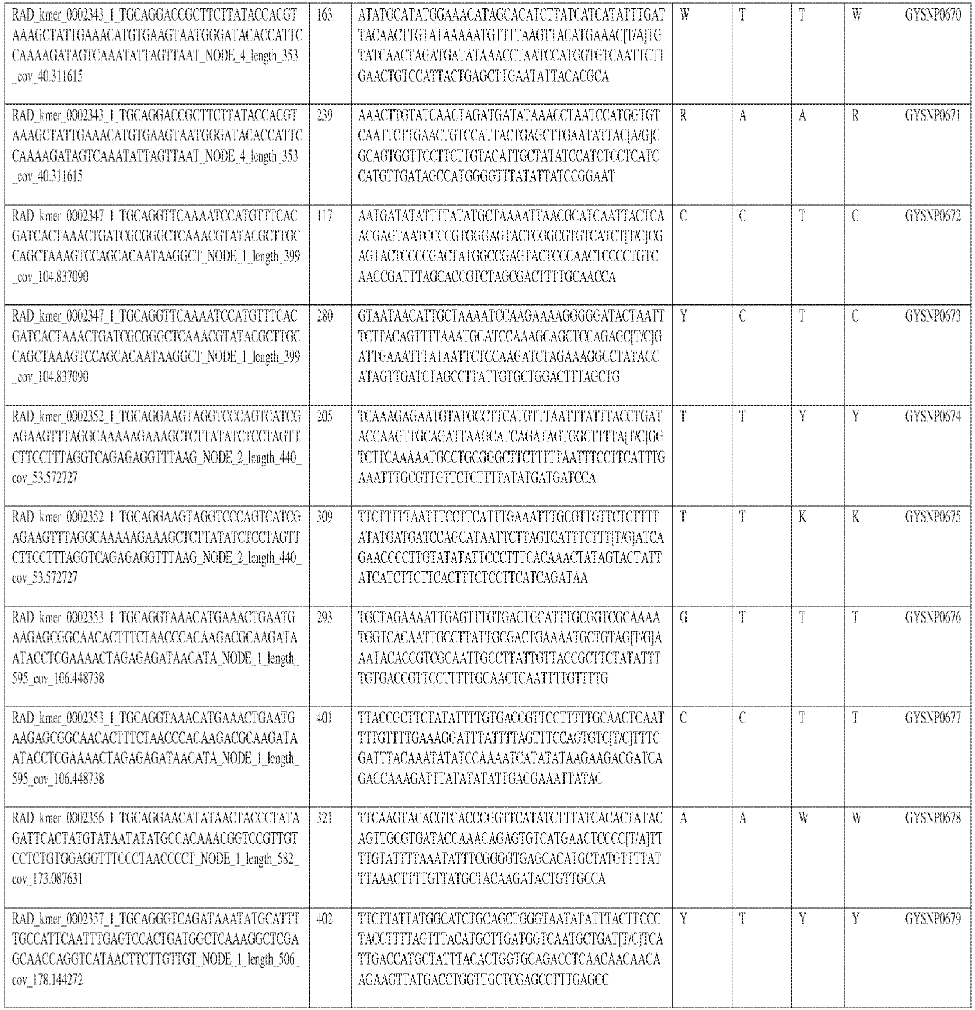

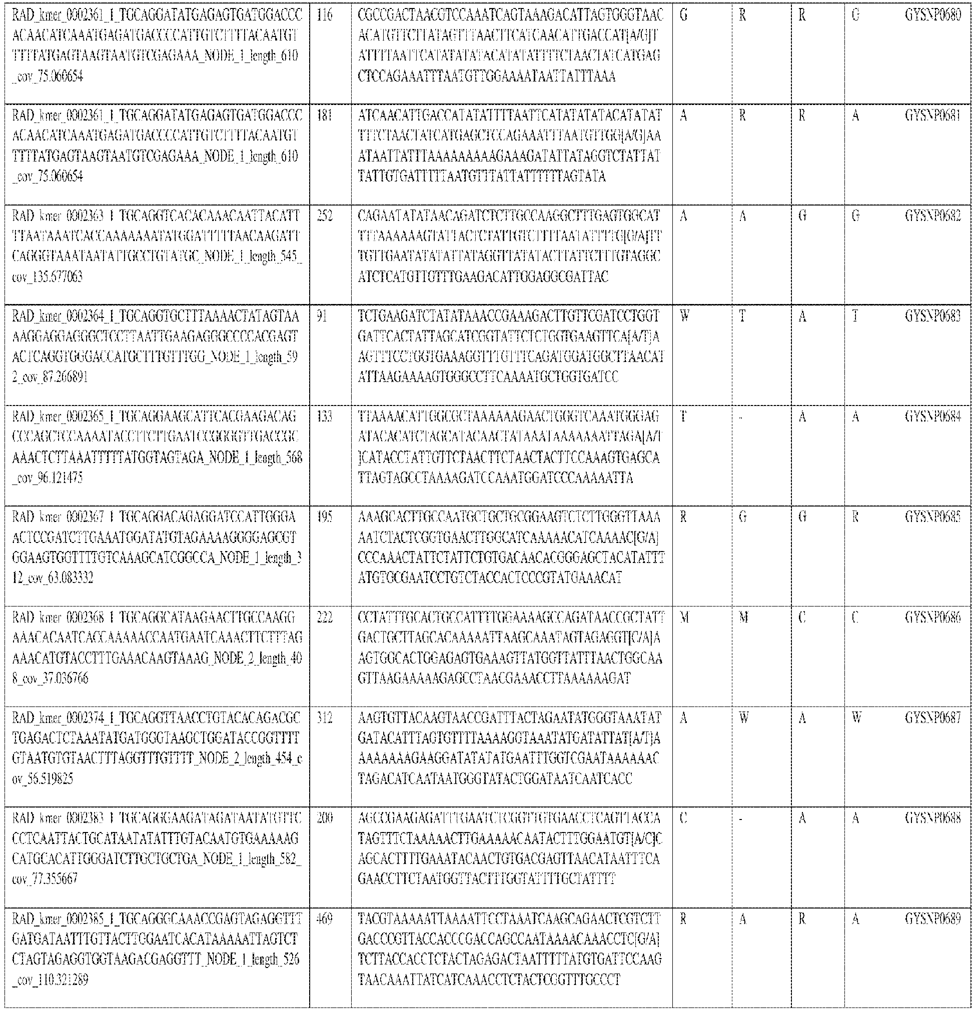

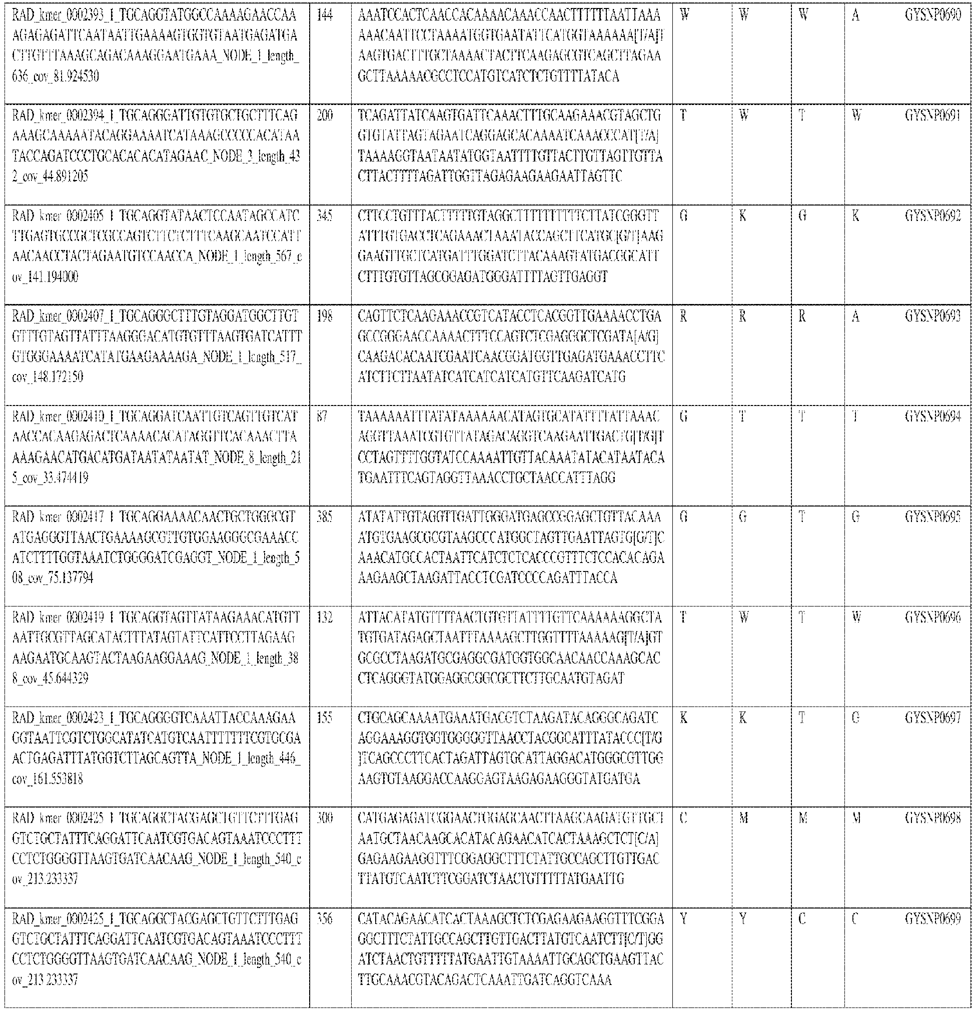

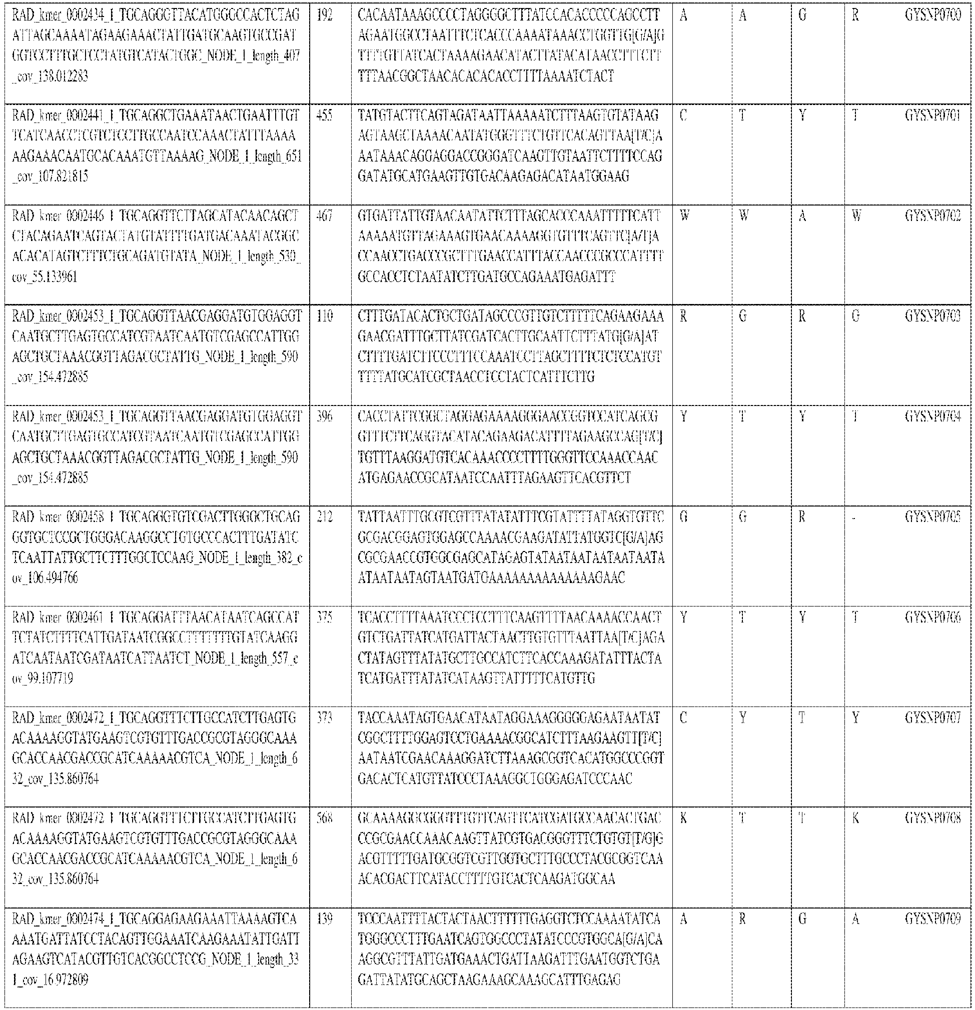

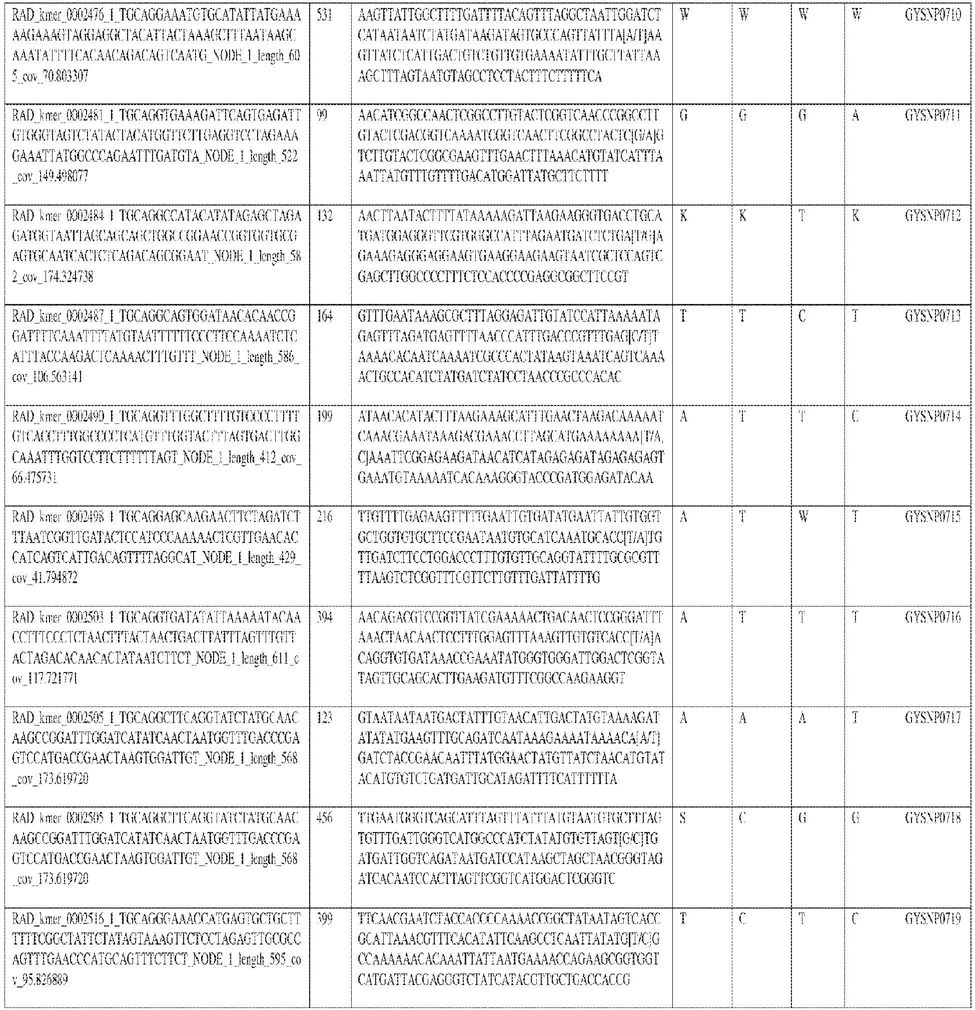

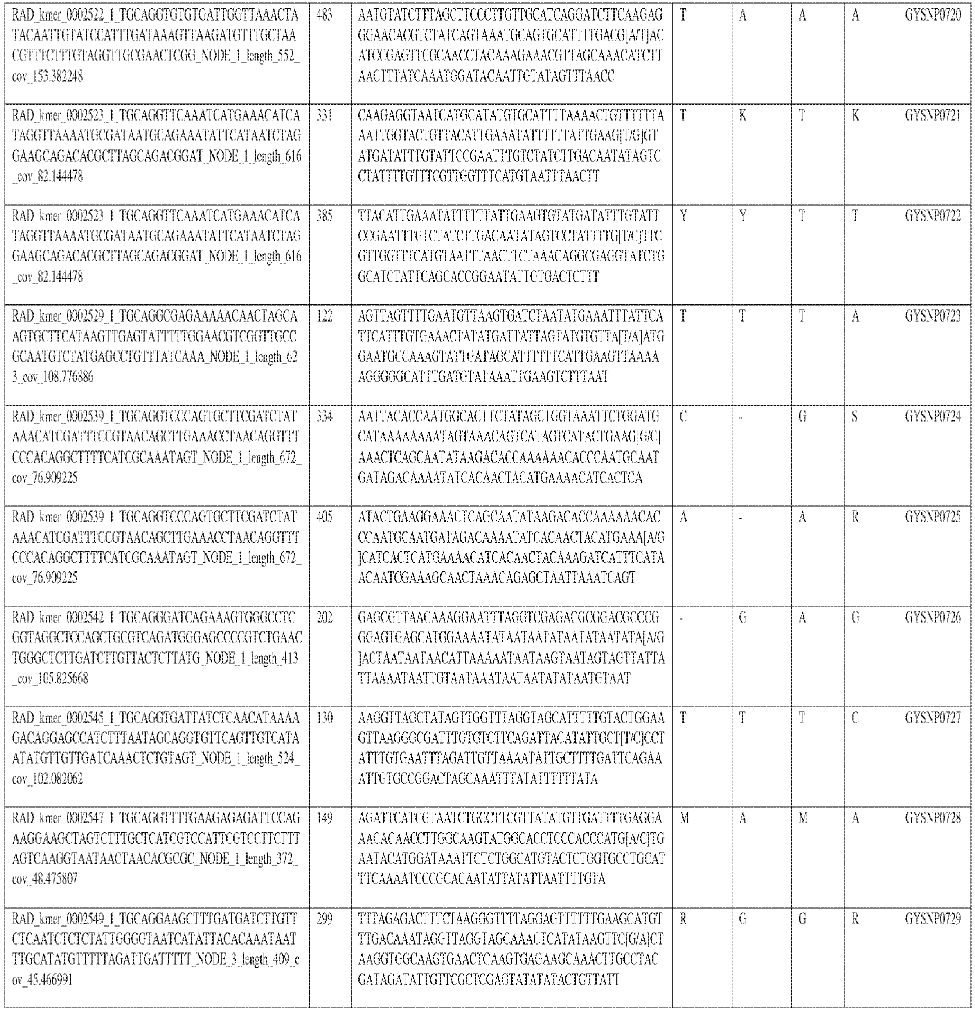

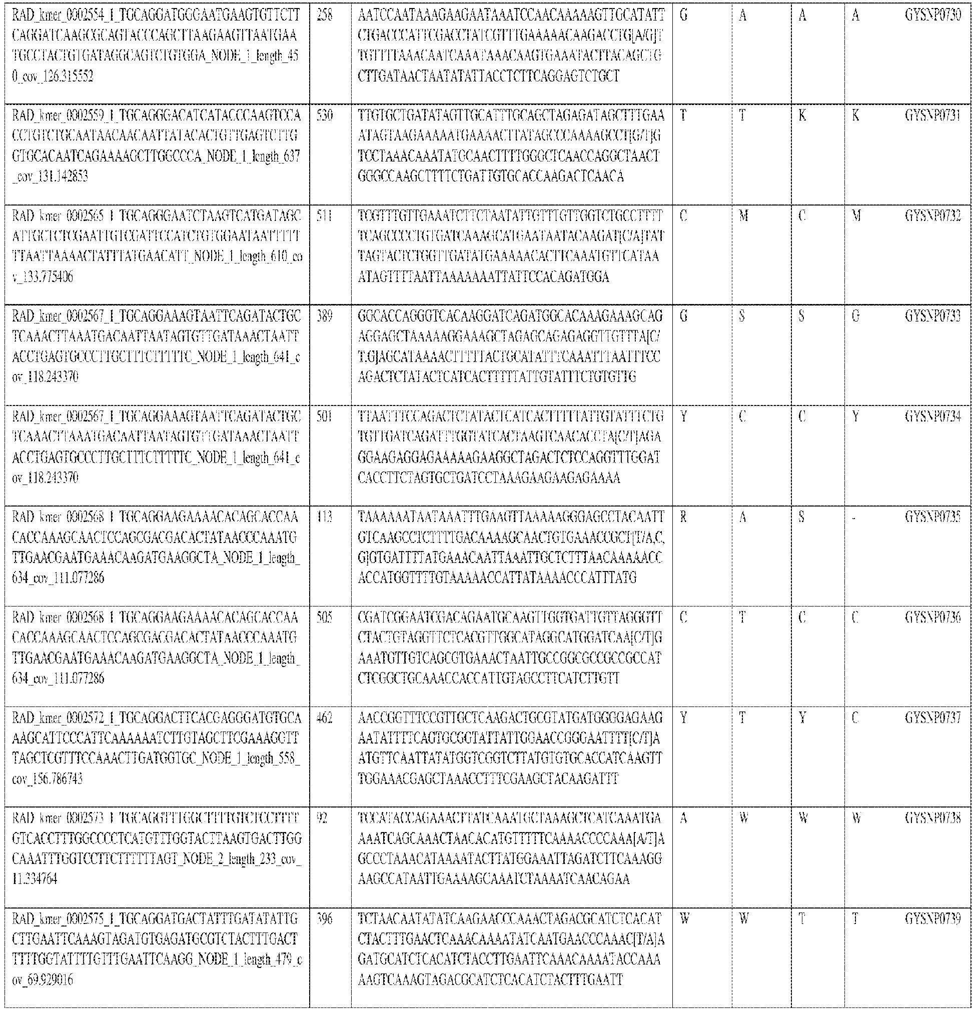

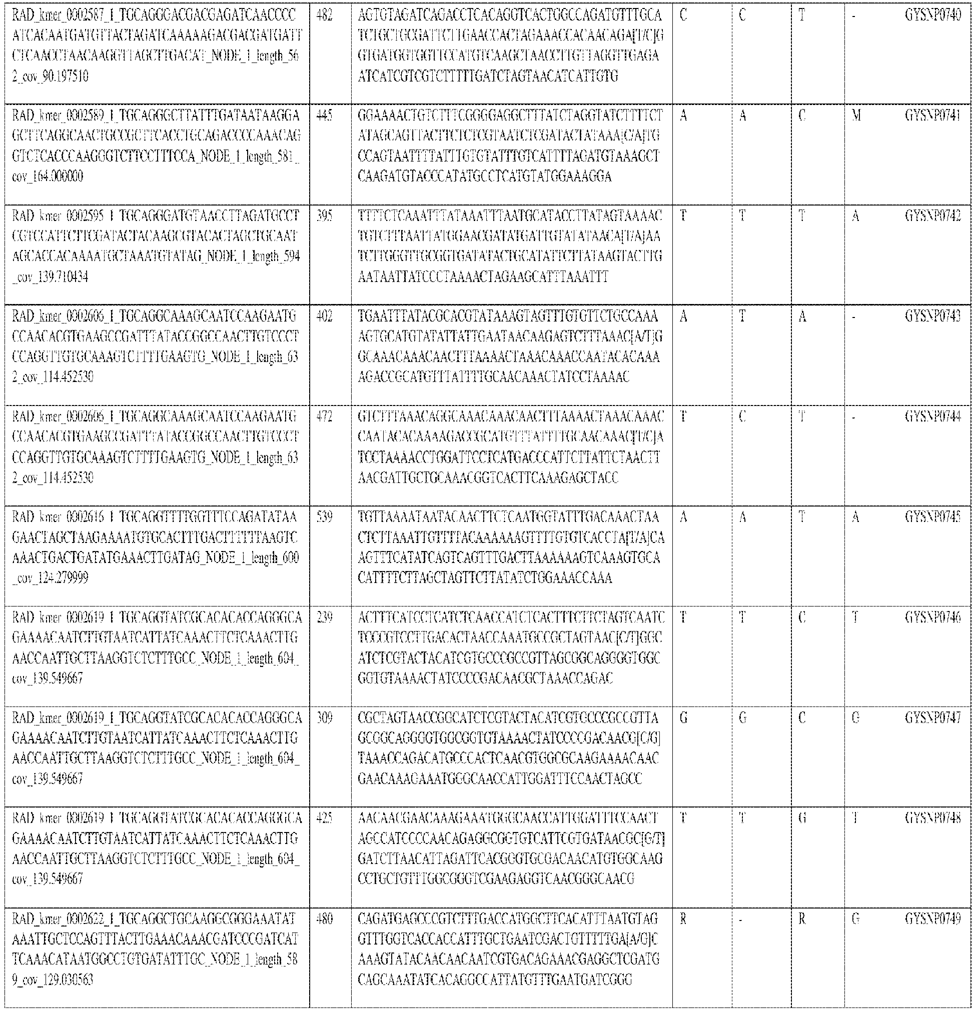

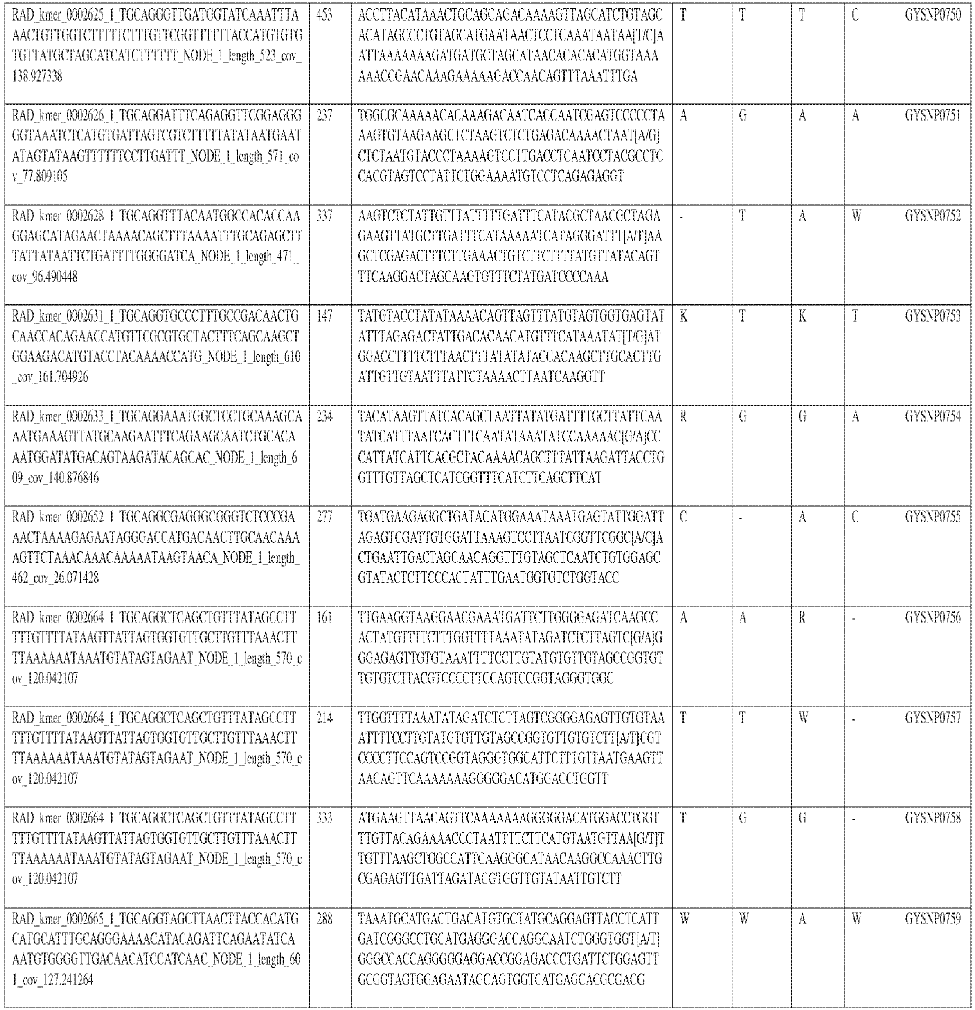

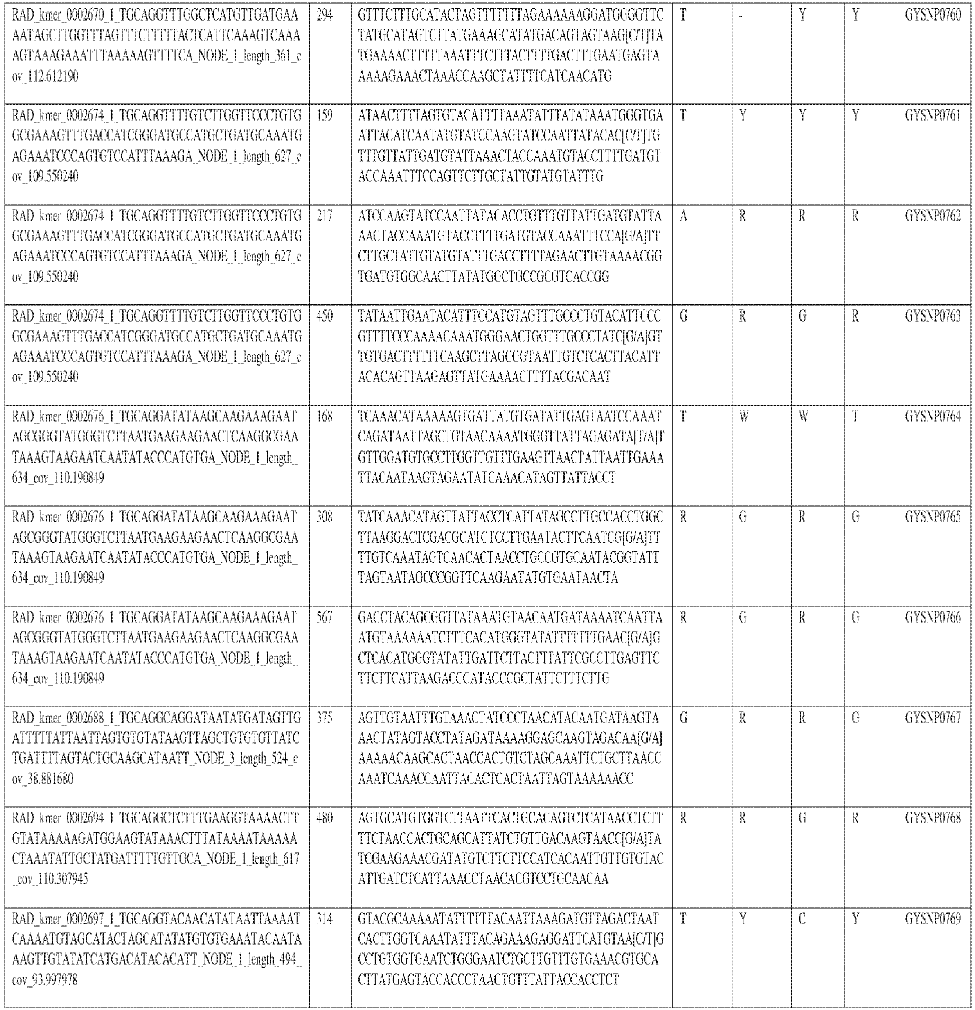

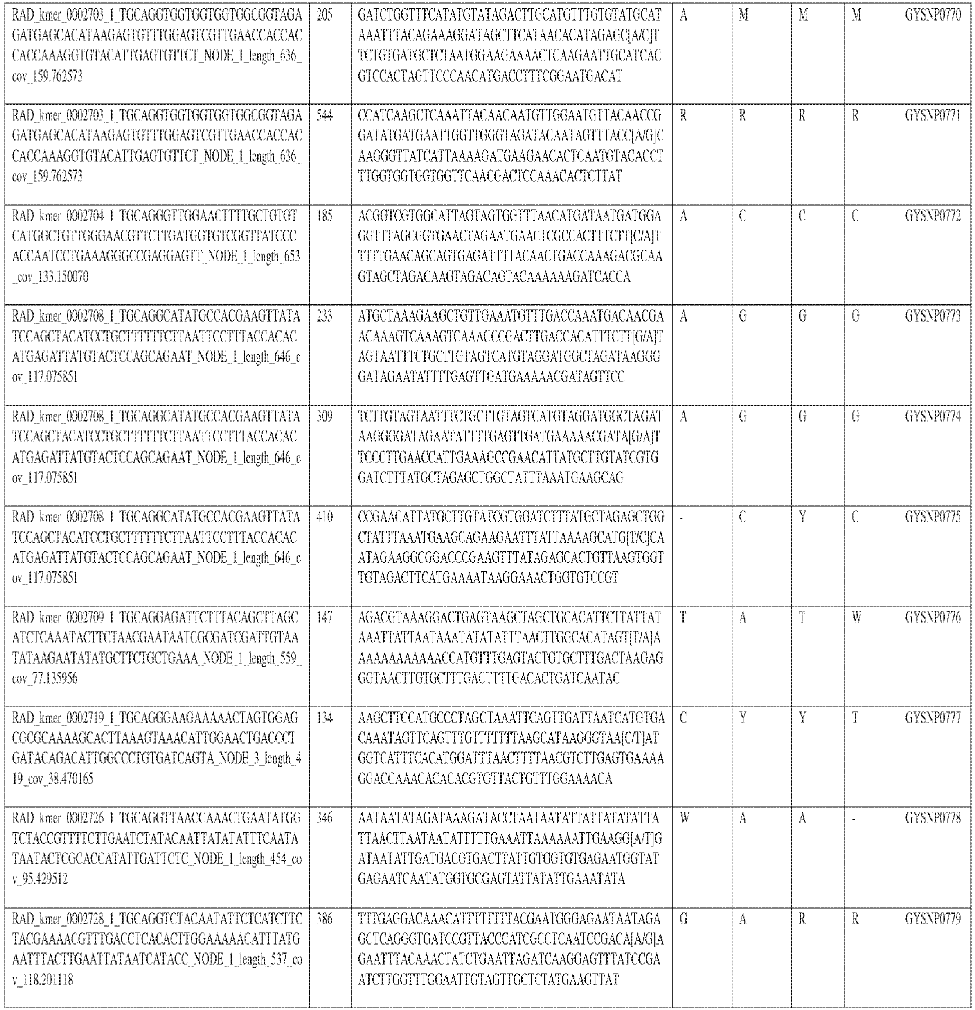

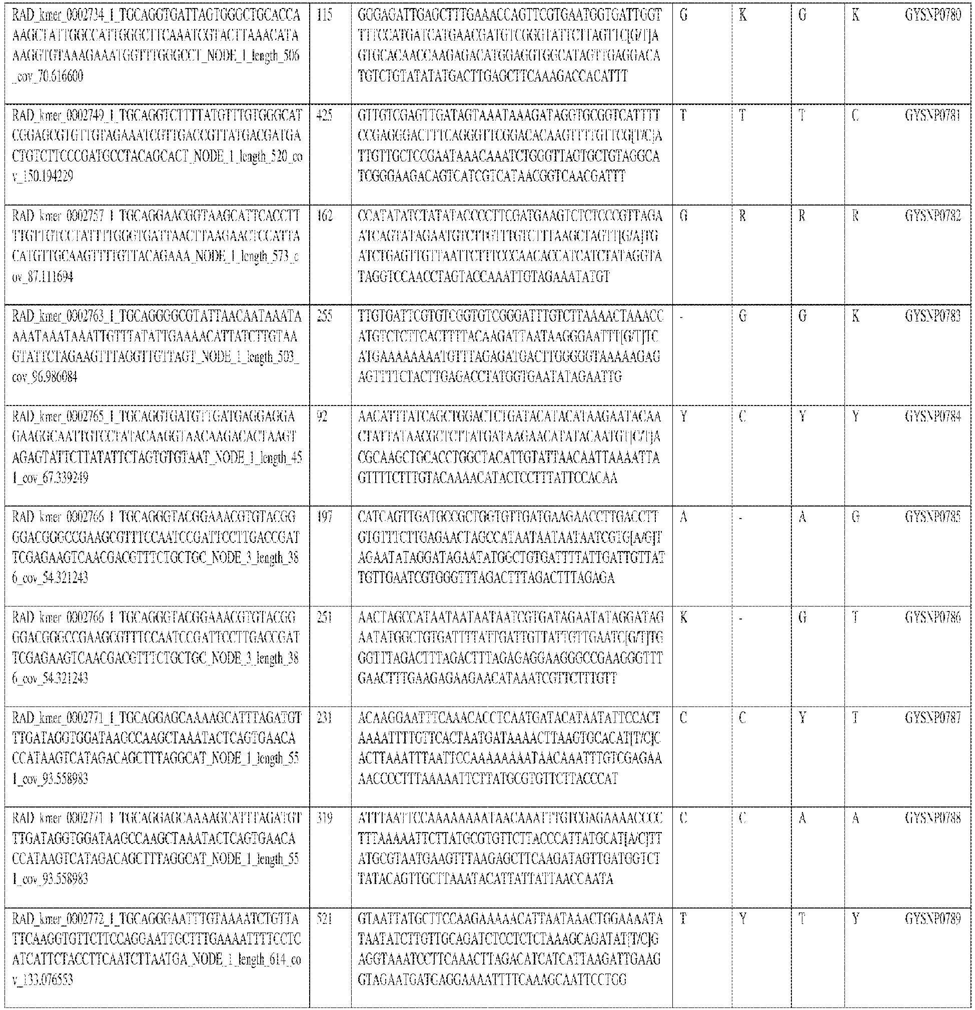

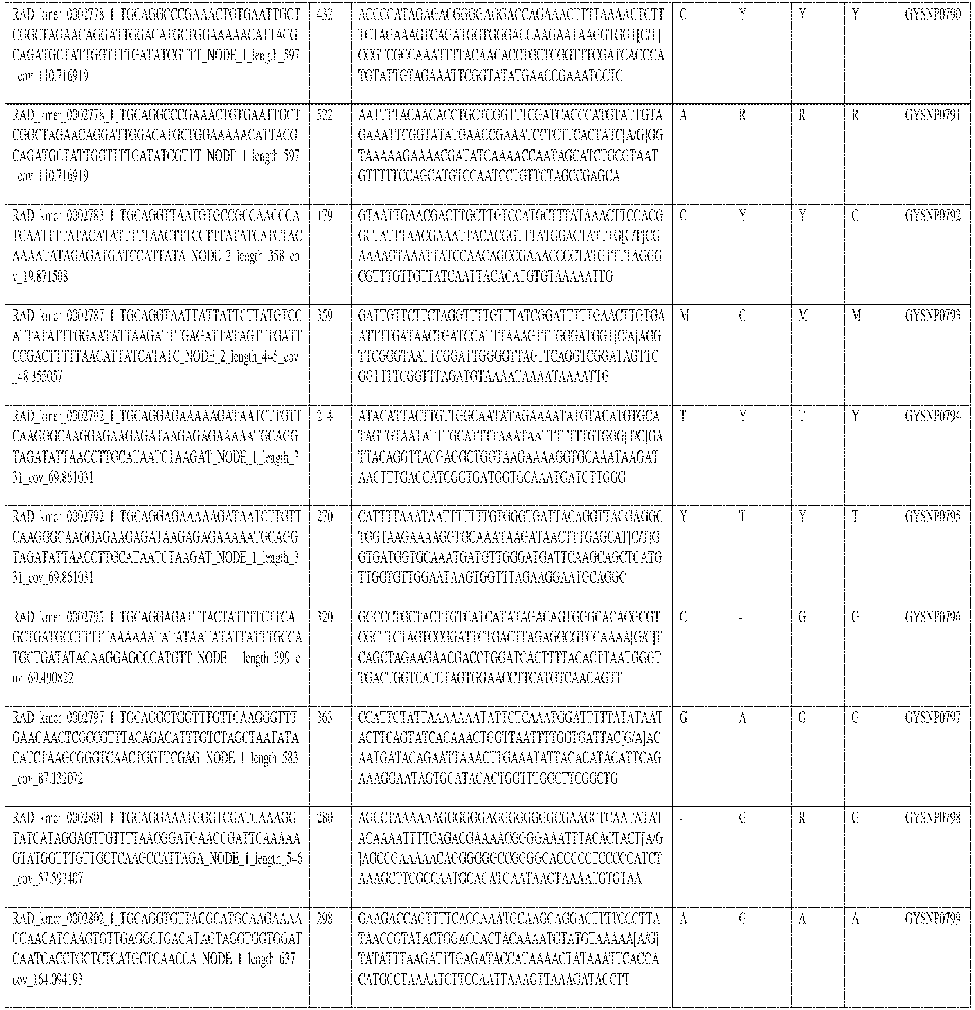

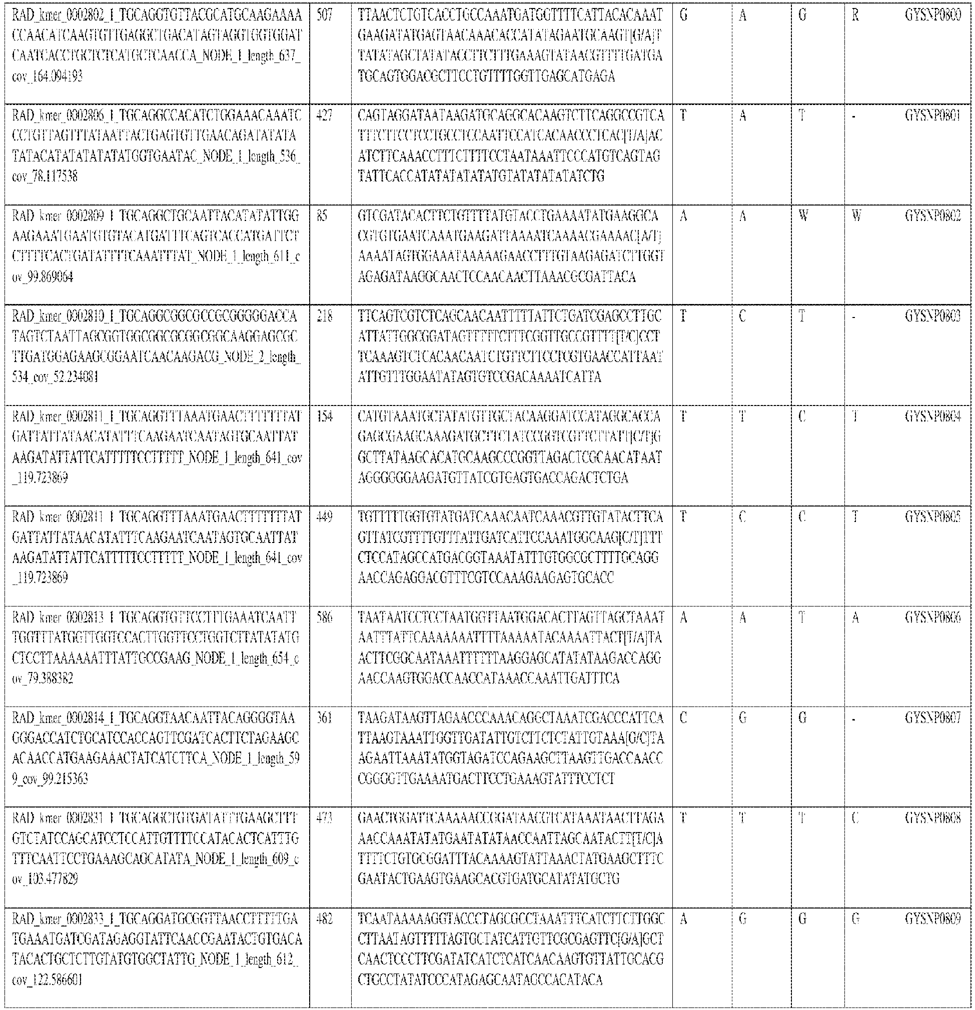

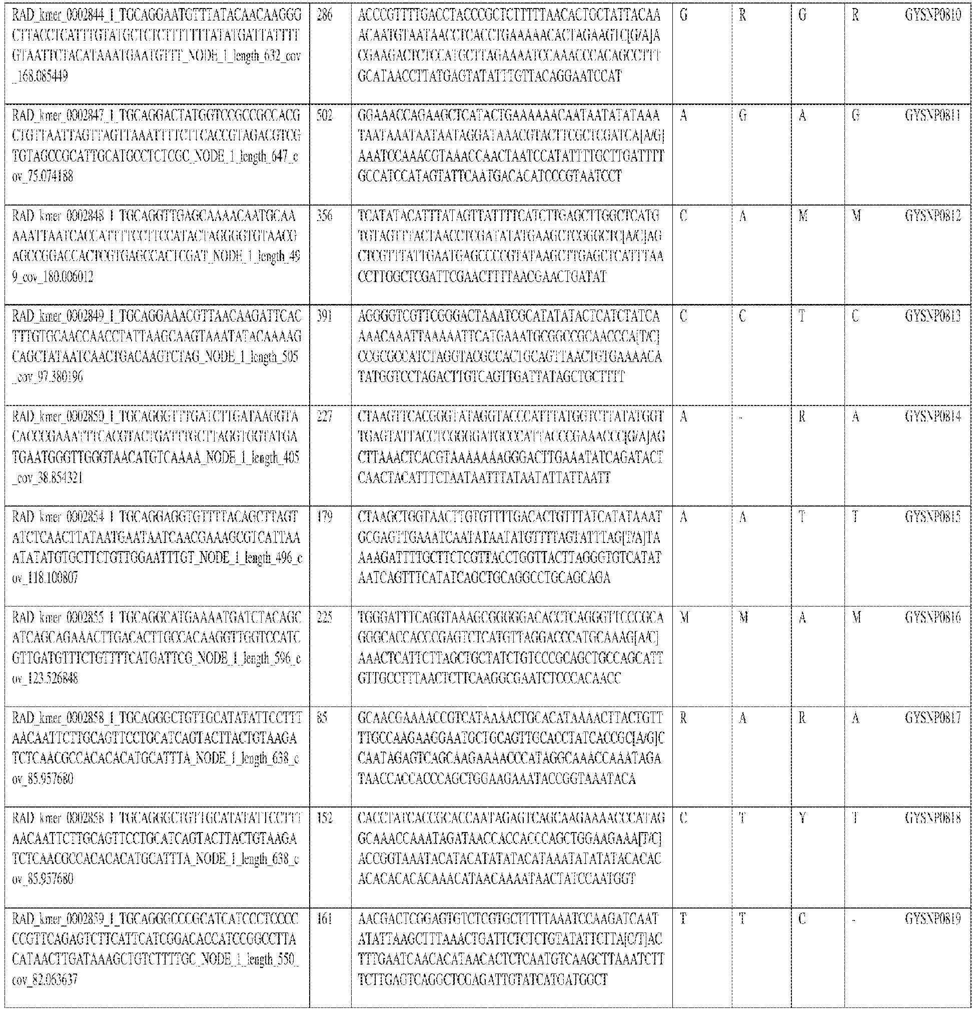

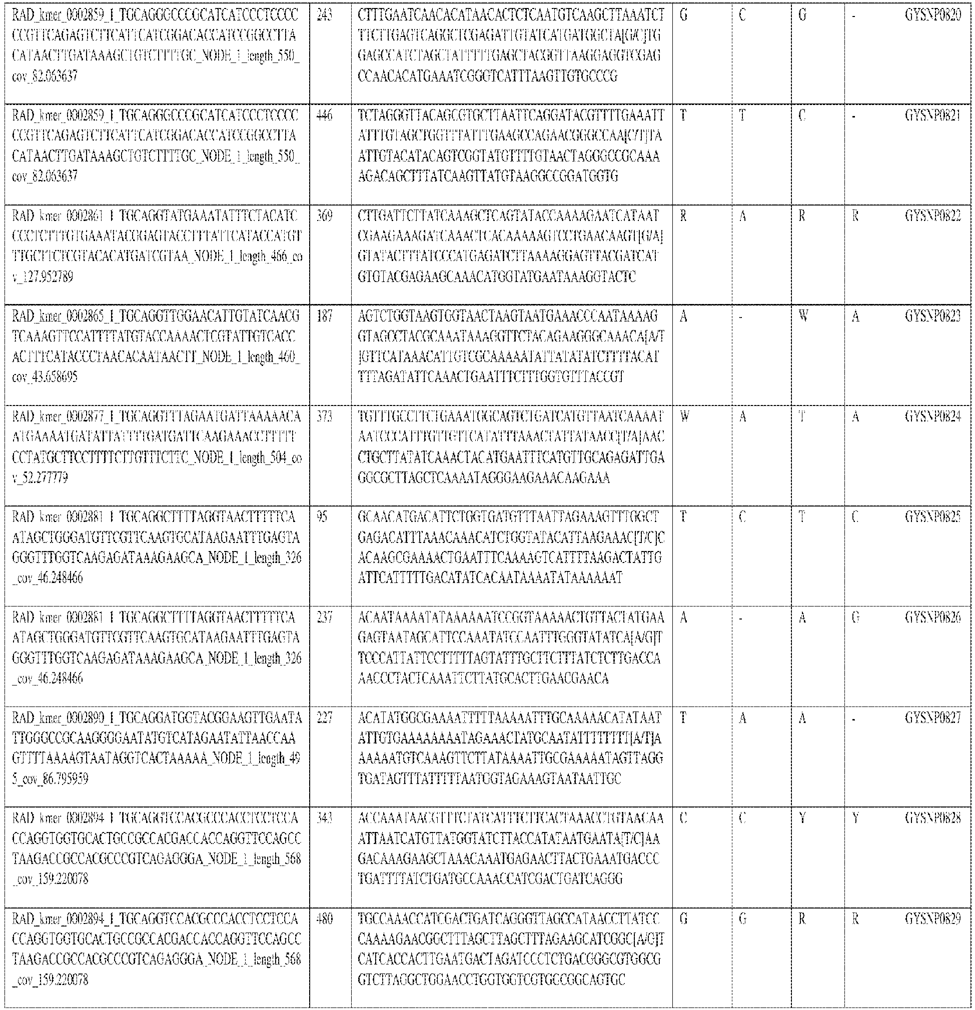

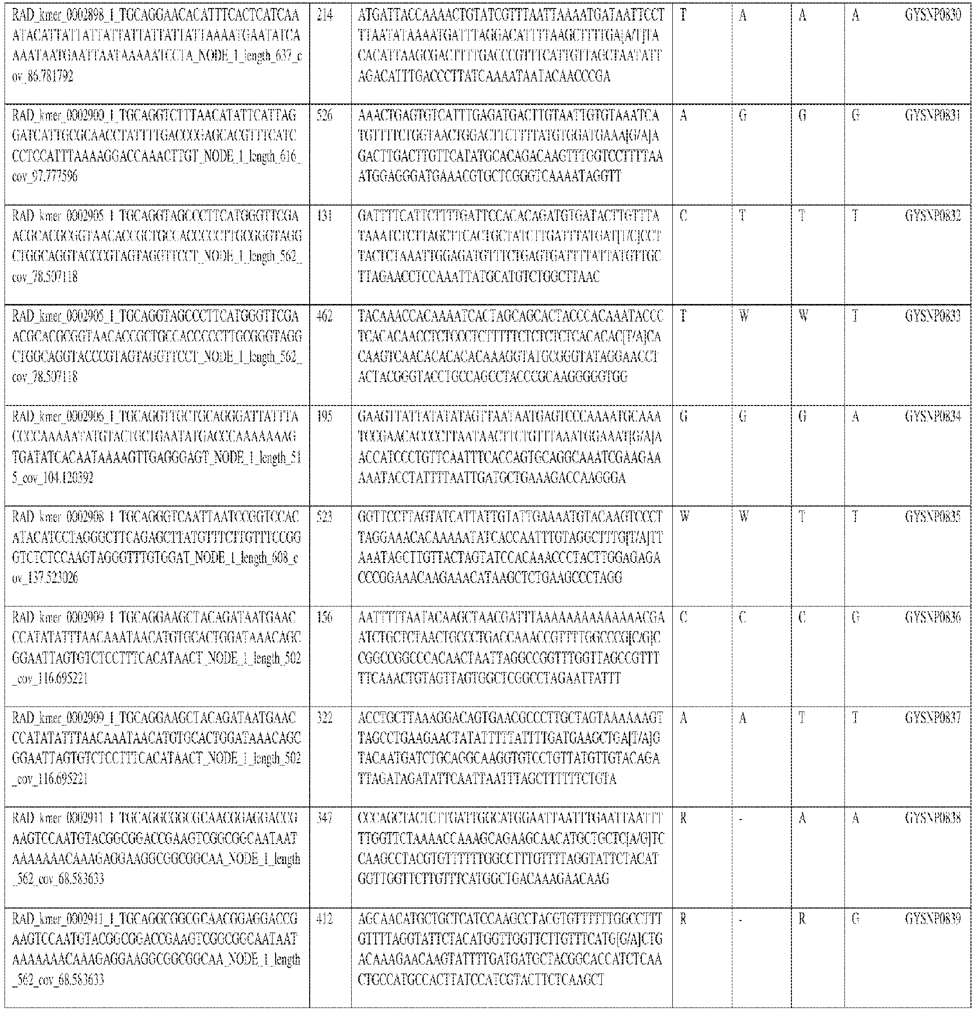

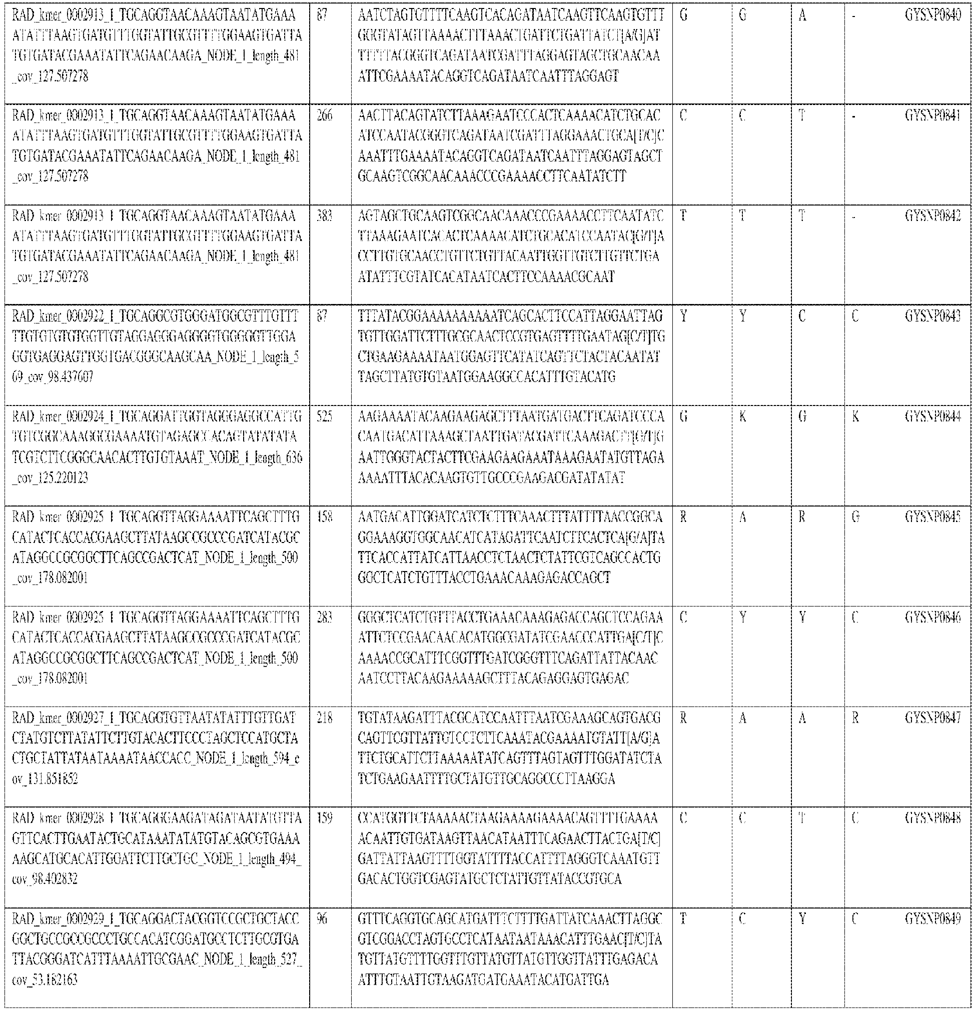

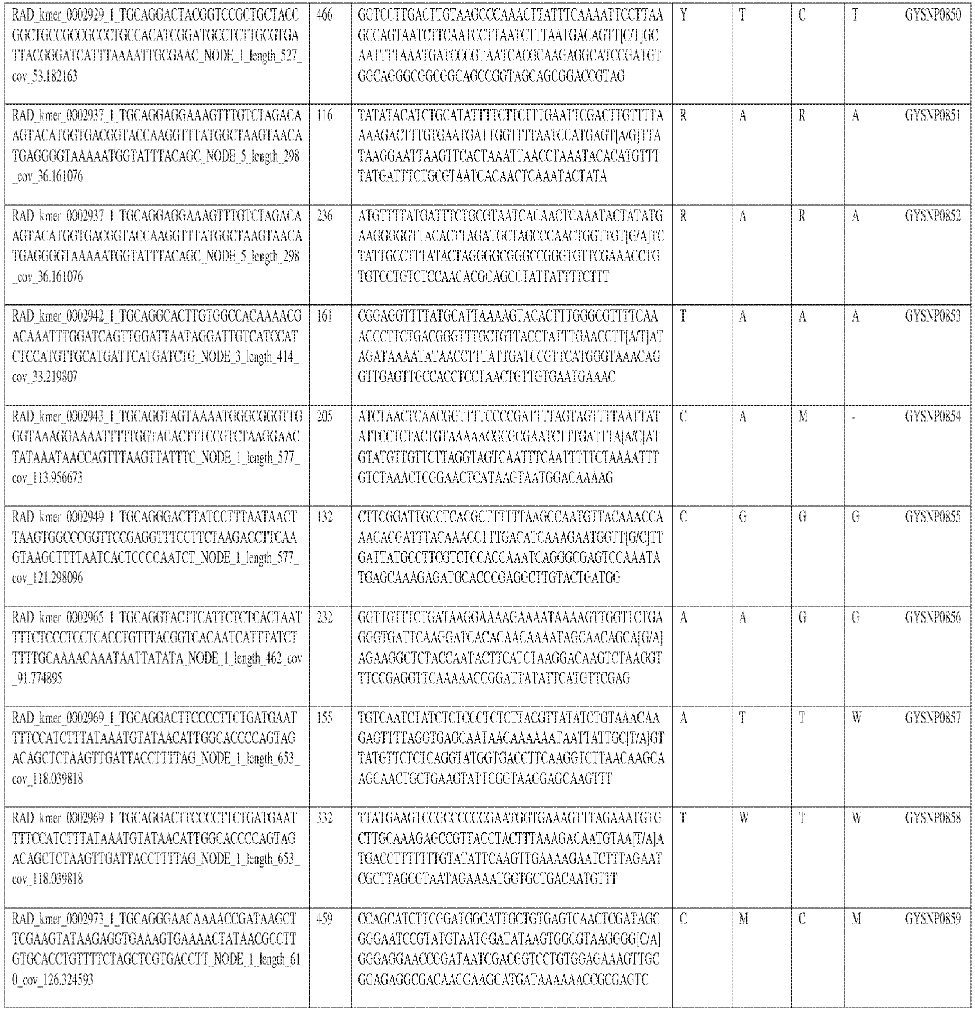

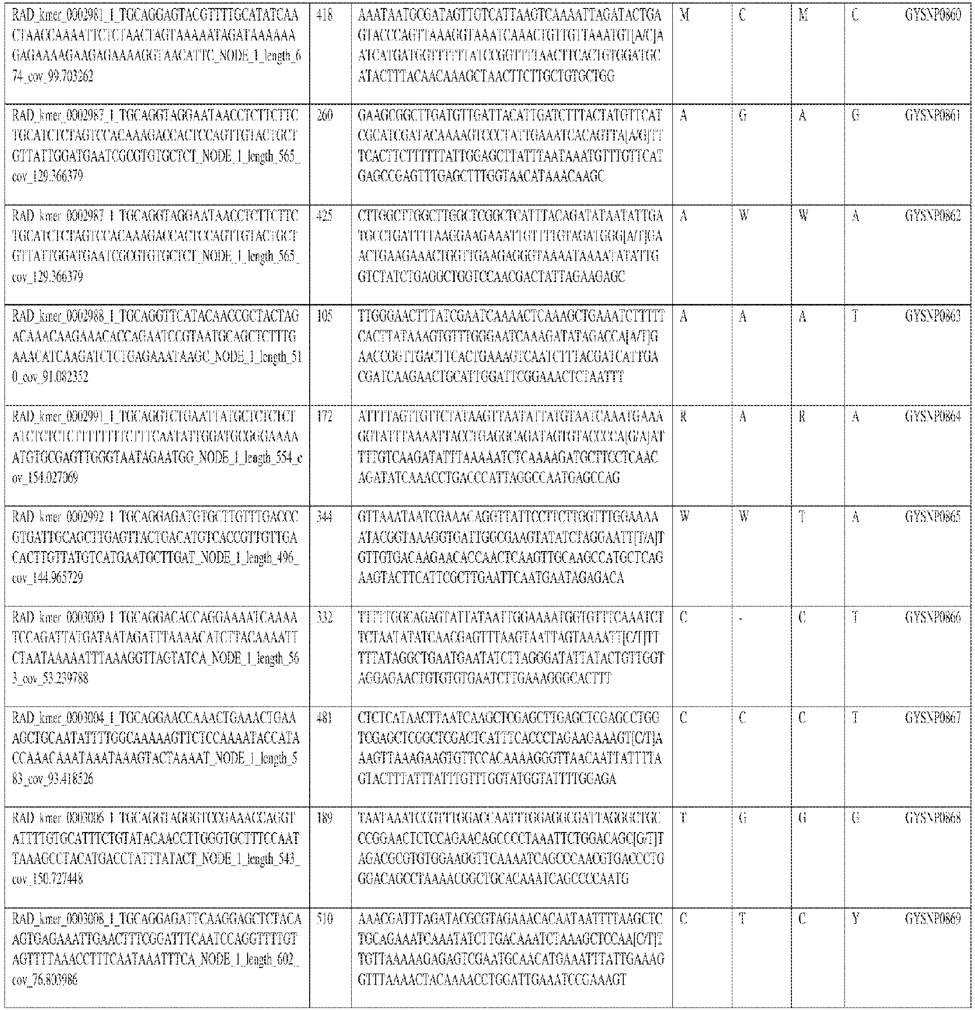

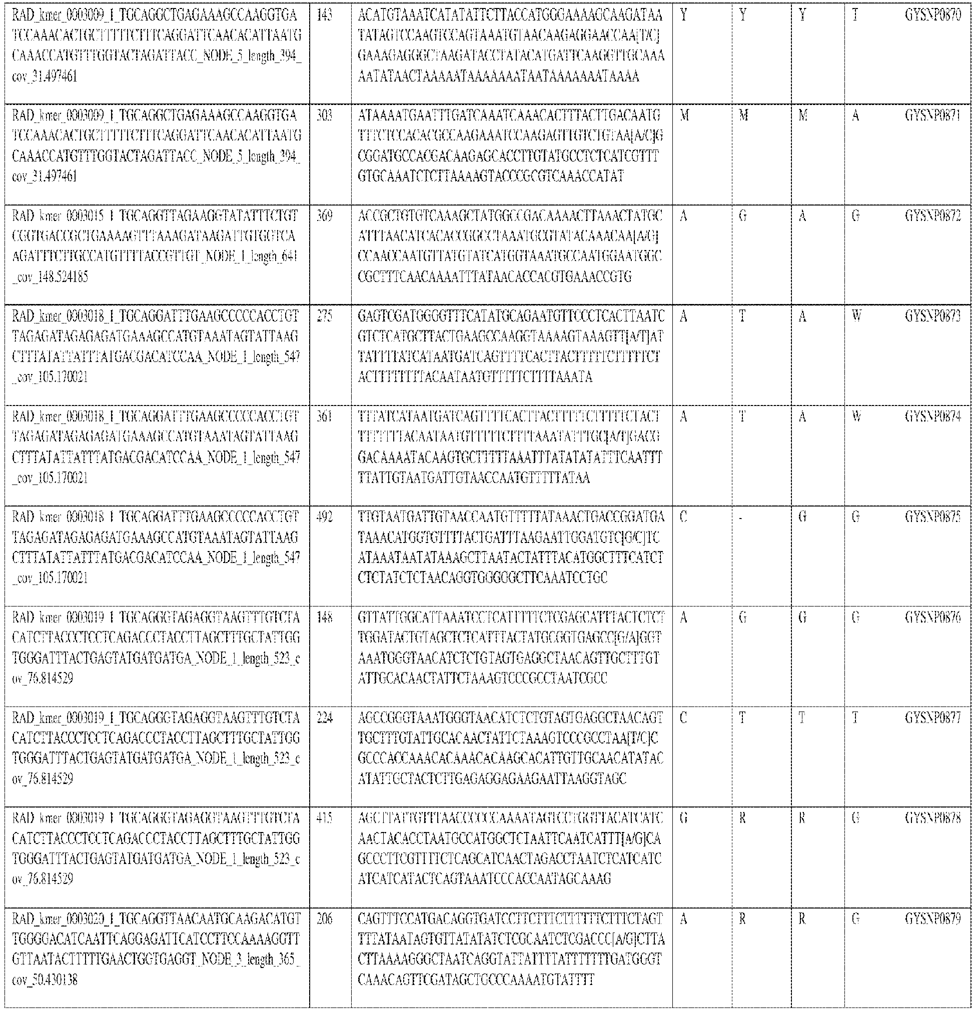

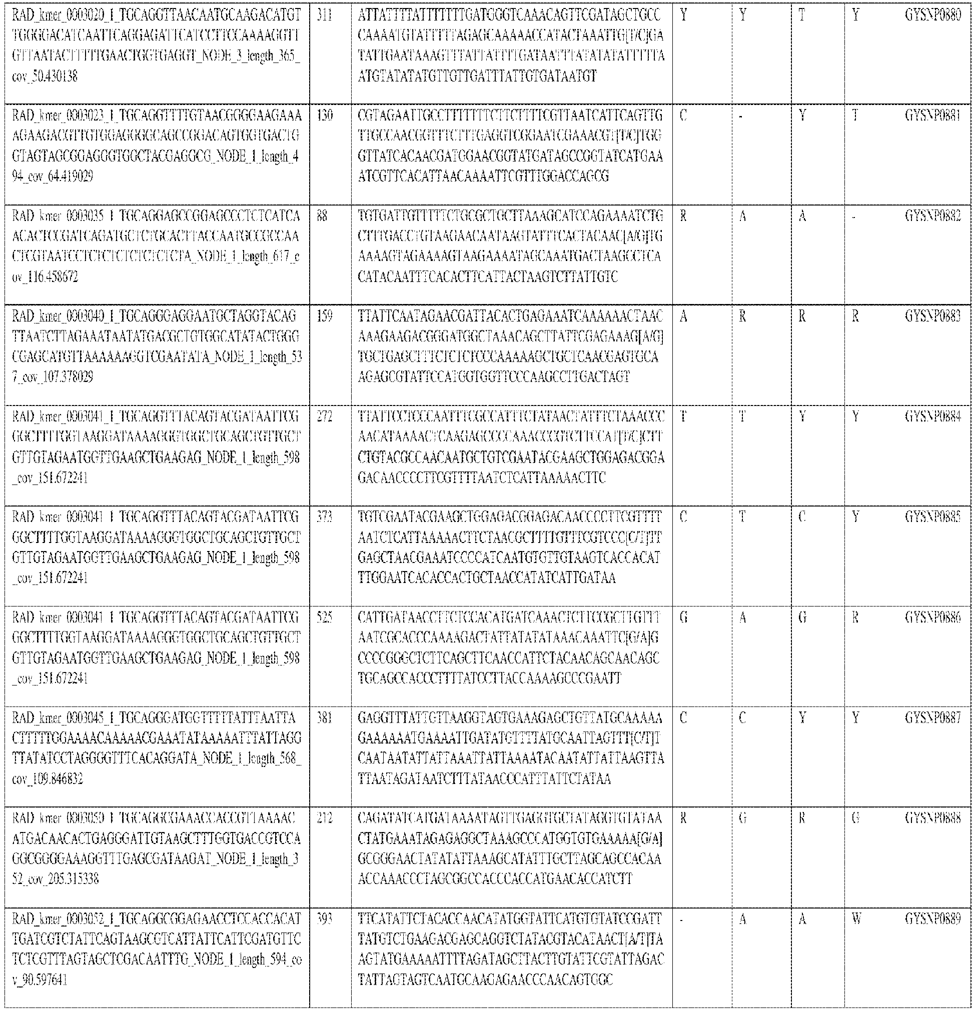

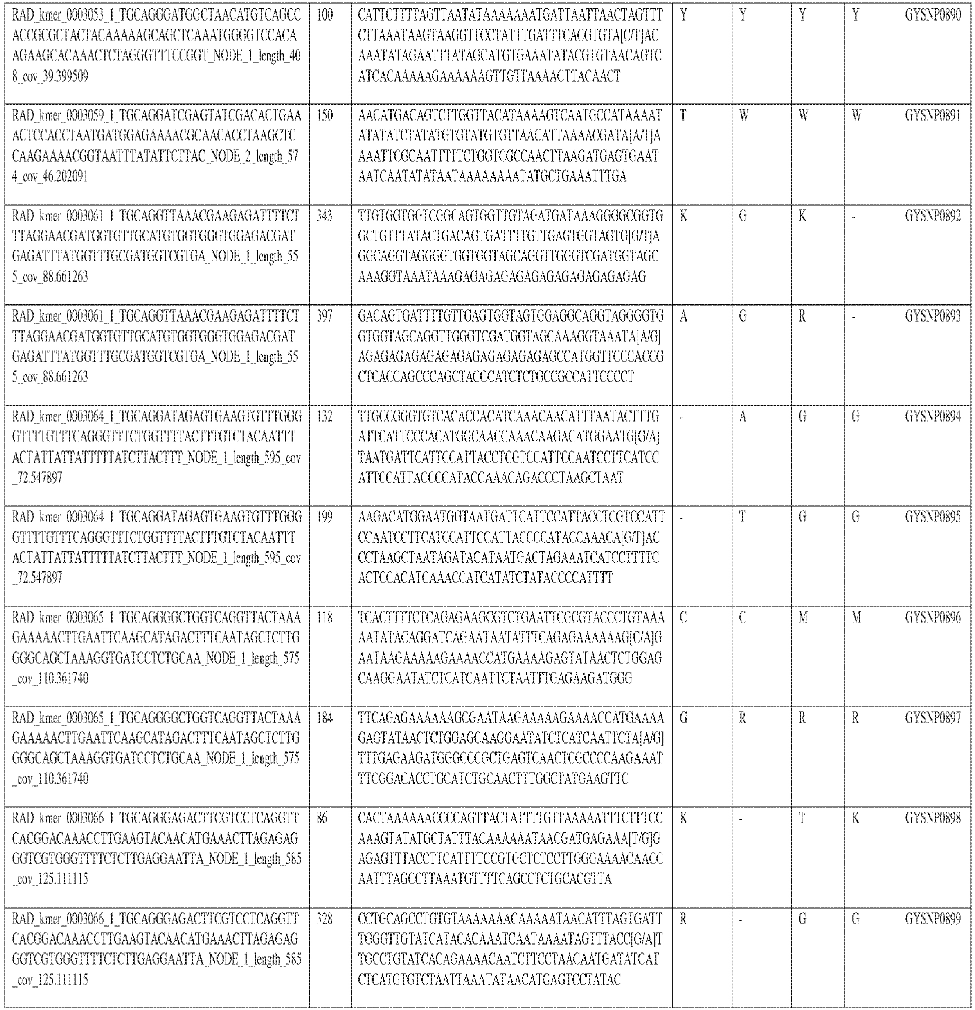

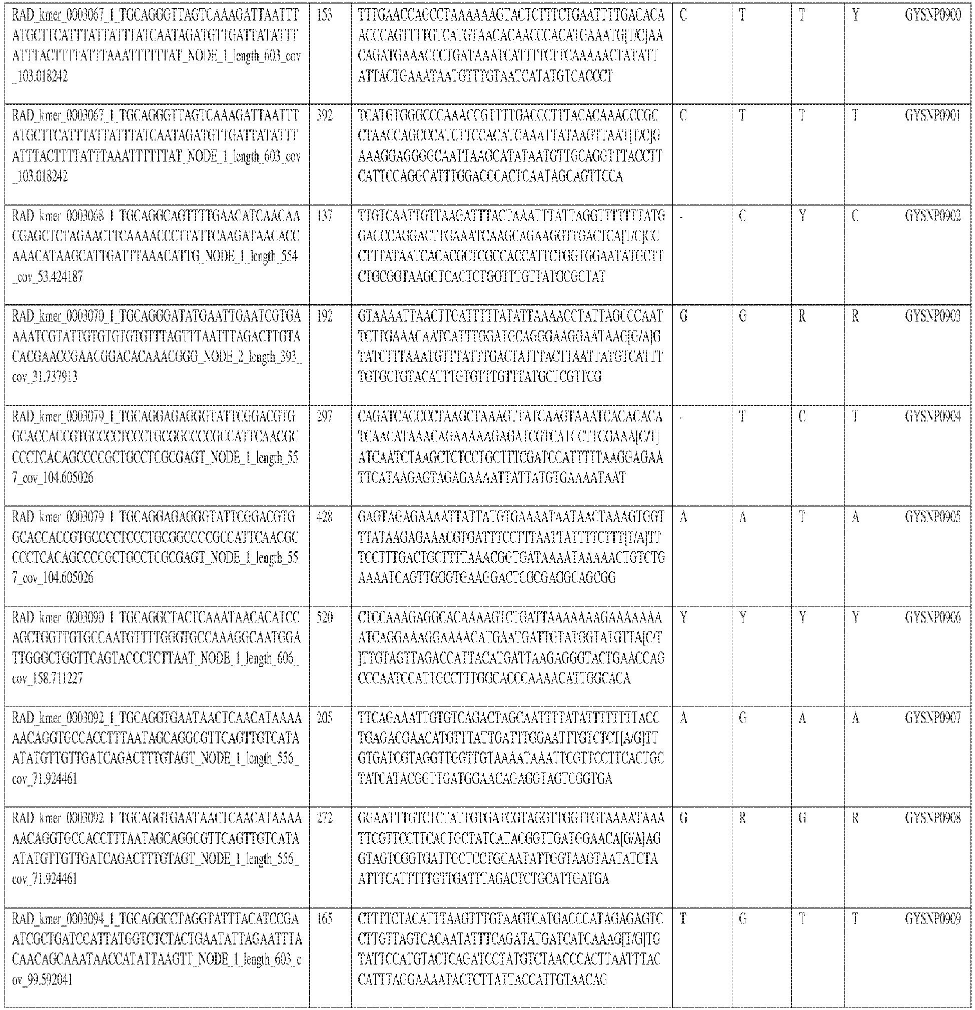

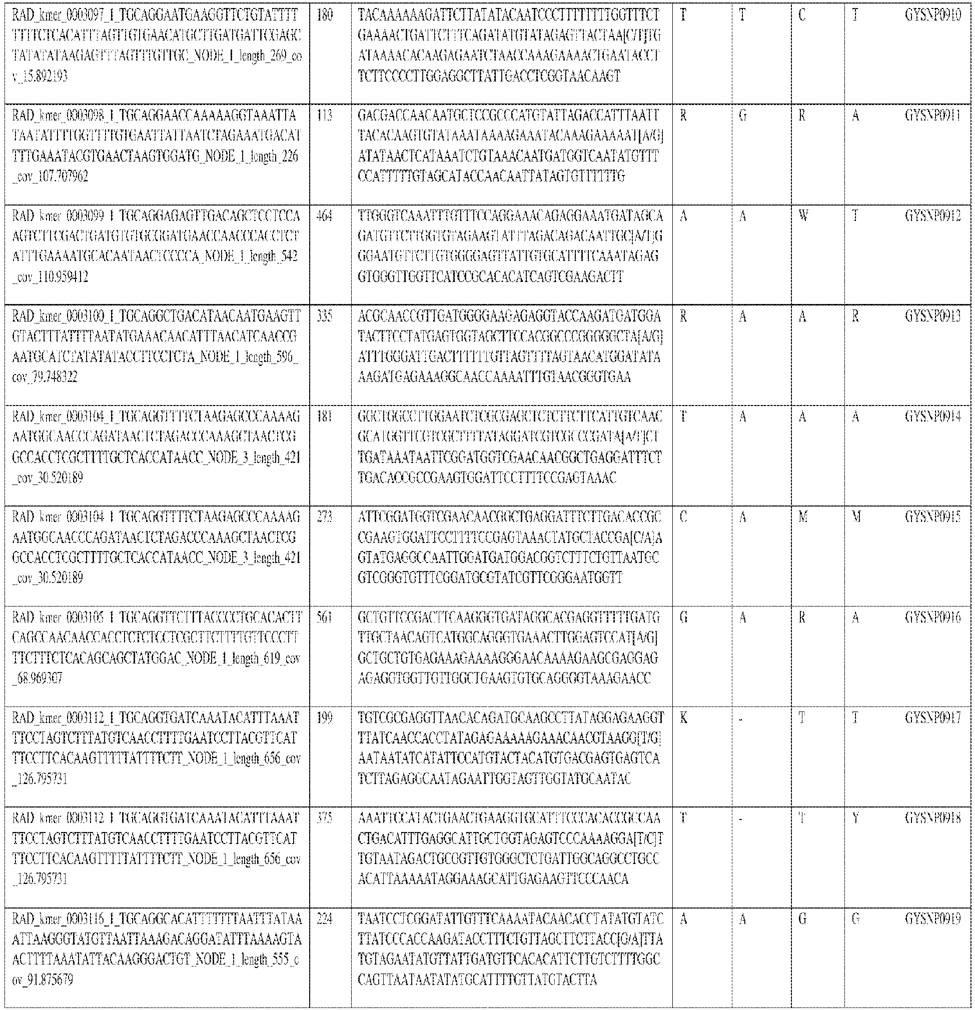

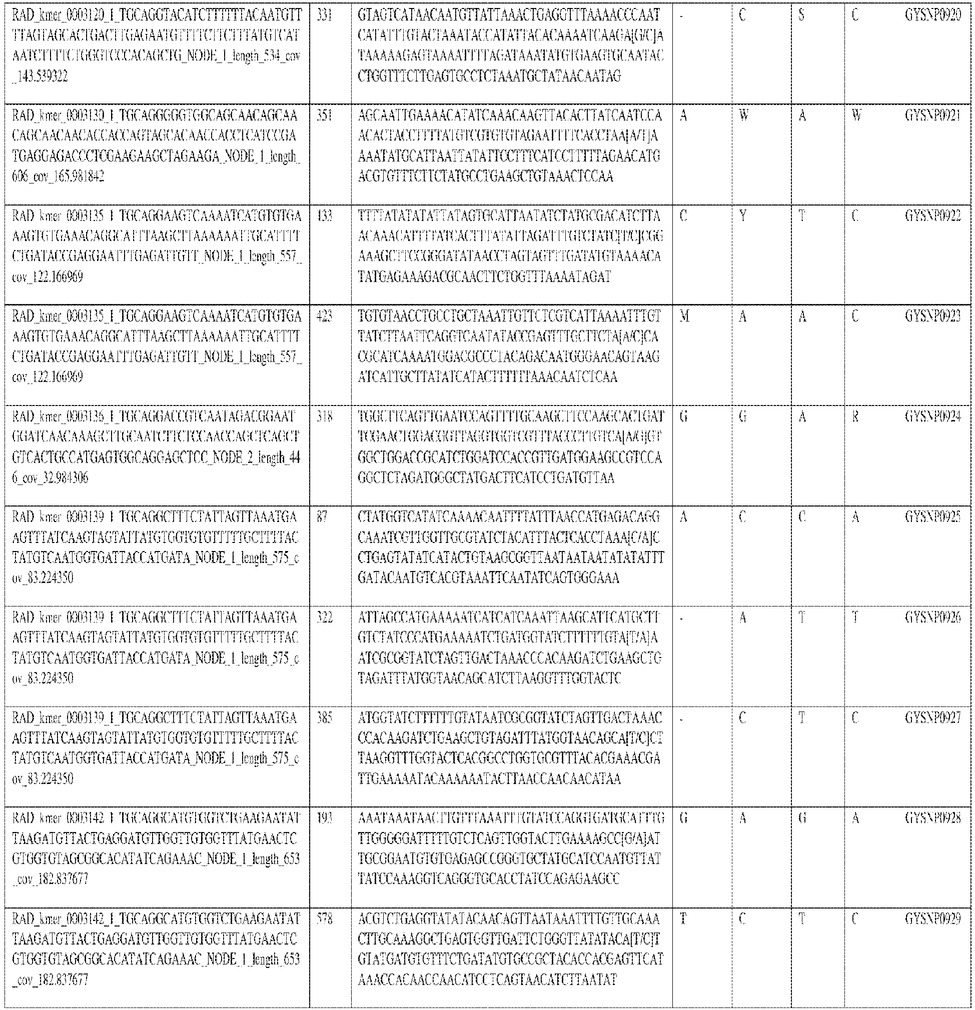

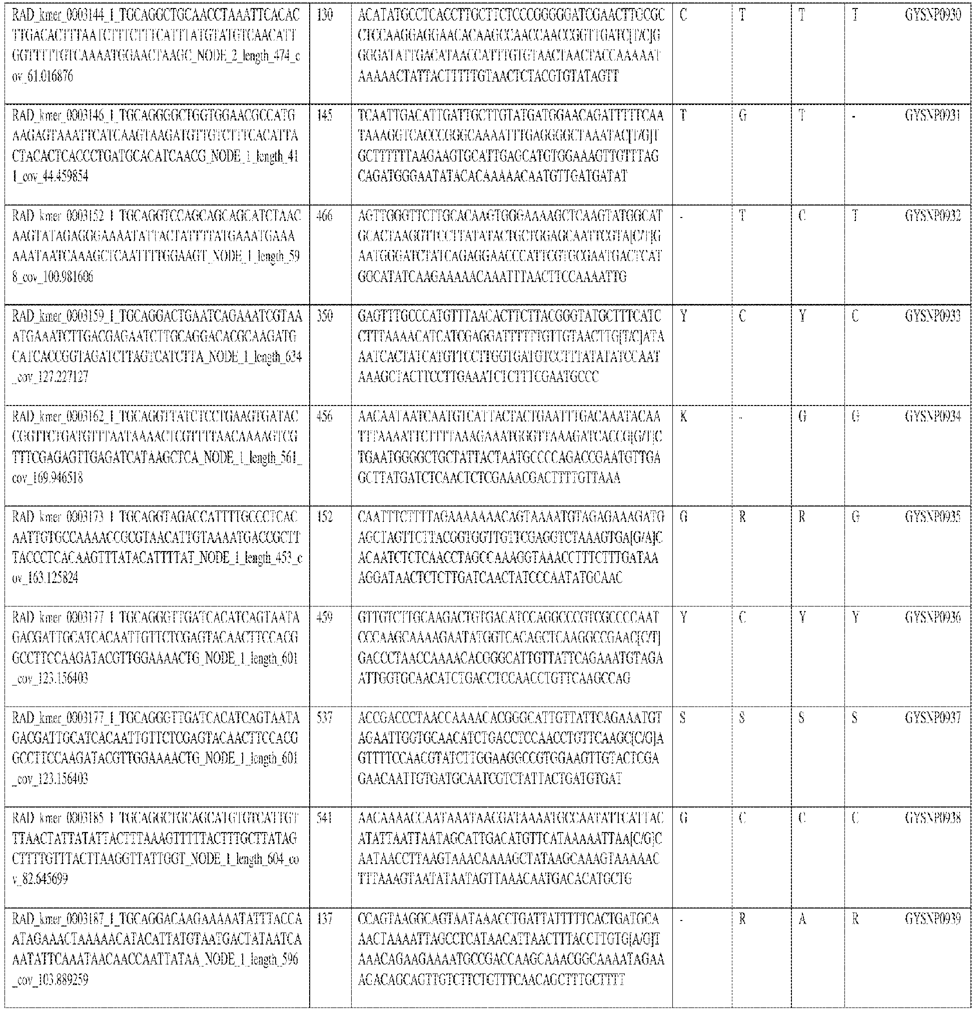

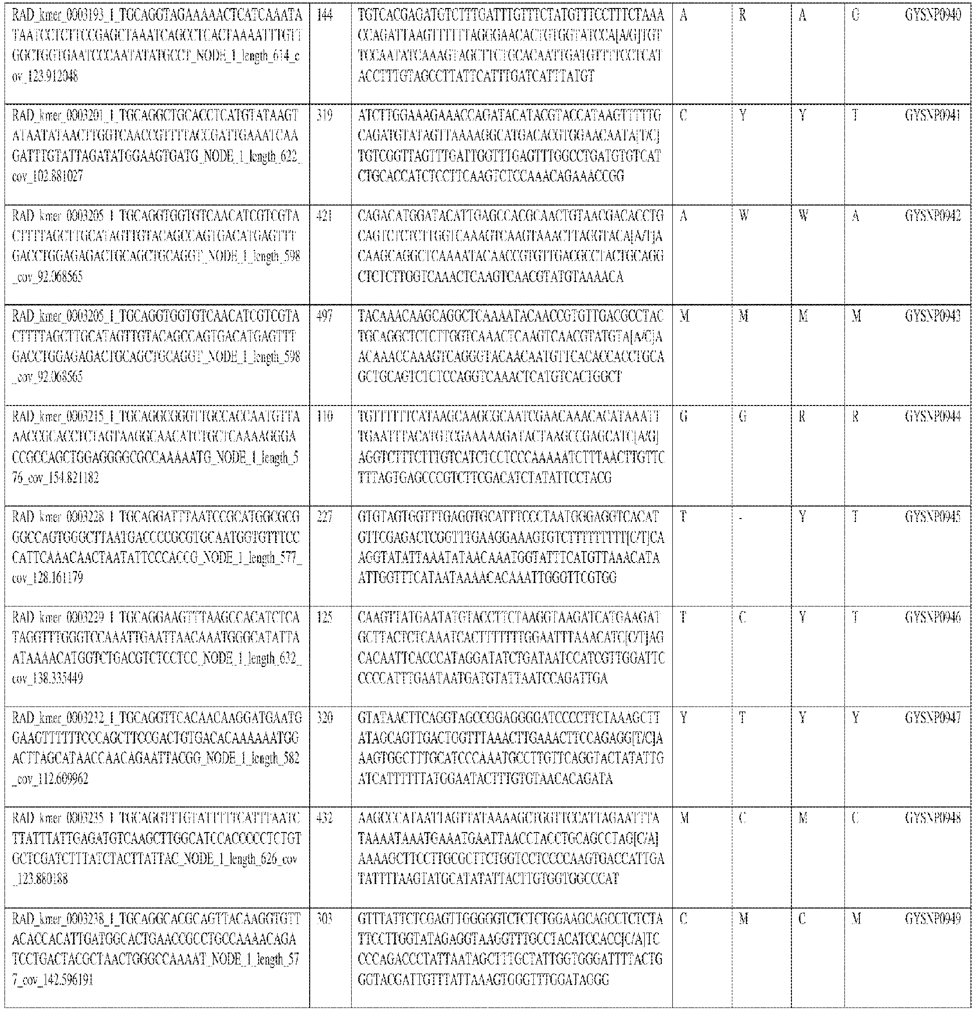

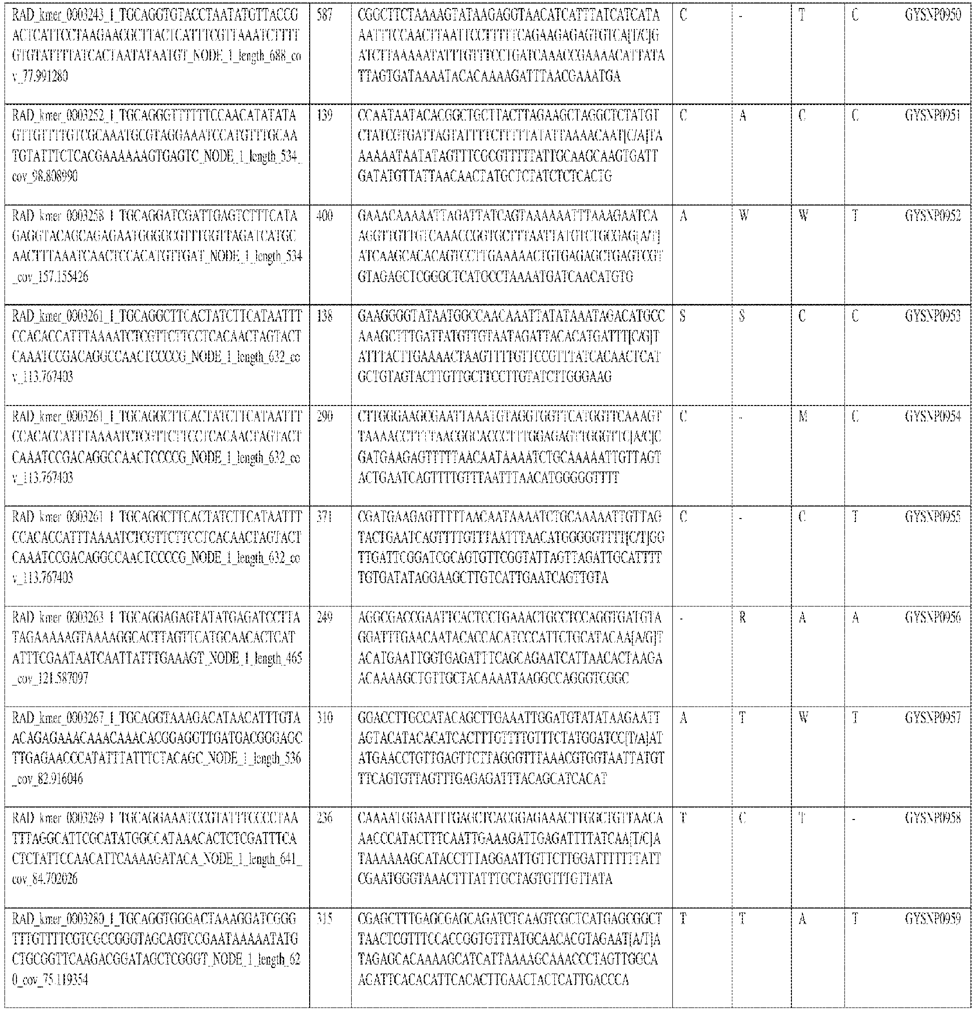

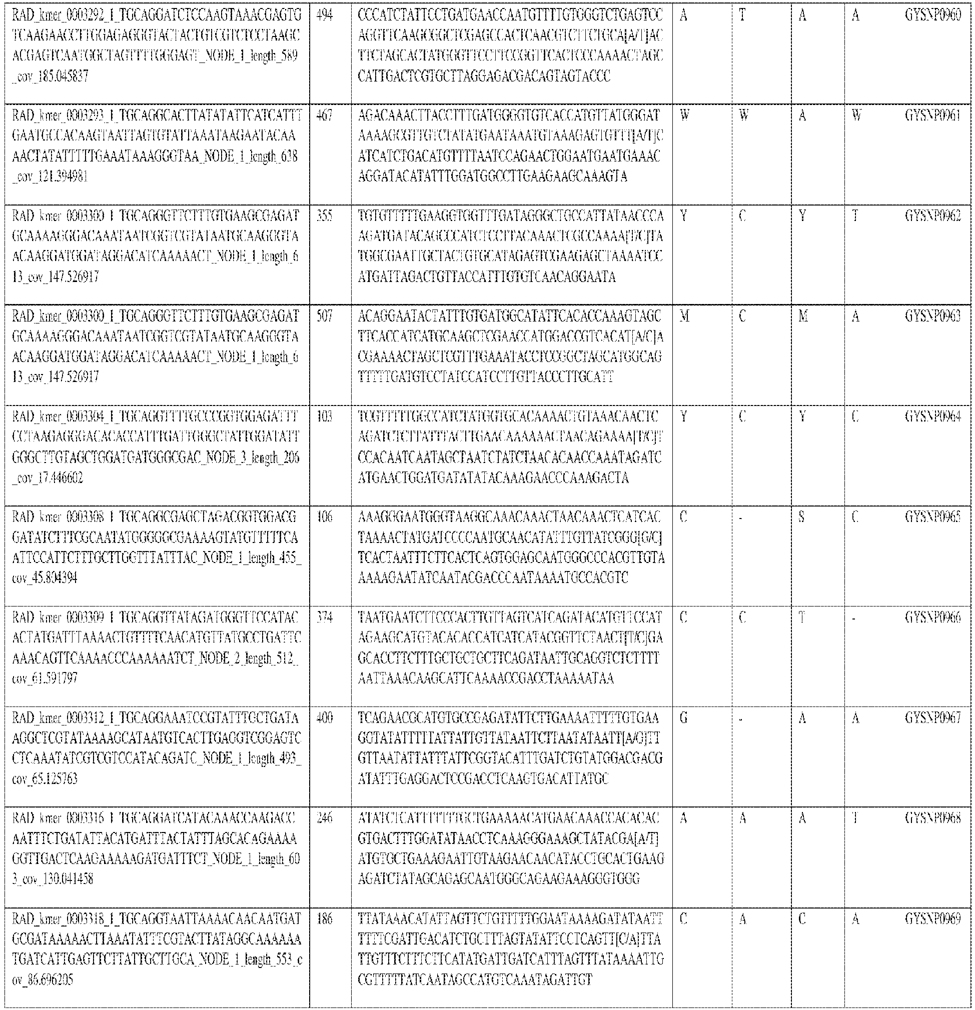

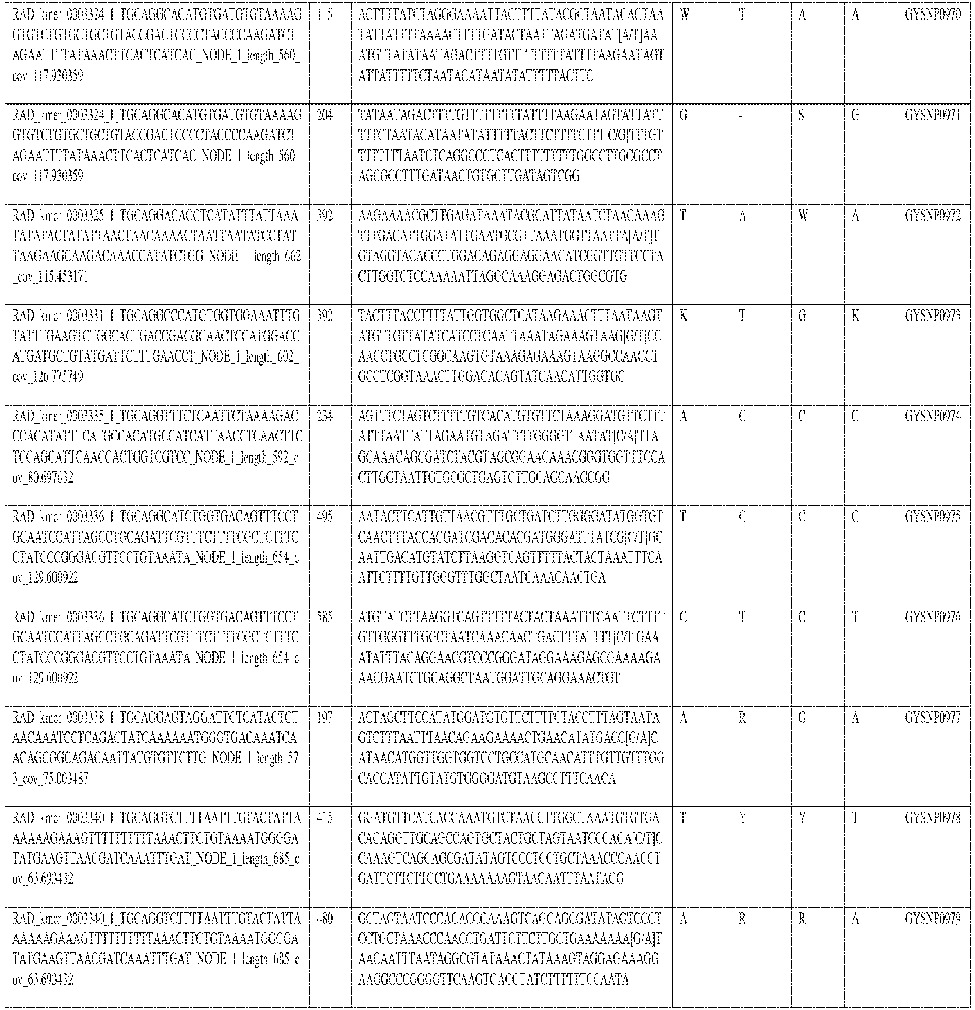

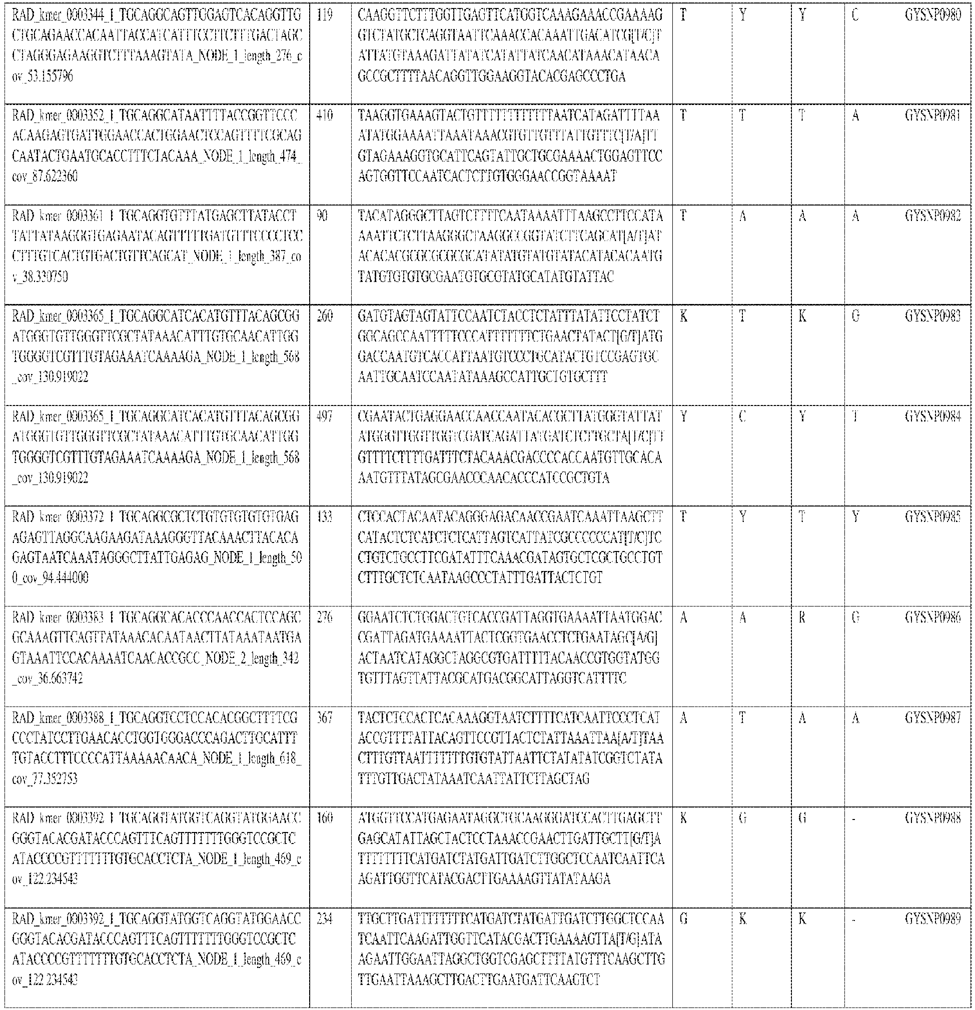

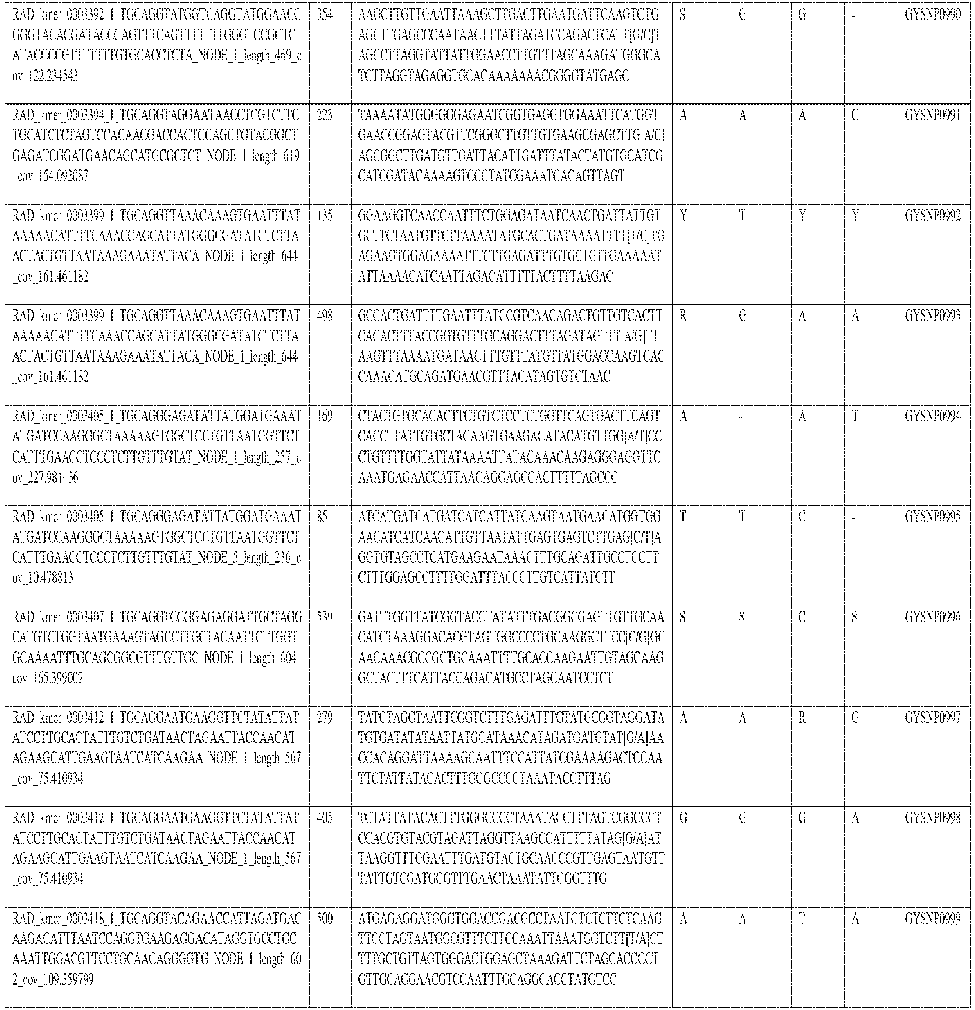

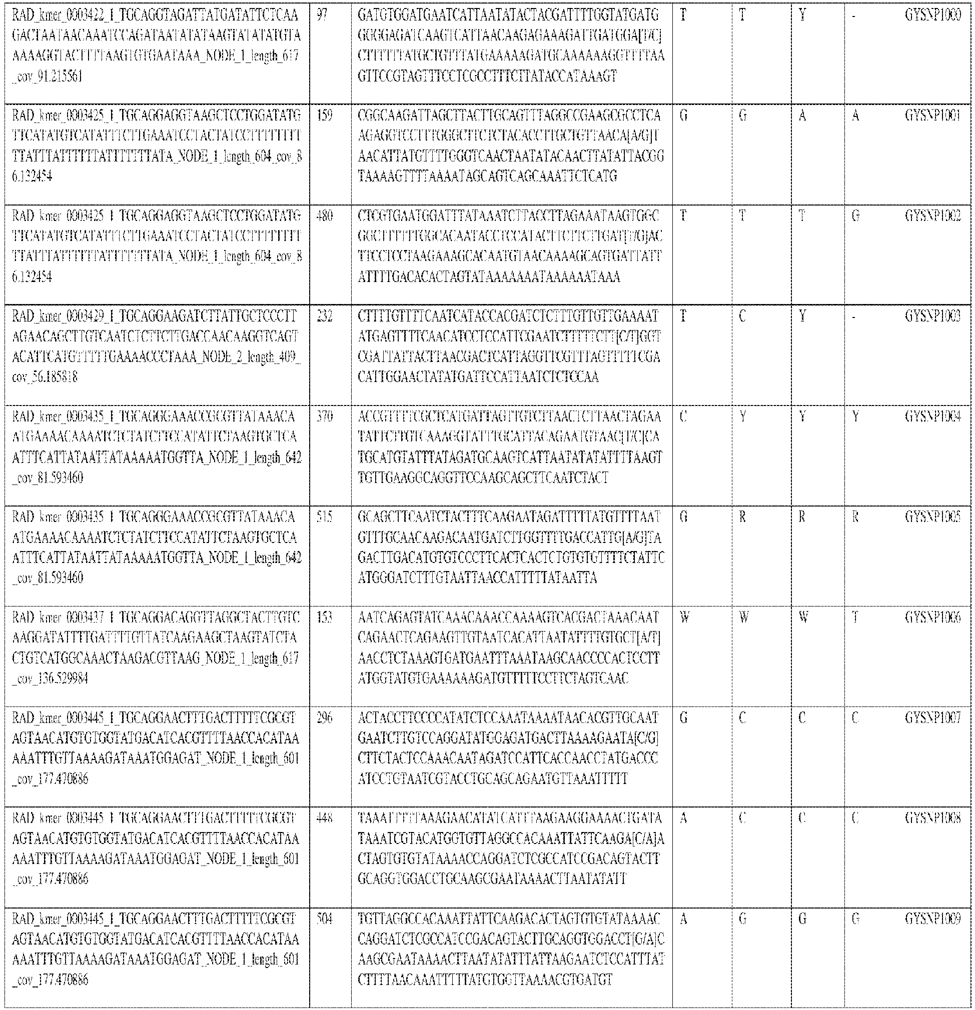

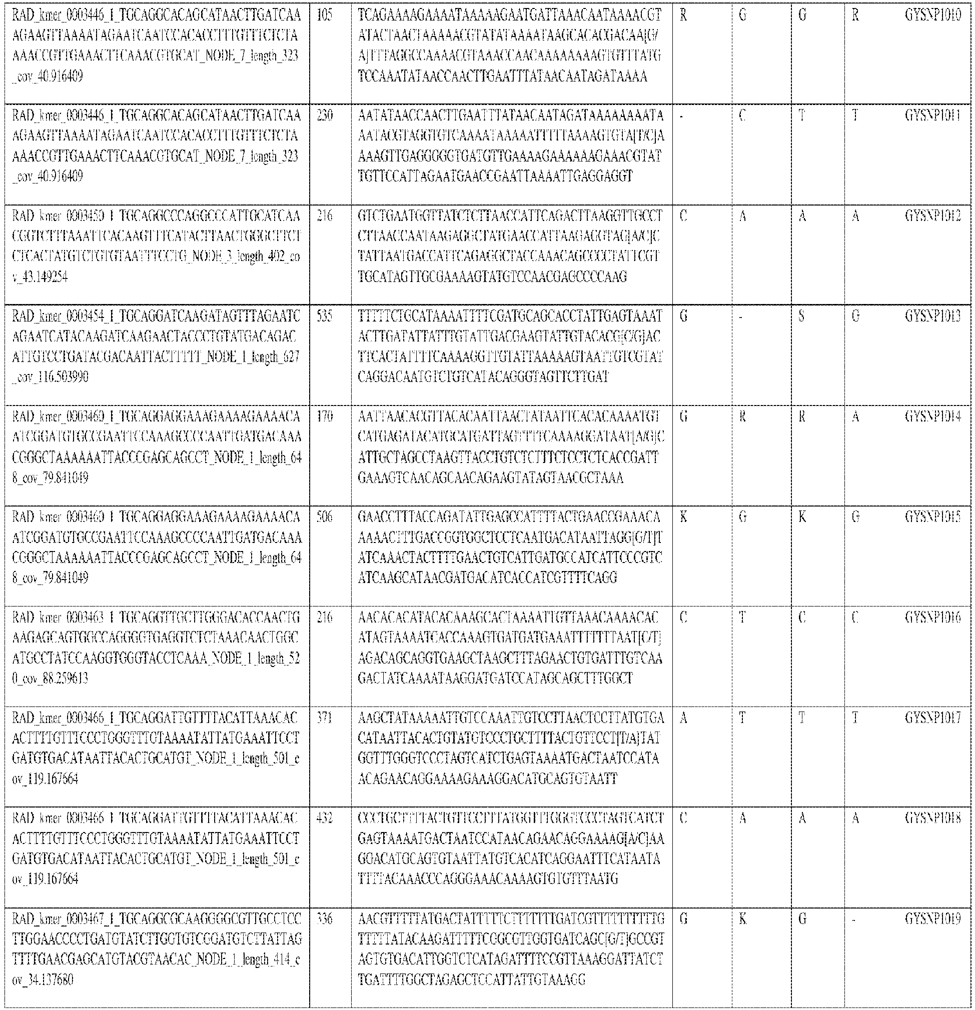

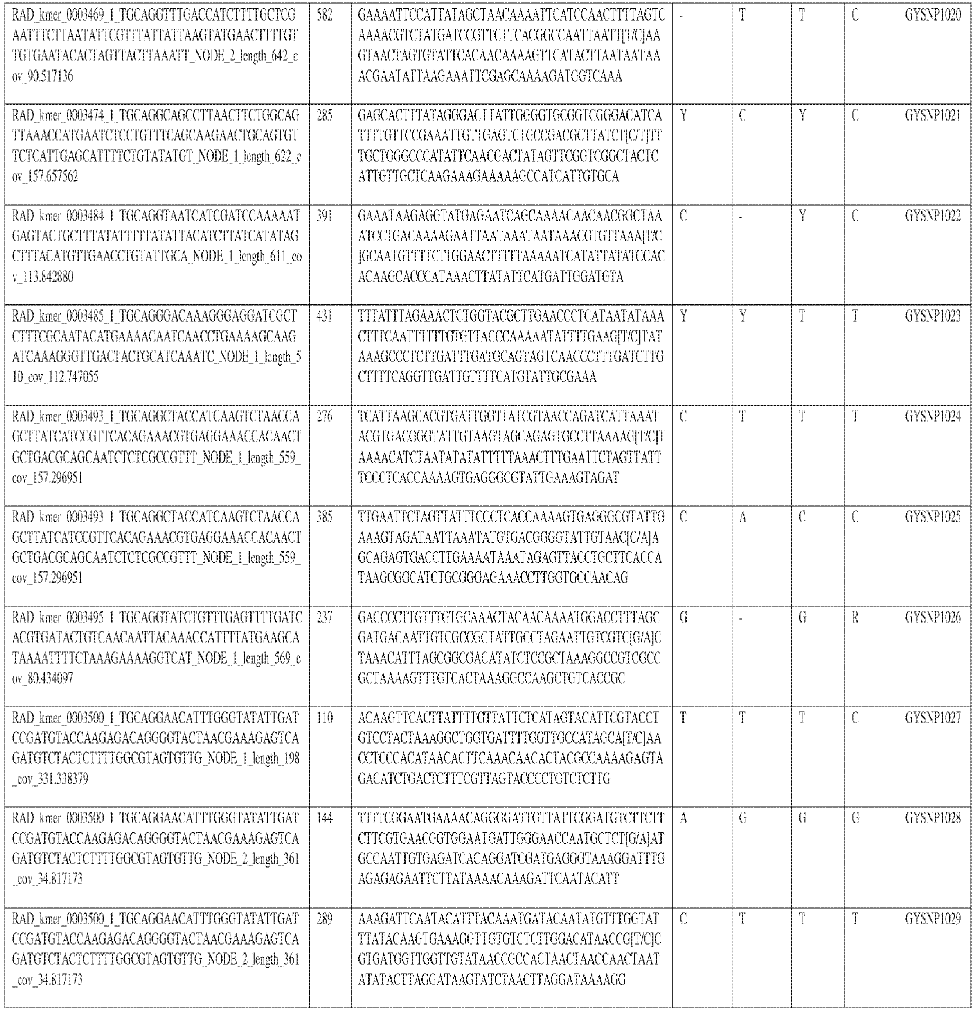

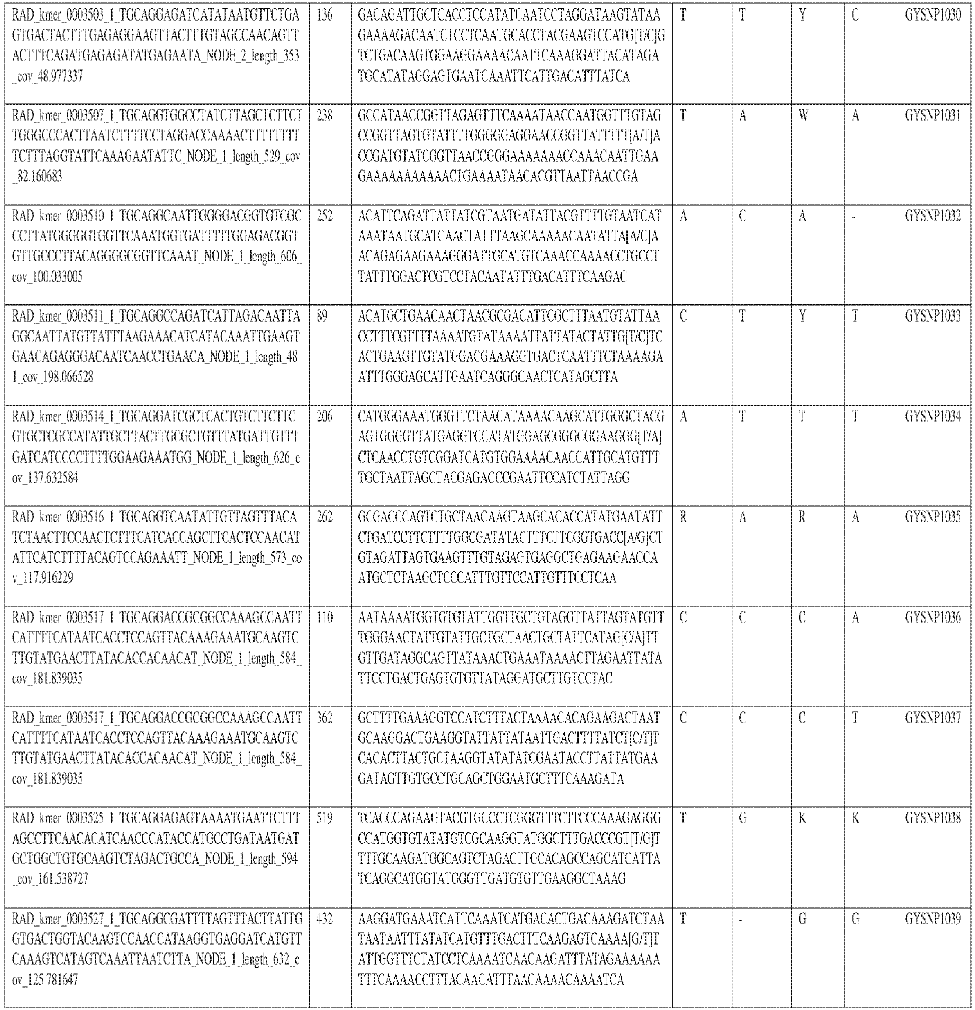

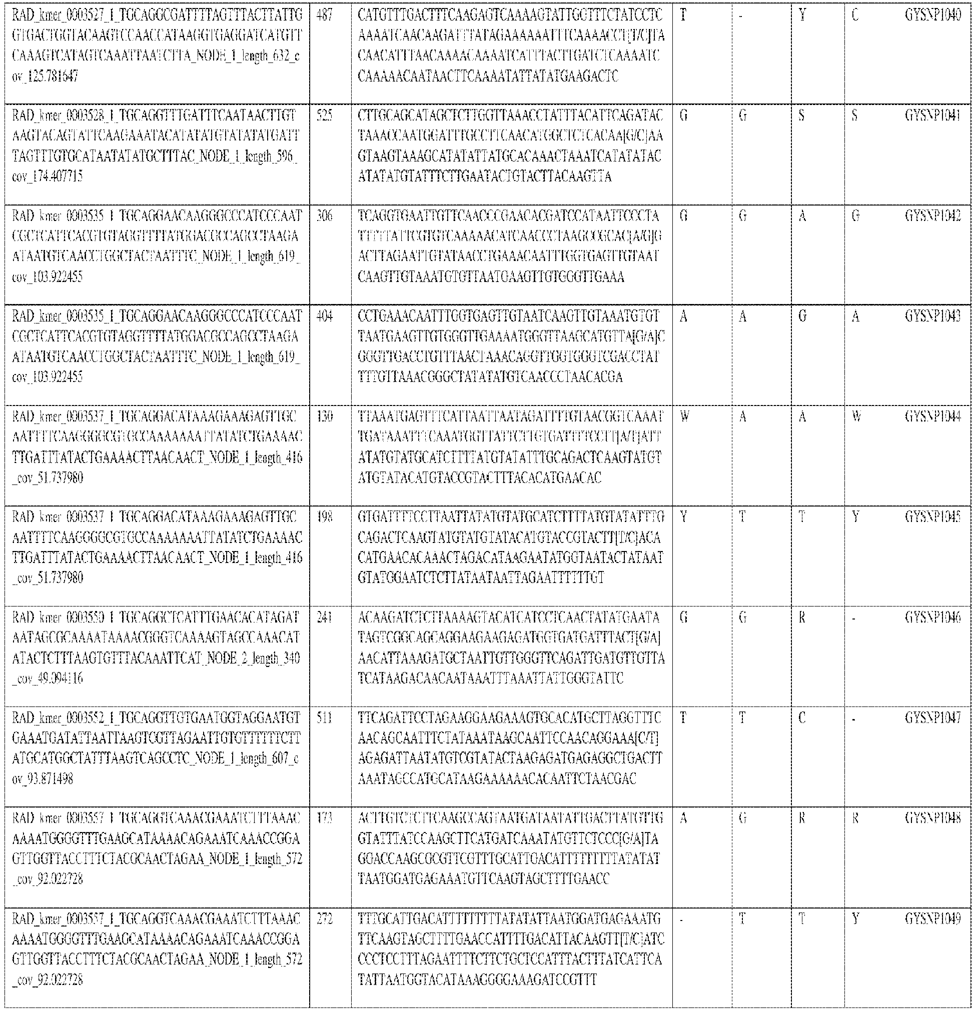

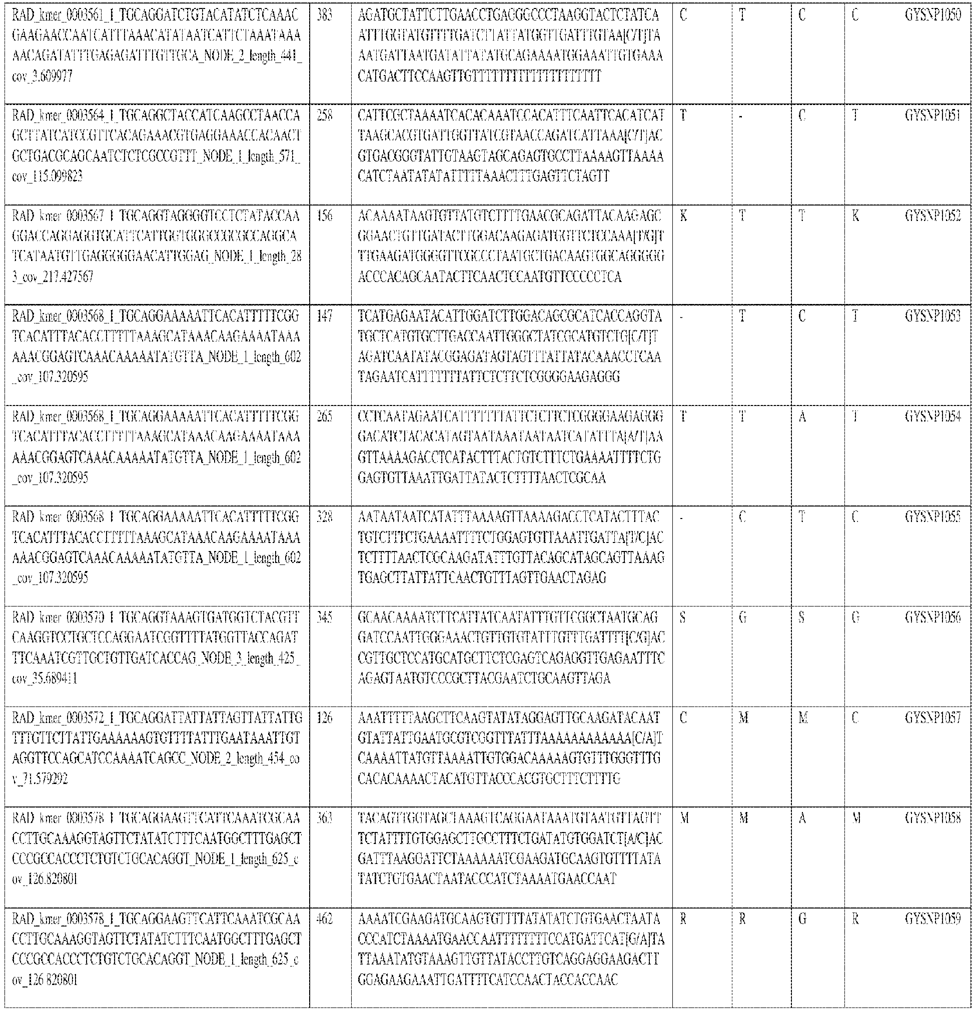

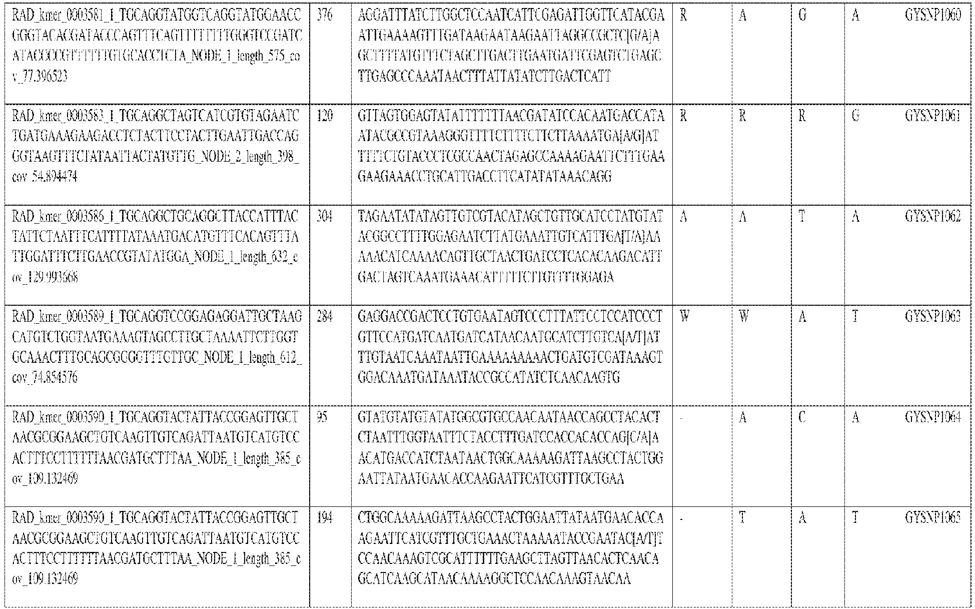

| TABLE 10 Information Regarding Guayule SNP Molecular Markers | | | | | | |
|---|---|---|---|---|---|---|
| | | | | | | |

| **Marker** | **Major.Allele.Frquency** | **GenotypeNo** | **No. of obs.** | **GeneDiversity** | **Heterozygosity** | **PIC** |
|---|---|---|---|---|---|---|
| SNP001 | 0.500 | 1 | 12 | 0.500 | 1.000 | 0.375 |
| SNP002 | 0.500 | 1 | 12 | 0.500 | 1.000 | 0.375 |
| SNP003 | 1.000 | 1 | 12 | 0.000 | 0.000 | 0.000 |
| SNP004 | 1.000 | 1 | 12 | 0.000 | 0.000 | 0.000 |
| SNP005 | 1.000 | 1 | 12 | 0.000 | 0.000 | 0.000 |
| SNP006 | 0.542 | 3 | 12 | 0.497 | 0.417 | 0.373 |
| SNP007 | 0.500 | 1 | 12 | 0.500 | 1.000 | 0.375 |
| SNP008 | 1.000 | 1 | 11 | 0.000 | 0.000 | 0.000 |
| SNP009 | 0.958 | 2 | 12 | 0.080 | 0.083 | 0.077 |
| SNP010 | 1.000 | 1 | 12 | 0.000 | 0.000 | 0.000 |
| SNP011 | 0.792 | 3 | 12 | 0.330 | 0.250 | 0.275 |
| SNP012 | 0.958 | 2 | 12 | 0.080 | 0.083 | 0.077 |
| SNP013 | 0.792 | 2 | 12 | 0.330 | 0.417 | 0.275 |
| SNP014 | 0.958 | 2 | 12 | 0.080 | 0.083 | 0.077 |
| SNP015 | 0.500 | 1 | 12 | 0.500 | 1.000 | 0.375 |
| SNP016 | 0.792 | 3 | 12 | 0.330 | 0.083 | 0.275 |
| SNP017 | 1.000 | 1 | 12 | 0.000 | 0.000 | 0.000 |
| SNP018 | 0.583 | 2 | 12 | 0.486 | 0.833 | 0.368 |
| SNP019 | 0.500 | 1 | 12 | 0.500 | 1.000 | 0.375 |
| SNP020 | 0.500 | 1 | 12 | 0.500 | 1.000 | 0.375 |
| SNP021 | 0.500 | 1 | 12 | 0.500 | 1.000 | 0.375 |
| SNP022 | 0.500 | 1 | 12 | 0.500 | 1.000 | 0.375 |
| SNP023 | 1.000 | 1 | 12 | 0.000 | 0.000 | 0.000 |
| SNP024 | 0.917 | 2 | 12 | 0.153 | 0.167 | 0.141 |
| SNP025 | 1.000 | 1 | 12 | 0.000 | 0.000 | 0.000 |
| SNP026 | 0.500 | 1 | 12 | 0.500 | 1.000 | 0.375 |
| SNP027 | 0.500 | 1 | 12 | 0.500 | 1.000 | 0.375 |
| SNP028 | 0.500 | 1 | 12 | 0.500 | 1.000 | 0.375 |
| SNP029 | 1.000 | 1 | 12 | 0.000 | 0.000 | 0.000 |
| SNP030 | 1.000 | 1 | 12 | 0.000 | 0.000 | 0.000 |
| SNP031 | 1.000 | 1 | 12 | 0.000 | 0.000 | 0.000 |
| SNP032 | 1.000 | 1 | 10 | 0.000 | 0.000 | 0.000 |
| SNP033 | 1.000 | 1 | 8 | 0.000 | 0.000 | 0.000 |
| SNP034 | 0.958 | 2 | 12 | 0.080 | 0.083 | 0.077 |
| SNP035 | 1.000 | 1 | 8 | 0.000 | 0.000 | 0.000 |
| SNP036 | 1.000 | 1 | 12 | 0.000 | 0.000 | 0.000 |
| SNP037 | 1.000 | 1 | 12 | 0.000 | 0.000 | 0.000 |
| SNP038 | 0.542 | 2 | 12 | 0.497 | 0.917 | 0.373 |
| SNP039 | 0.500 | 1 | 12 | 0.500 | 1.000 | 0.375 |
| SNP040 | 0.917 | 2 | 12 | 0.153 | 0.000 | 0.141 |
| SNP041 | 0.500 | 1 | 12 | 0.500 | 1.000 | 0.375 |
| SNP042 | 0.500 | 1 | 12 | 0.500 | 1.000 | 0.375 |
| SNP043 | 0.833 | 2 | 12 | 0.278 | 0.333 | 0.239 |
| SNP044 | 0.500 | 1 | 12 | 0.500 | 1.000 | 0.375 |
| SNP045 | 0.833 | 2 | 12 | 0.278 | 0.000 | 0.239 |
| SNP046 | 1.000 | 1 | 12 | 0.000 | 0.000 | 0.000 |
| SNP047 | 0.500 | 1 | 12 | 0.500 | 1.000 | 0.375 |
| SNP048 | 0.500 | 1 | 12 | 0.500 | 1.000 | 0.375 |
| SNP049 | 0.500 | 1 | 12 | 0.500 | 1.000 | 0.375 |
| SNP050 | 1.000 | 1 | 12 | 0.000 | 0.000 | 0.000 |
| SNP051 | 1.000 | 1 | 12 | 0.000 | 0.000 | 0.000 |
| SNP052 | 1.000 | 1 | 11 | 0.000 | 0.000 | 0.000 |
| SNP053 | 1.000 | 1 | 12 | 0.000 | 0.000 | 0.000 |
| SNP054 | 0.625 | 3 | 12 | 0.469 | 0.250 | 0.359 |
| SNP055 | 0.708 | 3 | 12 | 0.413 | 0.250 | 0.328 |
| SNP056 | 0.667 | 3 | 12 | 0.444 | 0.500 | 0.346 |
| SNP057 | 0.500 | 1 | 11 | 0.500 | 1.000 | 0.375 |
| SNP058 | 0.500 | 1 | 11 | 0.500 | 1.000 | 0.375 |
| SNP059 | 0.542 | 2 | 12 | 0.497 | 0.917 | 0.373 |
| SNP060 | 0.500 | 1 | 12 | 0.500 | 1.000 | 0.375 |
| SNP061 | 0.500 | 3 | 11 | 0.500 | 0.818 | 0.375 |
| SNP062 | 0.917 | 2 | 12 | 0.153 | 0.167 | 0.141 |
| SNP063 | 0.542 | 2 | 12 | 0.497 | 0.917 | 0.373 |
| SNP064 | 0.500 | 1 | 12 | 0.500 | 1.000 | 0.375 |
| SNP065 | 0.500 | 1 | 12 | 0.500 | 1.000 | 0.375 |
| SNP066 | 0.500 | 1 | 11 | 0.500 | 1.000 | 0.375 |
| SNP067 | 0.500 | 1 | 12 | 0.500 | 1.000 | 0.375 |
| SNP068 | 1.000 | 1 | 12 | 0.000 | 0.000 | 0.000 |
| SNP069 | 0.500 | 1 | 12 | 0.500 | 1.000 | 0.375 |
| SNP070 | 0.500 | 1 | 12 | 0.500 | 1.000 | 0.375 |
| SNP071 | 0.500 | 1 | 12 | 0.500 | 1.000 | 0.375 |
| SNP072 | 1.000 | 1 | 12 | 0.000 | 0.000 | 0.000 |
| SNP073 | 0.500 | 1 | 12 | 0.500 | 1.000 | 0.375 |
| SNP074 | 0.500 | 1 | 12 | 0.500 | 1.000 | 0.375 |
| SNP075 | 0.500 | 1 | 12 | 0.500 | 1.000 | 0.375 |
| SNP076 | 0.500 | 1 | 12 | 0.500 | 1.000 | 0.375 |
| SNP077 | 0.909 | 2 | 11 | 0.165 | 0.000 | 0.152 |
| SNP078 | 1.000 | 1 | 12 | 0.000 | 0.000 | 0.000 |
| SNP079 | 0.583 | 3 | 12 | 0.486 | 0.667 | 0.368 |
| SNP080 | 0.500 | 1 | 12 | 0.500 | 1.000 | 0.375 |
| SNP081 | 0.500 | 1 | 12 | 0.500 | 1.000 | 0.375 |
| SNP082 | 0.500 | 1 | 10 | 0.500 | 1.000 | 0.375 |
| SNP083 | 0.500 | 1 | 11 | 0.500 | 1.000 | 0.375 |
| SNP084 | 1.000 | 1 | 11 | 0.000 | 0.000 | 0.000 |
| SNP085 | 0.545 | 2 | 11 | 0.496 | 0.909 | 0.373 |
| SNP086 | 0.750 | 2 | 12 | 0.375 | 0.500 | 0.305 |
| SNP087 | 1.000 | 1 | 11 | 0.000 | 0.000 | 0.000 |
| SNP088 | 0.500 | 1 | 12 | 0.500 | 1.000 | 0.375 |
| SNP089 | 0.500 | 1 | 12 | 0.500 | 1.000 | 0.375 |
| SNP090 | 0.917 | 2 | 12 | 0.153 | 0.167 | 0.141 |
| SNP091 | 0.500 | 1 | 11 | 0.500 | 1.000 | 0.375 |
| SNP092 | 0.958 | 2 | 12 | 0.080 | 0.083 | 0.077 |
| SNP093 | 0.500 | 3 | 12 | 0.500 | 0.833 | 0.375 |
| SNP094 | 1.000 | 1 | 11 | 0.000 | 0.000 | 0.000 |
| SNP095 | 0.500 | 1 | 12 | 0.500 | 1.000 | 0.375 |
| SNP096 | 0.625 | 2 | 12 | 0.469 | 0.750 | 0.359 |
| SNP097 | 0.542 | 2 | 12 | 0.497 | 0.917 | 0.373 |
| SNP098 | 0.792 | 2 | 12 | 0.330 | 0.417 | 0.275 |
| SNP099 | 0.500 | 1 | 12 | 0.500 | 1.000 | 0.375 |
| SNP100 | 0.500 | 1 | 12 | 0.500 | 1.000 | 0.375 |
| SNP101 | 0.500 | 1 | 12 | 0.500 | 1.000 | 0.375 |
| SNP102 | 0.625 | 2 | 12 | 0.469 | 0.750 | 0.359 |
| SNP103 | 0.500 | 1 | 12 | 0.500 | 1.000 | 0.375 |
| SNP104 | 0.500 | 1 | 12 | 0.500 | 1.000 | 0.375 |
| SNP105 | 0.500 | 1 | 12 | 0.500 | 1.000 | 0.375 |
| SNP106 | 0.500 | 1 | 12 | 0.500 | 1.000 | 0.375 |
| SNP107 | 0.500 | 1 | 12 | 0.500 | 1.000 | 0.375 |
| SNP108 | 0.958 | 2 | 12 | 0.080 | 0.083 | 0.077 |
| SNP109 | 1.000 | 1 | 12 | 0.000 | 0.000 | 0.000 |
| SNP110 | 0.500 | 1 | 12 | 0.500 | 1.000 | 0.375 |
| SNP111 | 0.542 | 2 | 12 | 0.497 | 0.917 | 0.373 |
| SNP112 | 0.500 | 1 | 12 | 0.500 | 1.000 | 0.375 |
| SNP113 | 0.500 | 1 | 12 | 0.500 | 1.000 | 0.375 |
| SNP114 | 0.500 | 1 | 12 | 0.500 | 1.000 | 0.375 |
| SNP115 | 1.000 | 1 | 12 | 0.000 | 0.000 | 0.000 |
| SNP116 | 0.500 | 1 | 12 | 0.500 | 1.000 | 0.375 |
| SNP117 | 0.958 | 2 | 12 | 0.080 | 0.083 | 0.077 |
| SNP118 | 1.000 | 1 | 12 | 0.000 | 0.000 | 0.000 |
| SNP119 | 0.500 | 1 | 12 | 0.500 | 1.000 | 0.375 |
| SNP120 | 0.500 | 1 | 12 | 0.500 | 1.000 | 0.375 |
| SNP121 | 0.958 | 2 | 12 | 0.080 | 0.083 | 0.077 |
| SNP122 | 0.500 | 1 | 12 | 0.500 | 1.000 | 0.375 |
| SNP123 | 0.500 | 1 | 12 | 0.500 | 1.000 | 0.375 |
| SNP124 | 0.875 | 2 | 12 | 0.219 | 0.250 | 0.195 |
| SNP125 | 0.500 | 1 | 12 | 0.500 | 1.000 | 0.375 |
| SNP126 | 1.000 | 1 | 12 | 0.000 | 0.000 | 0.000 |
| SNP127 | 0.500 | 1 | 12 | 0.500 | 1.000 | 0.375 |
| SNP128 | 0.500 | 1 | 12 | 0.500 | 1.000 | 0.375 |
| SNP129 | 0.500 | 1 | 12 | 0.500 | 1.000 | 0.375 |
| SNP130 | 0.909 | 2 | 11 | 0.165 | 0.182 | 0.152 |
| SNP131 | 0.500 | 1 | 12 | 0.500 | 1.000 | 0.375 |
| SNP132 | 0.500 | 1 | 12 | 0.500 | 1.000 | 0.375 |
| SNP133 | 0.750 | 2 | 12 | 0.375 | 0.500 | 0.305 |
| SNP134 | 0.500 | 1 | 12 | 0.500 | 1.000 | 0.375 |
| SNP135 | 0.500 | 1 | 11 | 0.500 | 1.000 | 0.375 |
| SNP136 | 0.500 | 1 | 12 | 0.500 | 1.000 | 0.375 |
| SNP137 | 0.500 | 1 | 12 | 0.500 | 1.000 | 0.375 |
| SNP138 | 0.500 | 1 | 11 | 0.500 | 1.000 | 0.375 |
| SNP139 | 0.500 | 1 | 12 | 0.500 | 1.000 | 0.375 |
| SNP140 | 0.864 | 2 | 11 | 0.236 | 0.273 | 0.208 |
| SNP141 | 0.500 | 1 | 12 | 0.500 | 1.000 | 0.375 |
| SNP142 | 0.500 | 1 | 12 | 0.500 | 1.000 | 0.375 |
| SNP143 | 0.500 | 1 | 12 | 0.500 | 1.000 | 0.375 |
| SNP144 | 0.500 | 1 | 12 | 0.500 | 1.000 | 0.375 |
| SNP145 | 0.500 | 1 | 12 | 0.500 | 1.000 | 0.375 |
| SNP146 | 0.500 | 1 | 12 | 0.500 | 1.000 | 0.375 |
| SNP147 | 0.500 | 1 | 12 | 0.500 | 1.000 | 0.375 |
| SNP148 | 0.500 | 1 | 12 | 0.500 | 1.000 | 0.375 |
| SNP149 | 0.500 | 1 | 12 | 0.500 | 1.000 | 0.375 |
| SNP150 | 0.950 | 2 | 10 | 0.095 | 0.100 | 0.090 |
| SNP151 | 0.958 | 2 | 12 | 0.080 | 0.083 | 0.077 |
| SNP152 | 0.500 | 1 | 12 | 0.500 | 1.000 | 0.375 |
| SNP153 | 0.500 | 1 | 11 | 0.500 | 1.000 | 0.375 |
| SNP154 | 0.500 | 1 | 12 | 0.500 | 1.000 | 0.375 |
| SNP156 | 0.500 | 1 | 12 | 0.500 | 1.000 | 0.375 |
| SNP157 | 0.500 | 1 | 12 | 0.500 | 1.000 | 0.375 |
| SNP158 | 0.500 | 1 | 12 | 0.500 | 1.000 | 0.375 |
| SNP159 | 0.500 | 1 | 12 | 0.500 | 1.000 | 0.375 |
| SNP160 | 0.917 | 2 | 12 | 0.153 | 0.167 | 0.141 |
| SNP161 | 0.500 | 1 | 10 | 0.500 | 1.000 | 0.375 |
| SNP162 | 0.917 | 2 | 12 | 0.153 | 0.167 | 0.141 |
| SNP163 | 1.000 | 1 | 11 | 0.000 | 0.000 | 0.000 |
| SNP164 | 0.958 | 2 | 12 | 0.080 | 0.083 | 0.077 |
| SNP165 | 0.500 | 1 | 12 | 0.500 | 1.000 | 0.375 |
| SNP166 | 0.682 | 2 | 11 | 0.434 | 0.636 | 0.340 |
| SNP167 | 0.500 | 1 | 12 | 0.500 | 1.000 | 0.375 |
| SNP168 | 0.833 | 2 | 12 | 0.278 | 0.333 | 0.239 |
| SNP169 | 0.542 | 2 | 12 | 0.497 | 0.917 | 0.373 |
| SNP170 | 0.500 | 1 | 12 | 0.500 | 1.000 | 0.375 |
| SNP171 | 0.500 | 1 | 11 | 0.500 | 1.000 | 0.375 |
| SNP172 | 0.500 | 1 | 12 | 0.500 | 1.000 | 0.375 |
| SNP173 | 0.500 | 1 | 12 | 0.500 | 1.000 | 0.375 |
| SNP174 | 0.583 | 2 | 12 | 0.486 | 0.833 | 0.368 |
| SNP175 | 0.500 | 1 | 12 | 0.500 | 1.000 | 0.375 |
| SNP176 | 0.500 | 1 | 12 | 0.500 | 1.000 | 0.375 |
| SNP177 | 0.818 | 2 | 11 | 0.298 | 0.364 | 0.253 |
| SNP178 | 0.667 | 3 | 9 | 0.444 | 0.444 | 0.346 |
| SNP179 | 0.792 | 2 | 12 | 0.330 | 0.417 | 0.275 |
| SNP180 | 0.500 | 1 | 10 | 0.500 | 1.000 | 0.375 |
| SNP181 | 0.500 | 1 | 12 | 0.500 | 1.000 | 0.375 |
| SNP182 | 0.500 | 1 | 12 | 0.500 | 1.000 | 0.375 |
| SNP183 | 0.500 | 1 | 12 | 0.500 | 1.000 | 0.375 |
| SNP184 | 0.500 | 1 | 12 | 0.500 | 1.000 | 0.375 |
| SNP185 | 0.500 | 1 | 12 | 0.500 | 1.000 | 0.375 |
| SNP186 | 1.000 | 1 | 12 | 0.000 | 0.000 | 0.000 |
| SNP187 | 1.000 | 1 | 9 | 0.000 | 0.000 | 0.000 |
| SNP188 | 0.500 | 1 | 11 | 0.500 | 1.000 | 0.375 |
| SNP189 | 0.500 | 1 | 12 | 0.500 | 1.000 | 0.375 |
| SNP190 | 0.500 | 1 | 12 | 0.500 | 1.000 | 0.375 |
| SNP191 | 0.500 | 1 | 12 | 0.500 | 1.000 | 0.375 |
| SNP192 | 0.500 | 1 | 12 | 0.500 | 1.000 | 0.375 |
| SNP193 | 0.500 | 1 | 12 | 0.500 | 1.000 | 0.375 |
| SNP194 | 0.500 | 1 | 12 | 0.500 | 1.000 | 0.375 |
| SNP195 | 0.500 | 1 | 12 | 0.500 | 1.000 | 0.375 |
| SNP196 | 0.500 | 1 | 12 | 0.500 | 1.000 | 0.375 |
| SNP197 | 0.583 | 3 | 12 | 0.486 | 0.667 | 0.368 |
| SNP198 | 0.500 | 1 | 12 | 0.500 | 1.000 | 0.375 |
| SNP199 | 0.500 | 1 | 12 | 0.500 | 1.000 | 0.375 |
| SNP200 | 0.500 | 1 | 12 | 0.500 | 1.000 | 0.375 |
| SNP201 | 0.500 | 1 | 12 | 0.500 | 1.000 | 0.375 |
| SNP202 | 0.591 | 2 | 11 | 0.483 | 0.818 | 0.367 |
| SNP203 | 0.500 | 1 | 12 | 0.500 | 1.000 | 0.375 |
| SNP204 | 0.500 | 1 | 9 | 0.500 | 1.000 | 0.375 |
| SNP205 | 0.500 | 1 | 12 | 0.500 | 1.000 | 0.375 |
| SNP206 | 0.500 | 1 | 12 | 0.500 | 1.000 | 0.375 |
| SNP207 | 0.545 | 2 | 11 | 0.496 | 0.909 | 0.373 |
| SNP208 | 0.625 | 2 | 12 | 0.469 | 0.750 | 0.359 |
| SNP209 | 0.500 | 1 | 12 | 0.500 | 1.000 | 0.375 |
| SNP210 | 0.875 | 2 | 12 | 0.219 | 0.250 | 0.195 |
| SNP211 | 1.000 | 1 | 11 | 0.000 | 0.000 | 0.000 |
| SNP212 | 1.000 | 1 | 8 | 0.000 | 0.000 | 0.000 |
| SNP213 | 0.500 | 1 | 12 | 0.500 | 1.000 | 0.375 |
| SNP214 | 0.500 | 1 | 12 | 0.500 | 1.000 | 0.375 |
| SNP215 | 0.500 | 1 | 11 | 0.500 | 1.000 | 0.375 |
| SNP216 | 0.500 | 1 | 11 | 0.500 | 1.000 | 0.375 |
| SNP217 | 0.500 | 1 | 12 | 0.500 | 1.000 | 0.375 |
| SNP218 | 0.500 | 1 | 12 | 0.500 | 1.000 | 0.375 |
| SNP219 | 0.500 | 1 | 12 | 0.500 | 1.000 | 0.375 |
| SNP221 | 0.500 | 1 | 11 | 0.500 | 1.000 | 0.375 |
| SNP222 | 0.500 | 1 | 12 | 0.500 | 1.000 | 0.375 |
| SNP223 | 0.833 | 2 | 12 | 10.278 | 0.333 | 0.239 |
| SNP224 | 0.500 | 1 | 12 | 0.500 | 1.000 | 0.375 |
| SNP225 | 1.000 | 1 | 12 | 0.000 | 0.000 | 0.000 |
| SNP226 | 0.500 | 1 | 12 | 0.500 | 1.000 | 0.375 |
| SNP227 | 0.500 | 1 | 12 | 0.500 | 1.000 | 0.375 |
| SNP228 | 1.000 | 1 | 9 | 0.000 | 0.000 | 0.000 |
| SNP229 | 0.500 | 1 | 12 | 0.500 | 1.000 | 0.375 |
| SNP230 | 0.500 | 1 | 11 | 0.500 | 1.000 | 0.375 |
| SNP231 | 0.500 | 1 | 12 | 0.500 | 1.000 | 0.375 |
| SNP232 | 0.950 | 2 | 10 | 0.095 | 0.100 | 0.090 |
| SNP233 | 0.500 | 1 | 11 | 0.500 | 1.000 | 0.375 |
| SNP234 | 0.917 | 2 | 12 | 0.153 | 0.167 | 0.141 |
| SNP235 | 0.917 | 2 | 12 | 0.153 | 0.167 | 0.141 |
| SNP236 | 0.500 | 1 | 12 | 0.500 | 1.000 | 0.375 |
| SNP237 | 0.500 | 1 | 12 | 0.500 | 1.000 | 0.375 |
| SNP238 | 0.500 | 1 | 12 | 0.500 | 1.000 | 0.375 |
| SNP239 | 0.500 | 1 | 12 | 0.500 | 1.000 | 0.375 |
| SNP240 | 0.813 | 2 | 8 | 0.305 | 0.375 | 0.258 |
| SNP241 | 0.500 | 1 | 8 | 0.500 | 1.000 | 0.375 |
| SNP242 | 0.500 | 1 | 12 | 0.500 | 1.000 | 0.375 |
| SNP243 | 0.833 | 2 | 12 | 0.278 | 0.333 | 0.239 |
| SNP244 | 0.500 | 1 | 11 | 0.500 | 1.000 | 0.375 |
| SNP245 | 0.500 | 1 | 12 | 0.500 | 1.000 | 0.375 |
| SNP246 | 0.500 | 1 | 11 | 0.500 | 1.000 | 0.375 |
| SNP247 | 0.500 | 1 | 10 | 0.500 | 1.000 | 0.375 |
| SNP248 | 0.500 | 1 | 11 | 0.500 | 1.000 | 0.375 |
| SNP249 | 0.500 | 1 | 12 | 0.500 | 1.000 | 0.375 |
| SNP250 | 0.556 | 3 | 9 | 0.494 | 0.444 | 0.372 |
| SNP251 | 0.500 | 1 | 11 | 0.500 | 1.000 | 0.375 |
| SNP252 | 0.500 | 1 | 12 | 0.500 | 1.000 | 0.375 |
| SNP253 | 0.600 | 2 | 10 | 0.480 | 0.800 | 0.365 |
| SNP254 | 0.958 | 2 | 12 | 0.080 | 0.083 | 0.077 |
| SNP255 | 0.500 | 1 | 12 | 0.500 | 1.000 | 0.375 |
| SNP256 | 0.500 | 1 | 12 | 0.500 | 1.000 | 0.375 |
| SNP257 | 0.500 | 1 | 10 | 0.500 | 1.000 | 0.375 |
| SNP258 | 0.500 | 1 | 11 | 0.500 | 1.000 | 0.375 |
| SNP259 | 0.500 | 1 | 11 | 0.500 | 1.000 | 0.375 |
| SNP260 | 0.500 | 1 | 11 | 0.500 | 1.000 | 0.375 |
| SNP262 | 0.500 | 1 | 12 | 0.500 | 1.000 | 0.375 |
| SNP263 | 1.000 | 1 | 12 | 0.000 | 0.000 | 0.000 |
| SNP264 | 0.500 | 1 | 12 | 0.500 | 1.000 | 0.375 |
| SNP265 | 0.750 | 2 | 12 | 0.375 | 0.500 | 0.305 |
| SNP266 | 0.500 | 1 | 9 | 0.500 | 1.000 | 0.375 |
| SNP267 | 0.708 | 2 | 12 | 0.413 | 0.583 | 0.328 |
| SNP268 | 0.909 | 2 | 11 | 0.165 | 0.000 | 0.152 |
| SNP269 | 0.583 | 2 | 12 | 0.486 | 0.833 | 0.368 |
| SNP270 | 0.500 | 1 | 12 | 0.500 | 1.000 | 0.375 |
| SNP271 | 0.875 | 2 | 12 | 0.219 | 0.250 | 0.195 |
| SNP272 | 0.500 | 1 | 11 | 0.500 | 1.000 | 0.375 |
| SNP273 | 0.500 | 1 | 12 | 0.500 | 1.000 | 0.375 |
| SNP274 | 1.000 | 1 | 12 | 0.000 | 0.000 | 0.000 |
| SNP275 | 0.500 | 1 | 12 | 0.500 | 1.000 | 0.375 |
| SNP276 | 0.500 | 1 | 12 | 0.500 | 1.000 | 0.375 |
| SNP277 | 1.000 | 1 | 12 | 0.000 | 0.000 | 0.000 |
| SNP278 | 1.000 | 1 | 12 | 0.000 | 0.000 | 0.000 |
| SNP279 | 0.500 | 1 | 12 | 0.500 | 1.000 | 0.375 |
| SNP280 | 0.889 | 2 | 9 | 0.198 | 0.000 | 0.178 |
| SNP281 | 0.500 | 1 | 12 | 0.500 | 1.000 | 0.375 |
| SNP282 | 1.000 | 1 | 10 | 0.000 | 0.000 | 0.000 |
| SNP284 | 0.500 | 1 | 11 | 0.500 | 1.000 | 0.375 |
| SNP285 | 0.958 | 2 | 12 | 0.080 | 0.083 | 0.077 |
| SNP286 | 0.500 | 1 | 10 | 0.500 | 1.000 | 0.375 |
| SNP287 | 0.750 | 2 | 12 | 0.375 | 0.500 | 0.305 |
| SNP288 | 0.500 | 1 | 11 | 0.500 | 1.000 | 0.375 |
| SNP289 | 0.542 | 2 | 12 | 10.497 | 0.917 | 0.373 |
| SNP290 | 0.500 | 1 | 12 | 0.500 | 1.000 | 0.375 |
| SNP291 | 1.000 | 1 | 12 | 0.000 | 0.000 | 0.000 |
| SNP292 | 1.000 | 1 | 12 | 0.000 | 0.000 | 0.000 |
| SNP293 | 0.500 | 1 | 11 | 0.500 | 1.000 | 0.375 |
| SNP294 | 0.500 | 1 | 9 | 0.500 | 1.000 | 0.375 |
| SNP295 | 1.000 | 1 | 9 | 0.000 | 0.000 | 0.000 |
| SNP296 | 0.500 | 1 | 12 | 0.500 | 1.000 | 0.375 |
| SNP297 | 0.500 | 1 | 12 | 0.500 | 1.000 | 0.375 |
| SNP298 | 0.625 | 2 | 12 | 0.469 | 0.750 | 0.359 |
| SNP299 | 0.958 | 2 | 12 | 0.080 | 0.083 | 0.077 |
| SNP300 | 0.727 | 2 | 11 | 0.397 | 0.545 | 0.318 |
| SNP301 | 0.958 | 2 | 12 | 0.080 | 0.083 | 0.077 |
| SNP302 | 0.625 | 2 | 12 | 0.469 | 0.750 | 0.359 |
| SNP303 | 0.500 | 1 | 12 | 0.500 | 1.000 | 0.375 |
| SNP304 | 0.917 | 2 | 12 | 0.153 | 0.167 | 0.141 |
| SNP305 | 0.500 | 1 | 10 | 0.500 | 1.000 | 0.375 |
| SNP306 | 1.000 | 1 | 12 | 0.000 | 0.000 | 0.000 |
| SNP307 | 0.500 | 1 | 12 | 0.500 | 1.000 | 0.375 |
| SNP308 | 1.000 | 1 | 12 | 0.000 | 0.000 | 0.000 |
| SNP309 | 0.500 | 1 | 12 | 0.500 | 1.000 | 0.375 |
| SNP310 | 0.500 | 1 | 12 | 0.500 | 1.000 | 0.375 |
| SNP312 | 0.600 | 2 | 10 | 0.480 | 0.800 | 0.365 |
| SNP313 | 0.500 | 1 | 12 | 0.500 | 1.000 | 0.375 |
| SNP314 | 0.625 | 2 | 12 | 0.469 | 0.750 | 0.359 |
| SNP315 | 1.000 | 1 | 12 | 0.000 | 0.000 | 0.000 |
| SNP316 | 0.500 | 1 | 10 | 0.500 | 1.000 | 0.375 |
| SNP317 | 1.000 | 1 | 11 | 0.000 | 0.000 | 0.000 |
| SNP318 | 1.000 | 1 | 12 | 0.000 | 0.000 | 0.000 |
| SNP319 | 0.792 | 2 | 12 | 0.330 | 0.417 | 0.275 |
| SNP320 | 0.667 | 3 | 12 | 0.444 | 0.500 | 0.346 |
| SNP321 | 0.917 | 2 | 12 | 0.153 | 0.167 | 0.141 |
| SNP322 | 0.500 | 1 | 12 | 0.500 | 1.000 | 0.375 |
| SNP323 | 0.500 | 1 | 12 | 0.500 | 1.000 | 0.375 |
| SNP324 | 1.000 | 1 | 12 | 0.000 | 0.000 | 0.000 |
| SNP325 | 1.000 | 1 | 12 | 0.000 | 0.000 | 0.000 |
| SNP326 | 0.500 | 1 | 11 | 0.500 | 1.000 | 0.375 |
| SNP327 | 0.864 | 2 | 11 | 0.236 | 0.273 | 0.208 |
| SNP328 | 0.500 | 1 | 11 | 0.500 | 1.000 | 0.375 |
| SNP329 | 0.545 | 2 | 11 | 0.496 | 0.909 | 0.373 |
| SNP330 | 0.583 | 2 | 12 | 0.486 | 0.000 | 0.368 |
| SNP331 | 0.500 | 1 | 9 | 0.500 | 1.000 | 0.375 |
| SNP332 | 0.500 | 1 | 11 | 0.500 | 1.000 | 0.375 |
| SNP333 | 0.958 | 2 | 12 | 0.080 | 0.083 | 0.077 |
| SNP334 | 0.500 | 1 | 12 | 0.500 | 1.000 | 0.375 |
| SNP335 | 0.500 | 1 | 12 | 0.500 | 1.000 | 0.375 |
| SNP336 | 0.500 | 1 | 12 | 0.500 | 1.000 | 0.375 |
| SNP337 | 0.500 | 1 | 12 | 0.500 | 1.000 | 0.375 |
| SNP338 | 0.545 | 2 | 11 | 0.496 | 0.909 | 0.373 |
| SNP339 | 0.792 | 2 | 12 | 0.330 | 0.417 | 0.275 |
| SNP340 | 0.917 | 2 | 12 | 0.153 | 0.000 | 0.141 |
| SNP341 | 1.000 | 1 | 12 | 0.000 | 0.000 | 0.000 |
| SNP342 | 0.563 | 2 | 8 | 0.492 | 0.875 | 0.371 |
| SNP343 | 1.000 | 1 | 12 | 0.000 | 0.000 | 0.000 |
| SNP344 | 0.650 | 2 | 10 | 0.455 | 0.700 | 0.351 |
| SNP345 | 0.500 | 1 | 12 | 0.500 | 1.000 | 0.375 |
| SNP346 | 1.000 | 1 | 11 | 0.000 | 0.000 | 0.000 |
| SNP347 | 0.571 | 2 | 7 | 0.490 | 0.857 | 0.370 |
| SNP348 | 1.000 | 1 | 11 | 0.000 | 0.000 | 0.000 |
| SNP349 | 0.545 | 2 | 11 | 0.496 | 0.909 | 0.373 |
| SNP350 | 0.500 | 1 | 11 | 0.500 | 1.000 | 0.375 |
| SNP351 | 1.000 | 1 | 12 | 0.000 | 0.000 | 0.000 |
| SNP352 | 0.682 | 2 | 11 | 0.434 | 0.636 | 0.340 |
| SNP353 | 0.500 | 1 | 11 | 0.500 | 1.000 | 0.375 |
| SNP354 | 1.000 | 1 | 11 | 0.000 | 0.000 | 0.000 |
| SNP355 | 0.500 | 1 | 9 | 0.500 | 1.000 | 0.375 |
| SNP356 | 0.500 | 1 | 9 | 0.500 | 1.000 | 0.375 |
| SNP357 | 0.773 | 2 | 11 | 0.351 | 0.455 | 0.290 |
| SNP358 | 0.542 | 3 | 12 | 0.497 | 0.083 | 0.373 |
| SNP359 | 0.500 | 1 | 9 | 0.500 | 1.000 | 0.375 |
| SNP360 | 0.563 | 2 | 8 | 0.492 | 0.875 | 0.371 |
| SNP361 | 0.583 | 3 | 12 | 0.486 | 0.667 | 0.368 |
| SNP362 | 0.500 | 1 | 12 | 0.500 | 1.000 | 0.375 |
| SNP363 | 0.667 | 2 | 12 | 0.444 | 0.000 | 0.346 |
| SNP364 | 0.500 | 1 | 12 | 0.500 | 1.000 | 0.375 |
| SNP365 | 0.542 | 2 | 12 | 0.497 | 0.917 | 0.373 |
| SNP366 | 0.542 | 2 | 12 | 0.497 | 0.917 | 0.373 |
| SNP367 | 1.000 | 1 | 12 | 0.000 | 0.000 | 0.000 |
| SNP368 | 0.500 | 1 | 10 | 0.500 | 1.000 | 0.375 |
| SNP369 | 0.500 | 1 | 12 | 0.500 | 1.000 | 0.375 |
| SNP370 | 0.500 | 1 | 11 | 0.500 | 1.000 | 0.375 |
| SNP371 | 1.000 | 1 | 12 | 0.000 | 0.000 | 0.000 |
| SNP372 | 0.500 | 1 | 10 | 0.500 | 1.000 | 0.375 |
| SNP373 | 0.500 | 1 | 12 | 0.500 | 1.000 | 0.375 |
| SNP374 | 0.500 | 1 | 12 | 0.500 | 1.000 | 0.375 |
| SNP375 | 0.500 | 1 | 10 | 0.500 | 1.000 | 0.375 |
| SNP376 | 0.500 | 1 | 12 | 0.500 | 1.000 | 0.375 |
| SNP377 | 0.500 | 1 | 12 | 0.500 | 1.000 | 0.375 |
| SNP378 | 0.500 | 1 | 12 | 0.500 | 1.000 | 0.375 |
| SNP379 | 0.750 | 2 | 12 | 0.375 | 0.500 | 0.305 |
| SNP380 | 0.500 | 1 | 12 | 0.500 | 1.000 | 0.375 |
| SNP381 | 1.000 | 1 | 12 | 0.000 | 0.000 | 0.000 |
| SNP382 | 0.875 | 2 | 12 | 0.219 | 0.250 | 0.195 |
| SNP383 | 0.500 | 1 | 11 | 0.500 | 1.000 | 0.375 |
| SNP384 | 0.500 | 1 | 11 | 0.500 | 1.000 | 0.375 |
| **Mean** | **0.652** | **1.322** | **11.551** | **0.369** | **0.677** | **0.281** |

| TABLE 11 SSRs and SSR Primer Sequences from Contigs and Annealing Temperatures | | | | | | | |
|---|---|---|---|---|---|---|---|
| | | | | | | | |

| **SSR** | **Contig** | **Forward** | **TM Forward** | **Reverse** | **TM Reverse** | **Product Size** | **Repeat** |
|---|---|---|---|---|---|---|---|
| 1 | Contig5 | ACACAACGACCCAGAAAACC | 59.867 | GACCCATCATCACCAGACTT | 57.314 | 475 | (CAT)4 |
| 2 | Contig8 | CCATCCTTCCTTCCTTCTTC | 57.77 | CAAGATATGCITCGGTGAGG | 58.325 | 455 | (CAT)4 |
| 3 | Contig10 | AAGGAGAGGAGAAGAATGGG | 57.373 | GCCACTAGTAGGCTCAACCA | 57.98 | 435 | (TCT)5-(TCT)6-(ACA)4 |
| 4 | Contig26 | ACCACCTTCCCCTTAACAAC | 57.882 | GACCCTAACCACTTCCGAAT | 57.974 | 351 | (CAC)4 |
| 5 | Contig33 | TTAACCACTTAACCCTCCCC | 57.904 | CAGGAAGCGGAGACTCATAA | 58.048 | 429 | (CA)9 |
| 6 | Contig34 | TGTCGGCCTCTATCTCTCTG | 58.129 | ATGGCATCTTCACTCTCAGC | 57.969 | 180 | (AAG)4 |
| 7 | Contig39 | CCAACAAGTCCTCAAACACG | 59.183 | AATCAGAAGGGCAGAACTCC | 58.324 | 491 | (TAT)4 |
| 8 | Contig47 | ATAACTGGACTCACCAACCG | 57.514 | GATAGCACCGATGGAGAAGA | 57.834 | 360 | (GTC)4 |
| 9 | Contig69 | CAGTGTAGCCATCCTTCACC | 58.158 | TGTGAGGTITGTGTGGAGTG | 58.063 | 107 | (CAACCA)5 |
| 10 | Contig74 | CTCTCTCCACATCAATGGC | 56.593 | GCAAGTGGGTCTITACCCIT | 58.174 | 337 | (GAA)5 |
| 11 | Contig86 | CTAAATGGTCCTCCTGCTCA | 57.894 | GAAGCTCAACCTGCCTATGA | 58.048 | 498 | (CCG)4 |
| 12 | Contig93 | CCATCAATATCCACCGTACC | 57.61 | GTGTCAGATAGCGGCAGAAT | 57.921 | 202 | (CA)8 |
| 13 | Contig94 | GTCAAGTGGAGCTGTGGAGT | 57.844 | AAAGGTTGGGTAGATCCGTC | 57.974 | 454 | (TTC)4 |
| 14 | Contig104 | CTCAATCACTCACTCCACCA | 57.09 | CAGAGCTGATGCTAGCCAGT | 58.371 | 307 | (ACC)4 |
| 15 | Contig110 | CTCCGCCCATAAAACAAGAC | 59.569 | TCTGCCACACTTGACTCTGA | 58.03 | 283 | (TCT)4-(AC)6 |
| 16 | Contig132 | CCCACCACCTCCATTACATA | 58.145 | GCTCCAGATGGAGTCGTTTA | 57.893 | 293 | (ACA)4 |
| 17 | Contig147 | GATACTCCTGGGCTGTTTGA | 57.737 | CGATTCAGGGCAGTCTTCfA | 58.048 | 279 | (AAG)4 |
| 18 | Contig152 | ACACCTTTTCCCGTCCTTAG | 58.174 | GAAAGAGACAGCGGTTGAGA | 58.158 | 163 | (TCC)4 |
| 19 | Config154 | CCCTTTCTTCTTCATCCCTC | 57.77 | GATGTTGACGATGACGAGTG | 57.573 | 134 | (CCA)4 |
| 20 | Contig155 | CTCCATCTTCCAAACCCACT | 58.989 | CCTCTTATCCCTCCCCATAA | 57.94 | 207 | (AAC)4 |
| 21 | Contig158 | TCAGGGCTAACTTCCTCTCA | 57.612 | GCTACAACTGGTGGGTGAAC | 58.058 | 355 | (CAC)5-(CCA)4 |
| 22 | Contig167 | GTTATGGACGCCGTTACTTG | 58.174 | CTCGCTTCGACAATCTCTGT | 58.187 | 226 | (TAA)5 |
| 23 | Contig180 | CCTTAACTCCACAGTTCCCC | 58.498 | AATCCGGTTOACTGGCTTAC | 59.056 | 438 | (ACAT)4 |
| 24 | Contig184 | TACTTTCAAGTCTCACCGCC | 57.951 | CAAGGGATAGCGTCACAGTT | 57.826 | 417 | (AAC)6 |
| 25 | Contig197 | GTGGTTGTAATCAAGGACGG | 57.923 | GAGACACAGGCACACAGATG | 57.714 | 385 | (TG)6 |
| 26 | Contig221 | CTTCTCTCTCCACATCCCAA | 57.78 | GATCCAACACCACCACTCTC | 57.886 | 419 | (GAG)4 |
| 27 | Contig232 | TGAGTTTCGTGGCTCTAAGG | 58.105 | CTCGTCGACTCAACGGTACT | 57.959 | 150 | (ATA)4 |
| 28 | Contig234 | CTGGCTGAAACAGGTGAAGT | 57.93 | GGCTACTTTCCTTTGCCCT | 58.398 | 440 | (GCT)4 |
| 29 | Contig236 | ATGGAGGGACCTTAAAACCC | 59.148 | GTCATTAACGACACGTTCCC | 57.922 | 225 | (TCT)4 |
| 30 | Contig238 | CCTCTGCCTACTCGTCAAAA | 58.105 | AGACGACGAACAAGACGAAG | 58.087 | 261 | (CA)6 |
| 31 | Contig245 | CGAGTTCCTTCAAGTCCTCA | 58.001 | CGACGAGTTAACGCTTCTGT | 58.187 | 187 | (AAT)5 |
| 32 | Contig265 | TCTGTAAGGCGGTTTCTGTC | 57.951 | ATAGAAGCAGGCATGGTGAG | 57.922 | 346 | (ACA)4 |
| 33 | Contig277 | GATCCTATTGCTCGTTGCC | 58.257 | ACTGCCCGATATCTCCTTTC | 58.214 | 371 | (ATT)4-(CA)6 |
| 34 | Contig294 | CTCCTATCAAAGGACGGAGA | 56.925 | ATCCTIGGACAGTAGCACCA | 58.158 | 459 | (TAA)6 |
| 35 | Contig295 | ACTGAGCCACCACTAACCAG | 57.801 | TAGACTTAGTGGCACCAGCC | 57.98 | 434 | (TTG)4 |
| 36 | Contig298 | ATTCAAAGGCCAGGGTAGAG | 58.266 | CGACTACAAACCGGACACTC | 58.215 | 297 | (TCA)4 |
| 37 | Contig312 | GCTGAGGAAGAGCATGGATA | 57.99 | CTCCACCATGTACCCGTT | 56.656 | 440 | (ACA)4 |
| 38 | Contig321 | CAGTGTAGCCATCCITCACC | 58.158 | CACAGACCCAGCAAAGAACT | 57.93 | 202 | (ACAACC)4 |
| 39 | Contig335 | GAATGGGAGTGTGAATGGAG | 57.91 | TACTCCAAGATCAAGCCTGC | 58.048 | 308 | (AGA)4 |
| 40 | Contig339 | CAATTCTCTCTCCTCCCCAT | 58.118 | GAGGTGGTTTCTGGTTGTTG | 58.03 | 110 | (CTC)5 |
| 41 | Contig343 | ACAATAAGGAAGAGGGCCAC | 58.121 | GGCTGAAGTCTTGTGCTCAT | 58.028 | 365 | (TGG)5 |
| 42 | Contig349 | CCGTCTCATTGACCTACCTT | 56.702 | ACCIGGCCCCACITCIATAC | 58.001 | 427 | (GAC)5 |
| 43 | Contig353 | CCTCCCTCCTTCTTCTGGTA | 58.323 | CAGCCTTGGATCITGTGAAC | 58.287 | 347 | (TAC)4 |
| 44 | Contig357 | CAGACGTCATTGCTGAAGTG | 57.977 | ACTGGAAGTCTGCCCTTTTC | 58.377 | 405 | (CCA)4 |
| 45 | Contig363 | AAATATCTCCCCAACCCCTC | 59.104 | TCAGGCTTAGTTGTTCCAGG | 57.952 | 356 | (CT)6 |
| 46 | Contig366 | ACGAAGCAGTTTACCACCAC | 57.729 | CACCGTATCTTCGCACTTCT | 57.98 | 161 | (AGG)4 |
| 47 | Contig370 | CCATGGTTAGGACTCAGGTC | 57.001 | CCCAAGTTCAGACCGTAGAT | 56.702 | 133 | (TC)6 |
| 48 | Contig371 | CGTATCACTTTCAACCCGAC | 58.076 | TCTCGCCTTCTCTATTGGTG | 58.048 | 408 | (GAG)4 |
| 49 | Contig375 | ATCCTGTTCTTGACGAGCTG | 58.029 | AGCTCGTTACGTTGGTTGTC | 57.883 | 454 | (TGA)4 |
| 50 | Contig380 | CCATCGATCATCAGTTAGGG | 57.962 | GTTGCTCCACCAAGTACACC | 58.058 | 365 | (TCG)4 |
| 51 | Contig382 | GCCTGAAATCAAGTGTGTCC | 58.129 | CATAGGTTCCATGGGTTACG | 57.823 | 478 | (TTC)4 |
| 52 | Contig390 | CTCAAGCCCTACATCTTCCC | 58.741 | TCTTAGGAGCACGTCCAAAC | 57.951 | 164 | (TAA)4-(TGCC)4 |
| 53 | Contig390 | GGTGGAGACCCFGAGAAGTT | 58.159 | CATACCAGAACCTTGGCATC | 58.019 | 396 | (TGG)4 |
| 54 | Contig392 | CAACACTCAACACCTCGTGA | 58.232 | TCATACTGCATACCCGAACC | 58.45 | 340 | (GAT)4 |
| 55 | Contig393 | GTATTGTTCAAGTGTGGGGG | 57.768 | GGGTGACATATITGGGTTGG | 59.91 | 497 | (TA)6 |
| 56 | Contig395 | GCAACAACAATGGCAACC | 58.458 | AGGTGGTCCACTATGCTCTG | 57.74 | 386 | (TCC)5 |
| 57 | Contig401 | TTACCCACTGACGTCATCCT | 58 | TGTATCCGTCACCTCCTCAT | 57.94 | 343 | (ATA)6 |
| 58 | Contig403 | GTTTCTTCAAGCGTCTGCTG | 58.802 | GCTCTTCACCCAGTCAAAGA | 58 | 490 | (GAT)5 |
| 59 | Contig414 | ACGCTTCTTCAGTGCTTCTG | 58.398 | CCTGTTCCCAGCTTTTAACC | 58.694 | 495 | (AC)9 |
| 60 | Contig421 | AACCGTCTAAAACCCGACTC | 58.174 | GTTACCGGAGAGGATGGATT | 57.918 | 213 | (ACC)4 |
| 61 | Contig422 | CTCACTTGTATCATTGCCCC | 58.019 | GTCAAGCCCATCTGTGACTC | 58.216 | 432 | (AAC)6-(TGA)4 |
| 62 | Contig467 | GCAGGCCGTATCACAAATAC | 58.12 | TAGGTGGATAGAACACCGGA | 58.02 | 241 | (CCA)4 |
| 63 | Contig468 | AAACCCTAGACACCGAAACC | 58.029 | CGTCACCAAAACCCTACAAC | 57.98 | 435 | (GCC)5 |
| 64 | Contig468 | GTTGTAGGGTTTTGGTGACG | 57.98 | GACCAATAGCAAGAGCAAGC | 57.725 | 243 | (GGA)4 |
| 65 | Contig471 | ACACCCCTCTCTCTCTTGCT | 58.06 | AATATGGGCTATCAGAGGGC | 58.109 | 245 | (TTG)5 |
| 66 | Contig473 | ACAACACTGTGTAATGGCCC | 58.343 | CCCCATATCCATAACFTCCC | 58.057 | 342 | (ACC)5 |
| 67 | Contig482 | TCTCATCTCAATGCTCCTCC | 57.879 | GCATCTGACGCTTGTAGCTC | 58.78 | 325 | (AAT)4-(TCC)5 |
| 68 | Contig484 | AACAATACCTCGCACTCTGC | 57.979 | GTTGGCTATGATTGGACGAC | 58.019 | 411 | (AAC)6 |
| 69 | Contig487 | ATCTTGCCTCTCTAAAGCCC | 57.639 | CGGAAGCACGATGAACTAGA | 59.024 | 327 | (AG)6 |
| 70 | Contig496 | TACCCTCCFCAGACCCTACC | 58.05 | AGCCCCACTTACCATGATCT | 58.473 | 143 | (ATG)6 |
| 71 | Contig498 | CTCAAGCGTATGAGTTGGCT | 58.132 | CCGGGACTTTTTACTCCTTC | 57.787 | 229 | (GAT)4 |
| 72 | Contig525 | CTGTTTCACCGATTCTGACC | 58.129 | ATGCACCTAAGTCCACAGGA | 58.158 | 473 | (TAA)5 |
| 73 | Contig534 | GACCAACACCCCCTTTTC | 57.718 | GACCAGCCAACACAAAGAAC | 58.187 | 294 | (TC)8 |
| 74 | Contig539 | AACAGCTGGAGACCCAAGTA | 57.387 | TAACCTACCAACTCGCTTGC | 58.054 | 278 | (GGT)4 |
| 75 | Contig546 | ACCCTAGATCCTCGGTTCAC | 58.049 | GCTTCGACAGGCAGTTATGT | 57.979 | 440 | (CCA)4 |
| 76 | Contig548 | GACGATGAGTTCAGAAACGG | 58.287 | GACACGTCATTTCCACCTTC | 57.97 | 370 | (TAC)5 |
| 77 | Contig570 | ATCCTCCCAAGAAGATGACC | 57.964 | TCTCTCCTGCAAGAAGCTGT | 57.9 | 230 | (AAC)4 |
| 78 | Contig577 | AAGAAGAGGAGCCTGGGTTA | 58.002 | GAAGGAAAAAGAGGCGTACC | 57.929 | 253 | (TCT)4 |
| 79 | Contig582 | TGGCGAAAGAAGAAGAGG | 56.587 | GAGAGGATGGCTATAAGGGG | 57.702 | 466 | (ATG)5 |
| 80 | Contig582 | CATGCTACCCCTTATAGCCA | 57.754 | GTITIGTAGCACAAGTCCGC | 58.433 | 177 | (AACCGA)4 |
| 81 | Contig591 | TGGAGTCTCCTGCTTCAAAC | 58 | GTGTTGGTGTGTGCTATCCA | 57.945 | 335 | (CAG)4 |
| 82 | Contig592 | CTACTGGTGGAGAAGGATGC | 57.323 | ACCTTCACTCACAAAGCTCG | 58.087 | 281 | (CCA)5 |
| 83 | Contig606 | GTCATCTCAACCACCACCAT | 58.186 | GGATGACAAGAGGACGTGAG | 58.216 | 242 | (CAC)4 |
| 84 | Contig612 | ACAAACCCTAACTGGACCGT | 58.429 | TTCACGCTCGAGTCTTAACC | 58.104 | 157 | (CCA)4 |
| 85 | Contig619 | TCTCTCTCCTCACCTACGTCAT | 58.01 | AAATCCTCAAGACTGGTCCC | 58.021 | 390 | (AAT)4 |
| 86 | Contig629 | TCAGGGTAAGGAGGAAGGAC | 58.173 | CGAGAATCTTCCCCGAGTAT | 58.214 | 315 | (TGA)4 |
| 87 | Contig629 | AGATTCTCGAGTGTGCTTGG | 58.029 | GACCTCGCTCCCTAATTACA | 56.909 | 318 | (CCA)4 |
| 88 | Contig632 | CTGCCTGCGTTTTCTCAT | 57.474 | GGAGATTCTTGGAGGAAGGA | 58.242 | 398 | (TTA)4 |
| 89 | Contig635 | GAAAGCGGATCACACAGC | 57.817 | TTCTCCAGAACATCCGAGTG | 58.8 | 314 | (CTAT)4 |
| 90 | Contig639 | TTCAACCTTAGGTCCAGACG | 57.799 | CTAGCACCAGCCGTAACAAT | 57.933 | 327 | (CAC)4 |
| 91 | Contig645 | GTCACCACTTCCTCCAAATG | 57.971 | TGGTGCTGATGATACAGTGG | 58.05 | 445 | (TAC)4 |
| 92 | Contig647 | TTACGGACGGAGACAAGAAG | 57.952 | TCATCATAGTGCTTGCGG | 56.735 | 259 | (CAC)4 |
| 93 | Contig663 | CCAAATCAACACCACCACC | 59.641 | CACTGCTCTTACGGATTGCT | 58.132 | 229 | (TCAC)4 |
| 94 | Contig686 | CTCATTCACCTCCCACCTAA | 57.582 | TACTTGACCIACTGGCAGGC | 57.98 | 468 | (AAT)4 |
| 95 | Contig711 | ACGAAGATGCACTCCTGAAG | 58.029 | AGCGGTCATGAGTCGATTAG | 57.922 | 315 | (ATG)4 |
| 96 | Contig716 | CCTGTTGCTTCTAGGGTGAA | 57.952 | GAACGGGTCTCCAAGGTTAT | 57.974 | 306 | (AGC)6-(TGA)5 |
| 97 | Contig717 | CCACCTCCATCTTAACCCAT | 58.715 | CATAAGCCGAAGTCACCCTA | 57.85 | 373 | (ATA)4 |
| 98 | Contig720 | TTTGTAACCCTCACACACCC | 58.315 | CAGTACTGACGGCGACTTCT | 58.116 | 224 | (AGG)4 |
| 99 | Contig722 | CTCACTCTCTCTCCAGAAGCA | 57.464 | GCACAGCTCCACTCATCTTT | 58.028 | 260 | (GAT)4 |
| 100 | Contig727 | CCCATGTACCGATCAAAGAG | 58.02 | AGGATAACCATAGCCCGGT | 58.347 | 326 | (CAA)4 |
| 101 | Contig728 | GACGGAAATAAACCGTCGTC | 59.434 | GAGAAGTTTTCTCGCOTGTG | 58.65 | 430 | (GGT)4 |
| 102 | Contig734 | CACGTTCCTCCTACCGTAAA | 57.758 | CTAGAACGTCGGAAGGTTGA | 57.952 | 108 | (CTA)5 |
| 103 | Contig737 | GAACGTTGACAAGGTGGAAC | 58.029 | TCGTGTCACGTGGAACATAC | 57.945 | 242 | (TGG)4 |
| 104 | Contig739 | ACGGTATCGTCCTTTCCTTC | 58.121 | AGCATCTCAACGATGCCTAC | 57.921 | 404 | (AGA)5 |
| 105 | Contig744 | TAGTACCACTCCACAAGCCC | 57.671 | TGATGACACCCTTGACAGTG | 57.999 | 204 | (TCC)4 |
| 106 | Contig747 | GACACTCTCTCTCATCCCCA | 57.686 | GCCTCTGCTCTCCFCTTTCT | 57.953 | 302 | (TC)6-(CAC)4 |
| 107 | Contig750 | GACCCATACACAGGCGTTAG | 58.104 | CTGTGAAGCCACATCAACAC | 57.64 | 274 | (TGA)4 |
| 108 | Contig754 | CTCCCTCACTCTTCGAAACA | 58.001 | GCCGACGTAACAGAACAGAT | 57.824 | 369 | (CT)8 |
| 109 | Contig757 | AGCAAATTGGGGCTGTGT | 59.09 | GAGGCCCATATTCTCCAATC | 58.402 | 484 | (ATI)4 |
| 110 | Contig762 | GTTGCTGTCGAAAAGCTCC | 58.585 | CCGAGTGAGCFGTGGTTAGT | 57.959 | 179 | (TC)8 |
| 111 | Contig780 | CTCTCACTCCTCTCAGCAGC | 57.979 | TGTTAAACTCACCTCCGGTC | 57.641 | 439 | (ATG)5 |
| 112 | Contig808 | CCCCTTTCATCTCTCTAGCC | 57.946 | CCGAGCTCTTTCCTAGCTTT | 57.983 | 406 | (AG)8 |
| 113 | Contig816 | GGCCCAGAATCAAGGAAGTA | 59.126 | GGATCCTCACATTCATCTGC | 58.01 | 354 | (GTG)4 |
| 114 | Contig826 | CCCCAATCAAAGCCCTAA | 58.492 | CAAACACACCTTGTTCCTCC | 58.03 | 371 | (TTC)5-(ACC)4 |
| 115 | Contig829 | GTGAAAGGGATGTGTTGGAG | 57.971 | CCCAACCTCATCTTCTCCTC | 58.664 | 122 | (GAT)4 |
| 116 | Contig830 | CAAGGAAGTGGTTGAAGGAG | 57.36 | GTAGGACGCGTAACGATCTC | 57.449 | 298 | (AAG)4 |
| 117 | Contig836 | AATAAGACCCACACCCAACC | 58.62 | GTGACGATGGATGTAAAGGC | 58.019 | 175 | (TCA)5-(TTA)5 |
| 118 | Contig849 | GGTAGGTCGCCfAAGAGAGG | 58.029 | CAGACCACACTATTTCCCCC | 59.259 | 457 | (CTT)4 |
| 119 | Contig854 | GTAACCACATGGACGATGCT | 58.444 | GCAGCTGAATCTCCAACACT | 58.028 | 412 | (CATTCA)4-(TCA)5-(ATG)4 |
| 120 | Contig868 | CCCTACACTCCACCACACTC | 57.979 | TGATCGGAGGAGGTAGTCAG | 57.81 | 483 | (CCA)5 |
| 121 | Contig880 | GGGAAGGAAAGAAAGTGAGG | 57.827 | GTCTTCAGGATGCACCATTC | 58.07 | 332 | (TAA)4-(TCC)6 |
| 122 | Contig880 | TACfCAGGTGATGGTGGGTI | 57.84 | GTCTTCAGGATGCACCATTC | 58.07 | 241 | (TGT)4 |
| 123 | Contig904 | ACCCTAGGGGACGATTGTAG | 58.002 | TICCCTTTAGGCTGTGAGTG | 57.952 | 257 | (ATA)4 |
| 124 | Contig912 | GTCATCTCCAACCACCGTA | 56.809 | GTGTGAGAGGGAGATTGTGG | 58.052 | 207 | (CA)7 |
| 125 | Contig919 | TGCTCCTACAGAGCCAAAAG | 58.256 | CCAACCAGATCCACAAGAAG | 58.13 | 323 | (TGCA)4 |
| 126 | Contig932 | CCCTACCACCTGTTGTCAAG | 58.059 | AGATCCACACAAGCTCCAAG | 57.87 | 328 | (CCA)4 |
| 127 | Contig955 | TGAGTTTCGTGGCTCTAAGG | 58.105 | GAACGGTAGACGGAGCACTA | 57.98 | 224 | (ATA)4 |
| 128 | Contig964 | CACCAAGCTTTTCTGGTCC | 58.272 | AGATGGAGGGGTATGAGAGG | 57.993 | 142 | (AG)8 |
| 129 | Contig974 | CTATTGATGCCCCTCTCTCTC | 57.96 | AAAACCCCACATCTCTCTCC | 58.021 | 111 | (TC)6 |
| 130 | Contig981 | GATTACTCGITCAACGGTCG | 58.228 | AGGAGGAAAAAGGGGGTACT | 58.074 | 327 | (TAG)6 |
| 131 | Contig1011 | AGAGAAATCTGCACCACTCG | 58.029 | CTCCTTCTTCTTCGCCTTCT | 57.882 | 465 | (ATG)4 |
| 132 | Config1048 | GCCGCCFCCACTAGATAAA | 57.929 | AGCATCTTAGCCTCAGGGTT | 58.028 | 283 | (GAA)4 |
| 133 | Contig1051 | CGTCTCCTATCATCCGATTG | 58.117 | GGAGAAGACGATAAGCACCA | 57.893 | 352 | (TTA)4 |
| 134 | Contig1053 | CCGAGTAACCCAACATTCTG | 58.077 | AAGATCCCAAGAACCCAGTC | 58.021 | 397 | (TGA)5 |
| 135 | Contig1054 | CCCTATGTTTCCGATCCTCT | 58.067 | ATCAGCGCTAGATGCTTCAC | 58.229 | 203 | (TCA)4 |
| 136 | Contig1063 | GCAAAACAGATCTACCACCG | 58.228 | CGACTAATGGGTCGAATGAG | 58.173 | 345 | (CTTCAC)-(CTC)4-(ACC)4 |
| 137 | Contig1068 | AACTCCAAACTCCACCTCTC | 55.784 | GCTCCAGATGGAGTCGTTTA | 57.893 | 205 | (ACA)4 |
| 138 | Contig1069 | CCCCTAGGGTTTCATTCACT | 57.975 | CCTGTTGTCCTGCTTGTTCT | 57.93 | 203 | (AC)6 |
| 139 | Contig1081 | AAAGCAGCAACACTCTCCC | 58.014 | ATCTCAACCTCCTCAAACCC | 58.021 | 226 | (TAA)4-(ATC)5 |
| 140 | Contig1095 | TGGCCTCTTCTTTCTAGGGTT | 58.002 | CACGTGTTCTTTCTCAGCCT | 58.087 | 101 | (TTC)4 |
| 141 | Contig1098 | GCAACCCATCTCACACATAC | 56.335 | CACACACGTGTACTCCTCCA | 58.094 | 416 | (AAT)4-(TAA)5 |
| 142 | Contig1098 | AGCTGTTGGAGGAGTACACG | 57.959 | CTGCTGGTACCGACATTCTT | 57.826 | 293 | (TGG)4 |
| 143 | Contig1107 | GGCTATCCACAAATGTACGC | 58.12 | GCATAGTGGCTCCTGTTGIC | 58.316 | 206 | (CCA)4 |
| 144 | Contig1117 | CACCCTCTTCATCGCAAAC | 59.23 | GTCATCCTAAACGCTGTCGT | 57.824 | 207 | (GAA)4 |
| 145 | Contig1123 | GACTCCACCAACCAACACTC | 57.948 | CAGTTGGACCACCAGTAACC | 57.901 | 395 | (AAT)5 |
| 146 | Contig1126 | CCGCTAACAATCACTTCACC | 58.228 | TACATGTGTAACTGTGGGCG | 58.057 | 419 | (ATA)5 |
| 147 | Contig1138 | CGTCTTGCAGGATGAAGTCT | 58.029 | GAGACCCGACAGATTGATTG | 58.071 | 464 | (ATT)4 |
| 148 | Contig1139 | CTCTTCTTCCATCTCACCCA | 57.78 | CCGACTCCTTCTTGAACAGA | 58.001 | 229 | (GAA)4 |
| 149 | Contig1146 | CCGAGTGAGTAGACCCAGAA | 57.871 | ACTGAGTCAACCCGGTCATA | 58 | 167 | (TAT)4 |
| 150 | Contig1146 | GTATGACCGGGTTGACTCAG | 58 | TGGTTCACACCCTCTGTTCT | 58.113 | 383 | (ATGGTC)6 |
| 151 | Contig1158 | GGGAGGATTAAAGGATCTCG | 57.675 | CGCTCAACCAGTTTAGCATC | 58.529 | 414 | (TC)8 |
| 152 | Contig1173 | CGGACCAAACGAAGAACA | 58.156 | CCACCTGATTATTCGACGAC | 58.019 | 221 | (TCT)4 |
| 153 | Contig1175 | CAACCCCAATTATACCCCTC | 58.11 | TAACACGTGGGTAACACCCT | 57.856 | 338 | (CAG)4 |
| 154 | Contig1189 | ACTCAGGCATCTGATCATGG | 58.616 | TCAGTTTCTTGCCCTGCTAC | 58.104 | 279 | (TCT)4 |
| 155 | Contig1197 | ATACAGGTCACTGGCTCTCG | 57.9 | CCTAGGAGAACCCCACTTGT | 58.106 | 403 | (GAA)5 |
| 156 | Contig1199 | ACCTAAACACACCGAGTCCA | 58.059 | GCAACGAGTTCAGACCACTC | 58.436 | 213 | (TCC)4 |
| 157 | Contig1202 | TAAGACGGAGCACAAGGAAG | 58.105 | ATAACAGCTCTGCCTTCACG | 58.132 | 196 | (AGC)4 |
| 158 | Contig1211 | TTACTTAAGCCCTGTCGTGG | 57.907 | GGATCAGAACCTTCATGCAC | 58.07 | 216 | (CAGGTT)4 |
| 159 | Contig1231 | TGCTCACCAGGTTAGGTGTT | 58.215 | GCTCTCAAGTTACTGCAGCC | 57.853 | 314 | (TTA)4 |
| 160 | Contig1233 | TGTGTGCTCTACGAACCAGA | 57.977 | CCTCTTAACCAGAGGCACAA | 57.952 | 498 | (ATG)5 |
| 161 | | GAACATCTGCAAACCCTAGC | 57.419 | CACCACTGTTATCAGCACCA | 58.112 | 454 | (CAT)4 |
| | Contig1234 | | | | | | |
| 162 | Contig1248 | GTTCTGGTGTTGATGGGAGA | 58.482 | ATCTCCACCATCCTTCCTCT | 57.555 | 480 | (ATG)5 |
| 163 | Contig1249 | GATGGCTATGTCTATGGCGA | 58.699 | GCTTCTCTAGTTCGCAGTCG | 58.008 | 284 | (TCC)4 |
| 164 | Contig1253 | GGAATCTCTGACCAATCCCT | 57.964 | CCACACACCTTCACAACTCA | 58.063 | 442 | (TAT)4 |
| 165 | Contig1260 | CAACCCTTGTGACCCACTAC | 57.901 | ACAGCCACCACTCTAACCAC | 57.639 | 384 | (GA)8 |
| 166 | Contig1264 | TACACCTTCACCCACTCCAT | 57.84 | AAACATAGACTCGAACCCCC | 57.974 | 467 | (AAT)4 |
| 167 | Contig1276 | ACCACCACATACACCACACA | 58.062 | CGGGTTTGCTGACTTACACT | 57.884 | 450 | (TAT)4 |
| 168 | Contig1277 | GTIATCCCATGGAAAGACCC | 58.16 | GAACCTCTTAACCGCTCTCC | 58.001 | 351 | (ACC)5 |
| 169 | Contig1278 | GGAAGTCTTACGTGTCACCG | 58.215 | GATTCAGGATTCAGGTCACG | 58.071 | 190 | (TTA)4 |
| 170 | Contig1279 | CTGGATGTTGAGATCCGACT | 57.647 | GTTACACCCAGGTTGGTCTG | 57.901 | 228 | (CAA)4 |
| 171 | Contig1279 | CGGAGTGAAGCTACAAGGAA | 58.105 | CAGACAACCCTGTTGAAACC | 58.03 | 194 | (AAT)5 |
| 172 | Contig1286 | GAGTTGGAGGGGTTCTTGTT | 58.078 | GTCTTCAGGATGCACCATTC | 58.07 | 231 | (CTC)4 |
| 173 | Contig1303 | CACGTTGTAAGCACGTCATC | 57.764 | TAGGGCTTGTGTGACCACTT | 58.215 | 495 | (TGTA)4 |
| 174 | Contig1307 | CCTTGCTCCAGTGTCTTGTA | 56.46 | TAAGTTAGCCCGTTGTCGAG | 58.054 | 444 | (CAG)4 |
| 175 | Contig1307 | TCGACAACGGGCTAACTTAC | 57.906 | CTTGTAGTGCATTTCCCACC | 58.076 | 472 | (CCT)4 |
| 176 | Contig1313 | CGAAGGAGAAGACTCCACAA | 58.001 | CACCTTCCTGAATGATCTGG | 58.071 | 443 | (CAA)5 |
| 177 | Contig1316 | CATCGATCGGAACTGCTAAC | 58.326 | CTTCCTTCTTCACTGGCGTA | 58.105 | 195 | (AAC)5 |
| 178 | Contig1318 | TTTCTATCGAGGGAAGGAGG | 58.333 | GCCTTCTGTCAAACACTGCT | 58.087 | 425 | (GAT)4 |
| 179 | Contig1333 | GGGTACAGAAACCAACCGAT | 58.766 | AGCACTGACGACTAACCCAC | 57.8 | 142 | (CAA)5 |
| 180 | Contig1354 | AGAAGAGCCTCCACAAACCT | 57.982 | CATCACCGTCATCACCAGTA | 57.883 | 262 | (AAG)4 |
| 181 | Contig1358 | ACTCTTCGCCTTTGATCCTC | 58.473 | CTACAGCCAAGGAATGTGCT | 57.98 | 377 | (CTT)4 |
| 182 | Contig1376 | TGACTGTATGGAGACGGAGC | 58.83 | CATCGATACACCGATCTTCC | 57.961 | 119 | (CGC)4 |
| 183 | Contig1379 | TCTTCCCCTCCATATCACAC | 57.357 | TCTGAGAACAGAGGACAGGG | 57.919 | 123 | (ATI)5 |
| 184 | Contig1381 | AAAAGAGGAACGAGGAGTGG | 58.376 | TGTCCTGAGCCTCATACTCC | 57.809 | 132 | (GAA)4 |
| 185 | Contig1384 | ACACAGGATCAACCCAGCTA | 58.158 | GAACAGGAGGCTGTTGAAGA | 58 | 289 | (CA)7 |
| 186 | Contig1394 | AGAGGCTAAAGAACCCGAAG | 57.697 | CCAACCAGATCCACAAGAAG | 58.13 | 155 | (TGCA)4 |
| 187 | Contig1397 | GGAACCTCAGGGTTACGAAT | 57.974 | ACCAGAGTCTCCCTCTGCTT | 58.06 | 379 | (GAA)5 |
| 188 | Contig1403 | CACCCTTACAACCAGACACC | 57.901 | GTGCTCCATATAATCCCCCT | 57.82 | 339 | (CGA)5 |
| 189 | Contig1407 | GCTTACCGTCATTCACTGCT | 57.979 | GTAGGTGTAATGTGGGGGTG | 57.641 | 444 | (CAC)4 |
| 190 | Contig1426 | GGTAACACCACCATCAGGAG | 57.84 | CAAGTGTAACCAGGAGGTGG | 58.059 | 324 | (CCA)4 |
| 191 | Contig1459 | CATAACCCAAGTTCTGGTGG | 57.924 | GAGCTGAATCTTTCGCTCTG | 57.922 | 377 | (CTT)5 |
| 192 | Contig1481 | GCAGATGGTCAGATICATCC | 58.01 | CAGTCGGAGTAAACCCTGAA | 57.799 | 355 | (AGA)4 |
| 193 | Contig1493 | CCGATCCTTCTCTCTCCTTC | 57.993 | CTCTTGAGAAGCATCACGGT | 58.029 | 278 | (AAG)4 |
| 194 | Contig1494 | GAAGAAAGAGGTAGACCGGG | 57.854 | ACCCCAACACGACTGTTTAC | 57.417 | 245 | (TAA)4 |
| 195 | Contig1501 | ATTCAGCGTCTGCTAGCCT | 57.809 | GATGGTGATCTCATGGAAGC | 58.01 | 328 | (ITC)4 |
| 196 | | CTCTAACTTTGGACGACGAC | 55.024 | TGAACATGAGCCTCCTTAGC | 58.048 | 334 | (CTT)4 |
| | Contig1503 | | | | | | |
| 197 | Config1503 | GCTAAGGAGGCTCATGTTCA | 58.048 | ACATTCATCCCCTCCTCTTC | 57.964 | 260 | (TCT)4 |
| 198 | Config1525 | TGGACTGAGGGCATTATAGC | 57.791 | AAACCACTTCTCACAGCAGC | 58.087 | 181 | (CTG)4 |

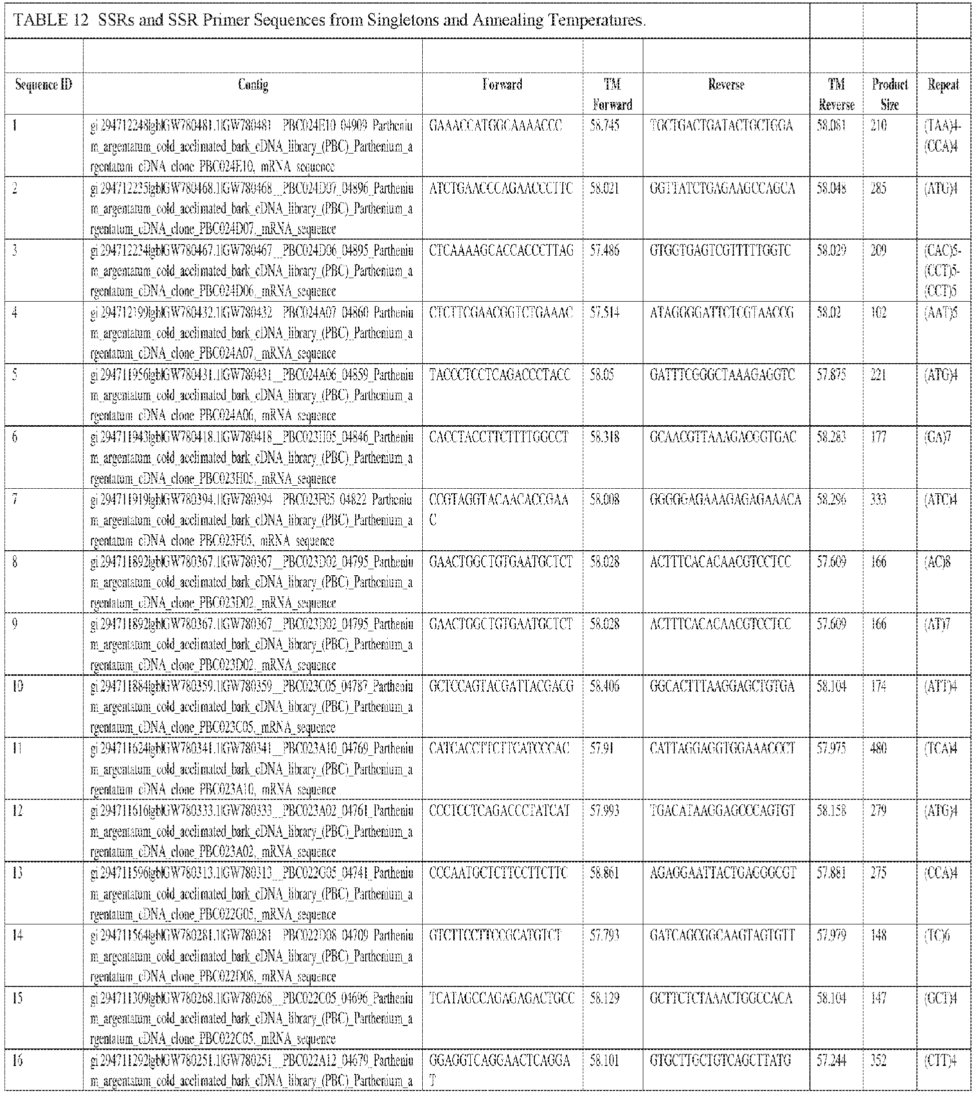

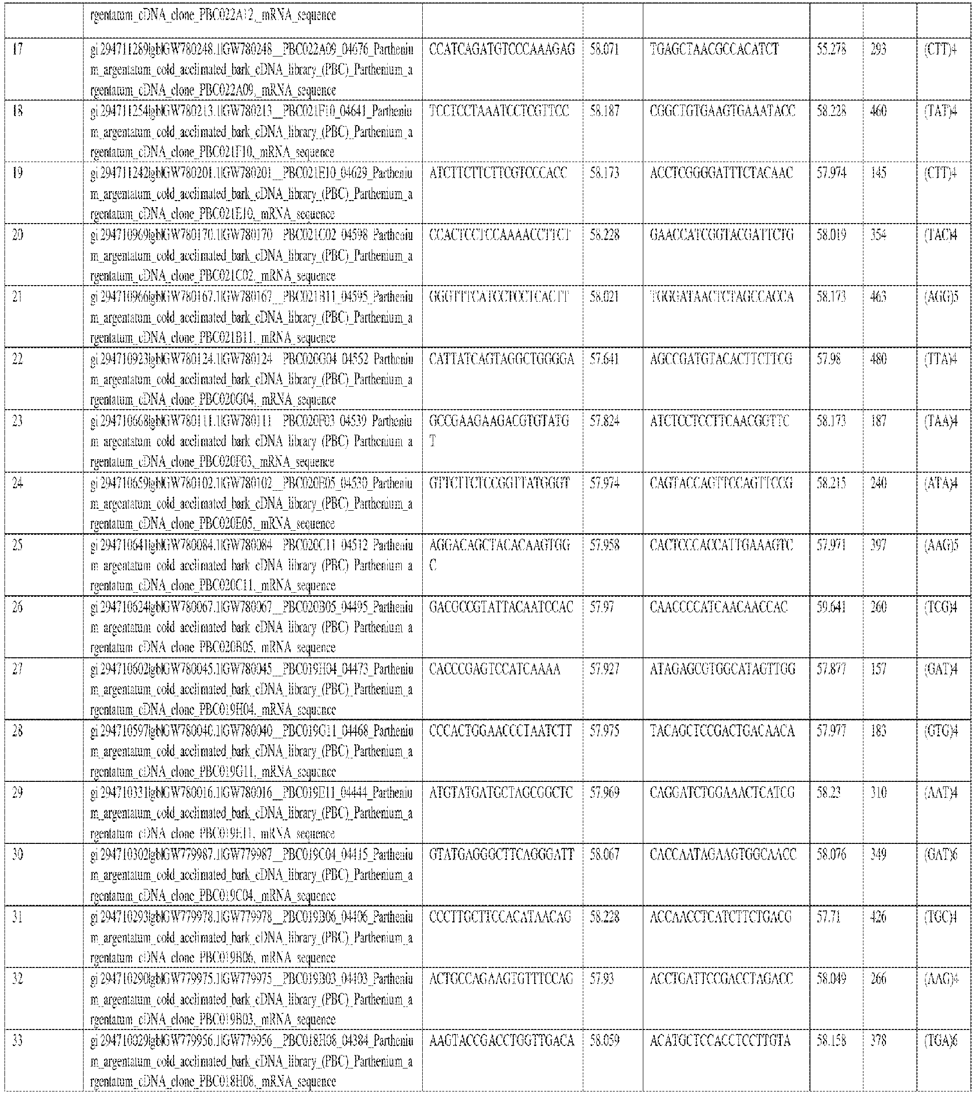

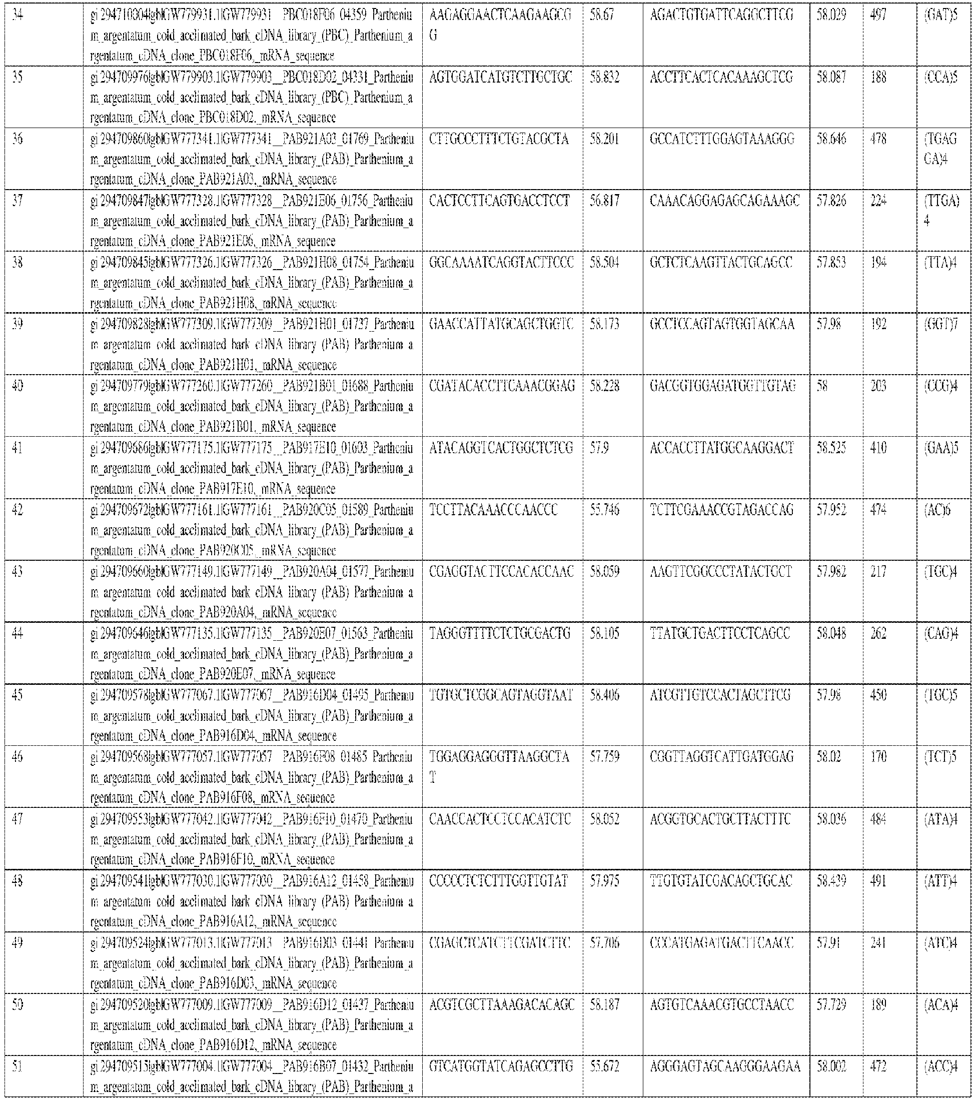

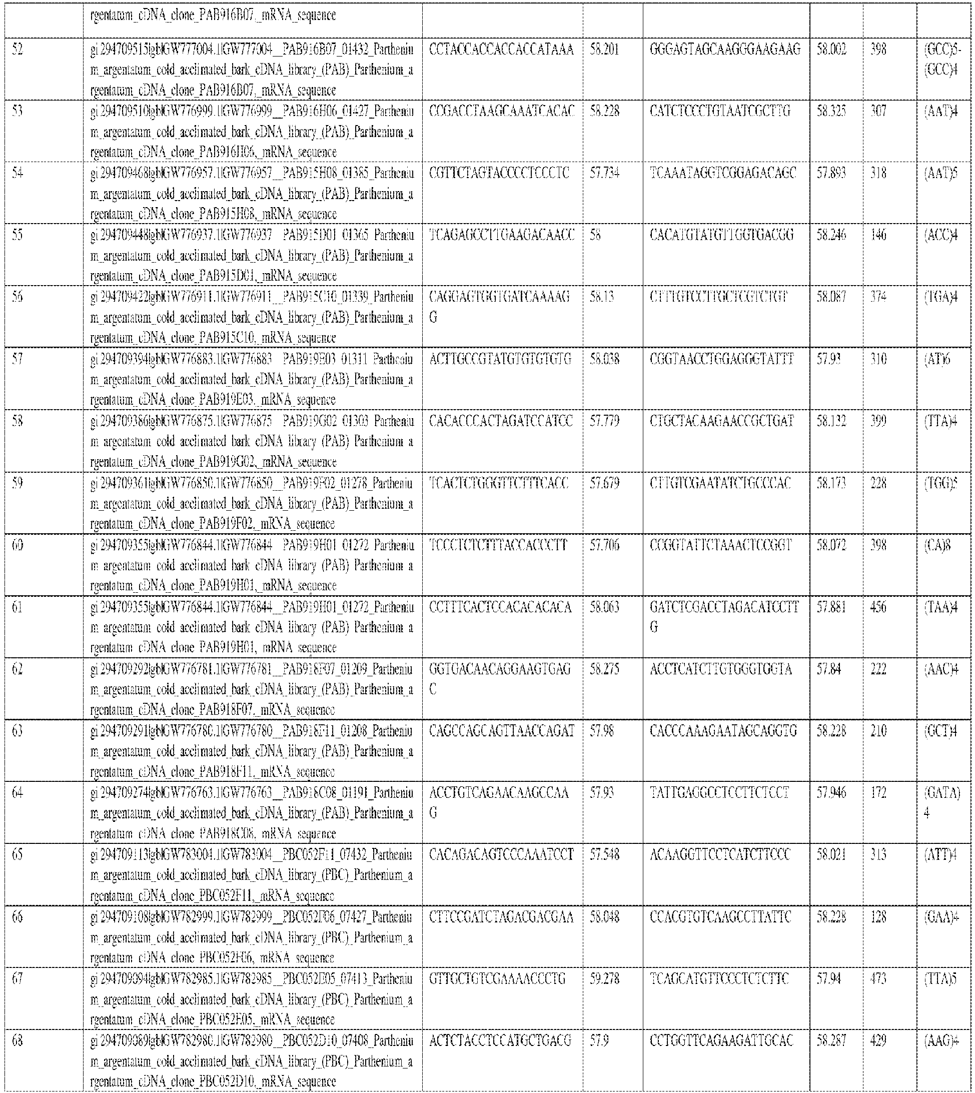

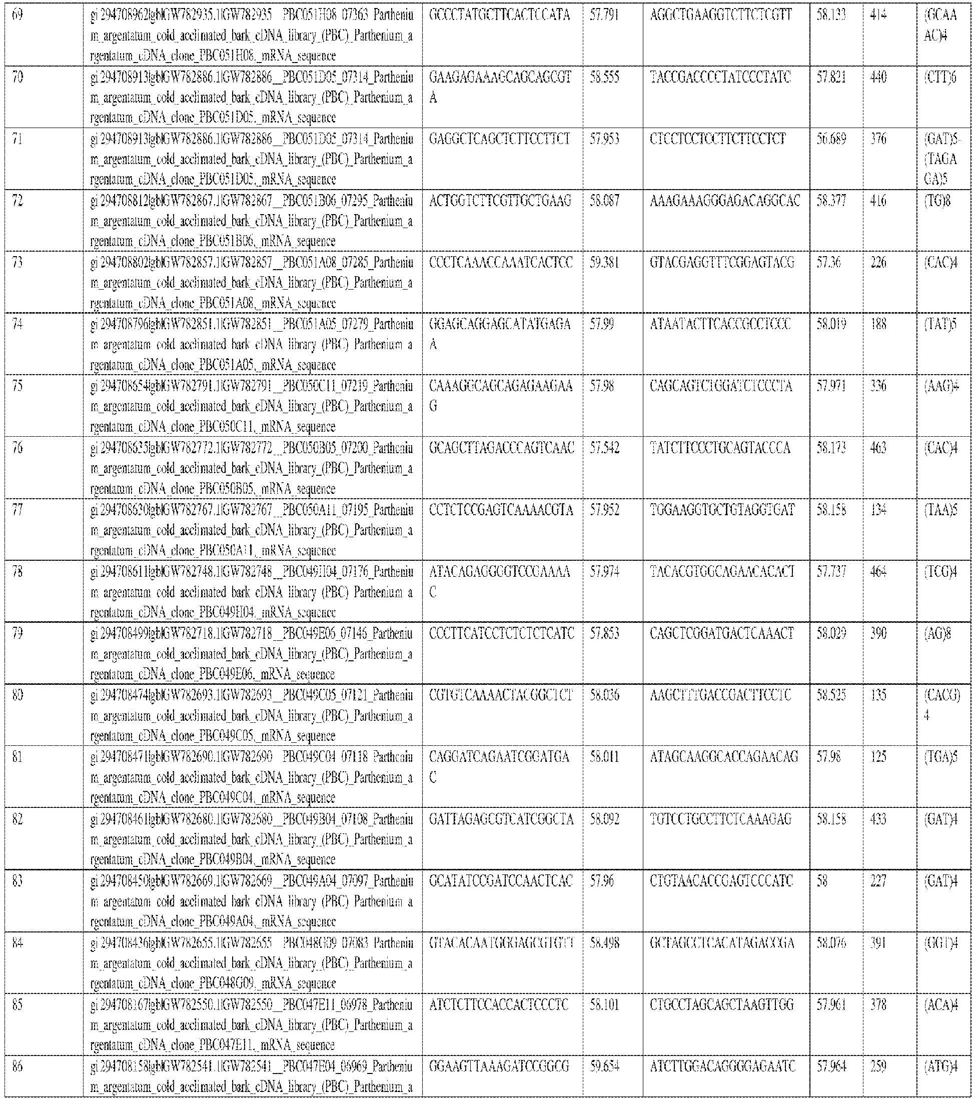

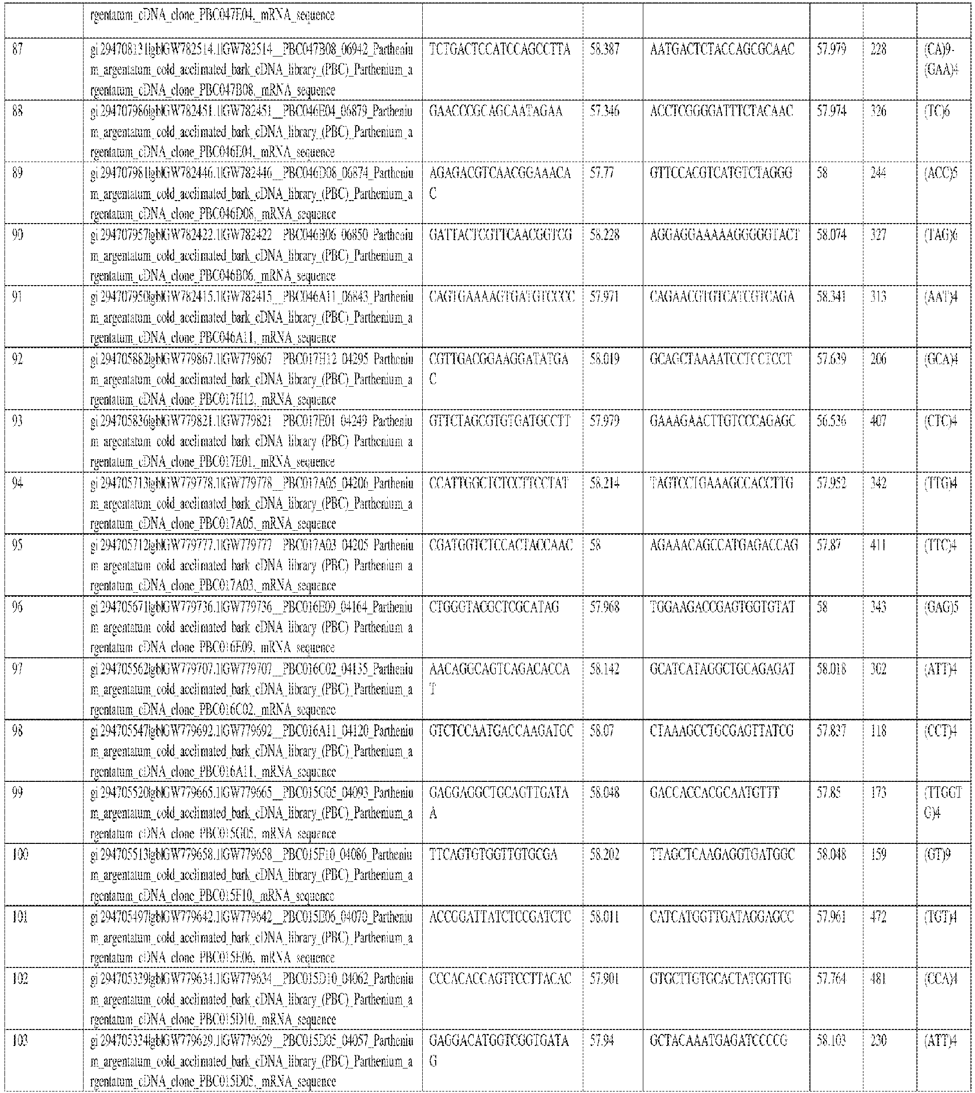

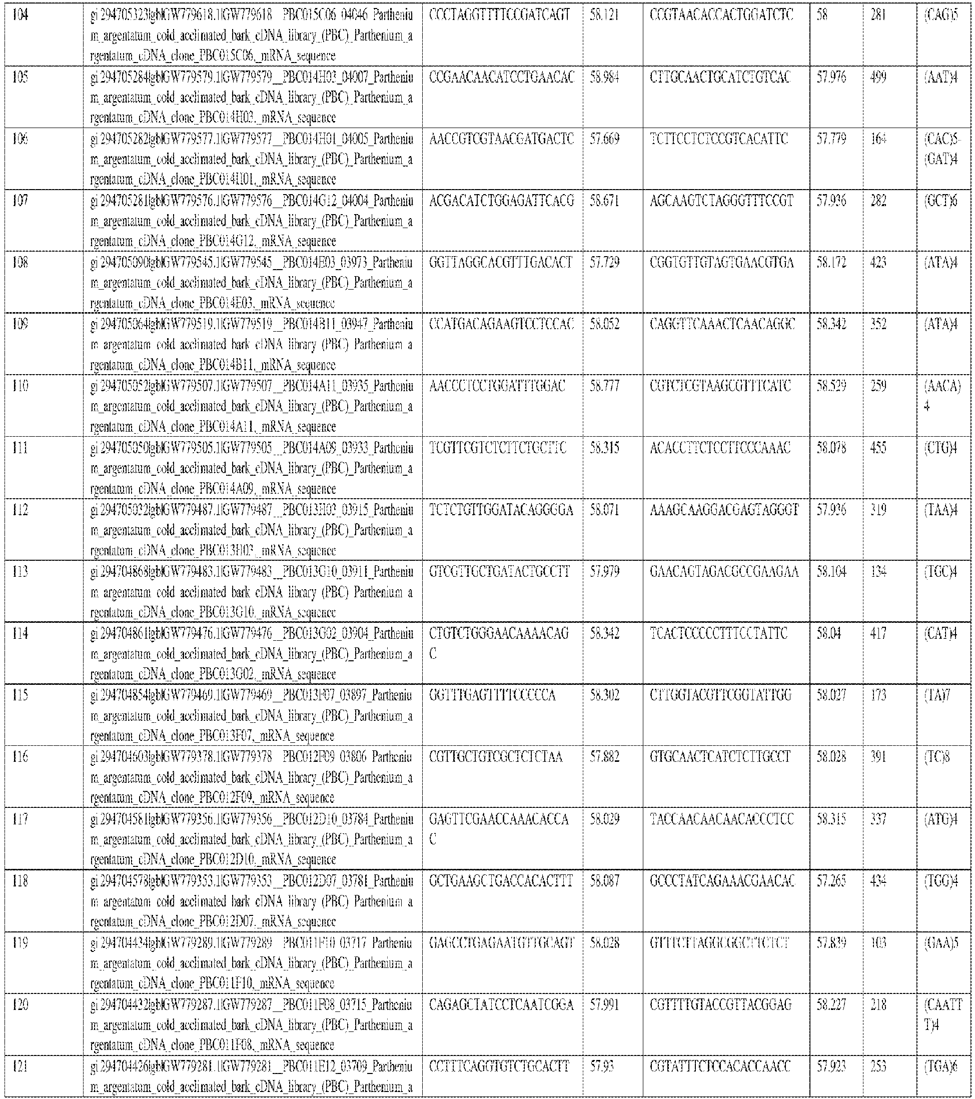

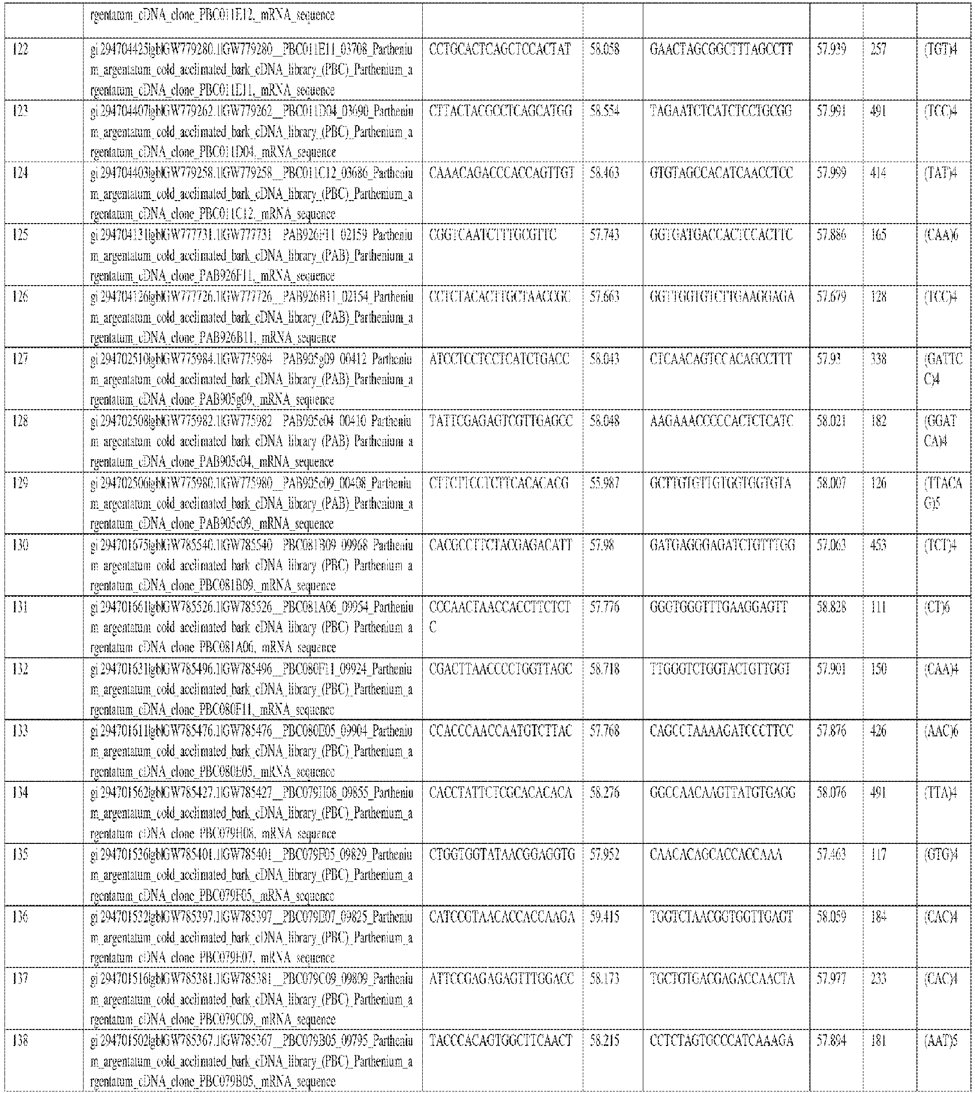

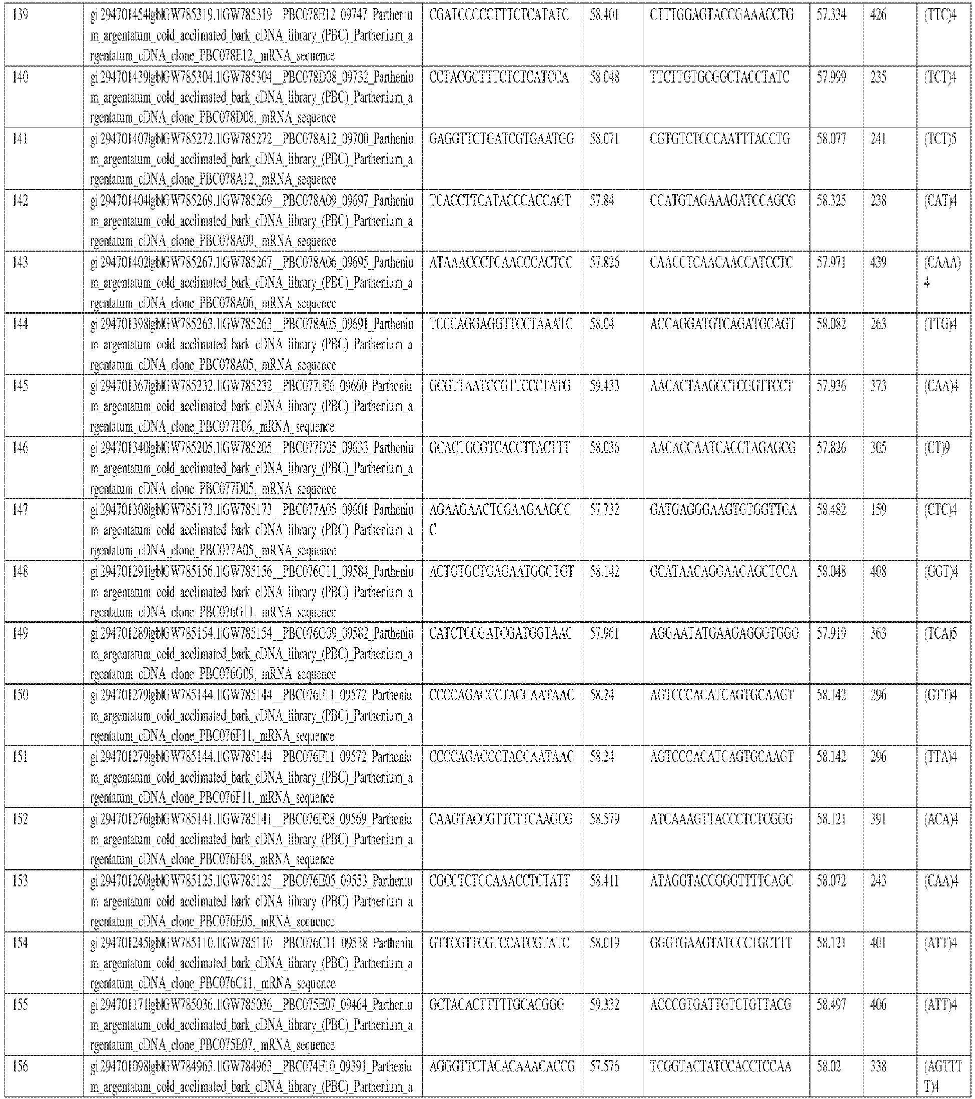

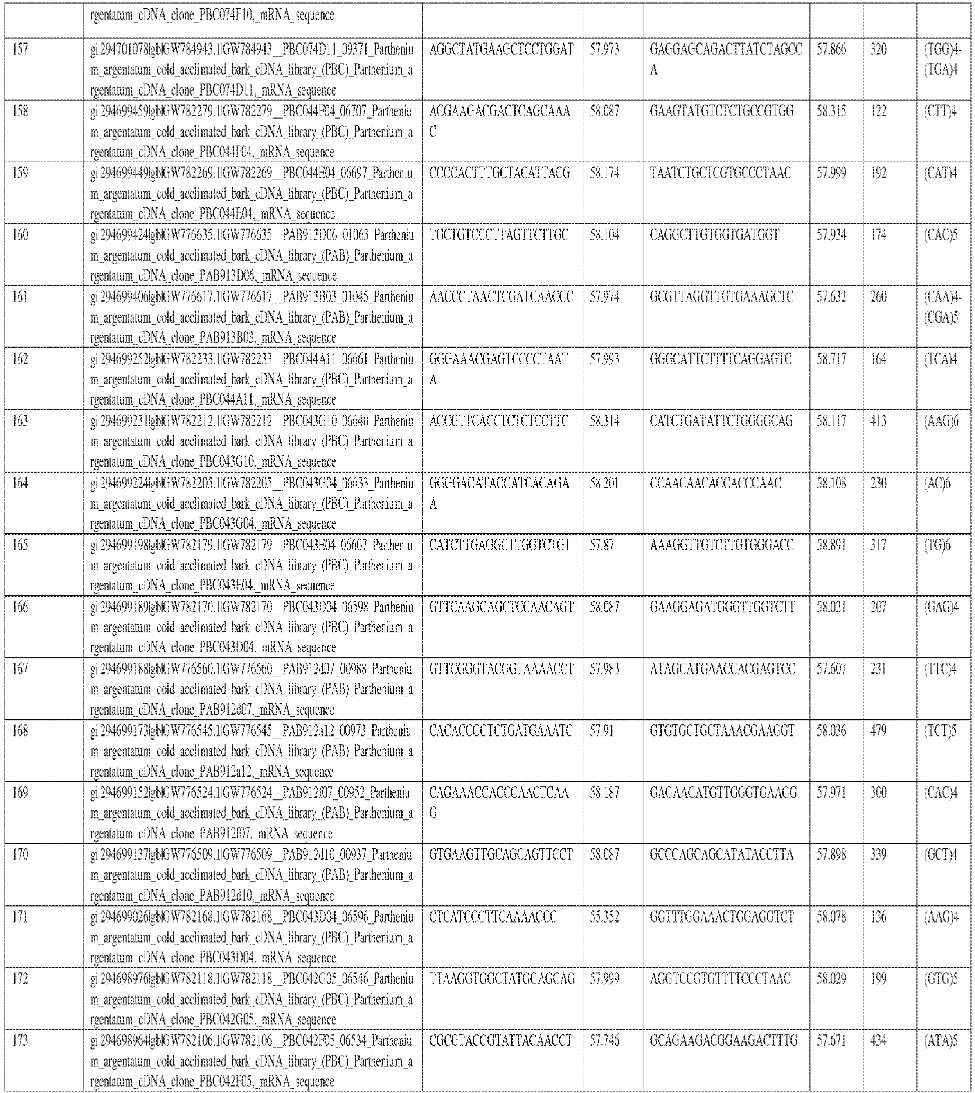

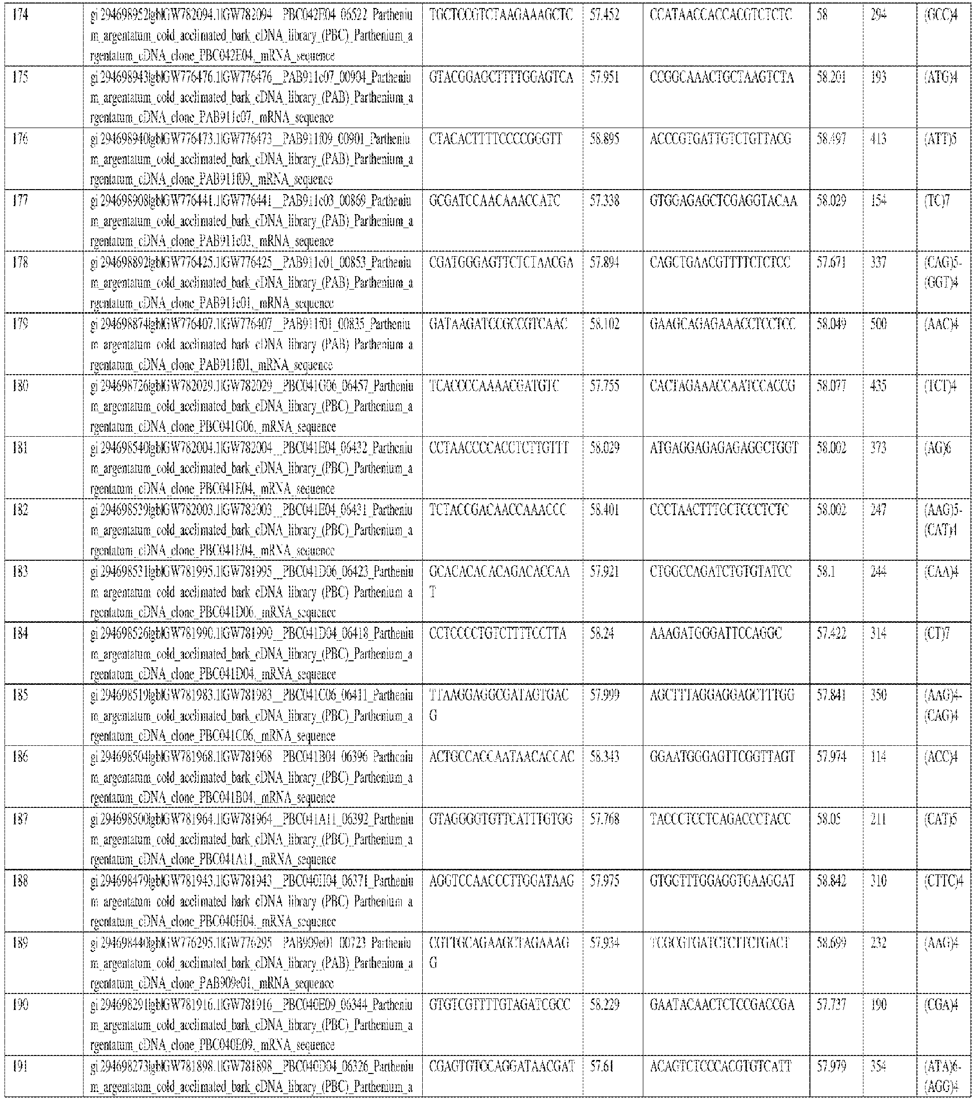

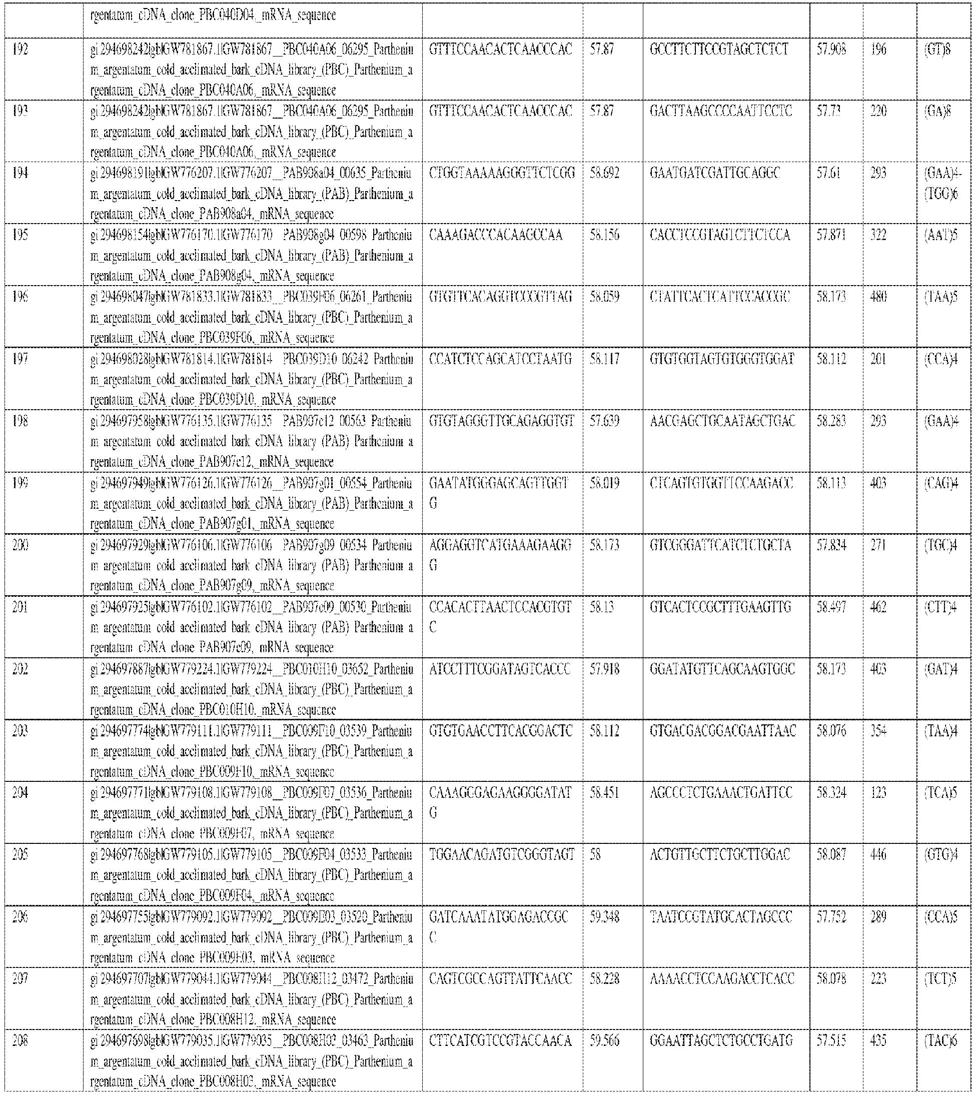

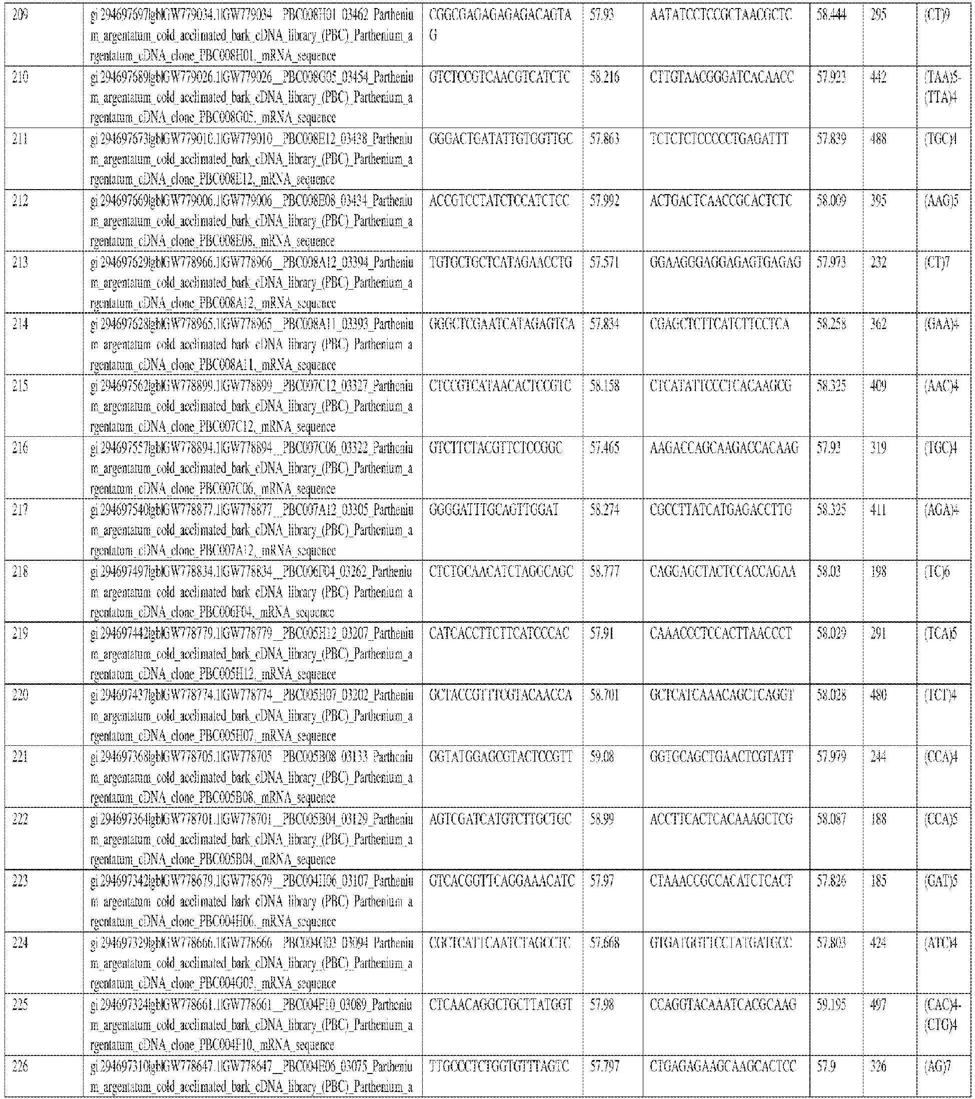

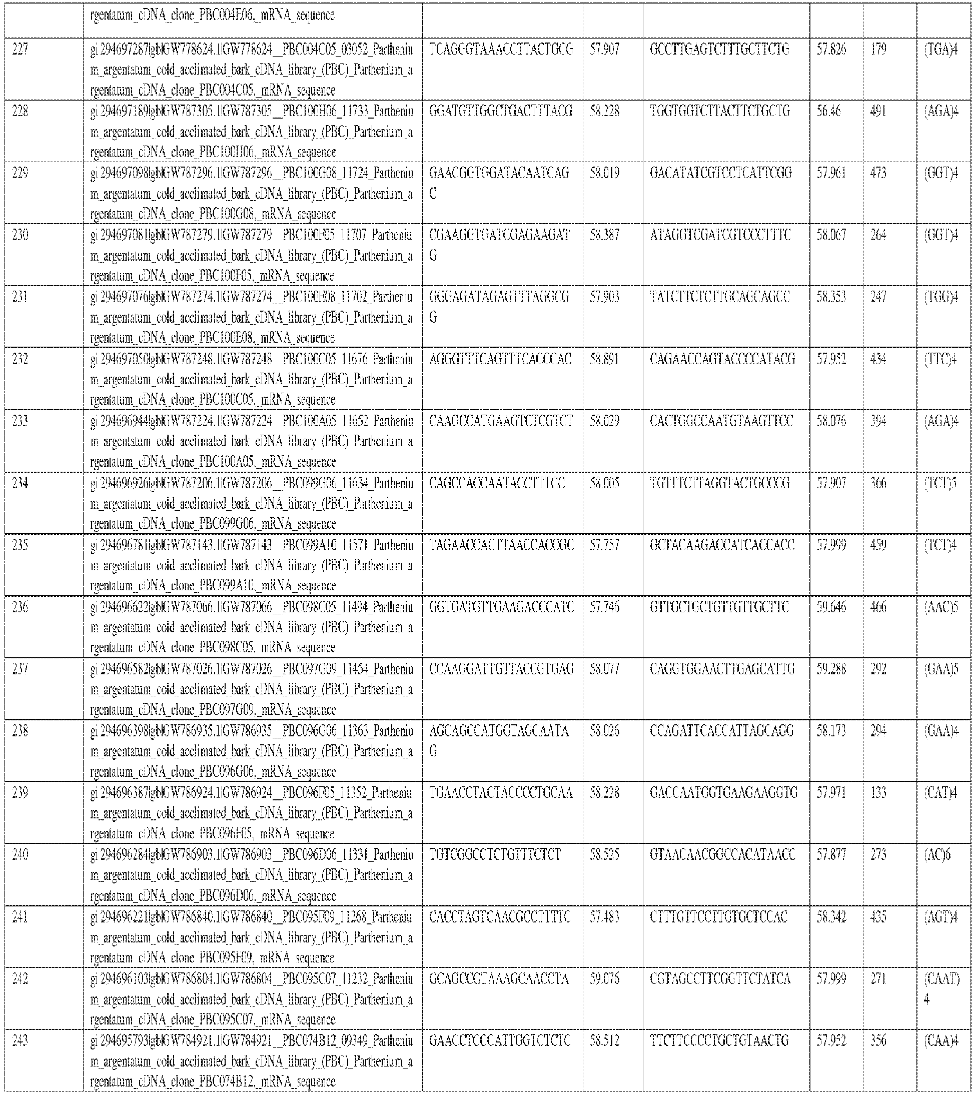

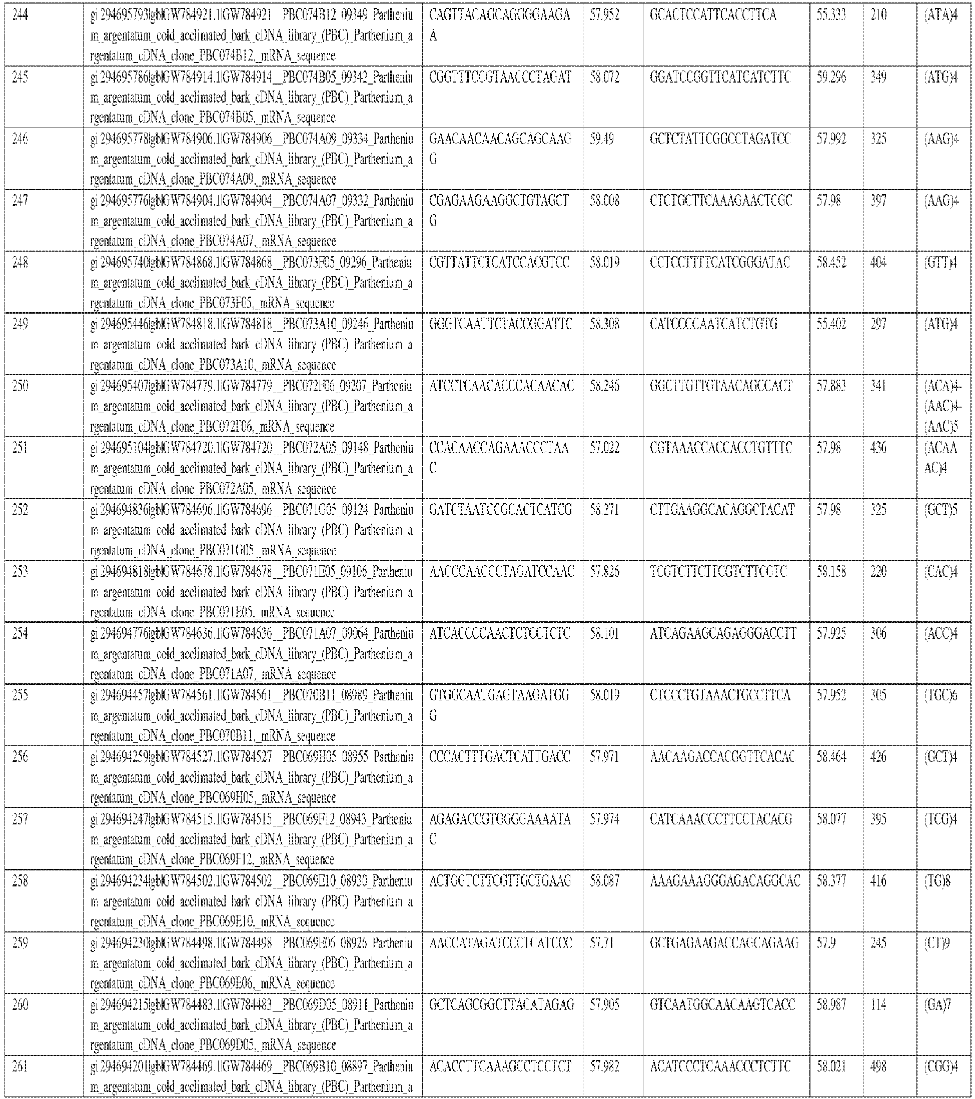

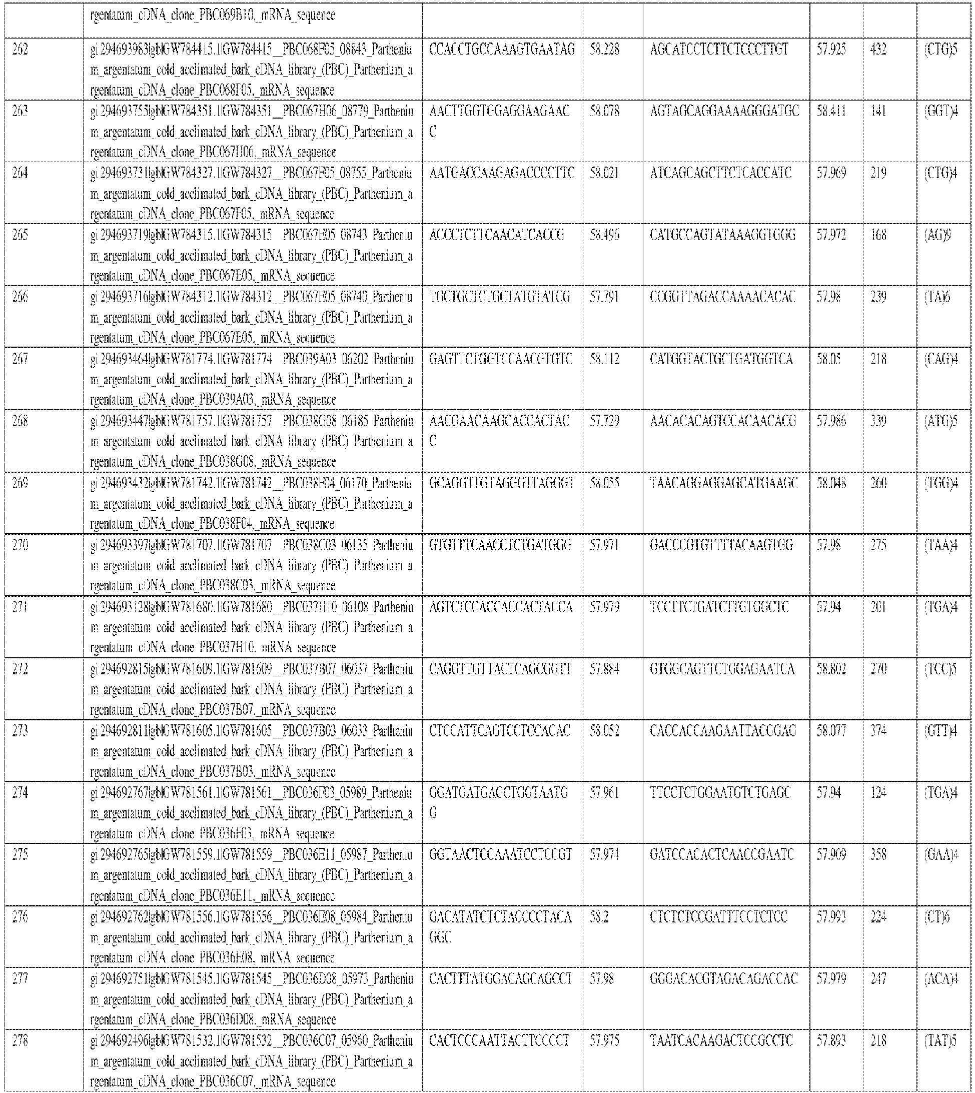

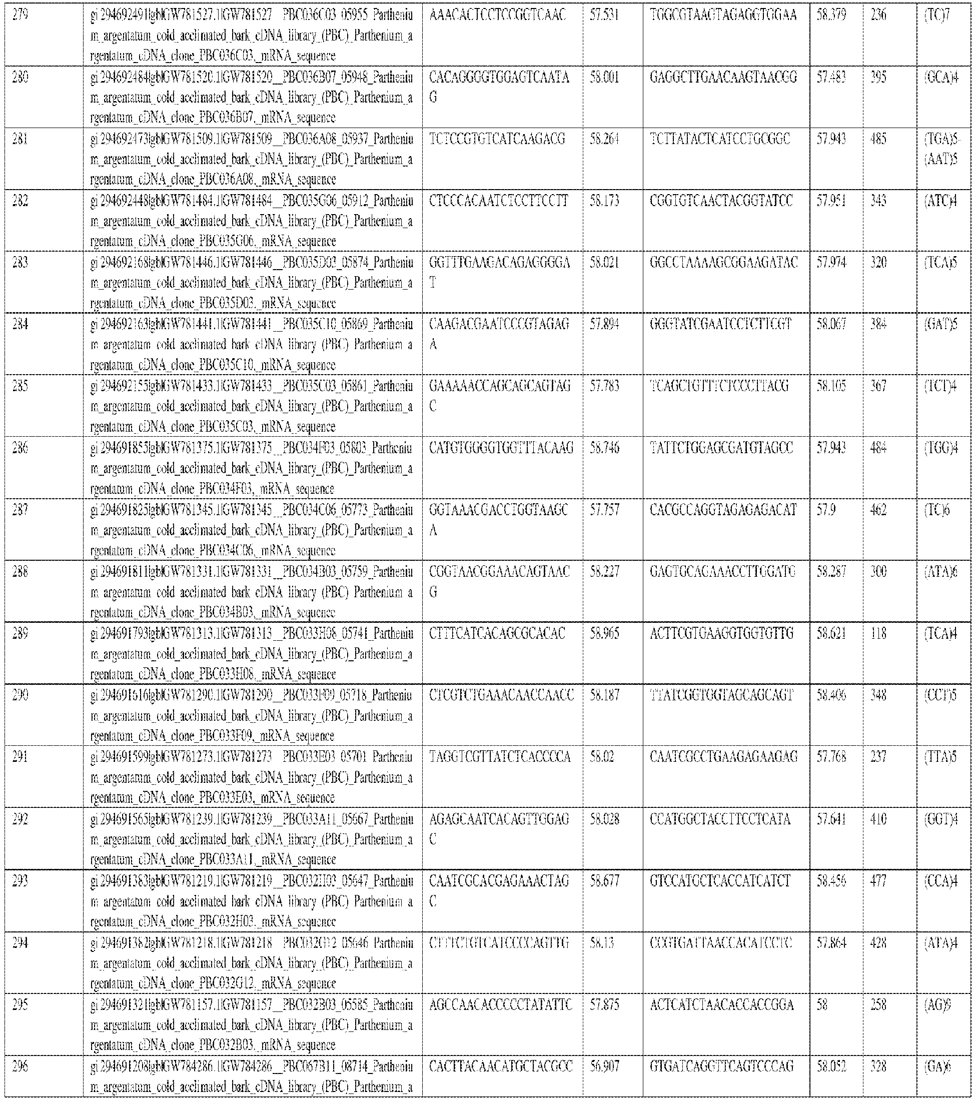

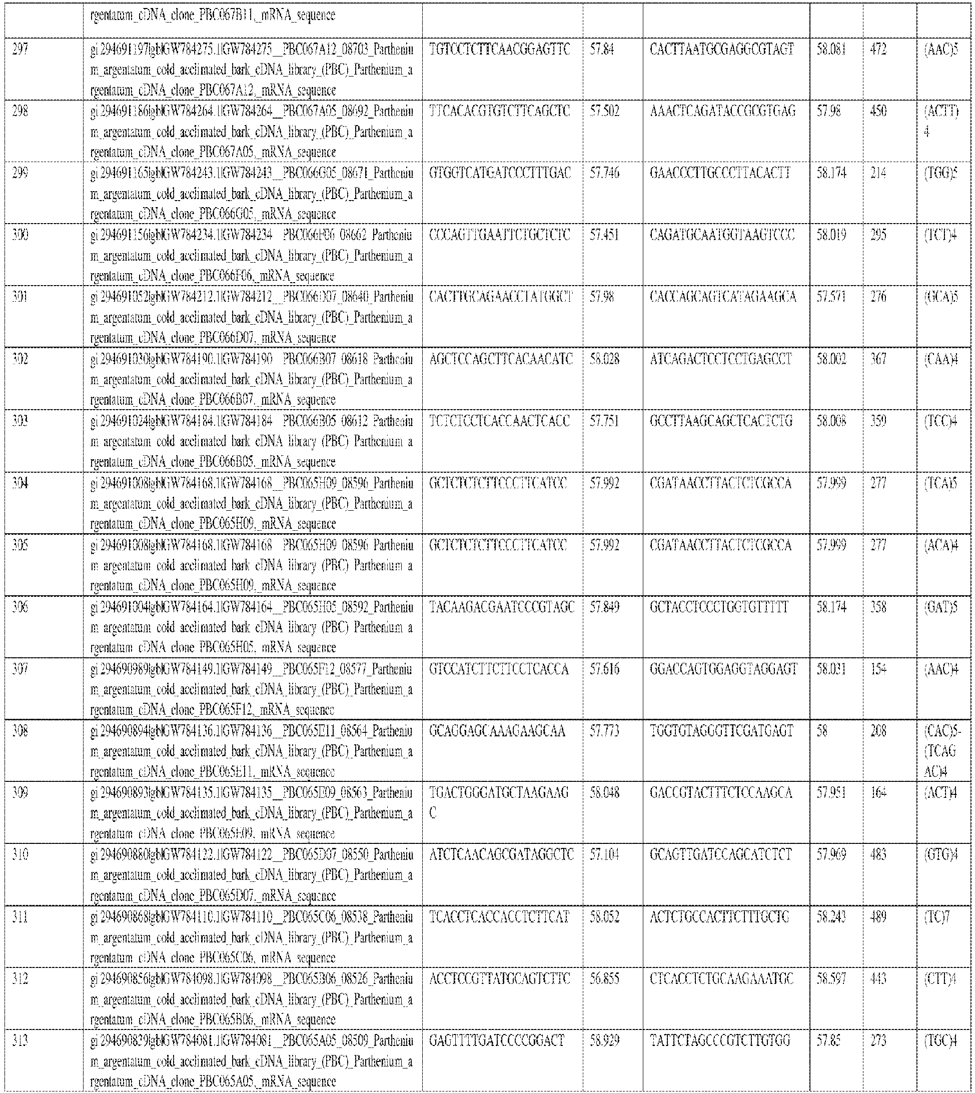

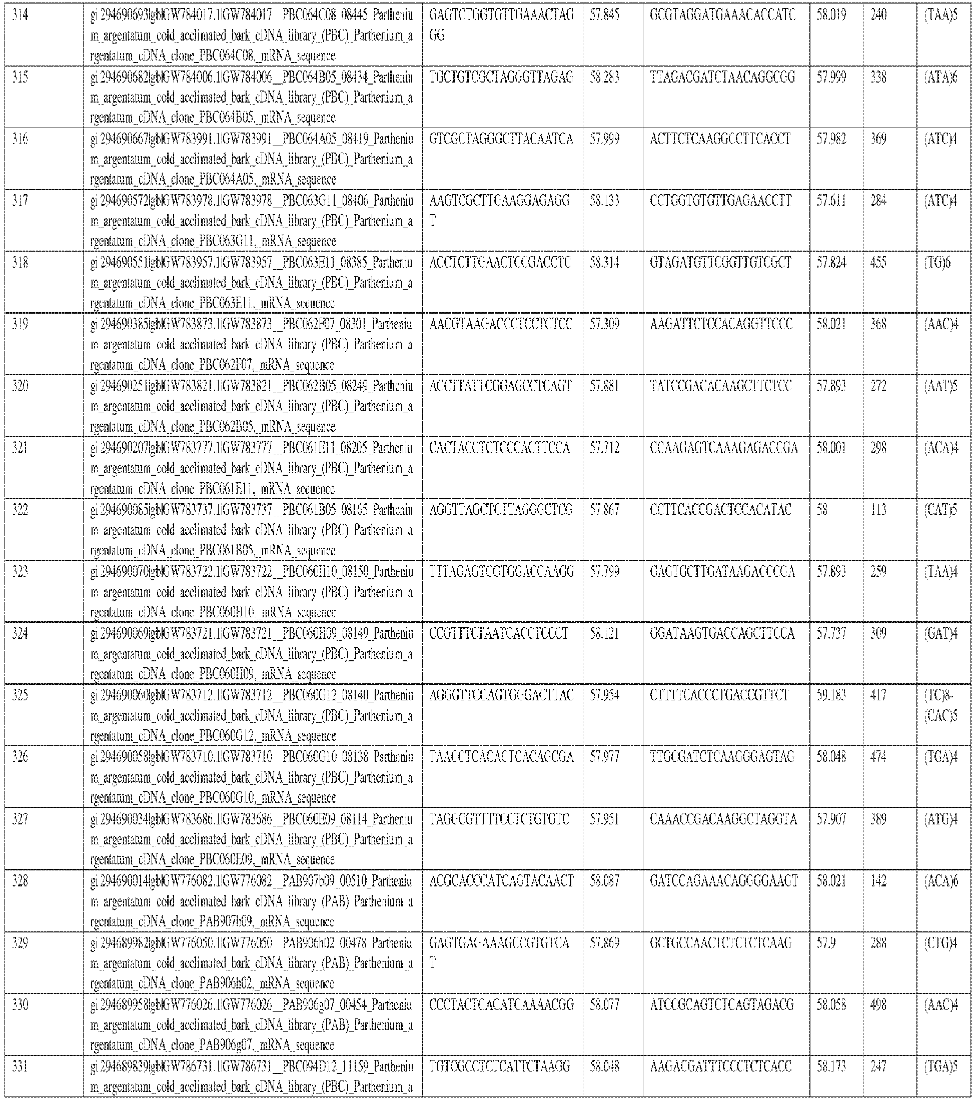

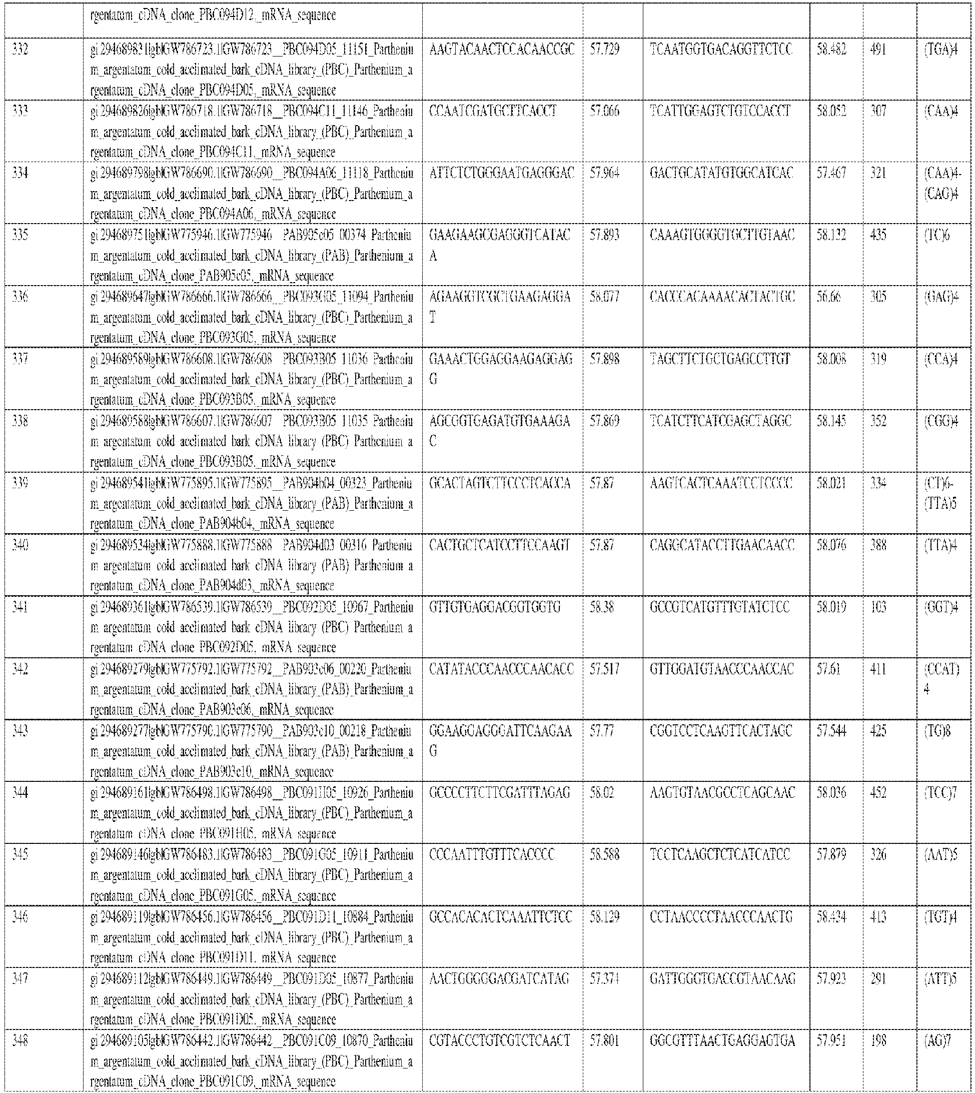

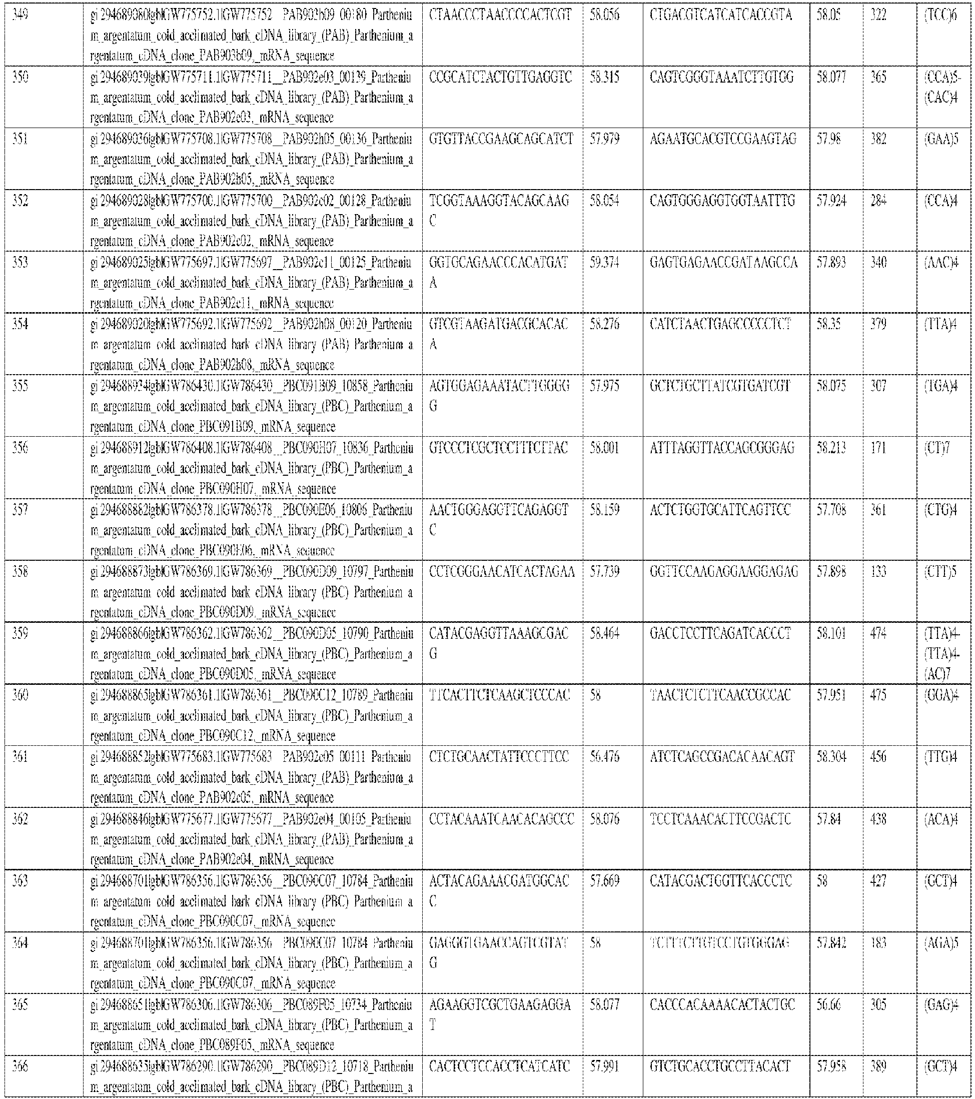

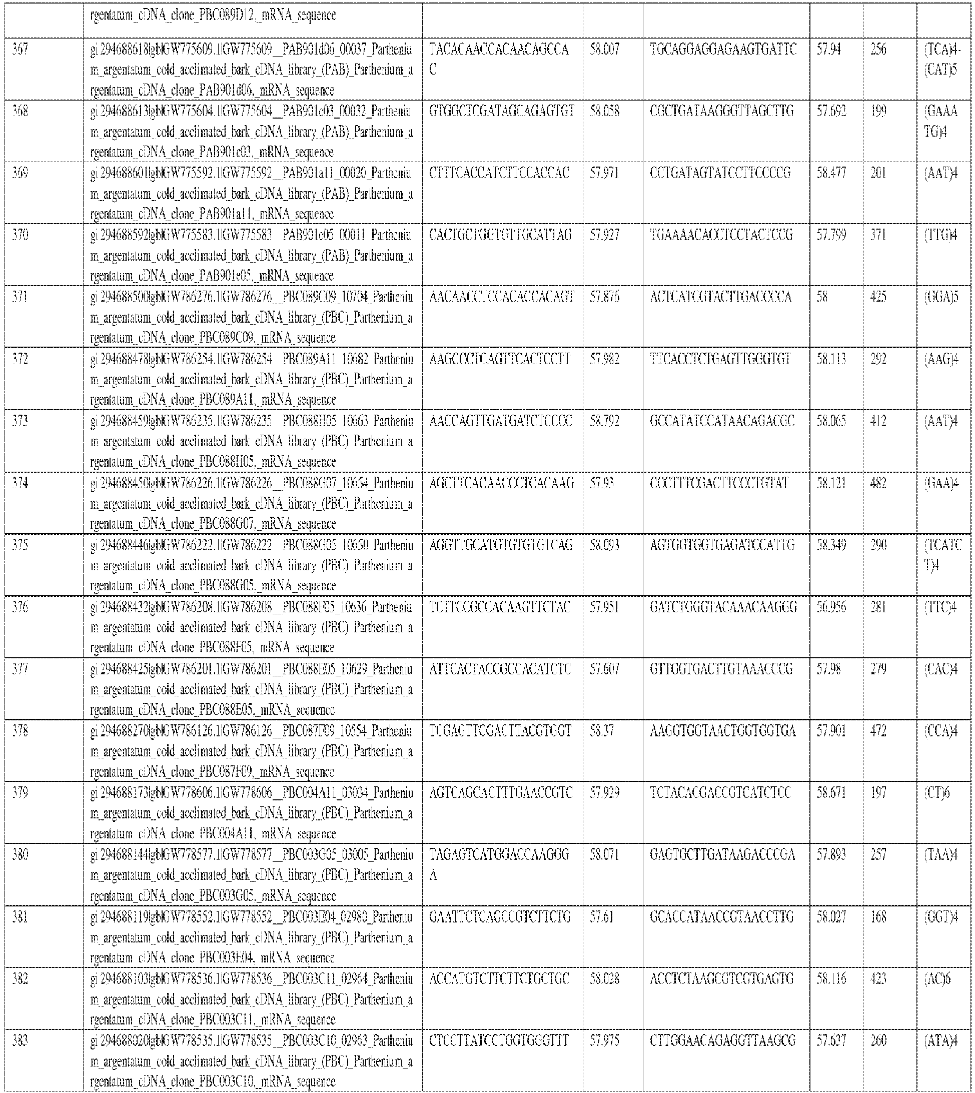

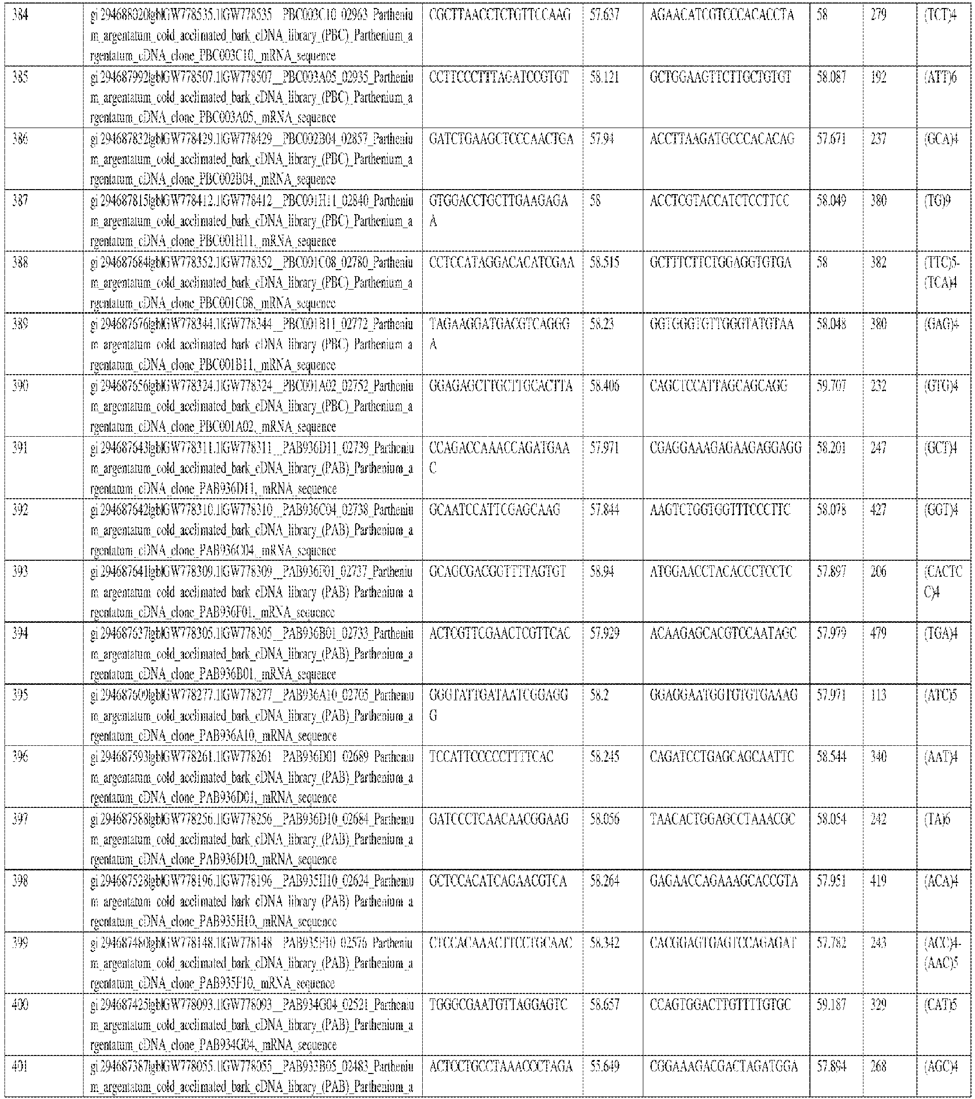

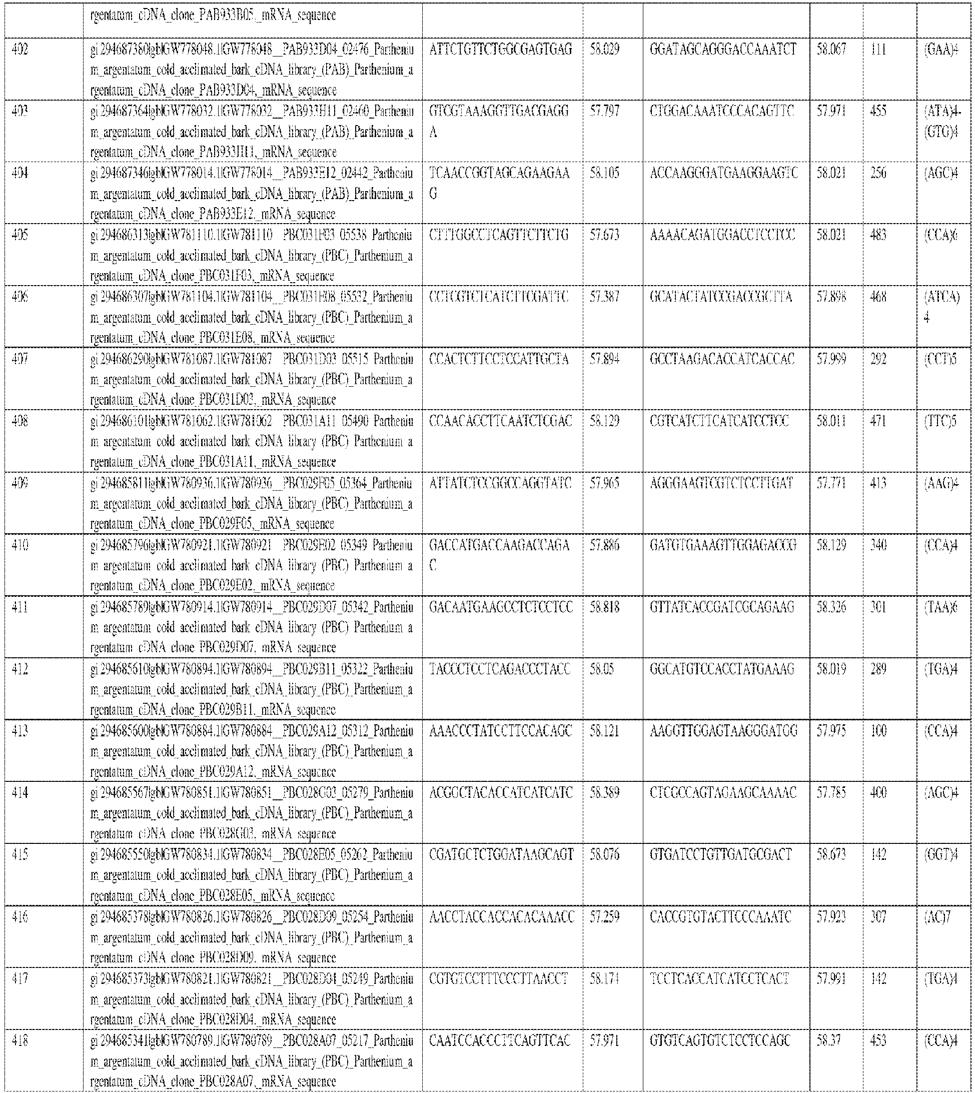

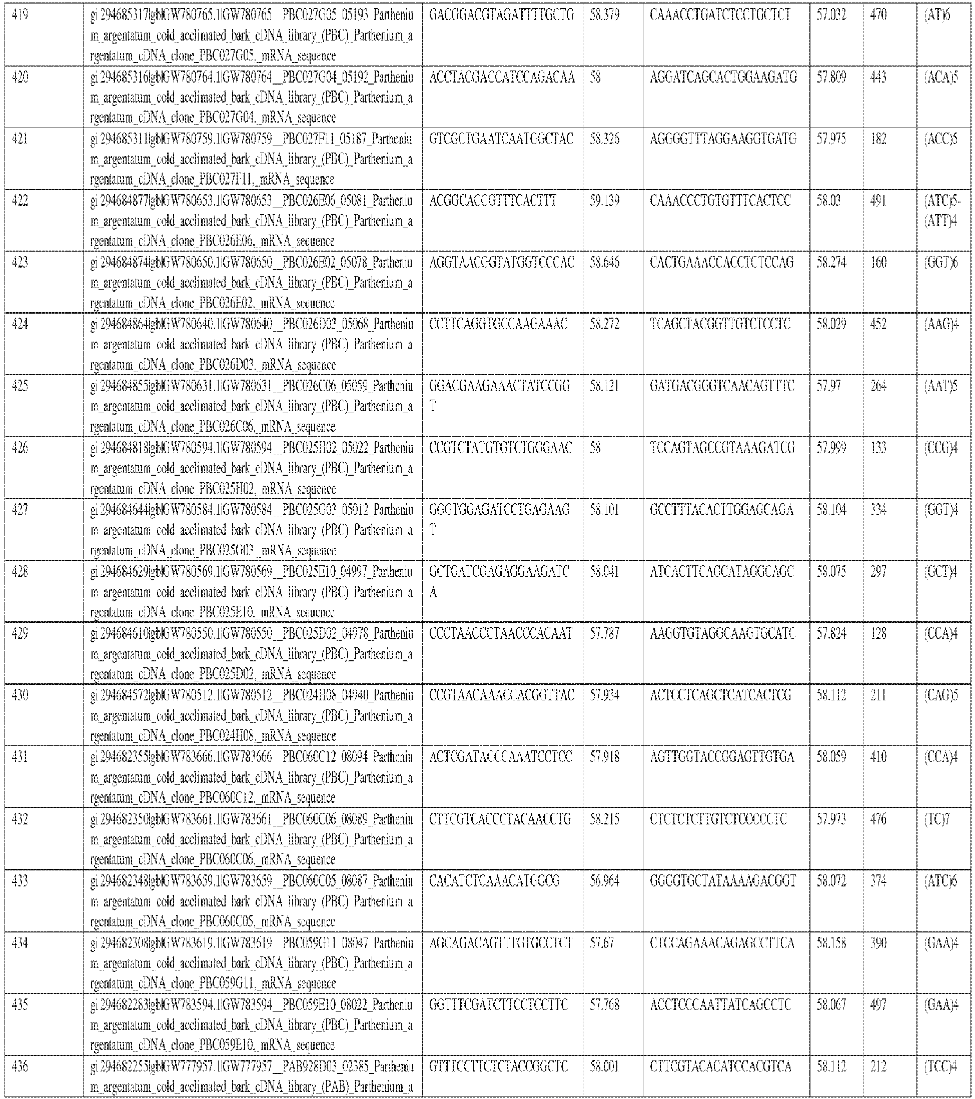

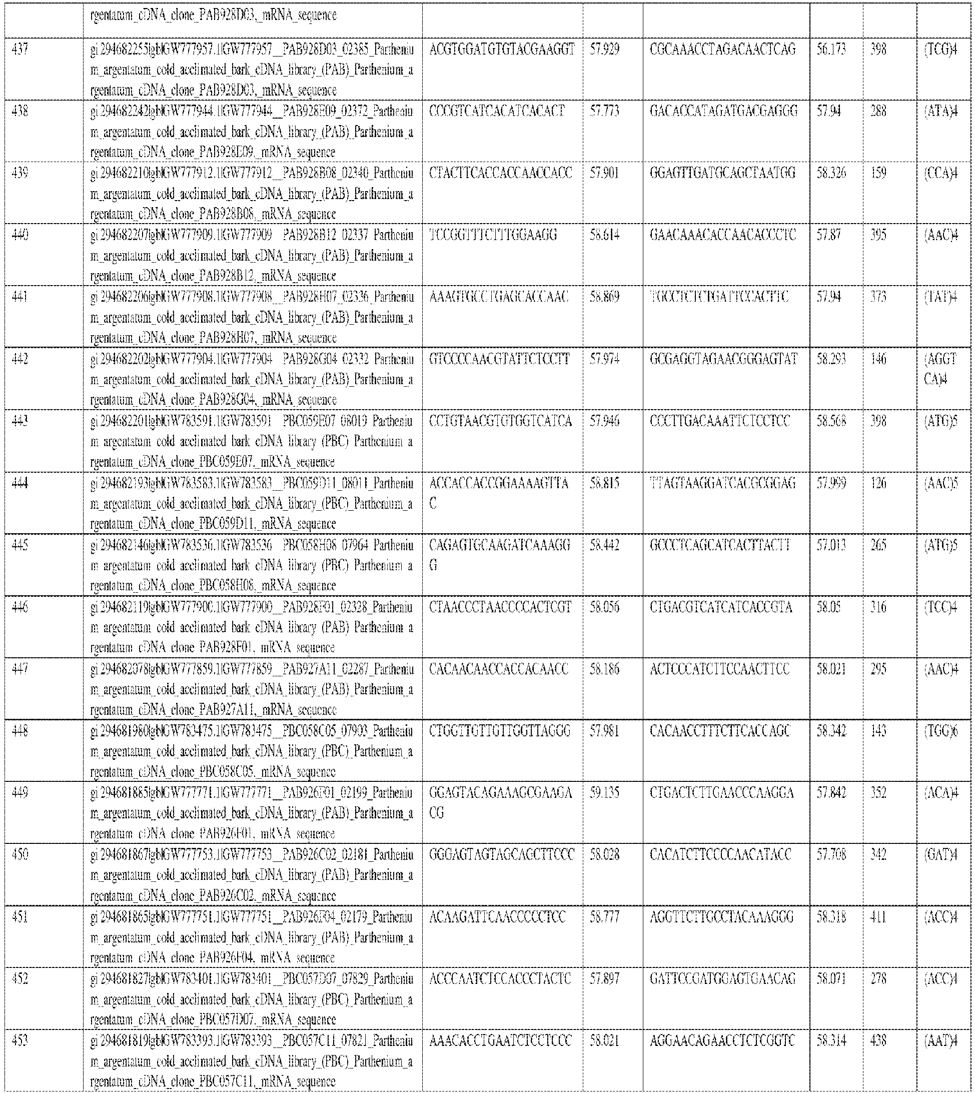

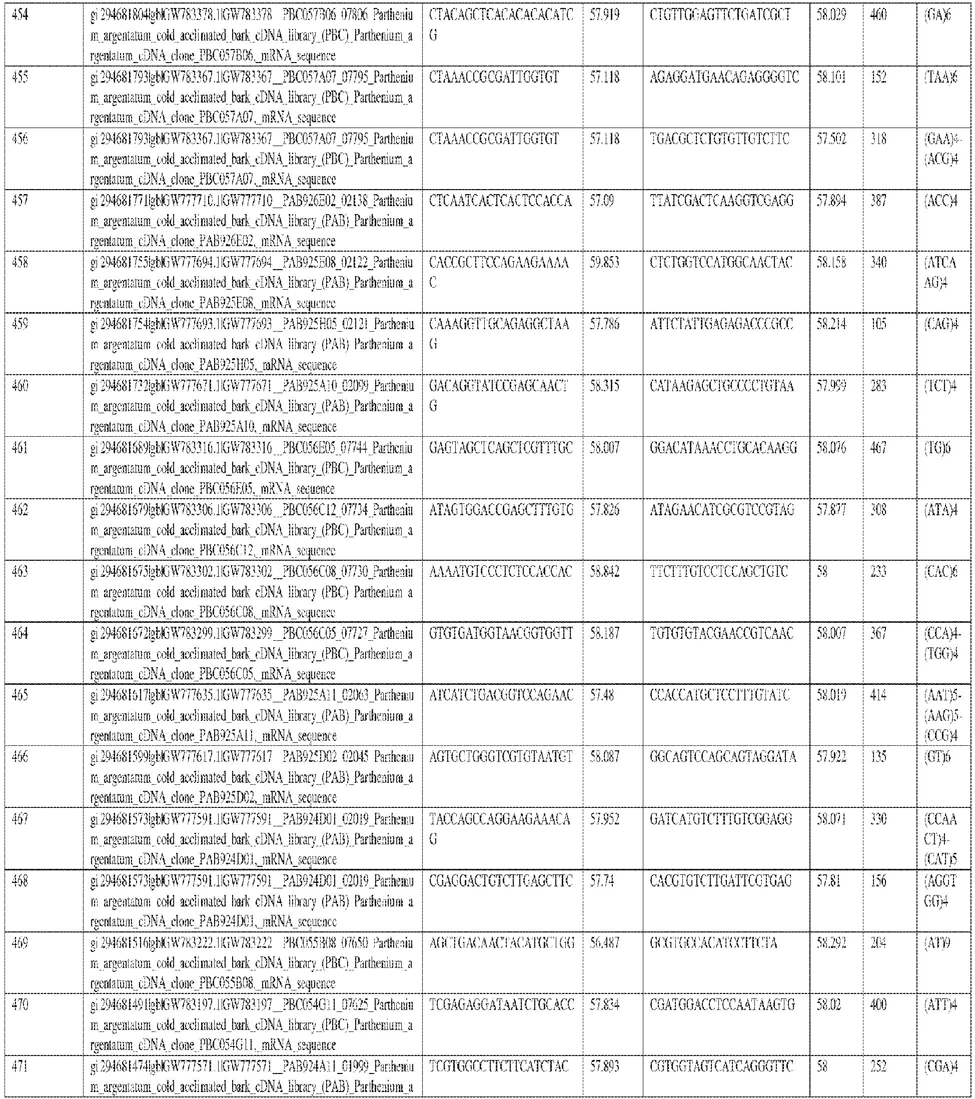

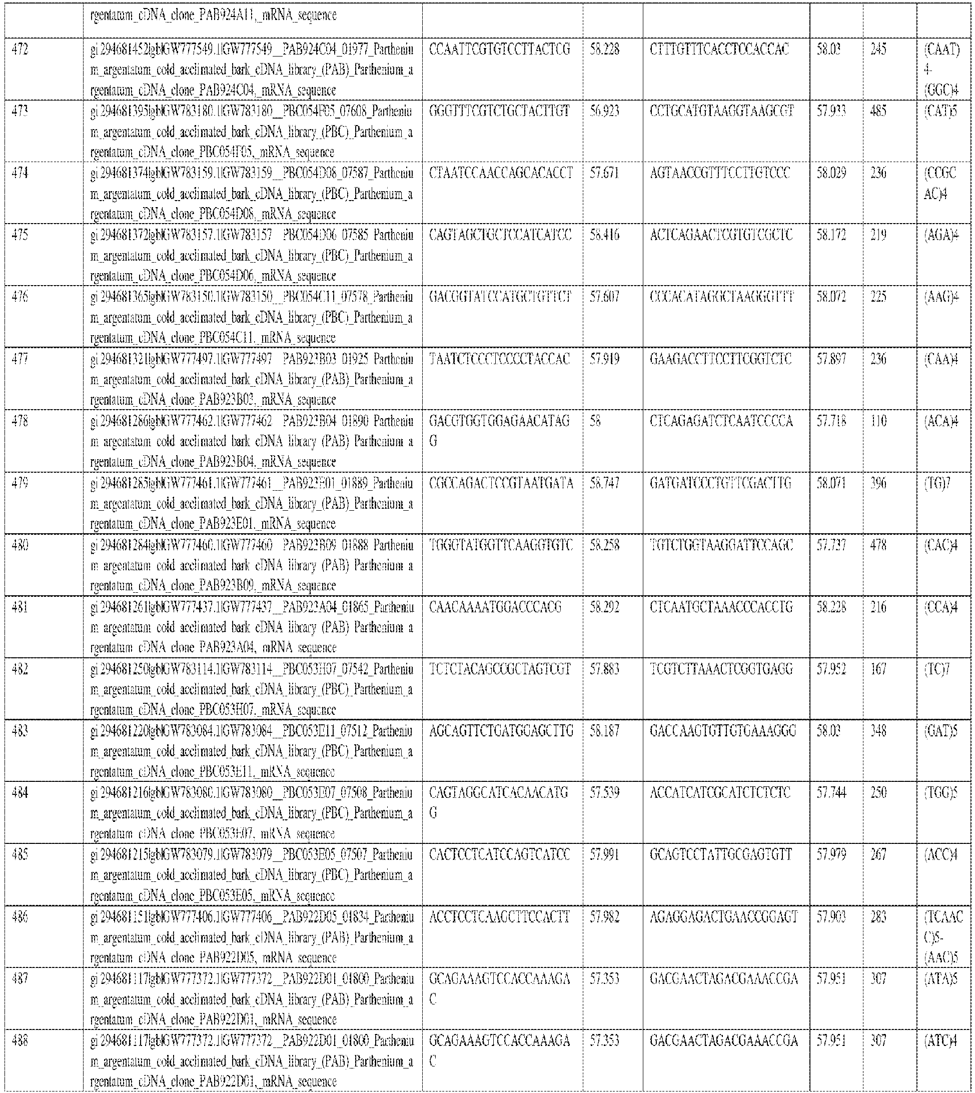

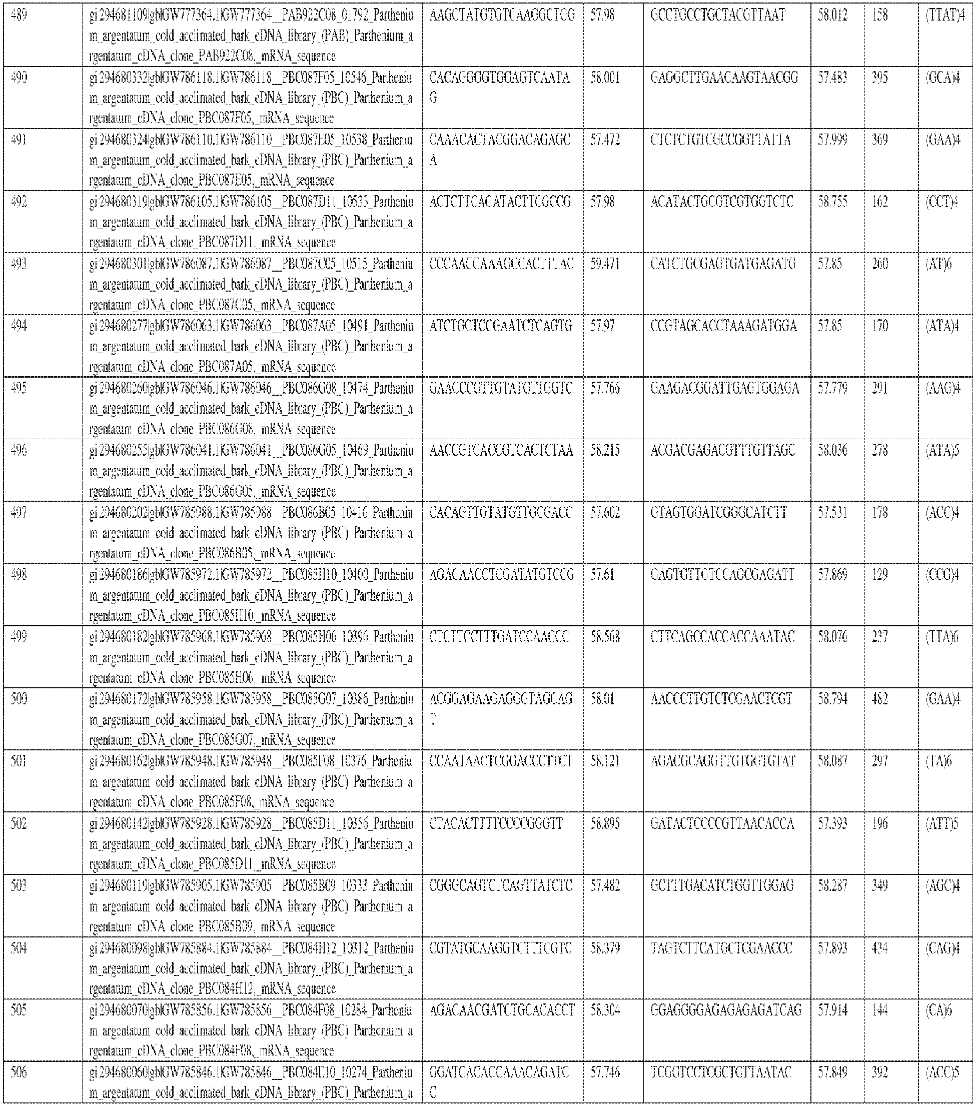

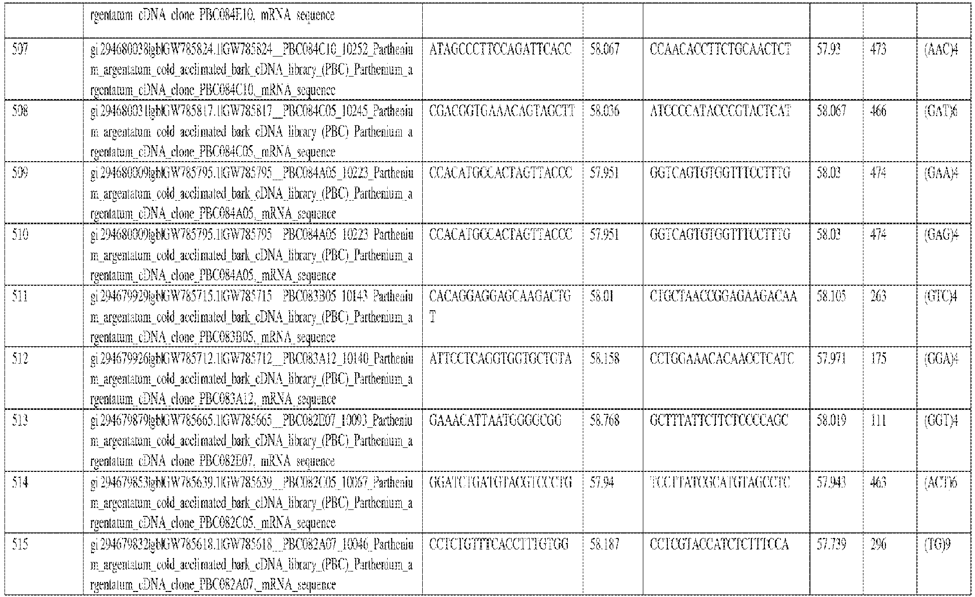

## Claims

1. A method of selecting two guayule plants for breeding comprising:
(a) determining the rate of apomixis of one or more guayule plants;
(b) determining the genetic pedigree of the guayule plants using DNA analysis; and
(c) using the information regarding the rate of apomixis and genetic pedigrees to identify two plants that are genetically dissimilar suitable for use in guayule plant breeding to produce an advanced guayule plant;
wherein the DNA analysis is performed using DNA of mono-, di-, tri-, tetra-, penta- or hexanucleotide simple sequence repeats (SSRs);
**characterized in that** the SSRs comprise the SSR markers corresponding to 4, 5, 10, 12, 18, 19, 68, 80, 93, 134, 139, 158, 185, 186 and 188 in Table 11.

2. The method of claim 1, wherein the rate of apomixis is determined by flow cytometry.

3. The method of claim 1 or 2, wherein the rate of apomixis is determined from leaf samples, seed samples, or both.

## Patentansprüche

1. Verfahren zum Auswählen von zwei Guayule-Pflanzen zum Züchten, umfassend:
(a) Bestimmen der Apomixisrate einer oder mehrerer Guayule-Pflanzen;
(b) Bestimmen des genetischen Stammbaums der Guayule-Pflanzen unter Verwendung von DNA-Analyse; und
(c) Verwenden der Informationen bezüglich der Apomixisrate und genetischer Stammbäume, um zwei Pflanzen zu identifizieren, die genetisch unähnlich sind, die sich zur Verwendung in der Guayule-Pflanzenzüchtung eignen, um eine fortgeschrittene Guayule-Pflanze zu erzeugen;
wobei die DNA-Analyse unter Verwendung von DNA von Mono-, Di-, Tri-, Tetra-, Penta- oder Hexanukleotid-Mikrosatelliten (SSRs) durchgeführt wird;
**dadurch gekennzeichnet, dass** die SSRs die SSR-Marker umfassen, die 4, 5, 10, 12, 18, 19, 68, 80, 93, 134, 139, 158, 185, 186 und 188 in Tabelle 11 entsprechen.

2. Verfahren nach Anspruch 1, wobei die Apomixisrate durch Durchflusszytometrie bestimmt wird.

3. Verfahren nach Anspruch 1 oder 2, wobei die Apomixisrate aus Blattproben, Samenproben oder beiden bestimmt wird.

## Revendications

1. Procédé de sélection de deux plantes de guayule pour le croisement comprenant :
(a) la détermination du taux d'apomixie d'un ou plusieurs plants de guayule ;
(b) la détermination du pedigree génétique des plants de guayule par analyse d'ADN ; et
(c) l'utilisation des informations concernant le taux d'apomixie et les généalogies génétiques pour identifier deux plantes qui sont génétiquement dissemblables appropriées pour une utilisation dans la sélection de plantes de guayule afin de produire une plante de guayule avancée ;
dans lequel l'analyse d'ADN est effectuée à l'aide de l'ADN de répétitions de séquences simples (SSR) de mono-, di-, tri-, tétra-, penta- ou hexanucléotides ;
**caractérisé en ce que** les SSR comprennent les marqueurs SSR correspondant à 4, 5, 10, 12, 18, 19, 68, 80, 93, 134, 139, 158, 185, 186 et 188 dans le Tableau 11.

2. Procédé selon la revendication 1, dans lequel le taux d'apomixie est déterminé par cytométrie en flux.

3. Procédé selon la revendication 1 ou 2, dans lequel le taux d'apomixie est déterminé à partir d'échantillons de feuilles, d'échantillons de graines, ou des deux.
